(19) Europäisches Patentamt
European Patent Office
Office européen des brevets

(11) EP 0 936 912 B1

(12) EUROPEAN PATENT SPECIFICATION

(45) Date of publication and mention of the grant of the patent:
**11.02.2004 Bulletin 2004/07**

(21) Application number: **97937146.5**

(22) Date of filing: **07.08.1997**

(51) Int Cl.[7]: **C07D 207/24**, A61K 31/40, C07D 401/12, A61K 31/44, C07D 401/06, C07D 401/10, A61K 31/47, C07D 211/74, A61K 31/445, C07D 401/14, C07D 405/12

(86) International application number:
**PCT/US1997/013875**

(87) International publication number:
**WO 1998/005336 (12.02.1998 Gazette 1998/06)**

(54) **INHIBITORS OF CYSTEINE PROTEASE**

CYSTEINE PROTEASE INHIBITOREN

INHIBITEURS DE LA PROTEASE A CYSTEINE

(84) Designated Contracting States:
**AT BE CH DE DK ES FI FR GB GR IE IT LI LU MC NL PT SE**
Designated Extension States:
**SI**

(30) Priority: **08.08.1996 US 23742 P**
**08.05.1997 US 46867 P**

(43) Date of publication of application:
**25.08.1999 Bulletin 1999/34**

(73) Proprietor: **SMITHKLINE BEECHAM CORPORATION**
**Philadelphia Pennsylvania 19101 (US)**

(72) Inventors:
- **MARQUIS, Robert, W., Jr.**
  **St. Davids, PA 19087 (US)**
- **VEBER, Daniel, F.**
  **Ambler, PA 19002 (US)**
- **RU, Yu**
  **Havertown, PA 19083 (US)**
- **LO CASTRO, Stephen**
  **Exton, PA 19341 (US)**

(74) Representative: **Waters, David Martin, Dr. et al**
**GlaxoSmithKline**
**Corporate Intellectual Property (CN9.25.1)**
**980 Great West Road**
**Brentford, Middlesex TW8 9GS (GB)**

(56) References cited:
**EP-A- 0 603 873**
**WO-A-94/04172**
**WO-A-97/16433**
**US-A- 4 994 471**
**US-A- 5 057 525**
**US-A- 5 206 251**
**US-A- 5 216 168**
**US-A- 5 374 637**
**US-A- 5 585 387**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 936 912 B1

## Description

### Field of the Invention

This invention relates to novel protease inhibitors, particularly inhibitors of cysteine and serine proteases, more particularly compounds which inhibit cysteine proteases, even more particularly compounds which inhibit cysteine proteases of the papain superfamily, yet more particularly compounds which inhibit cysteine proteases of the cathepsin family, most particularly compounds which inhibit cathepsin K. Such compounds are particularly useful for treating diseases in which cysteine proteases are implicated, especially diseases of excessive bone or cartilage loss, e.g., osteoporosis, periodontitis, and arthritis.

### Background of the Invention

Cathepsin K is a member of the family of enzymes which are part of the papain superfamily of cysteine proteases. Cathepsins B, H, L, N and S have been described in the literature. Recently, cathepsin K polypeptide and the cDNA encoding such polypeptide were disclosed in U.S. Patent No. 5,501,969 (called cathepsin O therein). Cathepsin K has been recently expressed, purified, and characterized. Bossard, M. J., et al., (1996) *J. Biol. Chem*. **271,** 12517-12524; Drake, F.H., et al., (1996) *J. Biol. Chem.* **271,** 12511-12516; Bromme, D., et al., (1996) *J. Biol. Chem*. **271,** 2126-2132.

Cathepsin K has been variously denoted as cathepsin O, cathepsin X or cathepsin O2 in the literature. The designation cathepsin K is considered to be the more appropriate one (name assigned by Nomenclature Committee of the International Union of Biochemistry and Molecular Biology).

Cathepsins of the papain superfamily of cysteine proteases function in the normal physiological process of protein degradation in animals, including humans, e.g., in the degradation of connective tissue. However, elevated levels of these enzymes in the body can result in pathological conditions leading to disease. Thus, cathepsins have been implicated in various disease states, including but not limited to, infections by pneumocystis carinii, trypsanoma cruzi, trypsanoma brucei brucei, and Crithidia fusiculata; as well as in schistosomiasis malaria, tumor metastasis, metachromatic leukodystrophy, muscular dystrophy, amytrophy, and the like. *See* International Publication Number WO 94/04172, published on March 3, 1994, and references cited therein. *See also* European Patent Application EP 0 603 873 A1, and references cited therein. Two bacterial cysteine proteases from P. gingivallis, called gingipains, have been implicated in the pathogenesis of gingivitis. Potempa, J., et al. (1994) *Perspectives in Drug Discovery and Design*, **2,** 445-458.

Cathepsin K is believed to play a causative role in diseases of excessive bone or cartilage loss. Bone is composed of a protein matrix in which spindle- or plate-shaped crystals of hydroxyapatite are incorporated. Type I Collagen represents the major structural protein of bone comprising approximately 90% of the structural protein. The remaining 10% of matrix is composed of a number of non-collagenous proteins, including osteocalcin, proteoglycans, osteopontin, osteonectin, thrombospondin, fibronectin, and bone sialoprotein. Skeletal bone undergoes remodeling at discrete foci throughout life. These foci, or remodeling units, undergo a cycle consisting of a bone resorption phase followed by a phase of bone replacement.

Bone resorption is carried out by osteoclasts, which are multinuclear cells of hematopoietic lineage. The osteoclasts adhere to the bone surface and form a tight sealing zone, followed by extensive membrane ruffling on their apical (i.e., resorbing) surface. This creates an enclosed extracellular compartment on the bone surface that is acidified by proton pumps in the ruffled membrane, and into which the osteoclast secretes proteolytic enzymes. The low pH of the compartment dissolves hydroxyapatite crystals at the bone surface, while the proteolytic enzymes digest the protein matrix. In this way, a resorption lacuna, or pit, is formed. At the end of this phase of the cycle, osteoblasts lay down a new protein matrix that is subsequently mineralized. In several disease states, such as osteoporosis and Paget's disease, the normal balance between bone resorption and formation is disrupted, and there is a net loss of bone at each cycle. Ultimately, this leads to weakening of the bone and may result in increased fracture risk with minimal trauma.

The abundant selective expression of cathepsin K in osteoclasts strongly suggests that this enzyme is essential for bone resorption. Thus, selective inhibition of cathepsin K may provide an effective treatment for diseases of excessive bone loss, including, but not limited to, osteoporosis, gingival diseases such as gingivitis and periodontitis, Paget's disease, hypercalcemia of malignancy, and metabolic bone disease. Cathepsin K levels have also been demonstrated to be elevated in chondroclasts of osteoarthritic synovium. Thus, selective inhibition of cathepsin K may also be useful for treating diseases of excessive cartilage or matrix degradation, including, but not limited to, osteoarthritis and rheumatoid arthritis. Metastatic neoplastic cells also typically express high levels of proteolytic enzymes that degrade the surrounding matrix. Thus, selective inhibition of cathepsin K may also be useful for treating certain neoplastic diseases.

It now has been discovered that a novel class of compounds are protease inhibitors, most particularly inhibitors

of cathepsin K, and these compounds are useful for treating diseases in which inhibition of bone resorption is indicated, such as osteoporosis and periodontal disease.

Summary of the Invention

An object of the present invention is to provide protease inhibitors, particularly such inhibitors of cysteine and serine proteases, more particularly such compounds which inhibit cysteine proteases, even more particularly such compounds which inhibit cysteine proteases of the papain superfamily, yet more particularly such compounds which inhibit cysteine proteases of the cathepsin family, most particularly such compounds which inhibit cathepsin K, and which are useful for treating diseases which may be therapeutically modified by altering the activity of such proteases.
Accordingly, in the first aspect, this invention provides a compound according to formula (I).
In another aspect, this invention provides a pharmaceutical composition comprising a compound according to formula (I) and a pharmaceutically acceptable carrier.
In yet another aspect, this invention provides a compound of formula (I) for use in treating diseases in which the disease pathology may be therapeutically modified by inhibiting proteases, particularly cysteine and serine proteases, more particularly cysteine proteases, even more particularly cysteine proteases of the papain superfamily, yet more particularly cysteine proteases of the cathepsin family, most particularly cathepsin K.
In a particular aspect, the compounds of this invention are especially useful for treating diseases characterized by bone loss, such as osteoporosis and gingival diseases, such as gingivitis and periodontitis, or by excessive cartilage or matrix degradation, such as osteoarthritis and rheumatoid arthritis.

Detailed Description of the Invention

The present invention provides compounds of formula (I):

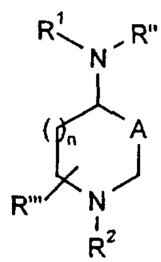

(I)

wherein:

A is C(O) or CH(OH);
$R^1$ is

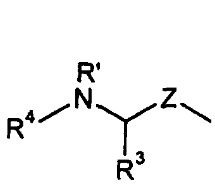

,

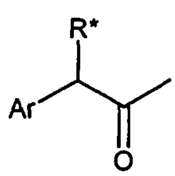

,

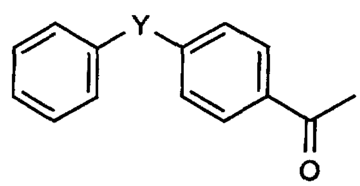

,

or

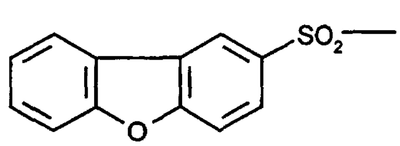

;

$R^2$ is H, $C_{1-6}$alkyl, optionally substituted independently by one or two halogens, SR, OR, $N(R)_2$, $C(O)N(R)_2$, carbamyl or $C_{1-4}$alkyl, $C_{3-6}$cycloalkyl-$C_{0-6}$alkyl, Ar-$C_{0-6}$alkyl, Het-$C_{0-6}$alkyl, $R^5C(O)$-, $R^5C(S)$-, $R^5SO_2$-, $R^5OC(O)$-, $R^5R'NC(O)$-, $R^5R'NC(S)$-, adamantyl-C(O)-, or

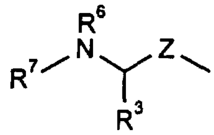
;

R" is H, $C_{1-6}$alkyl optionally substituted independently by one or two halogens, SR, OR, $N(R)_2$, $C(O)N(R)_2$, carbamyl or $C_{1-4}$alkyl, Ar-$C_{0-6}$alkyl, or Het-$C_{0-6}$alkyl;

R"' is H, $C_{1-6}$alkyl optionally substituted independently by one or two halogens, SR, OR, $N(R)_2$, $C(O)N(R)_2$, carbamyl or $C_{1-4}$alkyl, $C_{3-6}$cycloalkyl-$C_{0-6}$alkyl, Ar-$C_{0-6}$alkyl, or Het-$C_{0-6}$alkyl;

each $R^3$ independently is H, $C_{2-6}$alkenyl, $C_{2-6}$alkynyl, Het, Ar or $C_{1-6}$alkyl optionally substituted by OR', SR', $NR'_2$, $RNC(O)OR^5$, $CO_2R'$, $CO_2NR'_2$, $N(C{=}NH)NH_2$, Het or Ar;

$R^4$ is H, $C_{1-6}$alkyl optionally substituted independently by one or two halogens, SR, OR, $N(R)_2$, $C(O)N(R)_2$, carbamyl or $C_{1-4}$alkyl, $C_{3-6}$cycloalkyl-$C_{0-6}$alkyl, Ar-$C_{0-6}$alkyl, Het-$C_{0-6}$alkyl, $R^5C(O)$-, $R^5C(S)$-, $R^5SO_2$-, $R^5OC(O)$-, $R^5R'NC(O)$-, $R^5R'NC(S)$-, R'HNCH(R')C(O)-, or $R^5OC(O)NR'CH(R')C(O)$-;

each $R^5$ independently is $C_{3-6}$cycloalkyl-$C_{0-6}$alkyl, Ar-$C_{0-6}$alkyl, Het-$C_{0-6}$alkyl, Ar-$C_{0-6}$alkoxy, Het-$C_{0-6}$alkoxy, or $C_{1-6}$alkyl optionally substituted by OR', SR', $NR'_2$, $R'NC(O)OR^5$, $CO_2R'$, $CO_2NR'_2$, $N(C{=}NH)NH_2$, Het or Ar;

$R^6$ is H, $C_{1-6}$alkyl optionally substituted independently by one or two halogens, SR, OR, $N(R)_2$, $C(O)N(R)_2$, carbamyl or $C_{1-4}$alkyl, Ar-$C_{0-6}$alkyl, or Het-$C_{0-6}$alkyl and $R^7$ is H, $C_{1-6}$alkyl optionally substituted independently by one or two halogens, SR, OR, $N(R)_2$, $C(O)N(R)_2$, carbamyl or $C_{1-4}$alkyl, $C_{3-6}$cycloalkyl-$C_{0-6}$alkyl, Ar-$C_{0-6}$alkyl, Het-$C_{0-6}$alkyl, $R^5C(O)$-, $R^5C(S)$-, $R^5SO_2$-, $R^5OC(O)$-, $R^5R'NC(O)$-, $R^5R'NC(S)$-, R'HNCH(R')C(O)-, or $R^5OC(O)NR'CH(R')C(O)$-;

or $R^6$ and $R^7$ are connected to form a pyrrolidine, a piperidine, or a morpholine ring;

each R' independently is H, $C_{1-6}$alkyl, Ar-$C_{0-6}$alkyl, or Het-$C_{0-6}$alkyl;

R* is H, $C_{1-6}$alkyl, $C_{3-6}$cycloalkyl-$C_{0-6}$alkyl, Ar-$C_{0-6}$alkyl, or Het-$C_{0-6}$alkyl;

R is H or $C_{1-6}$ alkyl;

Y is a single bond or O;

each Z independently is CO or $CH_2$; and

n is 0, 1, or 2;

$C_{3-6}$cycloalkyl is optionally substituted independently by one or two halogens, SR, OR, $N(R)_2$, $C(O)N(R)_2$, carbamyl or $C_{1-4}$alkyl, where R is H or $C_{1-6}$alkyl;

Ar means unsubstituted phenyl or naphthyl; or phenyl or naphthyl substituted by one or more of Ph-$C_{0-6}$alkyl, Het-$C_{0-6}$alkyl, $C_{1-6}$alkoxy, Ph-$C_{0-6}$alkoxy, Het-$C_{0-6}$alkoxy, OH, $(CH_2)_{1-6}NR^aR^a$, $O(CH_2)_{1-6}NR^aR^a$; or phenyl or naphthyl substituted by one to three moieties selected from $C_{1-4}$alkyl, $OR^a$, $N(R^a)_2$, $SR^a$, $CF_3$, $NO_2$, CN, $CO_2R^a$, $CON(R^a)$, F, Cl, Br and I, or substituted by a methylenedioxy group; and

Het represents a stable 5- to 7-membered monocyclic or a stable 7- to 10-membered bicyclic heterocyclic ring, which is either saturated or unsaturated, and which consists of carbon atoms and from one to four heteroatoms selected from the group consisting of N, O and S, and wherein the N and S heteroatoms may optionally be oxidized, and the N heteroatom may optionally be quaternized, and including any bicyclic group in which any of the above-defined heterocyclic rings is fused to a benzene ring; wherein the Het group may be optionally substituted with one or two moieties selected from $C_{1-4}$alkyl, $OR^a$, $N(R^a)_2$, $SR^a$, $CF_3$, $NO_2$, CN, $CO_2R^a$, $CON(R^a)$, F, Cl, Br and I, and wherein each $R^a$ independently is H, $C_{1-6}$alkyl, Ar-$C_{0-6}$alkyl, or Het-$C_{0-6}$alkyl;

or a pharmaceutically acceptable salt thereof.

The present invention includes all hydrates, solvates, and complexes of the compounds of this invention. If a chiral center or another form of an isomeric center is present in a compound of the present invention, all forms of such isomer or isomers, including enantiomers and diastereomers, are intended to be covered herein. Inventive compounds containing a chiral center may be used as a racemic mixture, an enantiomerically enriched mixture, or the racemic mixture may be separated using well-known techniques and an individual enantiomer may be used alone. In cases in which compounds have unsaturated carbon-carbon double bonds, both the cis (Z) and trans (E) isomers are within the scope of this invention. In cases wherein compounds may exist in tautomeric forms, such as keto-enol tautomers, each tautomeric form is contemplated as being included within this invention whether existing in equilibrium or predominantly in one form.

The meaning of any substituent at any one occurrence in formula (I) or any subformula thereof is independent of its meaning, or any other substituent's meaning, at any other occurrence, unless specified otherwise.
With respect to formula (I):
Preferably, A is C(O).
Suitably, $R^1$ is

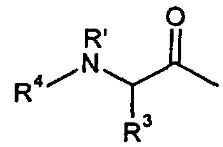

.

Particularly, in said $R^1$ group, R' is H or $CH_3$, $R^3$ is i-butyl and $R^4$ is $R^5C(O)$-, $R^5SO_2$-, $R^5OC(O)$-, preferably, $R^5$ is Ar-$C_{0-6}$alkyl or Het-$C_{0-6}$alkyl. In particular, in said $R^1$ group, $R^5$ is phenyl or benzyl which are unsubstituted or substituted by one or two of the group consisting of Cl, Br, F, $CF_3$, $C_{1-4}$alkyl, OH, $C_{1-4}$alkoxy, CN, $CONH_2$, $NH_2$, or $NO_2$, or substituted by methylenedioxy, or

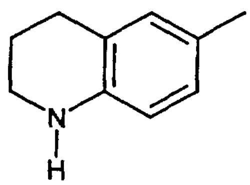

,

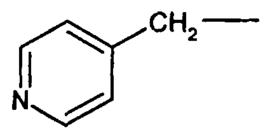

,

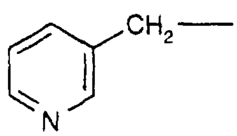

,

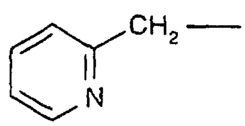

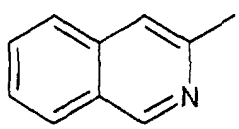

,

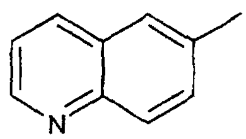

,

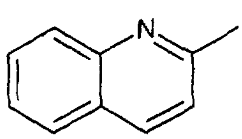

,

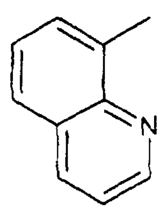

,

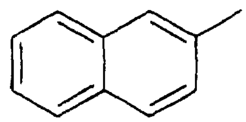
, or
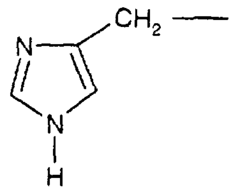
.

Alternately, $R^1$ is

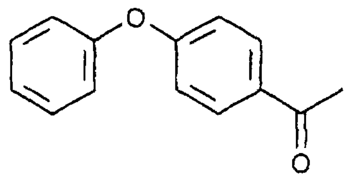
or
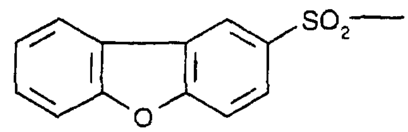
.

Suitably, $R^2$ is

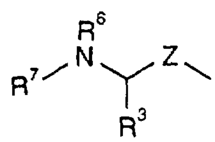
.

Suitably, in said $R^2$ group, $R^6$ is H or $CH_3$, $R^3$ is i-butyl and $R^7$ is $R^5OC(O)$- wherein $R^5$ in said $R^7$ group is Ar-$C_{0-6}$alkyl or Het-$C_{0-6}$alkyl. In particular, in said $R^1$ group, $R^5$ is phenyl or benzyl which are unsubstituted or substituted by one or two of the group consisting of Cl, Br, F, $CF_3$, $C_{1-4}$alkyl, OH, $C_{1-4}$alkoxy, CN, $CONH_2$, $NH_2$, or $NO_2$, or substituted by methylenedioxy; or 2-, 3-, or 4-pyridyl-$CH_2$-.
Alternately, $R^2$ is

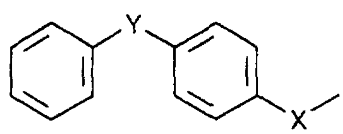

in which X is CO, $SO_2$, or $CH_2$-CO and Y is a single bond or O.
Alternately, $R^2$ is $C_{3-6}$cycloalkyl-$C_{0-6}$alkyl, Ar-$C_{0-6}$alkyl, Het-$C_{0-6}$alkyl, $R^5C(O)$-, $R^5C(S)$-, $R^5SO_2$-, $R^5OC(O)$-, $R^5R'NC(O)$-, $R^5R'NC(S)$-, $R'HNCH(R')C(O)$-, $R^5OC(O)NR'CH(R')C(O)$-, or adamantyl-C(O)-.

Specific representative compounds of this invention are named in Examples 1-198 detailed and claimed hereinafter.

Abbreviations and symbols commonly used in the peptide and chemical arts are used herein to describe the compounds of the present invention. In general, the amino acid abbreviations follow the IUPAC-IUB Joint Commission on Biochemical Nomenclature as described in *Eur. J. Biochem.*, 158, 9 (1984). The term "amino acid" as used herein refers to the D- or L- isomers of alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine and valine.

"$C_{1-6}$alkyl" as applied herein is meant to include substituted and unsubstituted methyl, ethyl, n-propyl, isopropyl, n-butyl, isobutyl and t-butyl, pentyl, n-pentyl, isopentyl, neopentyl and hexyl and the simple aliphatic isomers thereof. Any $C_{1-6}$alkyl group may be optionally substituted independently by one or two halogens, SR', OR', $N(R')_2$, $C(O)N(R')_2$, carbamyl or $C_{1-4}$alkyl, where R' is H or $C_{1-6}$alkyl. $C_0$alkyl means that no alkyl group is present in the moiety. Thus, Ar-$C_0$alkyl is equivalent to Ar.

"$C_{3-6}$cycloalkyl" as applied herein is meant to include substituted and unsubstituted cyclopropane, cyclobu-

tane, cyclopentane, and cyclohexane.

"$C_{2-6}$ alkenyl" as applied herein means an alkyl group of 2 to 6 carbons wherein a carbon-carbon single bond is replaced by a carbon-carbon double bond. $C_{2-6}$alkenyl includes ethylene, 1-propene, 2-propene, 1-butene, 2-butene, isobutene and the several isomeric pentenes and hexenes. Both cis and trans isomers are included.

"$C_{2-6}$alkynyl" means an alkyl group of 2 to 6 carbons wherein one carbon-carbon single bond is replaced by a carbon-carbon triple bond. $C_{2-6}$ alkynyl includes acetylene, 1-propyne, 2-propyne, 1-butyne, 2-butyne, 3-butyne and the simple isomers of pentyne and hexyne.

"Halogen" or "halo" means F, Cl, Br, and I.

"Ar" or "aryl" means unsubstituted phenyl or naphthyl; or phenyl or naphthyl substituted by one or more of Ph-$C_{0-6}$alkyl, Het-$C_{0-6}$alkyl, $C_{1-6}$alkoxy, Ph-$C_{0-6}$alkoxy, Het-$C_{0-6}$alkoxy, OH, $(CH_2)_{1-6}NR'R'$, $O(CH_2)_{1-6}NR'R'$; wherein each R' independently is H, $C_{1-6}$alkyl, Ar-$C_{0-6}$alkyl, or Het-$C_{0-6}$alkyl; or phenyl or naphthyl substituted by one to three moieties selected from $C_{1-4}$alkyl, OR', $N(R')_2$, SR', $CF_3$, $NO_2$, CN, $CO_2R'$, CON(R'), F, Cl, Br and I, or substituted by a methylenedioxy group.

As used herein "Het" or "heterocyclic" represents a stable 5- to 7-membered monocyclic or a stable 7- to 10-membered bicyclic heterocyclic ring, which is either saturated or unsaturated, and which consists of carbon atoms and from one to four heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure, and may optionally be substituted with one or two moieties selected from $C_{1-4}$alkyl, OR', $N(R')_2$, SR', $CF_3$, $NO_2$, CN, $CO_2R'$, CON(R'), F, Cl, Br and I, where R' is as defined hereinbefore. Examples of such heterocycles include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, pyridyl, pyrazinyl, oxazolidinyl, oxazolinyl, oxazolyl, isoxazolyl, morpholinyl, thiazolidinyl, thiazolinyl, thiazolyl, quinuclidinyl, indolyl, quinolinyl, isoquinolinyl, benzimidazolyl, benzopyranyl, benzoxazolyl, furyl, pyranyl, tetrahydrofuryl, tetrahydropyranyl, thienyl, benzoxazolyl, thiamorpholinyl sulfoxide, thiamorpholinyl sulfone, oxadiazolyl, benzothiazolyl, benzoisothiazolyl, benzisoxazolyl, pyrimidinyl, cinnolinyl, quinazolinyl, quinoxalinyl, 1,5-napthyridinyl, 1,6-napthyridinyl, 1,7-napthyridinyl, 1,8-napthyridinyl, tetrazolyl, 1,2,3-triazolyl, and 1,2,4-triazolyl.

"HetAr" or "heteroaryl" means any heterocyclic moiety encompassed by the above definition of Het which is aromatic in character, e.g., pyridinyl, quinolinyl, isoquinolinyl, pyrrolyl, pyrazolyl, imidazolyl, pyridyl, pyrazinyl, oxazolyl, isoxazolyl, thiazolyl, isothiazolyl, indolyl, quinolinyl, isoquinolinyl, benzimidazolyl, benzoxazolyl, furyl, thienyl, benzoxazolyl, oxadiazolyl, benzothiazolyl, benzoisothiazolyl, benzisoxazolyl, pyrimidinyl, cinnolinyl, quinazolinyl, quinoxalinyl, 1,5-napthyridinyl, 1,6- napthyridinyl, 1,7- napthyridinyl, 1,8- napthyridinyl, tetrazolyl, 1,2,3-triazolyl, and 1,2,4-triazolyl.

Certain radical groups are abbreviated herein. t-Bu refers to the tertiary butyl radical, Boc or BOC refers to the t-butyloxycarbonyl radical, Fmoc refers to the fluorenylmethoxycarbonyl radical, Ph refers to the phenyl radical, Cbz or CBZ refers to the benzyloxycarbonyl radical.

Certain reagents are abbreviated herein. DCC refers to dicyclohexylcarbodiimide, DMAP is 2,6-dimethylaminopyridine, EDC or EDCI refers to N-ethyl-N'(dimethylaminopropyl)-carbodiimide. HOBT or HOBt refers to 1-hydroxybenzotriazole, DMF refers to dimethyl formamide, BOP refers to benzotriazol-1-yloxy-tris(dimethylamino)phosphonium hexafluorophosphate, DMAP is dimethylaminopyridine, DIEA refers to di-isopropylethylamine, Lawesson's reagent is 2,4-bis(4-methoxyphenyl)-1,3-dithia-2,4-diphosphetane-2,4-disulfide, NMM is N-methylmorpholine, TFA refers to trifluoroacetic acid, TFAA refers to trifluoroacetic anhydride, KHMDS refers to potassium hexamethyldisilazide, and THF refers to tetrahydrofuran. Jones reagent is a solution of chromium trioxide, water, and sulfuric acid well-known in the art.

The compounds of formula (I) are generally prepared using a process which comprises:

(A) for compounds in which A is CH(OH):

(i) reacting a compound of the formula (III):

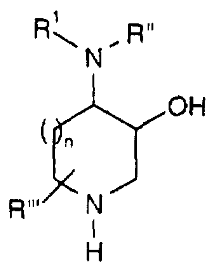

(III)

or a salt thereof,

wherein $R^1$, R", R"' and n are as defined in formula (I), with any reactive functional groups protected, with:

(a) $R^5C(O)Cl$, in which $R^5$is as defined in formula (I); or
(b) $R^5C(O)OH$, in which $R^5$is as defined in formula (1), in the presence of EDC and HOBT; or
(c) $R^5C(O)H$, in which $R^5$is as defined in formula (I), followed by reduction; or
(d) $R^5OC(O)Cl$, in which $R^5$is as defined in formula (I), in the presence of base; or
(e) $R^5SO_2Cl$, in which $R^5$is as defined in formula (I), in the presence of base; or
(f)

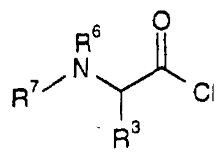

wherein $R^3$, $R^6$ and $R^7$ are as defined in formula (I); or
(g) adamantyl-C(O)Cl;

(ii) reacting a compound of the formual (IV):

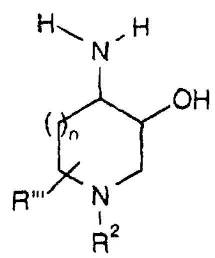

(IV)

wherein $R^2$, R"' and n are as defined in formula (I), with any reactive functional groups protected, with:

(a)

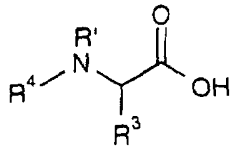

,

in which $R^3$, $R^4$ and $R^1$ are as defined in formula (I), in the presence of EDC and HOBT; or

(b)

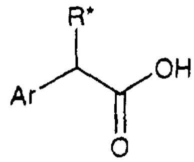

,

in which R* is as defined in formula (1), in the presence of EDC and HOBT; or

(c)

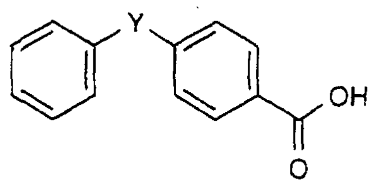

,

in which Y is as defined in formula (I), in the presence of EDC and HOBT; or

(d)

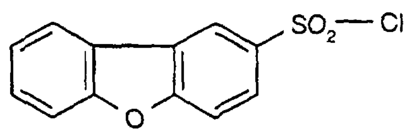

;

(iii) reacting a compound of the formual (V):

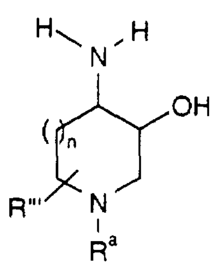

(V)

wherein R''' and n are as defined in formula (I), with any reactive functional groups protected, and $R^a$ is $C_{1-6}$alkyl, $C_{3-6}$cycloalkyl-$C_{0-6}$alkyl, Ar-$C_{0-6}$alkyl, or Het-$C_{0-6}$alkyl with:

(a)

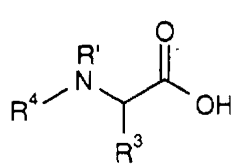

,

in which $R^3$, $R^4$ and R' are as defined in formula (1), in the presence of EDC and HOBT; or

(b)

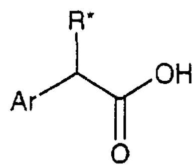

,

in which R* is as defined in formula (I), in the presence of EDC and HOBT; or

(c)

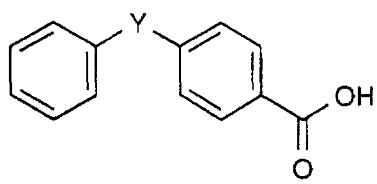

,

in which Y is as defined in formula (I), in the presence of EDC and HOBT; or

(d)

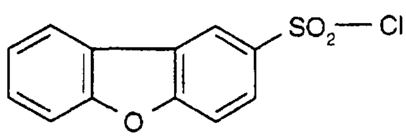

;

(B) for compounds in which A is C(O):

(i) reacting a compound of the formual (VI):

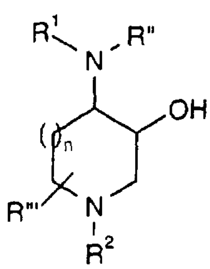

(VI)

wherein $R^1$, $R^2$, R'', R''' and n are as defined in formula (I), with any reactive functional groups protected, with an oxidizing agent;

and thereafter removing any protecting groups and optionally forming a pharmaceutically acceptable salt.

Compounds of the formula (I) are prepared by methods analogous to those described in Schemes 1-5.

Scheme 1

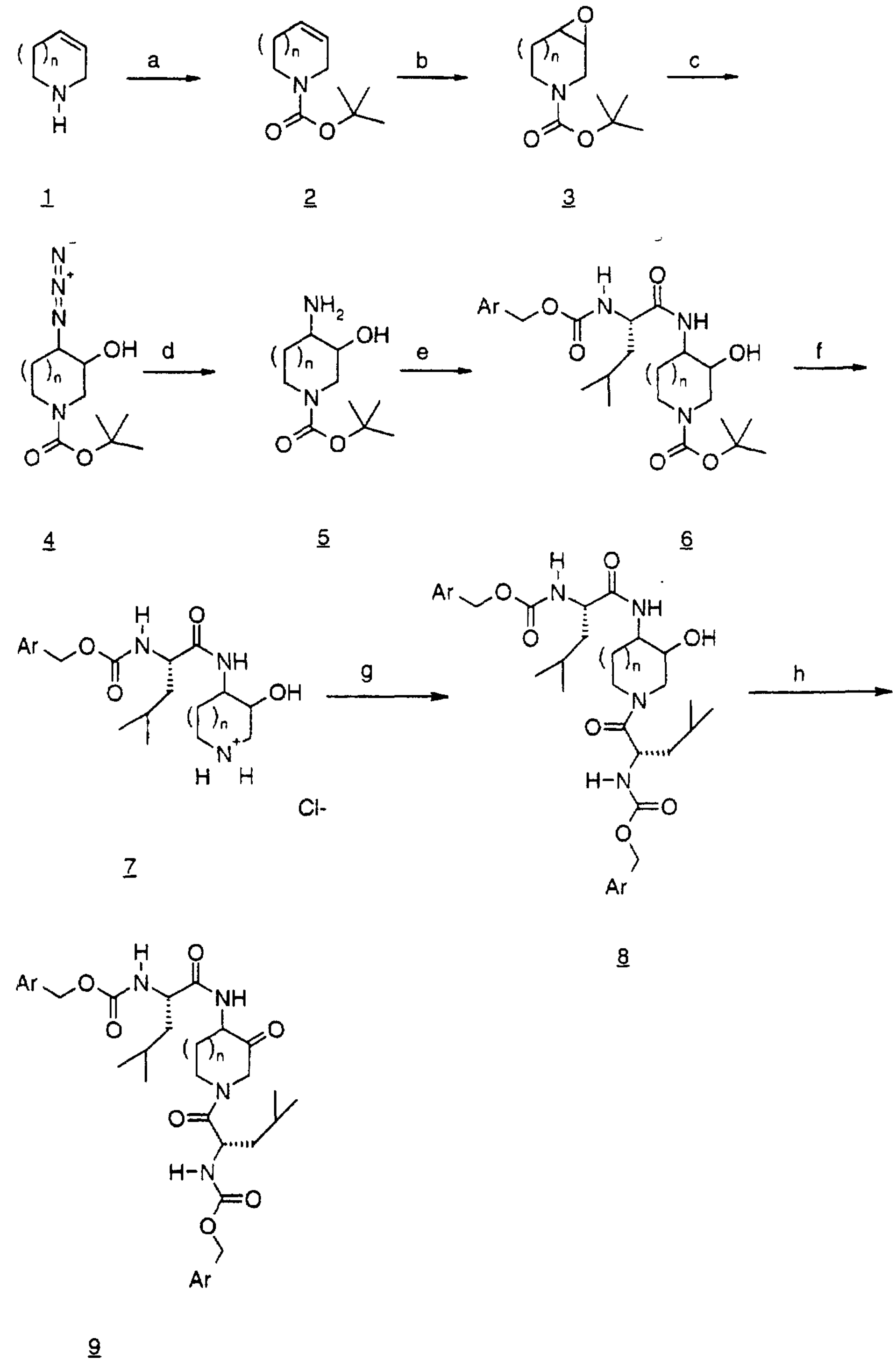


a) di-*tert*-butyl dicarbonate, $CH_2Cl_2$; b) m-chloroperoxybenzoic acid, $CH_2Cl_2$; c) $NaN_3$, $NH_4Cl$, $CH_3OH$:$H_2O$ (8:1); d) 10% Pd/C, $CH_3OH$, $H_2$; e) $RCO_2H$, EDC, HOBt, $CH_2Cl_2$ ; f) HCl/EtOAc or TFA, $CH_2Cl_2$; g) $RCO_2H$, EDC, HOBt, $CH_2Cl_2$ or RCOCl, TEA, $CH_2Cl_2$, TEA; h) $CrO_3$, HOAc or DMSO, $(COCl)_2$, $CH_2Cl_2$, TEA, -78°C to RT or DMSO, sulphur trioxide pyridine comple, TEA

Compounds of the general formula (1) wherein n is 0 or 1, $R^1$ and $R^2$ are amides and R" is hydrogen are prepared as outlined in Scheme 1. Treatment of the commercially available 3-pyrroline (1 -Scheme-1; n = 0) or 1,2,3,6-tetrahydropyridine (1-Scheme-1; n = 1) under conditions which are known in the art for nitrogen protection,

such as di-*tert*-butyl dicarbonate, gave 2-Scheme- 1 (n = 0, 1). 2-Scheme-1 is epoxidized by standard conditions, such as meta-chloroperoxybenzoic acid, to provide the epoxide 3-Scheme-1 (n = 0,1). The epoxide 3-Scheme-1 may be opened with sodium azide in a protic solvent, such as methanol and water, at an elevated temperature to give the azido alcohol 4-Scheme-1. The azide 4-Scheme-1 may be reduced to the amine 5-scheme-1 by methods that are common to the art, such as hydrogen with palladium on carbon as a catalyst, in a protic solvent, such as methanol or ethanol. The amine 3-Scheme-1 is acylated under standard conditions with EDC, HOBt and a carboxylic acid in an aprotic solvent, such as dichloromethane or DMF, to give 6-scheme-1. The amine 5-Scheme-1 may also be acylated with an acid chloride in the presence of an organic base such as triethylamine or N-methyl morpholine in an aprotic solvent such as dichloromethane to give the amide derivative 6-Scheme-1. The amine 5-Scheme-1 may also be suphonylated to give the sulphonamide via methods that are known in the art such as treatment with a sulphonyl chloride, in the presence of an organic base, such as N-methylmorpholine, in an aprotic solvent such as dichloromethane. Removal of the protecting group of 6-Scheme-1 may be accomplished by treatment with a strong acid, such as anhydrous hydrochloric acid or triflouroacetic acid, in an anhydrous aprotic solvent, such as ethyl acetate or dichloromethane, to give 7-Scheme-1. The amine or amine salt of 7-Scheme-1 may be acylated under standard conditions, such as EDC, HOBt and a carboxylic acid or with an acid chloride, to give amide derivatives 8-Scheme-1 (n = 0, 1). The amine 7-Scheme-1 may also be alkylated by treatment with an aldehyde in an aprotic solvent, such as dichloromethane, followed by reduction with sodium cyanoborohydride or sodium triacetoxyborohydride. Alternatively, the amine 7-Scheme-1 may also be converted to the carbamate by treatment with a chloroformate in the presence of base, such as triethylamine or pyridine. The amine 7-Scheme-1 may also be converted to the sulphonamide by treatment with a sulphonyl chloride in the presence of a base. The alcohol derivatives 8-Scheme-1 may be oxidized to the ketone 9-Scheme-1 under standard conditions such as chromium trioxide in acetic acid, in a solvent, such as acetone. These alcohols may also be oxidised with methylsulfoxide and oxalyl chloride in an aprotic solvent, such as dichlormethane at -78°C, followed by treatment with an organic base, such as triethylamine, and warming to room temperature. Alternatively, the alcohols may be oxidised with pyridine sulphur trioxide complex in methylsulphoxide with an organic base, such as triethylamine.

Scheme 2

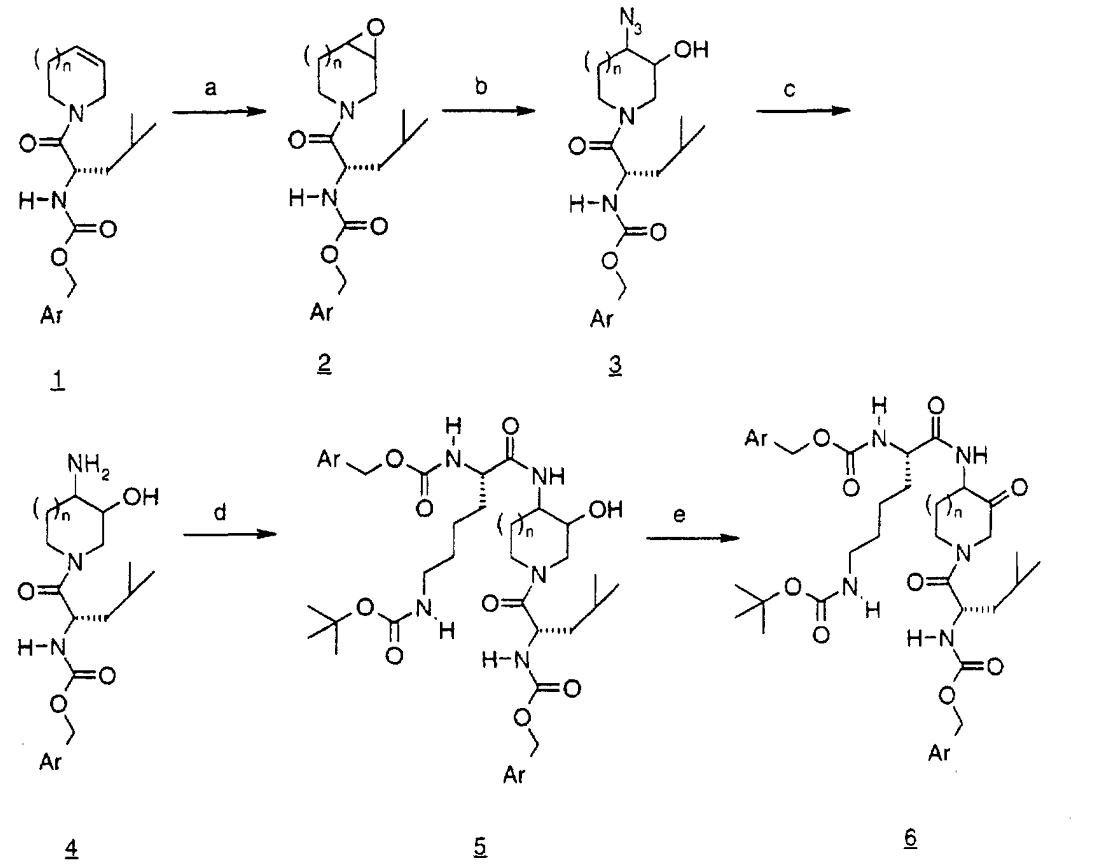


1 2 3 4 5 6

a) m-chloroperoxybenzoic acid, $CH_2Cl_2$; b) $NaN_3$, $NH_4Cl$, $CH_3OH$:$H_2O$ (8:1); c) 1,3-propanedithiol, TEA, $CH_3OH$; d) $RCO_2H$, EDC, HOBt, $CH_2Cl_2$; e) pyridine sulphur trioxide complex, DMSO, TEA

Compounds of the formula (I) wherein n is 1, $R^1$ and $R^2$ are amides and R" is hydrogen may also be prepared as detailed in Scheme 2. Coupling of 1,2,3,6-tetrahydropyridine with a carboxylic acid in the presence of EDC and HOBT or with an acid chloride provides the amide 1-Scheme-2. Epoxidation of 1-Scheme-2 with m-chloroperoxybenzoic acid yields the epoxide 2-Scheme-2 which is opened with sodium azide in the presence of ammonium chloride to provide the azido alcohol 3-Scheme -2. The azide is then reduced under standard conditions which are known in the art, such as 1,3-propanedithiol with triethylamine, in a protic solvent, such as methanol, to provide the amino alcohol 4-Scheme-2. Coupling of the amine 4-Scheme-2 with a carboxylic acid in the presence of EDC and HOBT provides 5-Scheme-2 which is oxidised by methods which are known in the art, such as DMSO, oxalyl chloride and triethylamine at low temperature, to yield the ketone 6-Scheme-2.

Scheme 3

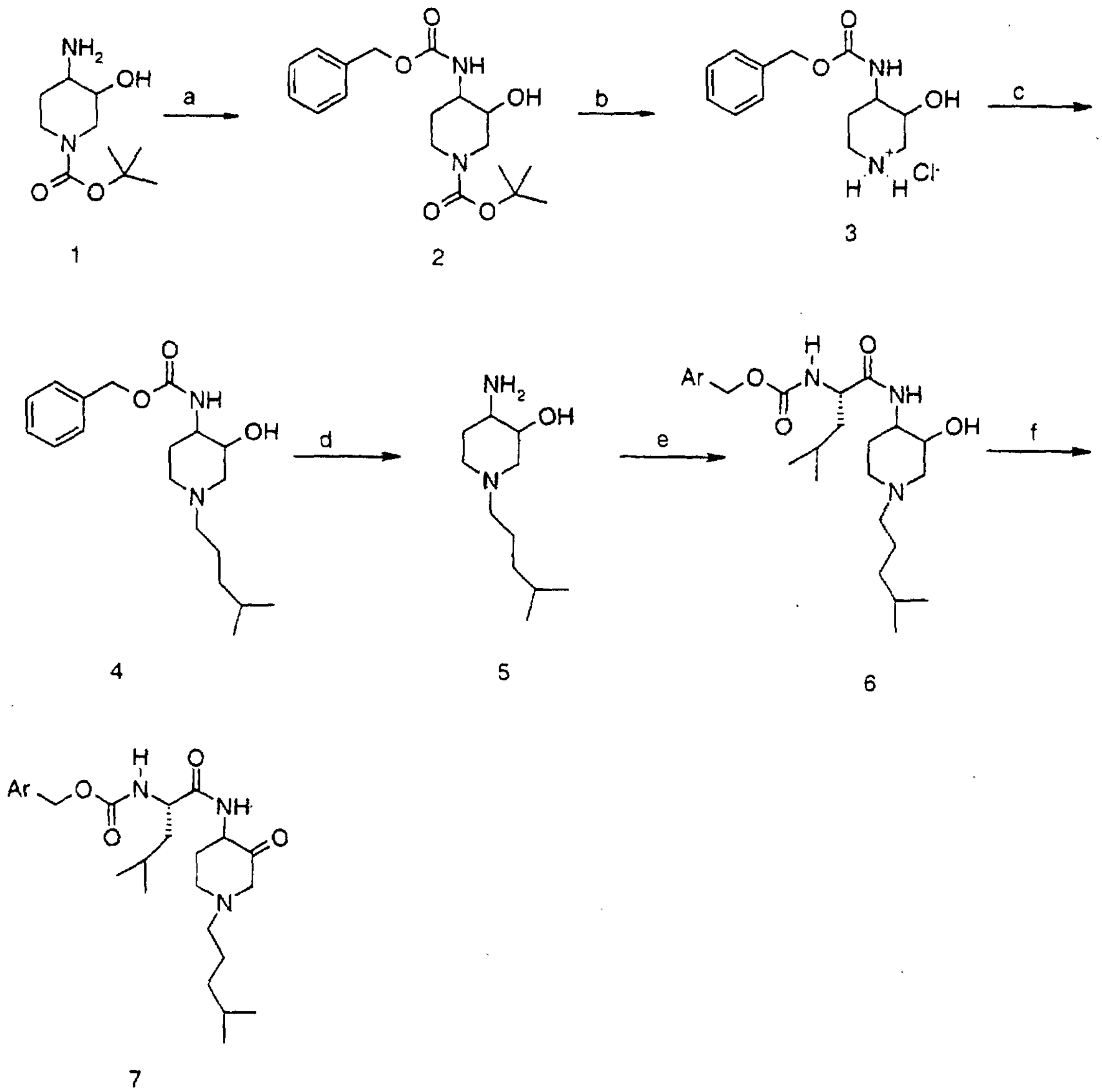


1 2 3 4 5 6 7

a) BnOC(O)Cl, pyridine or TEA, $CH_2Cl_2$; b) HCl, EtOAc; c) RCHO, TEA, $CH_2Cl_2$, sodium triacetoxyborohydride; d) $H_2$, ammonium formate, palladium black; e) $RCO_2H$, EDC, HOBT, DMF; f) sulphur trioxide pyridine complex, DMSO, TEA

Compounds of the formula (I) wherein n is 1, $R^1$ is amide, R" is hydrogen and $R^2$ is alkyl, may be synthesised as detailed in Scheme 3. Acylation of the amino alcohol 1-Scheme-3 with a chloroformate, such as benzyl chloroformate, in the presence of pyridine or triethylamine in an aprotic solvent, such as dichloromethane, provides 2-Scheme

3. Removal of the nitrogen protecting group from the secondary nitrogen by methods which are known in the art provides the amine 3-Scheme-3. This amine may be alkylated by treatment with an aldehyde followed by treatment with a reducing agent, such as sodium cyanoborohydride or sodium triacetaoxyborohydride, to yield 4-Scheme-3. Removal of the benzyloxycarbonyl protecting group via methods that are known in the art provides the amine 5-Scheme-3. This amine may be coupled with an acid in the presence of EDC and HOBT to provide the amide 5-Scheme-4. Oxidation of 5-Scheme-4 via methods that are known in the art, such as pyridine sulphur trioxide complex, provides the ketone 7-Scheme-3.

Scheme 4

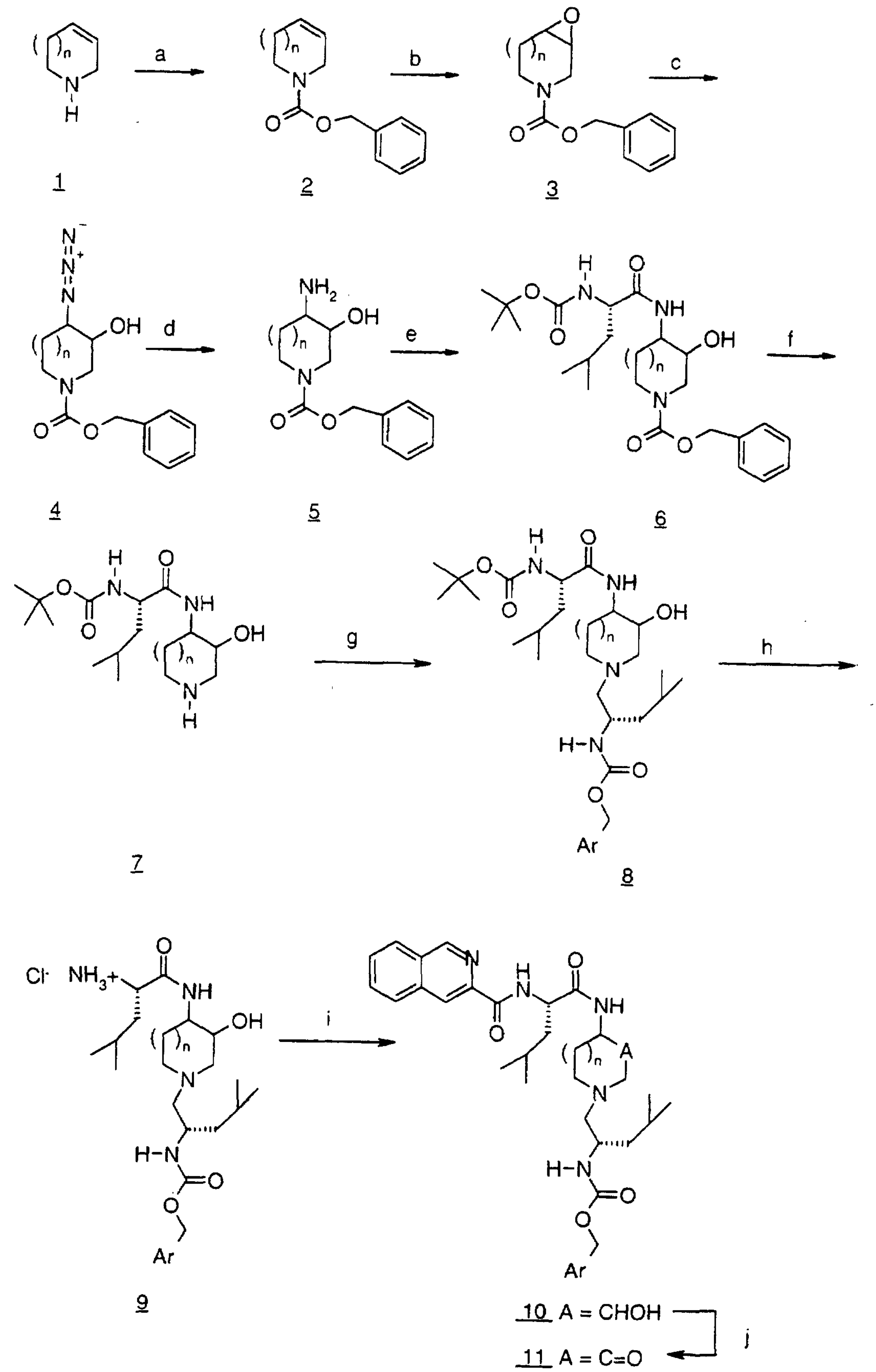


1 2 3 4 5 6 7 8 9

10 A = CHOH

11 A = C=O

a) BnOC(O)Cl, pyridine or TEA, $CH_2Cl_2$; b) m-chloroperoxybenzoic acid, $CH_2Cl_2$; c) $NaN_3$, $NH_4Cl$, $CH_3OH$:$H_2O$ (8:1); d) 1,3-propanedithiol, TEA, $CH_3OH$; e) $RCO_2H$, EDC, HOBt, $CH_2Cl_2$; f) HCl/EtOAc; g) RCHO, $CH_2Cl_2$, sodium triacetoxyborohydride; h) HCl, EtOAc, methanol; i) $RCO_2H$, EDC, HOBt, $CH_2Cl_2$; j) DMSO, sulphur troxide pyridine complex, TEA

Compounds of the formula (1) wherein n is 0 or 1, $R^1$ is an amide, R" is hydrogen and $R^2$ is alkyl may be prepared as outlined in Scheme 4. Protection of the amines 1-Scheme-4 with benzyl chloroformate in the presence of an organic base, such as pyridine or triethylamine, affords 2-Scheme-4. 2-Scheme-4 is epoxidized by standard conditions, such as meta-chloroperoxybenzoic acid, to provide the epoxide 3-Scheme-4 (n = 0,1). The epoxide 3-Scheme-4 may be opened with sodium azide in a protic solvent, such as methanol and water, at an elevated temperature to give the azido alcohol 4-Scheme-4. The azide 4-Scheme-4 may be reduced to the amine 5-scheme-4 by methods that are common to the art, such as 1,3-propanedithiol with triethylamine, in a protic solvent, such as methanol. The amine 5-Scheme-4 is acylated under standard conditions with EDC, HOBt and a carboxylic acid in an aprotic solvent, such as dichloromethane or DMF, to give 6-Scheme-4. The amine 5-Scheme-4 may also be acylated with an acid chloride in the presence of an organic base, such as triethylamine or N-methyl morpholine, in an aprotic solvent, such as dichloromethane, to give the amide derivative 6-Scheme-4. Removal of the protecting group of 6-Scheme-4 is accomplished by methods known in the art, such as treatment with 10% palladium on carbon under hydrogen, to give 7-Scheme-4 compounds. The amine 7-Scheme-4 may be alkylated by treatment with an aldehyde in an aprotic solvent, such as dichloromethane, followed by reduction with sodium cyanoborohydride or sodium triacetoxyborohydride. Alternatively, the amine 7-Scheme-4 may be acylated under standard conditions such as EDC, HOBt and a carboxylic acid or with an acid chloride, as described previously, to give amide derivatives of 8-Scheme-4 (n = 0,1). The *tert*-butoxycarbonyl protecting group of 8-Scheme-4 may be removed by methods that are known in the art, such as treament with hydrogen chloride or trifluoroacetic acid, in an aprotic solvent, such as dichloromethane or ethyl acetate. The amine salt may be coupled with an acid or acid chloride to give amides such as 10-Scheme-4. Alternatively, the amine salt 9-Scheme-4 may be converted to a carbamate by treatment with a chloroformate in the presence of base, such as triethylamine. 9-Scheme-4 may also be converted to sulphonamide by treatment with a sulphonyl chloride in the presence of base, such as triethylamine or N-methylmorpholine, in an aprotic solvent, such as dichloromethane. 9-Scheme-4 may also be converted to a urea by methods that are common to the art such as treatment with an isocyanate in the presence of base, such as triethyamine, in an aprotic solvent, such as dichloromethane. The alcohol 10-Scheme-4 may be oxidised by methods that are common in the art, such as pyridine sulphur trioxide complex with triethylamine in DMSO or methyl sulphoxide and oxalyl chloride at low temperature, in an aprotic solvent, such as dichloromethane, followed by tratment with an organic base, such as triethylamine, and warming.

Scheme 5

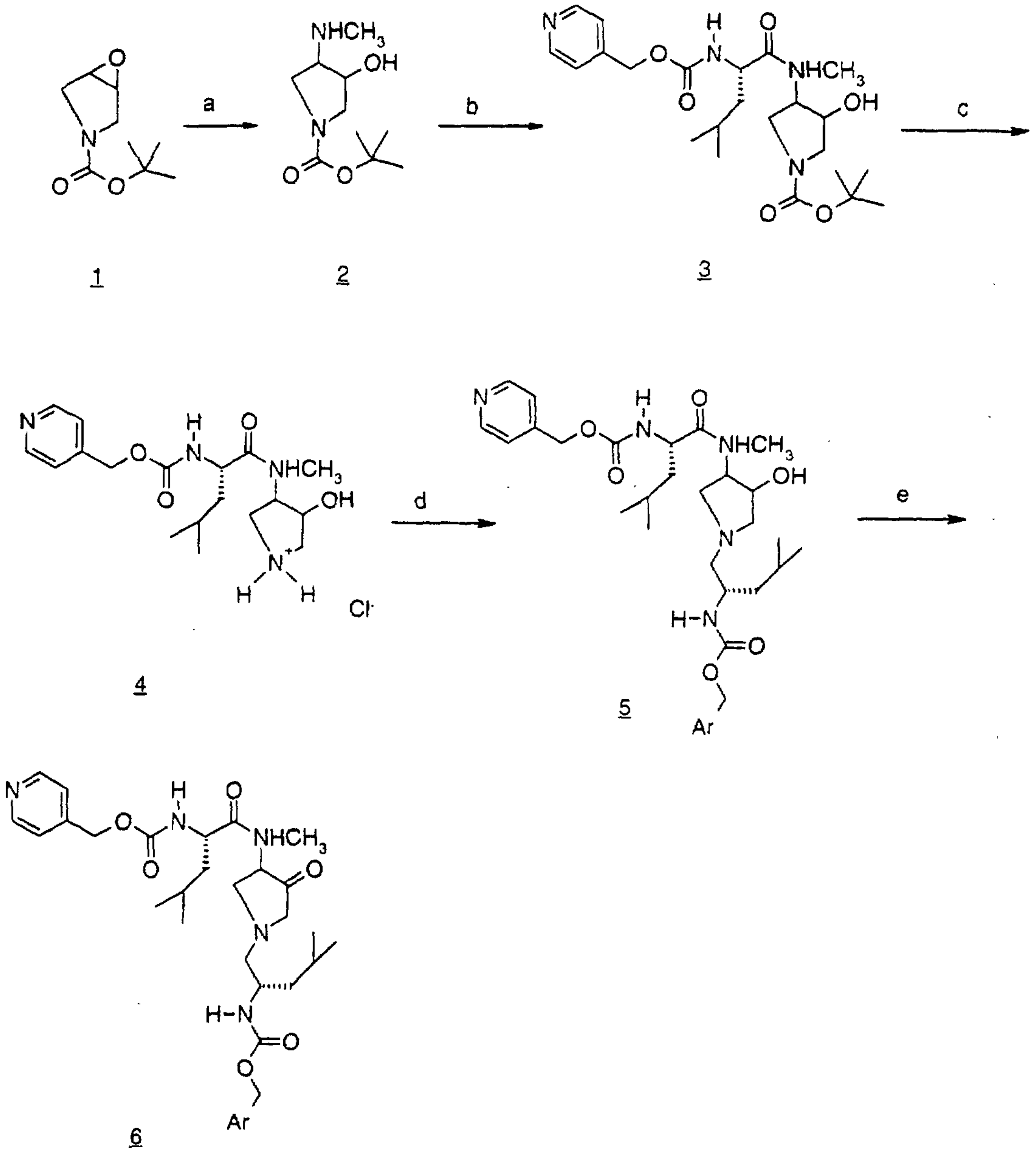


1 2 3 4 5 6

a) methylamine; b) $RCO_2H$, EDC, HOBT, $CH_2Cl_2$; c) HCl, EtOAc; d) RCHO, TEA, $CH_2Cl_2$, sodium triacetoxyborohydride; e) pyridine sulphur trioxide complex, DMSO, TEA

Compounds of the formula of (I) wherein n is 0, $R^1$ is an amide, R" is methyl and $R^2$ is alkyl were prepared as outlined in Scheme 5. The epoxide 1-Scheme-5 may be opened with methylamine to provide 2-Scheme-5. Acylaton of 2-Scheme -5 by methods that are known in the art, such coupling with a carboxylic acid with EDC and HOBt, provides the amide 3-Scheme-5. Removal of the protecting group may be accomplished by treating 3-Scheme-5 with a strong acid such as trifluoroacetic acid or hydrogen chloride, in an aprotic solvent, such as dicholormethane or ethyl acetate, provides the amine salt 4-Scheme-5. This salt may be alkylated by treating it with an aldehyde followed by reduction with a reducing agent, such as sodium cyanoborohydride or sodium triacetoxyborohydride, to provide 5-Scheme-5. The alcohol 5-Scheme-5 may be oxidised by methods that are common to the art, such as pyridine sulphur trioxide complex.

The starting materials used herein are commercially available amino acids or are prepared by routine methods well known to those of ordinary skill in the art and can be found in standard reference books, such as the COMPENDIUM OF ORGANIC SYNTHETIC METHODS, Vol. I-VI (published by Wiley-Interscience).

Coupling methods to form amide bonds herein are generally well known to the art. The methods of peptide synthesis generally set forth by Bodansky *et al*., THE PRACTICE OF PEPTIDE SYNTHESIS, Springer-Verlag, Berlin, 1984; E. Gross and J. Meienhofer, THE PEPTIDES, Vol. 1, 1-284 (1979); and J.M, Stewart and J.D. Young, SOLID PHASE PEPTIDE SYNTHESIS, 2d Ed., Pierce Chemical Co., Rockford, Ill., 1984. are generally illustrative of the technique and are incorporated herein by reference.

Synthetic methods to prepare the compounds of this invention frequently employ protective groups to mask a reactive functionality or minimize unwanted side reactions. Such protective groups are described generally in Green, T.W, PROTECTIVE GROUPS IN ORGANIC SYNTHESIS, John Wiley & Sons, New York (1981). The term "amino protecting groups" generally refers to the Boc, acetyl, benzoyl, Fmoc and Cbz groups and derivatives thereof as known to the art. Methods for protection and deprotection, and replacement of an amino protecting group with another moiety are well known.

Acid addition salts of the compounds of formula (I) are prepared in a standard manner in a suitable solvent from the parent compound and an excess of an acid, such as hydrochloric, hydrobromic, hydrofluoric, sulfuric, phosphoric, acetic, trifluoroacetic, maleic, succinic or methanesulfonic. Certain of the compounds form inner salts or zwitterions which may be acceptable. Cationic salts are prepared by treating the parent compound with an excess of an alkaline reagent, such as a hydroxide, carbonate or alkoxide, containing the appropriate cation; or with an appropriate organic amine. Cations such as $Li^+$, $Na^+$, $K^+$, $Ca^{++}$, $Mg^{++}$ and $NH_4^+$ are specific examples of cations present in pharmaceutically acceptable salts. Halides, sulfate, phosphate, alkanoates (such as acetate and trifluoroacetate), benzoates, and sulfonates (such as mesylate) are examples of anions present in pharmaceutically acceptable salts.

This invention also provides a pharmaceutical composition which comprises a compound according to formula (I) and a pharmaceutically acceptable carrier, diluent or excipient. Accordingly, the compounds of formula (I) may be used in the manufacture of a medicament. Pharmaceutical compositions of the compounds of formula (I) prepared as hereinbefore described may be formulated as solutions or lyophilized powders for parenteral administration. Powders may be reconstituted by addition of a suitable diluent or other pharmaceutically acceptable carrier prior to use. The liquid formulation may be a buffered, isotonic, aqueous solution. Examples of suitable diluents are normal isotonic saline solution, standard 5% dextrose in water or buffered sodium or ammonium acetate solution. Such formulation is especially suitable for parenteral administration, but may also be used for oral administration or contained in a metered dose inhaler or nebulizer for insufflation. It may be desirable to add excipients such as polyvinylpyrrolidone, gelatin, hydroxy cellulose, acacia, polyethylene glycol, mannitol, sodium chloride or sodium citrate.

Alternately, these compounds may be encapsulated, tableted or prepared in an emulsion or syrup for oral administration. Pharmaceutically acceptable solid or liquid carriers may be added to enhance or stabilize the composition, or to facilitate preparation of the composition. Solid carriers include starch, lactose, calcium sulfate dihydrate, terra alba, magnesium stearate or stearic acid, talc, pectin, acacia, agar or gelatin. Liquid carriers include syrup, peanut oil, olive oil, saline and water. The carrier may also include a sustained release material such as glyceryl monostearate or glyceryl distearate, alone or with a wax. The amount of solid carrier varies but, preferably, will be between about 20 mg to about 1 g per dosage unit. The pharmaceutical preparations are made following the conventional techniques of pharmacy involving milling, mixing, granulating, and compressing, when necessary, for tablet forms; or milling, mixing and filling for hard gelatin capsule forms. When a liquid carrier is used, the preparation will be in the form of a syrup, elixir, emulsion or an aqueous or non-aqueous suspension. Such a liquid formulation may be administered directly p. o. or filled into a soft gelatin capsule.

For rectal administration, the compounds of this invention may also be combined with excipients such as cocoa butter, glycerin, gelatin or polyethylene glycols and molded into a suppository.

The compounds of formula (I) are useful as protease inhibitors, particularly as inhibitors of cysteine and serine proteases, more particularly as inhibitors of cysteine proteases, even more particularly as inhibitors of cysteine proteases of the papain superfamily, yet more particularly as inhibitors of cysteine proteases of the cathepsin family, most particularly as inhibitors of cathepsin K. The present invention also provides useful compositions and formulations of said compounds, including pharmaceutical compositions and formulations of said compounds.

The present compounds are useful for treating diseases in which cysteine proteases are implicated, including infections by pneumocystis carinii, trypsanoma cruzi, trypsanoma brucei, and Crithidia fusiculata; as well as in schistosomiasis, malaria, tumor metastasis, metachromatic leukodystrophy, muscular dystrophy, amytrophy; and especially diseases in which cathepsin K is implicated, most particularly diseases of excessive bone or cartilage loss, including osteoporosis, gingival disease including gingivitis and periodontitis, arthritis, more specifically, osteoarthritis and rheumatoid arthritis, Paget's disease; hypercalcemia of malignancy, and metabolic bone disease.

Metastatic neoplastic cells also typically express high levels of proteolytic enzymes that degrade the surrounding matrix, and compounds of the present invention may also find use in the treatment of certain tumors and metastatic neoplasias.

The present invention also provides compounds of the present invention for use in treating diseases caused by pathological levels of proteases, particularly cysteine and serine proteases, more particularly cysteine proteases,

even more particularly cysteine proteases of the papain superfamily, yet more particularly cysteine proteases of the cathepsin family. The present invention especially provides for an inhibitor of cathepsin K, including a compound of the present invention, for use in treating diseases caused by pathological levels of cathepsin K. The present invention particularly provides compounds for use in treating diseases in which cysteine proteases are implicated, including infections by pneumocystis carinii, trypsanoma cruzi, trypsanoma brucei, and Crithidia fusiculata; as well as in schistosomiasis, malaria, tumor metastasis, metachromatic leukodystrophy, muscular dystrophy, amytrophy, and especially diseases in which cathepsin K is implicated, most particularly diseases of excessive bone or cartilage loss, including osteoporosis, gingival disease including gingivitis and periodontitis, arthritis, more specifically, osteoarthritis and rheumatoid arthritis, Paget's disease, hypercalcemia of malignancy, and metabolic bone disease.

This invention further provides a compound of formula (I) for use in treating osteoporosis or inhibiting bone loss which comprises internal administration to a patient of an effective amount of a compound of formula (I), alone or in combination with other inhibitors of bone resorption, such as bisphosphonates (i.e., allendronate), hormone replacement therapy, anti-estrogens, or calcitonin. In addition, treatment with a compound of this invention and an anabolic agent, such as bone morphogenic protein, iproflavone, may be used to prevent bone loss or to increase bone mass.

For acute therapy, parenteral administration of a compound of formula (I) is preferred. An intravenous infusion of the compound in 5% dextrose in water or normal saline, or a similar formulation with suitable excipients, is most effective, although an intramuscular bolus injection is also useful. Typically, the parenteral dose will be about 0.01 to about 100 mg/kg; preferably between 0.1 and 20 mg/kg, in a manner to maintain the concentration of drug in the plasma at a concentration effective to inhibit cathepsin K. The compounds are administered one to four times daily at a level to achieve a total daily dose of about 0.4 to about 400 mg/kg/day. The precise amount of an inventive compound which is therapeutically effective, and the route by which such compound is best administered, is readily determined by one of ordinary skill in the art by comparing the blood level of the agent to the concentration required to have a therapeutic effect.

The compounds of this invention may also be administered orally to the patient, in a manner such that the concentration of drug is sufficient to inhibit bone resorption or to achieve any other therapeutic indication as disclosed herein. Typically, a pharmaceutical composition containing the compound is administered at an oral dose of between about 0.1 to about 50 mg/kg in a manner consistent with the condition of the patient. Preferably the oral dose would be about 0.5 to about 20 mg/kg.

No unacceptable toxicological effects are expected when compounds of the present invention are administered in accordance with the present invention.

The compounds of this invention may be tested in one of several biological assays to determine the concentration of compound which is required to have a given pharmacological effect.

**Determination of cathepsin K proteolytic catalytic activity**

All assays for cathepsin K were carried out with human recombinant enzyme. Standard assay conditions for the determination of kinetic constants used a fluorogenic peptide substrate, typically Cbz-Phe-Arg-AMC, and were determined in 100 mM Na acetate at pH 5.5 containing 20 mM cysteine and 5 mM EDTA. Stock substrate solutions were prepared at concentrations of 10 or 20 mM in DMSO with 20 uM final substrate concentration in the assays. All assays contained 10% DMSO. Independent experiments found that this level of DMSO had no effect on enzyme activity or kinetic constants. All assays were conducted at ambient temperature. Product fluorescence (excitation at 360 nM; emission at 460 nM) was monitored with a Perceptive Biosystems Cytofluor II fluorescent plate reader. Product progress curves were generated over 20 to 30 minutes following formation of AMC product.

**Inhibition studies**

Potential inhibitors were evaluated using the progress curve method. Assays were carried out in the presence of variable concentrations of test compound. Reactions were initiated by addition of enzyme to buffered solutions of inhibitor and substrate. Data analysis was conducted according to one of two procedures depending on the appearance of the progress curves in the presence of inhibitors. For those compounds whose progress curves were linear, apparent inhibition constants ($K_{i,app}$) were calculated according to equation 1 (Brandt *et al.*, *Biochemitsry,* **1989**, *28*, 140):

$$\nu = V_m A/[K_a(1 + I/K_{i,\ app}) + A] \qquad (1)$$

where $\nu$ is the velocity of the reaction with maximal velocity $V_m$ , $A$ is the concentration of substrate with Michaelis constant of $K_a$, and $I$ is the concentration of inhibitor.

For those compounds whose progress curves showed downward curvature characteristic of time-dependent inhibition, the data from individual sets was analyzed to give $k_{obs}$ according to equation 2:

$$[AMC] = \nu_{ss}\, t + (\nu_0 - \nu_{ss})\, [1 - exp(-k_{obs}t)]/k_{obs} \qquad (2)$$

where [AMC] is the concentration of product formed over *time t*, $\nu_0$ is the initial reaction velocity and $\nu_{ss}$ is the final steady state rate. Values for $k_{obs}$ were then analyzed as a linear function of inhibitor concentration to generate an apparent second order rate constant ($k_{obs}$ / inhibitor concentration or $k_{obs}$ / [I]) describing the time-dependent inhibition. A complete discussion of this kinetic treatment has been fully described (Morrison *et al*., *Adv. Enzymol. Relat. Areas Mol. Biol*., **1988**, *61*, 201).

One skilled in the art would consider any compound with a $K_i$ of less than 50 micromolar to be a potential lead compound. Preferably, the compounds used in the method of the present invention have a $K_i$ value of less than 1 micromolar. Most preferably, said compounds have a $K_i$ value of less than 100 nanomolar. 4-(*R,S*)-Amino-N-[(8-quinolinesulfonyl)-*S*-leucine]-3-tetrahydrofuran-3-one, a compound of formula (I), has a $K_i$ value that is greater than 10 micromolar.

**Human Osteoclast Resorption Assay**

Aliquots of osteoclastoma-derived cell suspensions were removed from liquid nitrogen storage, warmed rapidly at 37°C and washed x1 in RPMI-1640 medium by centrifugation (1000 rpm, 5 min at 4°C). The medium was aspirated and replaced with murine anti-HLA-DR antibody, diluted 1:3 in RPMI-1640 medium, and incubated for 30 min on ice The cell suspension was mixed frequently.

The cells were washed x2 with cold RPMI-1640 by centrifugation (1000 rpm. 5 min at 4°C) and then transferred to a sterile 15 mL centrifuge tube. The number of mononuclear cells were enumerated in an improved Neubauer counting chamber.

Sufficient magnetic beads (5 / mononuclear cell), coated with goat anti-mouse IgG, were removed from their stock bottle and placed into 5 mL of fresh medium (this washes away the toxic azide preservative). The medium was removed by immobilizing the beads on a magnet and is replaced with fresh medium.

The beads were mixed with the cells and the suspension was incubated for 30 min on ice. The suspension was mixed frequently, The bead-coated cells were immobilized on a magnet and the remaining cells (osteoclast-rich fraction) were decanted into a sterile 50 mL centrifuge tube. Fresh medium was added to the bead-coated cells to dislodge any trapped osteoclasts. This wash process was repeated x10. The bead-coated cells were discarded.

The osteociasts were enumerated in a counting chamber, using a large-bore disposable plastic pasteur pipette to charge the chamber with the sample. The cells were pelleted by centrifugation and the density of osteoclasts adjusted to $1.5 \times 10^4$/mL in EMEM medium, supplemented with 10% fetal calf serum and 1.7g/litre of sodium bicarbonate. 3 mL aliquots of the cell suspension ( per treatment) were decanted into 15 mL centrifuge tubes. These cells were pelleted by centrifugation. To each tube 3 mL of the appropriate treatment was added (diluted to 50 uM in the EMEM medium). Also included were appropriate vehicle controls, a positive control (87MEM1 diluted to 100 ug/mL) and an isotype control (IgG2a diluted to 100 ug/mL). The tubes were incubate at 37°C for 30 min.

0.5 mL aliquots of the cells were seeded onto sterile dentine slices in a 48-well plate and incubated at 37°C for 2 h. Each treatment was screened in quadruplicate. The slices were washed in six changes of warm PBS (10 mL / well in a 6-well plate) and then placed into fresh treatment or control and incubated at 37°C for 48 h. The slices were then washed in phosphate buffered saline and fixed in 2% glutaraldehyde (in 0.2M sodium cacodylate) for 5 min., following which they were washed in water and incubated in buffer for 5 min at 37°C. The slices were then washed in cold water and incubated in cold acetate buffer / fast red garnet for 5 min at 4°C. Excess buffer was aspirated, and the slices were air dried following a wash in water.

The TRAP positive osteoclasts were enumerated by bright-field microscopy and were then removed from the surface of the dentine by sonication. Pit volumes were determined using the Nikon/Lasertec ILM21W confocal microscope.

Examples

Nuclear magnetic resonance spectra were recorded at either 250 or 400 MHz using, respectively, a Bruker AM 250 or Bruker AC 400 spectrometer. $CDCl_3$ is deuteriochloroform, DMSO-$d_6$ is hexadeuteriodimethylsulfoxide, and $CD_3OD$ is tetradeuteriomethanol. Chemical shifts are reported in parts per million (d) downfield from the internal standard tetramethylsilane. Abbreviations for NMR data are as follows: s = singlet, d = doublet, t = triplet, q = quartet, m =

multiplet, dd = doublet of doublets, dt = doublet of triplets, app = apparent, br = broad. J indicates the NMR coupling constant measured in Hertz. Continuous wave infrared (IR) spectra were recorded on a Perkin-Elmer 683 infrared spectrometer, and Fourier transform infrared (FTIR) spectra were recorded on a Nicolet Impact 400 D infrared spectrometer. IR and FTIR spectra were recorded in transmission mode, and band positions are reported in inverse wavenumbers ($cm^{-1}$). Mass spectra were taken on either VG 70 FE, PE Syx API III, or VG ZAB HF instruments, using fast atom bombardment (FAB) or electrospray (ES) ionization techniques. Elemental analyses were obtained using a Perkin-Elmer 240C elemental analyzer. Melting points were taken on a Thomas-Hoover melting point apparatus and are uncorrected. All temperatures are reported in degrees Celsius.

Analtech Silica Gel GF and E. Merck Silica Gel 60 F-254 thin layer plates were used for thin layer chromatography. Both flash and gravity chromatography were carried out on E. Merck Kieselgel 60 (230-400 mesh) silica gel.

Where indicated, certain of the materials were purchased from the Aldrich Chemical Co., Milwaukee, Wisconsin, Chemical Dynamics Corp., South Plainfield, New Jersey, and Advanced Chemtech, Louisville, Kentucky.

In the following synthetic examples, temperature is in degrees Centigrade (°C). Unless otherwise indicated, all of the starting materials were obtained from commercial sources. Without further elaboration, it is believed that one skilled in the art can, using the preceding description, utilize the present invention to its fullest extent. These Examples are given to illustrate the invention, not to limit its scope. Reference is made to the claims for what is reserved to the inventors hereunder.

Example 1

Preparation of 4-[[N$^{\alpha}$-(benzyloxycarbonyl)-L-leucinyl]amino]-1-[(2S)-4-methyl-2-[[(benzyloxycarbonyl)]amino] pentanoyl]-3-pyrrolidinone

a.) 1-*tert*-butoxycarbonyl-3-pyrrolidine

To a solution of 3-pyrroline (5.0 g, 72.35 mmol) in $CH_2Cl_2$ (25 mL) at room was added di-t-butyl dicarbonate (16.58 g, 75.97 mmol) in $CH_2Cl_2$ (50 mL). The reaction was stirred for ca. 1 hour whereupon it was concentrated in vacuo to give the BOC protected 3-pyrroline which was used directly in the following step without further purification: $^1H$ NMR (200 MHz, $CD_3OD$) 5.12 (m, 2H), 3.92 (m, 4H), 1.38 (s, 9H).

b.) 1-*tert* -butoxycarbonyl-3,4-epoxypyrrolidine

To a solution of compound of Example 1(a) (5.0 g, 29.5 mmol) in $CH_2Cl_2$ (200 mL) was added $NaHCO_3$ (9.03 g, 118.2 mmol) and m-CPBA (15.29 g, 88.6 mmol). The reaction was stirred at room temperature overnight whereupon it was concentrated and filtered with petroleum ether. The petroleum ether layer was washed with saturated $K_2CO_3$ (2x's), water, brine, dried ($MgSO_4$) and concentrated to give a clear colorless oil. Column chromatography of the oil (4:1 hexanes:ethyl acetate) gave the title compound which was used directly in the following step: $^1H$ NMR (200 MHz, $CDCl_3$) 3.85-3.20 (m, 6H), 1.43 (s,9H).

c.) 1-*tert*-butoxycarbonyl-*trans*-3-azido-4-hydroxypyrrolidine

To a solution of the compound of Example 1(b) (2.03 g, 10.96 mmol) in methanol:water (18 mL of an 8:1 solution) was added ammonium chloride (2.5 g, 10.96 mmol) and sodium azide (3.56 g, 54.8 mmol). The reaction was heated at 60°C overnight whereupon it was diluted with petroleum ether, washed with pH=4 buffer, sat. sodium bicarbonate, brine, dried ($MgSO_4$) and concentrated to give 2.12 g of the azido alcohol which was carried onto the next step without further purification: $^1H$ NMR (400 MHz, $CDCl_3$) 4.21 (br s, 1H), 3.92 (br s, 1H), 3.71-3.30 (m, 4H), 1.43 (s, 9H).

d.) 1-*tert*-butoxycarbonyl-*trans*-3-amino-4-hydroxypyrrolidine

To a solution of the compound of Example 1(c) (210 mg, 0.92 mmol) in $CH_3OH$ (10 mL) was added 10% Pd on carbon. This mixture was stirred under an atmosphere of hydrogen until TLC analysis indicated the complete disappearence of the starting material. The reaction was filtered through a pad of celite with $CH_2Cl_2$ and concentrated to give 202 mg of the title compound which was used directly in the following reaction.

e.) (3RS,4RS)-4-[[N$^{\alpha}$-(benzyloxycarbonyl)-L-leucinyl]amino]-1-*tert*-butoxycarbonyl-3-pyrrolidinol

To a solution of the compound of Example 1(d) (202 mg, 1.14 mmol) in $CH_2Cl_2$ (5 mL) was added CBZ-leucine (302.9 mg, 1.14 mmol), HOBT (154 mg, 1.14 mmol) and EDC (262.2 mg, 1.37 mmol). The reaction was allowed to stir

until complete by TLC analysis whereupon it was diluted with EtOAc and washed sequentially with pH 4 buffer, sat. $K_2CO_3$, water and brine. The organic layer was dried ($MgSO_4$), filtered and concentrated. Column chromatography of the residue (3:1EtOAc:hexanes) gave 325 mg of the title compound: MS(ES+) 450.3 ($MH^+$), 472.2 (M+Na).

f.) (3RS,4RS)-4-[[$N^\alpha$-(benzyloxycarbonyl)-L-leucinyl]amino]-3-pyrrolidinol hydrochloride

To a solution of the compound of Example 1(e) (310 mg, 0.69 mmol) in dry EtOAc (5.0 mL) was bubbled HCl gas for approximately 5 minutes. The reaction was stirred until TLC analysis indicated the complete consumption of the starting material. The reaction was then concentrated in vacuo to give 249 mg of the title compound: MS(ES+) 350.3 ($MH^+$)

g.) (3RS,4RS)-4-[[$N^\alpha$-(benzyloxycarbonyl)-L-leucinyl]amino]-1-[(2S)-4-methyl-2-[[(benzyloxycarbonyl)]amino] pentanoyl]-3-pyrrolidinol

To a solution of the compound of Example 1(f) (249 mg, 0.64 mmol) in $CH_2Cl_2$ (10 mL) was added CBZ-leucine (170.4 mg, 0.64 mmol), HOBT (86.5 mg, 0.64 mmol), NMM (300 uL) and EDC (147.2 mg, 0.77 mmol). The reaction was allowed to stir at room temperature for 2 hours whereupon it was diluted with ethyl acetate and worked up as described previously. Column chromatography of the residue (3:1 EtOAc:hexanes) gave 104 mg of the title compound: MS(ES+) 597.1 ($MH^+$), 619.1 (M+Na).

h.) 4-[[$N^\alpha$-(benzyloxycarbonyl)-L-leucinyl]amino]-1-[(2S)-4-methyl-2-[[(benzyloxycarbonyl)]amino]pentanoyl]-3-pyrrolidinone

To a 0°C solution of the compound of Example 1(g) (100 mg, 0.17 mmol) in acetone (5.0 mL) was added Jones reagent dropwise until the brown color persisted. The reaction was allowed to warm to room temperature and stirred approximately 48 hours whereupon it was quenched with iso-propanol, diluted with EtOAc and washed sequentially with sa'd. $K_2CO_3$, water and brine. The organic layer was dried ($MgSO_4$), filtered and concentrated. Column chromatography of the residue (3:1 EtOAc:hexanes) gave 31 mg of the title compound: MS(ES+) 595.1 ($MH^+$), 617.0 (M+Na).

Example 2

Preparation of 4-[[$N^\alpha$-(benzyloxycarbonyl)-L-leucinyl]amino]-1-[4-(phenoxybenzamide)]-3-pyrrolidinone

Following the procedure of Example 1(g)-1(h) except substituting 4-phenoxybenzoic acid for CBZ-leucine in step 1(g), the title compound was prepared: MS(ES+) 544.3 ($MH^+$), 566.2 (M+Na).

Example 3

Preparation of 4-[[$N^\alpha$-(benzyloxycarbonyl)-L-leucinyl]amino]-1-[4-(biphenylethanoyl)]-3-pyrrolidinone

a.) (3RS,4RS)-4-[[$N^\alpha$-(benzyloxycarbonyl)-L-leucinyl]amino]-1-[4-(biphenylethanoyl)]-3-pyrrolidinol

Following the procedure of Example 1(g) except substituting 4-biphenylacetic acid for CBZ-leucine, the title compound was prepared: MS(ES+) 544.3 ($MH^+$).

b.) 4-[[$N^\alpha$-(benzyloxycarbonyl)-L-leucinyl]amino]-1-[4-(biphenylethanoyl)]-3-pyrrolidinone

To a -78°C solution of oxalyl chloride (0.026 mL, 0.29 mmol) in $CH_2Cl_2$ was added DMSO (0.042 mL, 0.59 mmol) dropwise. The reaction was maintained at -78°C for approximately 20 minutes whereupon a solution of the compound of Example 3(a) (65 mg, 0.12 mmol) in $CH_2Cl_2$ was added dropwise. The reaction was maintained at -78°C for 30 minutes whereupon triethylamine (0.16 mL, 1.19 mmol) was added. The reaction was allowed to warm to room temperature, diluted with EtOAc and washed sequentially with pH 4 buffer, water and brine. The organic layer was dried ($MgSO_4$) filtered and concentrated. Column chromatography of the residue (3:1 EtOAc:hexanes) gave 35 mg (54%)of the title compound: MS(ES+) 542.3, 564.3 (M+Na).

Example 4

Preparation of 4-[[$N^{\alpha}$-(benzyloxycarbonyl)-L-leucinyl]amino]-1-[(2S)-4-methyl-2-[[(benzyloxycarbonyl)]aminomethyl] pentanoyl]-3-pyrrolidinone

a.) (3RS,4RS)-4-[[$N^{\alpha}$-(benzyloxycarbonyl)-L-leucinyl]amino]-1-[(2S)-4-methyl-2-[[(benzyloxycarbonyl)]aminomethyl] pentanoyl]-3-pyrrolidinol

Following the procedure of Example 1(g) except substituting N-methyl-CBZ-leucine for CBZ-leucine, the title compound was prepared: MS(ES+) 611.3 ($MH^+$), 633.3 ($MH^+$+Na).

b.) 4-[[$N^{\alpha}$-(benzyloxycarbonyl)-L-leucinyl]amino]-1-[(2S)-4-methyl-2-[[(benzyloxycarbonyl)]aminomethyl]pentanoyl]-3-pyrrolidinone

Following the procedure of Example 3(b) except substituting the compound of Example 4(a), the title compound was produced: MS(ES+) 609.3 ($MH^+$).

Example 5

Preparation of 4-[[$N^{\alpha}$-(benzyloxycarbonyl)-L-leucinyl]amino]-1-[(2S)-4-methyl-2-[[(*tert*-butoxyoxycarbonyl)] aminomethyl]pentanoyl]-3-pyrrolidinone

a.) (3RS,4RS)-4-[[$N^{\alpha}$-(benzyloxycarbonyl)-L-leucinyl]amino]-1-[(2S)-4-methyl-2-[[(tert-butoxyoxycarbonyl)] aminomethyl]pentanoyl]-3-pyrrolidinol

Following the procedure of Example 1(g) except substituting N-methyl-N-BOC-leucine for CBZ-leucine, the title compound was produced: MS(ES+) 477.4 ($MH^+$-$CO_2$-t-Bu), 577.4 ($MH^+$), 599.4 (M+Na).

b.) 4-[[$N^{\alpha}$-(benzyloxycarbonyl)-L-leucinyl]amino]-1-[(2S)-4-methyl-2-[[(tert-butoxyoxycarbonyl)]aminomethyl] pentanoyl]-3-pyrrolidinone

Following the procedure of Example 3(b) except substituting the compound of Example 5(a), the title compound was produced: MS(ES+) 475.4 ($MH^+$-$CO_2$-t-Bu), 575.3 ($MH^+$), 597.4 (M+Na).

Example 6

Preparation of 4-[[$N^{\alpha}$-(benzyloxycarbonyl)-L-leucinyl]amino]-1-[(2S)-4-methyl-2-(aminomethyl)pentanoyl]-3-pyrrolidinone hydrochloride

To a solution of the compound of Example 5(b) in dry ethyl acetate was bubbled HCl (g) for 2 minutes. The reaction was allowed to stir until complete as determined by TLC analysis. The reaction was concentrated and the residue was azeotropically dried with dry toluene (3x5 mL) to give the title compound: MS (ES+) 475.4 ($MH^+$).

Example 7

Preparation of 4-[[$N^{\alpha}$-(benzyloxycarbonyl)-L-leucinyl]amino]-1-*tert*-butoxycarbonyl-3-pyrrolidinone

Following the procedure of Example 3(b) except substituting the compound of Example 1(e), the title compound was produced: MS (ES+) 448.3 (MH+), 470.3 (M+Na)

Example 8

Preparation of 4-[[$N^{\alpha}$-(benzyloxycarbonyl)-L-leucinyl]amino]-3-pyrrolidinone hydrochloride

Following the procedure of Example 6 except substituting the compound of Example 7, the title compound was produced: MS(ES) 348.4 ($MH^+$).

Example 9

Preparation of 4-[[N$^{\alpha}$-(benzyloxycarbonyl)-L-leucinyl]amino]-1-[(2S)-4-methyl-2-[[N-tert-butoxycarbonyl)ethanoyl] aminomethyl]pentanoyl]-3-pyrrolidinone

To a solution of the compound of Example 6 (50 mg, 0.098 mmol) in $CH_2Cl_2$ (5.0 mL) was added N-methyl morpholine (0.054 mL, 0.49 mmol), EDC (22.5 mg, 0.12 mmol), HOBT (13.3 mg, 0.098 mmol) and N-BOC-glycine (17.3 mg, 0.098 mmol). The reaction was allowed to stir at room temperature until complete by TLC analysis. Workup and chromatography (3: 1 ethyl acetate:hexanes) gave 24 mg of the title compound: MS (ES+) 632.4 (MH+), 654.3 (M+Na).

Example 10

Preparation of 4-[[N$^{\alpha}$-(benzyloxycarbonyl)-L-leucinyl]amino]-1-[(2S)-4-methyl-2-[(ethanoyl)aminomethyl]pentanoyl]-3-pyrrolidinone hydrochloride

Following the procedure of Example 6 except substituting the compound of Example 9, produced the title compound: MS (ES) 532.4 ($MH^+$).

Example 11

Preparation of 4-[[N$^{\alpha}$-(benzyloxycarbonyl)-L-leucinyl]amino]-1-[(2S)-4-methyl-2-[[(*tert*-butoxycarbonyl)]amino] pentanoyl]-3-pyrrolidinone

a.) (3RS,4RS)-4-[[N$^{\alpha}$-(benzyloxycarbonyl)-L-leucinyl]amino]-1-[(2S)-4-methyl-2-[[(tert-butoxyoxycarbonyl)]amino] pentanoyl]-3-pyrrolidinol

Following the procedure of Example 1(g) except substituting Boc-leucine for CBZ-leucine, the title compound was prepared. This material was carried onto the oxidation.

b.) 4-[[N$^{\alpha}$-(benzyloxycarbonyl)-L-leucinyl]amino]-1-[(2S)-4-methyl-2-[[(tert-butoxycarbonyl)]amino]pentanoyl]-3-pyrrolidinone

Following the procedure of Example 3(b) except substituting the compound of Example 11(a), the title compound was prepared: MS (ES+) 561.3 (MH+), 583.3 (M+Na).

Example 12

Preparation of 4-[[N$^{\alpha}$-(benzyloxycarbonyl)-L-leucinyl]amino]-1-[(2R)-4-methyl-2-[[(benzyloxycarbonyl)]amino] pentanoyl]-3-pyrrolidinone

a.) (3RS,4RS)-4-[[N$^{\alpha}$-(benzyloxycarbonyl)-L-leucinyl]amino]-1-[(2R)-4-methyl-2-[[(benzyloxycarbonyl)]amino] pentanoyl]-3-pyrrolidinol

Following the procedure of Example 1(g) except substituting CBZ-D-leucine for CBZ-leucine, the title compound was prepared: This compound was used directly in the following step.

b.) 4-[[N$^{\alpha}$-(benzyloxycarbony)-L-leucinyl]amino]-1-[(2R)-4-methyl-2-[[(benzyloxycarbonyl)]amino]pentanoyl]-3-pyrrolidinone

Following the procedure of Example 3(b) except substituting the compound of Example 12(a), the title compound was prepared: MS(ES+) 595.5 ($MH^+$), 633.6 (M+Na).

Example 13

Preparation of 4-[[Nα-(benzyloxycarbonyl)-L-leucinyl]amino]-1-[2-[(benzyloxycarbonyl)amino]ethanoyl]-3-pyrrolidinone

a.) (3RS,4RS)-4-[[Nα-(benzyloxycarbonyl)-L-leucinyl]amino]-1-[2-[(benzyloxycarbonyl)amino]ethanoyl]-3-pyrrolidinonol

Following the procedure of Example 1(g) except substituting CBZ-glycine for CBZ-leucine, the title compound was prepared: This material was used directly in the following step.

b.) 4-[[Nα-(benzyloxycarbonyl)-L-leucinyl]amino]-1-[2-[(benzyloxycarbonyl)amino]ethanoyl]-3-pyrrolidinonone

Following the procedure of Example 3(b) except substituting the compound of Example 13(a), the title compound was prepared: MS(ES+) 539.3 ($MH^+$), 561.3 (M+Na).

Example 14

Preparation of 4-[[Nα-(benzyloxycarbonyl)-L-leucinyl]amino]-1-[(2S)-3-tert-butoxy-[[(benzyloxycarbonyl)]amino]propanoyl]-3-pyrrolidinone

a.) (3RS,4RS)-4-[[Nα-(benzyloxycarbonyl)-L-leucinyl]amino]-1-[(2S)-3-tert-butoxy-[[(benzyloxycarbonyl)]amino]propanoyl]-3-pyrrolidinol

Following the procedure of Example 1(g) except substituting CBZ-Ser(t-Bu)-OH for CBZ-leucine, the title compound was prepared: This material was used directly in the following step.

b.) 4-[[Nα-(benzyloxycarbonyl)-L-leucinyl]amino]-1-[(2S)-3-tert-butoxy-[[(benzyloxycarbonyl)]amino]propanoyl]-3-pyrrolidinone

Following the procedure of Example 3(b) except substituting the compound of Example 14(a), the title compound was prepared: MS(ES+) 625.4 ($MH^+$), 647.3 (M+Na).

Example 15

Preparation of 4-[[Nα-(benzyloxycarbonyl)-L-leucinyl]amino]-1-[(2S)-2-[[benzyloxycarbonyl)]amino]propanoyl]-3-pyrrolidinone

a.) (3RS,4RS)-4-[[Nα-(benzyloxycarbonyl)-L-leucinyl]amino]-1-[(2S)-2-[[(benzyloxycarbonyl)]amino]propanoyl]-3-pyrrolidinol

Following the procedure of Example 1(g) except substituting CBZ-alanine for CBZ-leucine, the title compound was prepared. This material was used directly in the following step.

b.) 4-[[Nα-(benzyloxycarbonyl)-L-leucinyl]amino]-1-[(2S)-2-[[(benzyloxycarbonyl)]amino]propanoyl]-3-pyrrolidinone

Following the procedure of Example 3(b) except substituting the compound of Example 15(a), the title compound was prepared: MS(ES+) 553.3 ($MH^+$), 575.3 (M+Na).

Example 16

Preparation of 4-[[Nα-(benzyloxycarbonyl)-L-leucinyl]amino]-1-[cyclohexanepropanoyl]-3-pyrrolidinone

a.) (3RS,4RS)-4-[[Nα-(benzyloxycarbonyl)-L-leucinyl]amino]-1-[cyclohexanepropanoyl]-3-pyrrolidinol

Following the procedure of Example 1(g) except substituting cyclohexanepropionic acid for CBZ-leucine, the title compound was prepared. This material was used directly in the following step.

b.) 4-[[$N^{\alpha}$-(benzyloxycarbonyl)-L-leucinyl]amino]-1-[cyclohexanepropanoyl]-3-pyrrolidinone

Following the procedure of Example 3(b) except substituting the compound of Example 16(a), the title compound was prepared: MS(ES+) 486.4 ($MH^+$), 508.3 (M+Na).

Example 17

Preparation of 4-[[$N^{\alpha}$-(benzyloxycarbonyl)-L-leucinyl]amino]-1-[(2S)-4-methyl-2-[[(4-pyridinylmethoxycarbonyl)]amino]pentanoyl]-3-pyrrolidinone

a.) (3RS,4RS)-4-[[$N^{\alpha}$-(benzyloxycarbonyl)-L-leucinyl]amino]-1-[(2S)-4-methyl-2-[[(4-pyridinylmethoxycarbonyl)]amino]pentanoyl]-3-pyrrolidinol

Following the procedure of Example 1(g) except substituting N-(4-pyridylmethoxycarbonyl)-L-leucine for CBZ-leucine, the title compound was produced: MS (ES+) 598.2 ($MH^+$)

b.) 4-[[$N^{\alpha}$-(benzyloxycarbonyl)-L-leucinyl]amino]-1-[(2S)-4-methyl-2-[[(4-pyridinylmethoxycarbonyl)]amino]pentanoyl]-3-pyrrolidinone

To a solution of the alcohol of Example 17(a) (200 mg, 0.34 mmol) in DMSO (3 mL) was added TEA (0.30 mL) and sulphur trioxide pyridine complex (162 mg). The reaction was stirred at room temperature for 2 hours whereupon it was partitioned between ethyl acetate and water. The organic layer as washed with water (2x's), brine, dried ($MgSO_4$), concentrated and the residue chromatographed (5% $CH_3OH$:$CH_2Cl_2$) to give 67.3 mg of the title compound: MS (ES+) 596 ($MH^+$).

Example 18

Preparation of 4-[[$N^{\alpha}$-(benzyloxycarbonyl)-L-leucinyl]amino]-1-[(2S)-4-methyl-2-[[(2-pyridinylmethoxycarbonyl)]amino]pentanoyl]-3-pyrrolidinone

a.) (3RS,4RS)-4-[[$N^{\alpha}$-(benzyloxycarbonyl)-L-leucinyl]amino]-1-[(2S)-4-methyl-2-[[(2-pyridinyloxycarbonyl)]amino]pentanoyl]-3-pyrrolidinol

Following the procedure of Example 1(g) except substituting N-(2-pyridylmethoxycarbonyl)-L-leucine for CBZ-leucine, the title compound was produced: MS(ES+) 598 ($MH^+$).

b.) 4-[[$N^{\alpha}$-(benzyloxycarbonyl)-L-leucinyl]amino]-1-[(2S)-4-methyl-2-[[(2-pyridinylmethoxycarbonyl)]amino]pentanoyl]-3-pyrrolidinone

Following the procedure of Example 17(b) except substituting the compound of Example 18(a), the title compound was produced: MS(ES+) 596 ($MH^+$).

Example 19

Preparation of 4-[[$N^{\alpha}$-(benzyloxycarbonyl)-L-leucinyl]amino]-1-[(2S)-4-methyl-2-[[(3-pyridinylmethoxycarbonyl)]amino]pentanoyl]-3-pyrrolidinone

a.) (3RS,4RS)-4-[[$N^{\alpha}$-(benzyloxycarbonyl)-L-leucinyl]amino]-1-[(2S)-4-methyl-2-[[(3-pyridinylmethoxycarbonyl)]amino]pentanoyl]-3-pyrrolidinol

Following the procedure of Example 1(g) except substituting N-(3-pyridylmethoxycarbonyl)-L-leucine for CBZ-leucine, the title compound was prepared: MS(ES+) 598 ($MH^+$).

b.) 4-[[$N^{\alpha}$-(benzyloxycarbonyl)-L-leucinyl]amino]-1-[(2S)-4-methyl-2-[[(3-pyridinylmethoxycarbonyl)]amino]pentanoyl]-3-pyrrolidinone

Following the procedure of Example 17(b) except substituting except substituting the compound of Example 19(a), the title compound was produced: MS(ES+) 596 ($MH^+$).

Example 20

Preparation of 4-[[N$^{\alpha}$-(benzyloxycarbonyl)-L-leucinyl]amino]-1-(2-pyridy)carbonyl)-3-pyrrolidinone

a.) (3RS,4RS)-4-[[N$^{\alpha}$-(benzyloxycarbonyl)-L-leucinyl]amino]-1-(2-pyridylcarbonyl)-3-pyrrolidinol

Following the procedure of Example 1(g) except substituting picolinic acid for CBZ-leucine and triethylamine for N-methylmorpholine, the title compound was produced: MS(ES+) 469 ($MH^+$).

b. 4-[[N$^{\alpha}$-(benzyloxycarbonyl)-L-leucinyl]amino]-1-(2-pyridylcarbonyl)-3-pyrrolidinone

Following the procedure of Example 17(b) except substituting the compound of Example 20(a), the title compound was produced: MS(ES+) 467 (MH+).

Examnle 21

Preparation of 4-[[N$^{\alpha}$-(benzyloxycarbonyl)-L-leucinyl]amino]-1-[(2S)-4-methyl-2-[[(benzyloxycarbonyl)]amino] pentanoyl]-3-piperidinone

a.) 1-*tert*-butoxycarbonyl-1,2,3,6-tetrahydropyridine

To a solution of 1,2,3,6-tetrahydropyridine (5.0 g, 60.0 mmol) in $CH_2Cl_2$ (25 mL) at room was added di-t-butyl dicarbonate (13.75 g, 63.0 mmol) in $CH_2Cl_2$ (50 mL). The reaction was stirred for ca. 1 hour whereupon it was concentrated in vacuo to give 11.1 g of BOC protected amine: $^1$H NMR ($CDCl_3$) 5.8 (m, 1H), 5.6 (m, 1H), 6.88 (br s, 2H), 3.45 (m, 2H), 1.46 (s, 9H).

b.) 1-*tert* -butoxycarbonyl-3,4-epoxy-piperidine

To a solution of the compound of Example 21(a) (5.0 g, 27.3 mmol) in $CH_2Cl_2$ (250 mL) was added m-CPBA (18.83 g, 109.5 mmol) portionwise. The reaction was stirred at room temperature overnight whereupon it was concentrated and diluted and filtered with petroleum ether. The petroleum ether layer was washed with saturated $K_2CO_3$ (2x's), pH=4 buffer, water, brine, dried ($MgSO_4$) and concentrated to give a clear colorless oil. Column chromatography of the oil (4:1 hexanes:ethyl acetate) gave 3.70 g of the epoxide which was used directly in the following step.

c.) 1-*tert*-butoxycarbonyl-3-hydroxy-4-azido-piperidine

To a solution of the compound of Example 21(b) (3.70 g, 18.57 mmol) in methanol:water (18 mL of an 8:1 solution) was added ammonium chloride (2.08 g, 38.98 mmol) and sodium azide (6.03 g, 92.85 mmol). The reaction was heated at reflux overnight whereupon it was diluted with ethyl acetate, washed with 1N HCl, water, brine , dried ($MgSO_4$) and concentrated to give 3.25 g of the azido alcohol which was used directly in the following step.

d.) 1-*tert*-butoxycarbonyl-3-hydroxy-4-amino-piperidine

To a solution of the compound of Example 21(c) (3.25 g) in $CH_3OH$ (25 mL) was added 10% Pd on carbon (1 g). This mixture was stirred under an atmosphere of hydrogen until TLC analysis indicated the complete disappearence of the starting material. The reaction was filtered through a pad of celite with $CH_2Cl_2$ and concentrated to give the amino alcohol.

e.) (3RS,4RS)-4-[[N$^{\alpha}$-(benzyloxycarbonyl)-L-leucinyl]amino]-1-*tert*-butoxycarbonyl-3-piperidinol

To a solution of the compound of Example 21(d) (1.0 g, 4.62 mmol) was added CBZ-leucine (1.22 g, 4.62 mmol), EDC (1.07 g, 5.58 mmol) and HOBT (624 mg, 4.62 mmol). The reaction was allowed to stir until complete as indicated by TLC analysis. Workup and column chromatography (1:1 hexanes:EtOAc) gave 883 mg of the title compound: MS(ES+) 464.4 ($MH^+$), 486.2 (M+Na).

f.) (3RS,4RS)-4-[[N$^{\alpha}$-(benzyloxycarbonyl)-L-leucinyl]amino]-3-piperidinol hydrochloride

To a solution of the compound of Example 21(e) (883 mg, 1.96 mmol) in dry EtOAc (10 mL) was bubbled HCl

gas for approximately 5 minutes. The reaction was stirred until TLC analysis indicated the complete consumption of the starting material. The reaction was then concentrated in vacuo to give 742 mg of the title compound: MS(ES+) 364.3 ($MH^+$).

g.) (3RS,4RS)-4-[[$N^{\alpha}$-(benzyloxycarbonyl)-L-leucinyl]amino]-1-[(2S)-4-methyl-2-[[(benzyloxycarbonyl)]amino] pentanoyl]-3-piperidinol

The compound of Example 21(f) (150 mg, 0.43 mmol) was coupled with CBZ-leucine (113.8 mg, 0.43 mmol), EDC (98.7 mg, 0.52 mmol), HOBT (57.9 mg, 0.43 mmol) and NMM (014 mL, 1.28 mmol). Workup and column chromatography (2:1 EtOAc:hexanes) gave 225 mg of the title compound: MS(ES+) 611.2 ($MH^+$), 633.2 (M+Na).

h.) 4-[[$N^{\alpha}$-(benzyloxycarbony)-L-leucinyl]amino]-1-[(2S)-4-methyl-2-[[(benzyloxycarbonyl)]amino]pentanoyl]-3-piperidinone

Following the procedure of Example 3(b) except substituting the compound of Example 21(g), the title compound was produced: MS(ES+) 609.3 ($MH^+$), 631.2 (M+Na).

Example 22

Preparation of 4-[[$N^{\alpha}$-(benzyloxycarbonyl)-L-leucinyl]amino]-1-[4-(biphenyl)ethanoyl]-3-piperidinone

a.) (3RS,4RS)-4-[[$N^{\alpha}$-(benzyloxycarbonyl)-L-leucinyl]amino]-1-[4-(biphenyl)ethanoyl]-3-piperidinol

Following the procedure of Example 21(g) except substituting 4-biphenylacetic acid for CBZ-leucine, the title compound was prepared: MS(ES+) 558.2 ($MH^+$), 580.1 (M+Na).

b. 4-[[$N^{\alpha}$-(benzyloxycarbonyl)-L-leucinyl]amino]-1-[4-(biphenyl)ethanoyl]-3-piperidinone

Following the procedure of Example 21(h) except substituting the compound of Example 22(a), the title compound was prepared: MS(ES+) 556.3 ($MH^+$), 578.2 (M+Na).

Example 23

Preparation of 4-[[$N^{\alpha}$-(benzyloxycarbonyl)-L-leucinyl]amino]-1-[(2S)-4-methyl-2-[[(benzyloxycarbonyl)]aminomethyl] pentanoyl]-3-piperidinone

a.) (3RS,4RS)-4-[[$N^{\alpha}$-(benzyloxycarbonyl)-L-leucinyl]amino]-1-[(2S)-4-methyl-2-[[(benzyloxycarbonyl)]aminomethyl] pentanoyl]-3-piperidinol

Following the procedure of Example 21(g) except substituting N-methyl-CBZ-leucine for CBZ-leucine, the title compound was prepared: MS(ES+) 625.4 ($MH^+$), 647.3 (M+Na).

b. 4-[[$N^{\alpha}$-(benzyloxycarbonyl)-L-leucinyl]amino]-1-[(2S)-4-methyl-2-[[(benzyloxycarbonyl)]aminomethyl]pentanoyl]-3-piperidinone

Following the procedure of Example 21 (h) except substituting the compound of Example 23(a), the title compound was prepared: MS(ES+) 623.3 ($MH^+$), 643.4 (M+Na).

Example 24

Preparation of 4-[[$N^{\alpha}$-(benzyloxycarbonyl)-L-leucinyl]amino]-1-*tert*-butoxycarbonyl-3-piperidinone

Following the procedure of Example 21(h) except substituting the compound of Example 21(e), the title compound was prepared: MS(ES+) 462.4 ($MH^+$), 484.4 (M+Na)

Example 25

Preparation of 4-[[Nα-(benzyloxycarbonyl)-L-leucinyl]amino]-1-[2-[[(benzyloxycarbonyl)]iso-butylamino]ethanoyl]-3-piperidinone

a.) (3RS,4RS)-4-[[Nα-(benzyloxycarbonyl)-L-leucinyl]amino]-1-[2-[[(benzyloxycarbonyl)]iso-butylamino]ethanoyl]-3-piperidinol

Following the procedure of Example 21(g) except substituting N-i-butyl-N-CBZ glycine for CBZ-leucine, the title compound was prepared: MS(ES+) 611.4 ($MH^+$), 633.5 (M+Na).

b. 4-[[Nα-(benzyloxycarbonyl)-L-leucinyl]amino]-1-[2-[[(benzyloxycarbonyl)]iso-butylamino]ethanoyl]-3-piperidinone

Following the procedure of Example 21(h) except substituting the compound of Example 25(a), the title compound was prepared: MS(ES+) 609.3 ($MH^+$), 631.4 (M+Na).

Example 26

Preparation of 4-[[Nα-(benzyloxycarbonyl)-L-leucinyl]amino]-1-[2-[(*tert* -butoxycarbonyl)amino]ethanoyl]-3-piperidinone

a.) (3RS,4RS)-4-[[Nα-(benzyloxycarbonyl)-L-leucinyl]amino]-1-[2-[(*tert*-butoxycarbonyl)amino]ethanoyl]-3-piperidinol

Following the procedure of Example 21(g) except substituting N-BOC-glycine for CBZ-leucine, the title compound was prepared: MS(ES+) 543.4 (M+Na).

b. 4-[[Nα-(benzyloxycarbonyl)-L-leucinyl]amino]-1-[2-[(*tert*-butoxycarbonyl)amino]ethanoyl]-3-piperidinone

Following the procedure of Example 21(h) except substituting the compound of Example 26(a), the title compound was prepared: MS(ES+) 519.5 ($MH^+$), 541.3 (M+Na).

Example 27

Preparation of 4-[[Nα-(benzyloxycarbonyl)-L-leucinyl]amino]-1-[2-(amino)ethanoyl]-3-piperidinone hydrochloride

Following the procedure of Example 21(f) except substituting the compound of Example 26(c), the title compound was prepared: MS(ES+) 419.4 (MH+)

Example 28

Preparation of 4-[[Nα-(benzyloxycarbonyl)-L-leucinyl]amino]-1-(4-methylpentanoyl)-3-piperidinone

a.) (3RS,4RS)-4-[[Nα-(benzyloxycarbonyl)-L-leucinyl]amino]-1-(4-methylpentanoyl)-3-piperidinol

To a solution of 4-methylvaleric acid (0.08 mL, 0.64 mL) in benzene (3 mL) was added oxalyl chloride (0.056 mL, 0.64 mmol) followed by the addition of 2 drops of DMF. The reaction was stirred for an additional 20 minutes whereupon it was concentrated in vacuo to give an oil. This oil was dissolved in $CH_2Cl_2$ (1.0 ml) and added to a 0°C solution of the compound from Example 21(f) (212 mg) in $CH_2Cl_2$ containing DIEA (0.27 mL). The reaction was warmed to room temperature and stirred for 90 minutes. The reaction was diluted with $CHCl_3$ and washed with 1N HCl, $H_2O$, brine and dried to give 142 mg of the title compound as an oil: MS (ES+) 462.5 ($MH^+$), 484.5 (M+Na).

b.) 4-[[Nα-(benzyloxycarbonyl)-L-leucinyl]amino]-1-(4-methylpentanoyl)-3-piperidinone

Following the procedure of Example 21(h) except substituting the compound of Example 28(a), the title compound was prepared: MS(ES+) 460.5 ($MH^+$), 482.5 (M+Na).

Example 29

Preparation of 4-[[N$^{\alpha}$-(benzyloxycarbonyl)-L-leucinyl]amino]-1-(benzoyl)-3-piperidinone

a.) (3RS,4RS)-4-[[N$^{\alpha}$-(benzyloxycarbonyl)-L-leucinyl]amino]-1-(benzoyl)-3-piperidinol

To a solution of the compound of Example 21(f) (161.1 mg, 0.40 mmol) in $CH_2Cl_2$ (5.0 mL) was added DIEA (0.21 mL) and benzoyl chloride (0.056 mL, 0.48 mmol). The reaction was stirred for 2.5 hours at room temperature, concentrated and the residue was chromatographed (5:95 $CH_3OH$:$CHCl_3$) to give 155 mg of the title compound: MS (ES+) 490.3 (M+Na).

b.) 4-[[N$^{\alpha}$-(benzyloxycarbonyl)-L-leucinyl]amino]-1-(benzoyl)-3-piperidinone

Following the procedure of Example 21(h) except substituting the compound of Example 29(a), the title compound was prepared: MS (ES) 466.4 ($MH^+$), 488.3 (M+Na).

Example 30

Preparation of 4-[[N$^{\alpha}$-(benzyloxycarbonyl)-L-leucinyl]amino]-1-(acetyl)-3-piperidinone

a.) (3RS,4RS)-4-[[N$^{\alpha}$-(benzyloxycarbonyl)-L-leucinyl]amino]-1-(acetyl)-3-piperidinol

Following the procedure of Example 29(a) except substituting acetyl chloride for benzoyl chloride, the title compound was prepared: MS(ES+) 428.5 (M+Na).

b.) 4-[[N$^{\alpha}$-(benzyloxycarbonyl)-L-leucinyl]amino]-1-(acetyl)-3-piperidinone

Following the procedure of Example 21(h) except substituting the compound of Example 30(a), the title compound was prepared: MS(ES+) 404.4 ($MH^+$).

Example 31

Preparation of 4-[[N$^{\alpha}$-(benzyloxycarbonyl)-L-leucinyl]amino]-1-(2-pyridoxyacetyl)-3-piperidinone

a.) (3RS,4RS)-4-[[N$^{\alpha}$-(benzyloxycarbonyl)-L-leucinyl]amino]-1-(2-pyridoxyacetyl)-3-piperidinol

Following the procedure of Example 21(g) except substituting 2-pyridoxyacetic acid for CBZ-leucine and DIEA for N-methylmorpholine, the title compound was prepared: MS(ES+) 499.1 ($MH^+$).

b.) 4-[[N$^{\alpha}$-(benzyloxycarbonyl)-L-leucinyl]amino]-1-(2-pyridoxyacetyl)-3-piperidinone

Following the procedure of Example 17(b) except substituting the compound of Example 31(a), the title compound was prepared: MS(ES+) 497.3 ($MH^+$).

Example 32

Preparation of 4-[[N$^{\alpha}$-(benzyloxycarbonyl)-L-leucinyl]amino]-1-[2-[(benzyloxycarbonyl)methylamino]ethanoyl]-3-piperidinone

a.) (3RS,4RS)-4-[[N$^{\alpha}$-(benzyloxycarbonyl)-L-leucinyl]amino]-1-[2-[(benzyloxycarbonyl)methylamino]ethanoyl]-3-piperidinol

Following the procedure of Example 21(g) except substituting CBZ-sarcosine for CBZ-leucine and DIEA for N-methylmorpholine, the title compound was prepared: MS(ES+) 591.3 ($MH^+$).

b.) 4-[[N$^{\alpha}$-(benzyloxycarbonyl)-L-leucinyl]amino]-1-[2-[( benzyloxycarbonyl)methylamino]ethanoyl]-3-piperidinone

Following the procedure of Example 17(b) except substituting the compound of Example 32(a), the title compound was prepared: MS(ES+) 567.6 ($MH^+$), 589.4 (M+Na).

Example 33

Preparation of 4-[[N$^{\alpha}$-(benzyloxycarbony)-L-leucinyl]amino]-1-[3-(2-pyridyl)phenylacetyl)]-3-piperidinone

a) 3-trifluoromethanesulphonyloxyphenylacetic acid methyl ester

To an oven-dried flask under Argon atmosphere containing sodium hydride (2.54 g, 60% dispersion in mineral oil, 63.5 mmol) was added anhydrous pentane (20 mL). The slurry was stirred for 5 min, allowed to settle, most of the pentane was removed, and anhydrous THF (40 mL) was added. To this suspension was added a solution of 3-hydroxyphenylacetic acid methyl ester (9.99 g, 60.1 mmol) in anhydrous THF (20 mL) and the reaction was stirred at room temperature for 20 min. To this mixture was then added a solution of N-phenyltrifluoromethanesulfonimide (22.53 g, 63.1 mmol) in anhydrous THF (40 mL) and the reaction was stirred at room temperature until TLC analysis indicated the complete consumption of starting material (1.5 h). The reaction was quenched by the addition of $H_2O$ (10 mL), concentrated to one half original volume, then diluted with $CHCl_3$ (200 mL) and washed with $H_2O$. The aqueous layer was washed with fresh $CHCl_3$ (50 mL), the combined organic layers were washed with 10% $Na_2CO_3$, $H_2O$, and brine, then dried ($MgSO_4$), filtered and concentrated. Column chromatography of the residue (silica gel, 5:95 EtOAc: hexanes, then 10:90 EtOAc: hexanes) gave 17.47 g of the title compound: $^1H$ NMR (400 MHz, $CDCl_3$) 7.42 (m, 1H), 7.31-7.19 (m, 3H), 3.72 (s, 3H), 3.68 (s, 2H)

b) 3-(2-pyridyl)phenyl acetic acid methyl ester

To a solution of the compound of Example 33(a) (6.86 g, 23.0 mmol) in anhydrous dioxane (100 mL) was added 2-pyridylstannane (8.89 g, 24.1 mmol), LiCl (2.94 g, 69.3 mmol), 2,6-di-tert-butyl-4-methylphenol (a few crystals), and $Pd(PPh_3)_4$ (632.1 mg, 0.55 mmol). The reaction was protected from light with foil and heated to reflux overnight. The reaction was allowed to cool to room temperature and concentrated. Column chromatography of the residue (silica gel, 1:3 EtOAc: hexanes, then 1:2 EtOAc: hexanes) gave 3.85 g of the title compound: MS(ES+) 228.1 (MH+).

c) 3-(2-pyridyl)phenyl acetic acid

To a solution of the compound of Example 33(b) (3.8 g, 16.7 mmol) in THF (50 mL) was added a solution of $LiOH{\bullet}H_2O$ (780.2 mg, 18.6 mmol) in $H_2O$ (10 mL). The reaction was stirred at room temperature until TLC analysis indicated the complete consumption of starting material (2 h). The reaction mixture was concentrated to remove the THF, then neutralized to pH=7 by the addition of 1N HCl, diluted with brine (50 mL), and washed with $CHCl_3$ (100 mL) The aqueous layer was readjusted back to pH 7 by the addition on 1N NaOH and washed with fresh $CHCl_3$ (100 mL). After repeating this procedure once more, the organic layers were combined, dried, filtered ($MgSO_4$) and concentrated to give 3.79 g of the title compound: MS (ES+) 214.3 (MH+).

d) (3RS,4RS)-4-[[N$^{\alpha}$-(benzyloxycarbony)-L-leucinyl]amino]-1-[3-(2-pyridyl)phenylacetyl)]-3-piperidinol

To a stirred suspension of the compound of Example 21(f) (1.21 g, 3.0 mmol) in DMF (10 mL) was added DIEA (523 uL, 3.0 mmol), HOBt (446.8 mg, 3.3 mmol), 3-(2-pyridyl)phenyl acetic acid (709.7 mg, 3.3 mmol), and EDC (634.9 mg, 3.3 mmol). The reaction mixture was stirred at room temperature overnight whereupon it was added to a rapidly-stirred mixture of EtOAc, 10% $Na_2CO_3$, and brine (100 mL) each) and allowed to stir for 1 h. The layers were separated, and the aqueous layer was washed with fresh EtOAc (100 mL), The combined organic layers were washed with 10% $Na_2CO_3$ and brine, dried, filtered ($MgSO_4$), and concentrated. Column chromatography (silica gel, EtOAc, then 5:95 MeOH: EtOAc) gave 1.12 g of the title compound: MS (ES+) 559.3 (MH+).

e) 4-[[N$^{\alpha}$-(benzyloxycarbony)-L-leucinyl]amino]-1-(3-(2-pyridyl)phenylacetyl)]-3-piperidinone

Following the procedure of Example 17(b), except substituting the compound of Example 33(d), the title compound was prepared: MS(ES+) 557.2 ($MH^+$), 589.3 (M+ Na).

Example 34

Preparation of 4-[[N$^{\alpha}$-(benzyloxycarbonyl)-L-leucinyl]amino]-1-[2-[(benzyloxycarbonyl)methylamino]ethanoyl]-3-pyrrolidinone

a.) (3RS,4RS)-4-[[N$^{\alpha}$-(benzyloxycarbonyl)-L-leucinyl]amino]-1-[2-[(benzyloxycarbonyl)methylamino]ethanoyl]-3-pyrrolidinol

Following the procedure of Example 1(g) except substituting CBZ-sarcosine for CBZ-leucine, the title compound was prepared: MS(ES+) 554.2 ($MH^+$), 577.2 (M+Na).

b.) 4-[[N$^{\alpha}$-(benzyloxycarbonyl)-L-leucinyl]amino]-1-[2-[( benzyloxycarbonyl)methylamino]ethanoyl]-3-pyrrolidinone

Following the procedure of Example 3(b) except substituting the compound of Example 34(a), the title compound was prepared: MS(ES+) 553.2 ($MH^+$), 575.2 (M+Na).

Example 35

Preparation of 4-[[N$^{\alpha}$-(benzyloxycarbonyl)-L-leucinyl]amino]-1-[2-(phenoxy)ethanoyl]-3-pyrrolidinone

a.) (3RS,4RS)-4-[[N$^{\alpha}$-(benzyloxycarbonyl)-L-leucinyl]amino]-1-[2-(phenoxy)ethanoyl]-3-pyrrolidinol

Following the procedure of Example 1(g) except substituting phenoxyacetic acid for CBZ-leucine, the title compound was prepared: MS(ES+) 484.3 ($MH^+$), 506.2 (M+Na).

b.) 4-[[N$^{\alpha}$-(benzyloxycarbonyl)-L-leucinyl]amino]-1-(2-(phenoxy)ethanoyl]-3-pyrrolidinone

Following the procedure of Example 3(b) except substituting the compound of Example 35(a), the title compound was prepared: MS(ES+) 482.3 ($MH^+$), 504.3 ($MH^+$+Na).

Example 36

Preparation of 4-[[N$^{\alpha}$-(4-pyridinylmethoxycarbonyl)-L-leucinyl]amino]-1-[(2-phenyl)ethanoyl]-3-pyrrolidinone

a.) (3RS,4RS)-4-[[N$^{\alpha}$-(4-pyridinylmethoxycarbonyl)-L-leucinyl]amino]-1-[(2-phenyl)ethanoyl]-3-pyrrolidinol

Following the procedure of Example 1(e)-1(g) except substituting N-(4-pyridylmethoxycarbonyl)-L-leucine for CBZ-leucine in step 1(e) and phenylacetic acid for CBZ-leucine in step 1(g), the title compound was prepared: MS (ES+) 469 ($MH^+$).

b.) 4-[[N$^{\alpha}$-(4-pyridinylmethoxycarbonyl)-L-leucinyl]amino]-1-[(2-phenyl)ethanoyl]-3-pyrrolidinone

Following the procedure of Example 17(b) except substituting the compound of Example 36(a), the title compound was prepared: MS(ES+) 467 ($MH^+$).

Example 37

Preparation of 4-[[N$^{\alpha}$-(4-pyridinylmethoxycarbonyl)-L-leucinyl]amino]-1-ethanoyl-3-pyrrolidinone

a.) (3RS,4RS)-4-[[N$^{\alpha}$-(4-pyridinylmethoxycarbonyl)-L-leucinyl]amino]-1-ethanoyl-3-pyrrolidinol

Following the procedure of Example 1(e)-1(g) except substituting N-(4-pyridylmethoxycarbonyl)-L-leucine for CBZ-leucine in step 1(e) and acetic acid for CBZ-leucine in step 1(g), the title compound was prepared: MS(ES+) 415 (M+Na).

b.) 4-[[N$^{\alpha}$-(4-pyridinylmethoxycarbonyl)-L-leucinyl]amino]-1-ethanoyl-3-pyrrolidinone

Following the procedure of Example 17(b) except substituting the compound of Example 37(a), the title compound was prepared: MS(ES+) 391 ($MH^+$).

Example 38

Preparation of 4-[[$N^\alpha$-(4-pyridinylmethoxycarbonyl)-L-leucinyl]amino]-1-(4-cyanobenzoyl)-3-pyrrolidinone

a.) (3RS,4RS)-4-[[$N^\alpha$-(4-pyridinylmethoxycarbonyl)-L-leucinyl]amino]-1-(4-cyanobenzoyl)-3-pyrrolidinol

Following the procedure of Example 1(e)-1(g) except substituting N-(4-pyridylmethoxycarbonyl)-L-leucine for CBZ-leucine in step 1(e) and 4-cyanobenzoic acid for CBZ-leucine in step 1(g), the title compound was prepared: MS (ES+) 480 ($MH^+$), 502 (M+Na).

b.) 4-[[$N^\alpha$-(4-pyridinylmethoxycarbonyl)-L-leucinyl]amino]-1-(4-cyanobenzoyl)-3-pyrrolidinone

Following the procedure of Example 17(b) except substituting the compound of Example 38(a), the title compound was prepared: MS(ES+) 478 ($MH^+$).

Example 39

Preparation of 4-[[$N^\alpha$-(4-pyridinylmethoxycarbonyl)-L-leucinyl]aminol-1-*tert*-butoxycarbonyl-3-pyrrolidinone

a.) (3RS,4RS)-4-[[$N^\alpha$-(4-pyridinylmethoxycarbonyl)-L-leucinyl]amino]-1-*tert*-butoxycarbonyl-3-pyrrolidinol

Following the procedure of Example 1(e) except substituting N-(4-pyridylmethoxycarbonyl)-L-leucine for CBZ-leucine, the title compound was prepared: MS(ES+) 451 ($MH^+$).

b.) 4-[[$N^\alpha$-(4-pyridinylmethoxycarbonyl)-L-leucinyl]amino]-1-*tert*-butoxycarbonyl-3-pyrrolidinone

Following the procedure of Example 17(b) except substituting the compound of Example 39(a), the title compound was prepared: MS(ES+) 449 ($MH^+$).

Example 40

Preparation of 4-[[$N^\alpha$-(3-pyridinylmethoxycarbonyl)-L-leucinyl]amino]-1-*tert*-butoxycarbonyl-3-pyrrolidinone

a.) (3RS,4RS)-4-[[$N^\alpha$-3-pyridinylmethoxycarbonyl)-L-leucinyl]amino]-1-*tert*-butoxycarbonyl-3-pyrrolidinol

Following the procedure of Example 1(e) except substituting N-(3-pyridylmethoxycarbonyl)-L-leucine for CBZ-leucine, the title compound was prepared: MS(ES+) 451 ($MH^+$).

b.) 4-[[$N^\alpha$-(3-pyridinylmethoxycarbonyl)-L-leucinyl]amino]-1-*tert*-butoxycarbonyl-3-pyrrolidinone

Following the procedure of Example 17(b) except substituting the compound of Example 40(a), the title compound was prepared: MS(ES+) 449 ($MH^+$).

Example 41

Preparation of 4-[[$N^\alpha$-(3-pyridinylmethoxycarbonyl)-L-leucinyl]amino]-3-pyrrolidinone bis hydrochloride

To a solution of the compound of Example 40(b) in ethyl acetate was added 4M HCl/dioxane (20 drops). The reaction was stirred at room temperature overnight whereupon it was concentrated to give the title compound: MS (ES+) 349 ($MH^+$)

Example 42

Preparation of 4-[[$N^\alpha$-(4-pyridinylmethoxycarbonyl)-L-leucinyl]amino]-3-pyrrolidinone bis hydrochloride

Following the procedure of Example 41 except substituting the compound of Example 39(b), the title compound was prepared: MS(ES+) 349 ($MH^+$)

Example 43

Preparation of 4-[[$N^{\alpha}$-(2-pyridinylmethoxycarbonyl)-L-leucinyl]amino]-1-[(2S)-4-methyl-2-[[(benzyloxycarbonyl)]aminomethyl]pentanoyl]-3-pyrrolidinone

a.) (3RS,4RS)-4-[[$N^{a}$-(2-pyridinylmethoxycarbonyl)-L-leucinyl]amino]-1-[(2S)-4-methyl-2-[[(benzyloxycarbonyl)]aminomethyl]pentanoyl]-3-pyrrolidinone

Following the procedure of Example 1(e)-1(g) except substituting N-(2-pyridylmethoxycarbonyl)-L-leucine for CBZ-leucine in step 1(e) and N-methyl-CBZ leucine for CBZ-leucine in step 1(g), the title compound was prepared: MS(ES) 612 ($MH^+$).

b.) 4-[[$N^{\alpha}$-(2-pyridiny]methoxycarbonyl)-L-leucinyl]amino]-1-[(2S)-4-methyl-2-[[(benzyloxycarbonyl)]aminomethyl]pentanoyl]-3-pyrrolidinone

Following the procedure of Example 17(b) except substituting the compound of Example 43(a), the title compound was prepared: MS(ES+) 610 ($MH^+$).

Example 44

Preparation of 4-[[$N^{\alpha}$-(4-pyridinylmethoxycarbonyl)-L-leucinyl]amino]-1-[(2S)-4-methyl-2-[[(4-pyridinylmethoxycarbonyl)]amino]pentanoyl]-3-pyrrolidinone

a.) (3RS,4RS)-4-[[$N^{\alpha}$-(4-pyridinylmethoxycarbonyl)-L-leucinyl]amino]-1-((2S)-4-methyl-2-[[(4-pyridinylmethoxycarbonyl)]amino]pentanoyl]-3-pyrrolidinol

Following the procedure of Example 1(e)-1(g) except substituting N-(4-pyridylmethoxycarbonyl)-L-leucine for CBZ-leucine in steps 1(e) and 1(g), the title compound was prepared: MS(ES+) 599 ($MH^+$),

b.) 4-[[$N^{\alpha}$-(4-pyridinylmethoxycarbonyl)-L-leucinyl]amino]-1-[(2S)-4-methyl-2-[[(4-pyridinylmethoxycarbonyl)]amino]pentanoyl]-3-pyrrolidinone

Following the procedure of Example 17(b) except substituting the compound of Example 44(a), the title compound was prepared: MS(ES+) 597 ($MH^+$).

Example 45

Preparation of 4-[[$N^{\alpha}$-(4-pyridinylmethoxycarbonyl)-L-leucinyl]amino]-1-[(2S)-4-methyl-2-[[(benzyloxycarbonyl)]aminomethyl]pentanoyl]-3-pyrrolidinone

a.) (3RS,4RS)-4-[[$N^{\alpha}$-(4-pyridinylmethoxycarbonyl)-L-leucinyl]amino]-1-[(2S)-4-methyl-2-[[(benzyloxycarbonyl)]aminomethyl]pentanoyl]-3-pyrrolidinol

Following the procedure of Example 1(e)-1(g) except substituting N-(4-pyridinylmethoxycarbonyl)-L-leucine for CBZ-leucine in step 1(e) and N-methyl-CBZ-leucine for CBZ-leucine in step 1(g), the title compound was prepared: MS(ES+) 612 ($MH^+$).

b.) 4-[[$N^{\alpha}$-(4-pyridinylmethoxycarbonyl)-L-leucinyl]amino]-1-[(2S)-4-methyl-2-[[(benzyloxycarbonyl)]aminomethyl]pentanoyl]-3-pyrrolidinone

Following the procedure of Example 17(b) except substituting the compound of Example 45(a), the title compound was prepared: MS (ES+) 610 ($MH^+$).

Example 46

Preparation of 4-[[N$^{\alpha}$-(3-isoquinolinecarbonyl)-L-leucinyl]amino]-1-[(2S)-4-methyl-2-[[(benzyloxycarbonyl)]amino] pentyl]-3-pyrrolidinone

a.) 1-benzyloxycarbonyl-3-pyrrolidine

To a solution of 3-pyrroline (25 g, 361.8 mmol) in $CH_2Cl_2$ (300 mL) at 0°C was added pyridine (33 mL 416 mmol) followed by benzyl chloroformate (57 mL, 380 mmol) in $CH_2Cl_2$ (100 mL). The reaction was stirred at 0°C for 1h and at room temperature for 1h. The reaction was diluted $CH_2Cl_2$, washed with 1N HCl, water, brine, dried ($MgSO_4$) and concentrated. The residue was chromatographed (50:50 $CH_2Cl_2$:hexane) to give 70 g of the title compound: MS (ES+) 226 (M+Na).

b.) 1-benzyloxycarbonyl-3,4-epoxy-pyrrolidine

To a solution of the compound of Example 46(a) (60 g, 295 mmol) in $CH_2Cl_2$ (1000 mL) was added m-CPBA (153 g, 886 mmol). The reaction was stirred at room temperature overnight whereupon it was concentrated, filtered with petroleum ether and orangic layer was washed with saturated $K_2CO_3$ (3 times), water, brine, dried ($MgSO_4$) and concentrated to give a clear cololess oil which was used directly in the next step: MS(ES+) 242(M+Na).

c.) 1-benzyloxycarbonyl-*trans*-3-azido-4-hydroxypyrrolidine

To a solution of the compound of Example 46(b) (60 g, 273 mmol) in methanol:water (800 mL of an 8:1 solution) was added ammonium chloride (29 g, 547 mmol) and sodium azide (35.6 g, 547 mmol). The reaction was heated at 50°C for 3h whereupon it was concentrated, diluted with ethyl acetate and washed sequentially with pH 4 buffer, saturated $NaHCO_3$, water and brine. The organic layer was dried ($MgSO_4$), filtered and concentrated to give the title compound: $^1H$ NMR (400MHz, $CDCl_3$) 7.35(m, 5H), 5.1(s, 2H), 4.2(m, 1H), 3.9(m, 1H), 3.3-3.7(m, 5H).

d.) 1-Benzyloxycarbonyl-*trans*-3-amino-4-hydroxypyrrolidine

To a solution of the compound of Example 46(c) (53 g, 201 mmol) in $CH_3OH$ (1200 mL) was added triethylamine (56 mL. 402 mmol) followed by 1,3-propanethiol (40.3 mL, 402 mmol). The reaction was stirred at room temperature overnight whereupon it was concentrated and purified by column chromatography (20:80 methanol: ethyl acetate) to give 38 g of the title compound: MS(ES) 237($MH^+$).

e.) (3RS,4RS)-4-[[N$^{\alpha}$-(*tert*-butoxycarbonyl)-L-leucinyl]amino]-1-benzyloxycarbonyl-3-pyrrolidinol

To a solution of the compound of Example 46(d) (20 g, 84.6 mmol) in $CH_2Cl_2$ (500 mL) was added Boc-L-leucine (22 g, 88.8 mmol), HOBT (12 g, 88.8 mmol) and EDC (20.28 g, 105.8 mmol). The reaction was allowed to stir at room temperature overnight whereupon it was diluted with $CH_2Cl_2$ and washed 0.5N HCl, sat'd $NaHCO_3$, water and brine. The organic was dried ($MgSO_4$), filtered and concentrated. Column chromatography of the residue (5:95 MeOH: $CH_2Cl_2$) gave 34 g of the title compound: MS(ES+) 450 ($MH^+$).

f.) (3RS,4RS)-4-[[N$^{\alpha}$-(*tert*-butoxycarbonyl)-L-leucinyl]amino]-3-pyrrolidinol

To a solution of the compound of Example 46(e) (24 g, 53.4 mmol) in methanol:ethyl acetate (300 mL of a 1: 2 solution) was added 10% Pd on carbon. The mixture shaken on a Parr hydrogenator for 2h whereupon it was filtered through a pad of celite with $CH_2Cl_2$ and concentrated to give 18 g of the title compound: MS(ES+) 316 ($MH^+$).

g.) CBZ-leucinal

To a solution of CBZ-Leu-OH (2 g, 7.54 mmol) in $CH_2Cl_2$ (100 mL) was added EDC (1.73 g, 9.05 mmol), HOBT (1.22 g, 9.05 mmol) and N,O-dimethylhydroxylamine (0.93 g, 15.08 mmol). The reaction was allowed to stir at room temperature overnight whereupon it was diluted with $CH_2Cl_2$ and washed with 1N HCl, sat'd $NaHCO_3$, water and brine. The organic layer was dried ($MgSO_4$) filtered and concentrated. Column chromatography of the residue (40:60 ethyl acetate: hexane ) gave 2.3 g of the CBZ-leucine Weinreb amide: MS(ES+) 309 ($MH^+$), 331($MH^+$+Na).
To a solution of the CBZ-leucine-N,O-dimethyl amide (1.2 g, 4 mmol) in THF (10 mL) at 0°C was added lithium aluminum hydride (10 mL of a 1.0 M solution in THF, 10 mmol) dropwise. The reaction was allowed to stir at 0°C for

1h whereupon it was quenched with potassium hydrogensulfate (953 mg, 7 mmol). The mixture was diluted with ethyl acetate, washed with 1N HCl, sat'd $NaHCO_3$, water and brine. The organic was dried ($MgSO_4$), filtered and concentrated to give 1.01 g of the title compound: $^1H$ NMR (400 MHz, $CDCl_3$) 9.5 (s, 1H) 7.35 (m, 5H), 5.1 (s, 2H), 4.3 (m, 1H), 1.6-1.8 (m, 2H), 1.5 (m, 1H), 1.0(m, 6H).

h.) (3RS,4RS)-4-[[N$^{\alpha}$-*tert*-butoxycarbonyl)-L-leucinyl]amino]-1-[(2S)-4-methyl-2-[[(benzyloxycarbonyl)]amino]pentyl]-3-pyrrolidinol

To a solution of compound from Example 46(f ) (950 mg, 3.01 mmol) in $CH_2Cl_2$ (10 mL) was added CBZ-leucinal (900 mg, 3.6 mmol). The reaction was allowed to stir at room temperature for 0.5h whereupon sodium triacetoxyborohydride (1.27 g, 6 mmol) was added. The reaction was stirred at room temperature for 2h whereupon it was diluted with ethyl acetate and washed with sat'd $NaHCO_3$, brine, dried ($Na_2SO_4$), filtered and concentrated. Column chromatography of the the residue (5:95 methanol: $CH_2Cl_2$) gave 1.3g of the title compound: MS(ES+) 549 ($MH^+$),

i.) (3RS,4RS)-4-[(L-leucinyl)amino]-1-[(2S)-4-methyl-2-[(benzyloxycarbonyl)amino]pentyl]-3-pyrrolidinol hydrochloride

To a solution of the compound of Example 46(h) (1.1 g, 2 mmol) in methanol (10 mL) was added 4M HCl in dioxane (10 mL). The reaction was stirred at room overnight whereupon it was concentrated to give the title compound: MS(ES) 449 (MH+).

j.) (3RS,4RS)-4-[[N$^{\alpha}$-(3-isoquinolinecarbonyl)-L-leucinyl]amino]-1-[(2S)-4-methyl-2-[[(benzyloxycarbonyl)]amino] pentyl]-3-pyrrolidinol

To a solution of above compound of Example 46(i) (250 mg, 0.48 mmol) in $CH_2Cl_2$ (10 mL) was added TEA (0.17 mL, 1.2 mmol) followed by 3-isoquinolinecarboxylic acid (96 mg, 0.5 mmol), EDC (115 mg, 0.6 mmol) and HOBT (68 mg, 0.5 mmol). The reaction was stirred until complete by TLC analysis. Workup and column chromatography (5% $CH_3OH/CH_2Cl_2$) gave 180 mg of the title compound: MS(ES+) 604 ($MH^+$).

k.) 4-[[N$^{\alpha}$-(3-isoquinolinecarbonyl)-L-leucinyl]amino]-1-[(2S)-4-methyl-2-[(benzyloxycarbonyl)amino]pentyl]-3-pyrrolidinone

To a solution of the compound of Example 46(j) (180 mg, 0.3 mmol) in DMSO (2.5 mL) was added TEA (0.25 mL, 1.8 mmol) and pyridine sulphur trioxide complex (143 mg, 0.9 mmol). The reaction was stirred at room temperature for 1h whereupon it was partitioned between ethyl acetate and sat'd $NaHCO_3$. The organic layer was washed with brine, dried ($Na_2SO_4$), filtered, concentrated and the residue chromatographed (5% $CH_3OH/CH_2Cl_2$) to give 110 mg of the title compound: MS(ES) 602 ($MH^+$).

Example 47

Preparation of 4-[[N$^{\alpha}$-(4-pyridinylmethoxycarbonyl)-L-leucinyl]amino]-1-[1-(adamantyl)carbonyl]-3-pyrrolidinone

a.) (3RS,4RS)-4-[[N$^{a}$-(4-pyridinylmethoxycarbonyl)-L-leucinyl]amino]-1-*tert*-butoxycarbonyl-3-pyrrolidinol

To a solution of the compound of Example 1(d) (1.0 g, 5.25 mmol) in $CH_2Cl_2$ was added EDC (1.0 g, 5.25 mmol), HOBT (0.71 g, 5.25 mmol) and N-(4-pyridylmethoxycarbonyl)-L-leucine (1.4 g, 5.25 mmol). The reaction was stirred at room temperature overnight. The following morning the rection was diluted with ethyl acetate and washed with water, brine, dried ($Na_2SO_4$), filtered and concentrated. Column chromatography of the residue (5% $CH_3OH$: $CH_2Cl_2$) gave the title compound: MS(ES+) 451 ($MH^+$).

b.) (3RS,4RS)-4-[[N$^{\alpha}$-(4-pyridinylmethoxycarbonyl)-L-leucinyl]amino]-3-pyrrolidinol bis-hydrochloride

Following the procedure of Example 1(f) except substituting the compound of Example 47(a), the title compound was prepared: MS(ES+) 351 ($MH^+$).

c.) (3RS,4RS)-4-[[N$^{\alpha}$-(4-pyridinylmethoxycarbonyl)-L-leucinyl]amino]-1-[1-(adamantyl)carbonyl]-3-pyrrolidinol

To a solution of the compound of Example 47(b) (300 mg, 0.71 mmol) in $CH_2Cl_2$ was added TEA (0.34 mL),

2.48 mmol) followed by 1-adamantanecarbonyl chloride (149 mg, 0.75 mmol). The reaction was stirred until complete as indicated by TLC analysis. Workup followed by column chromatography (5% $CH_3OH$:$CH_2Cl_2$) gave the title compound: MS(ES+) 513 ($MH^+$).

d.) 4-[[N$^{\alpha}$-(4-pyridinylmethoxycarbonyl)-L-leucinyl]amino]-1-[1-(adamantyl)carbonyl]-3-pyrrolidinone

Following the procedure of Example 17(b) except substituting the compound of Example 47(c), the title compound was prepared: MS(ES+) 511 ($MH^+$).

Example 48

Preparation of 4-[[N$^{\alpha}$-(benzyloxycarbonyl)-L-leucinyl]amino]-1-(4-methylpentanoyl)-3-pyrrolidinone

a.) (3RS,4RS)-4-[[N$^{\alpha}$-(benzyloxycarbonyl)-L-leucinyl]amino]-1-(4-methylpentanoyl)-3-pyrrolidinol

Following the procedure of Example 1(g) except substituting 4-methylvaleric acid for CBZ-leucine, the title compound was prepared: MS(ES+) 448.6 ($MH^+$), 470.4 (M+Na).

b.) 4-[[N$^{\alpha}$-(benzyloxycarbonyl)-L-leucinyl]amino]-1-(4-methylpentanoyl)-3-pyrrolidinone

Following the procedure of Example 3(b) except substituting the compound of Example 48(a), the title compound was prepared: MS(ES+) 446.3 ($MH^+$), 468.4 (M+Na).

Example 49

Preparation of 4-[[N$^{\alpha}$-(benzyloxycarbonyl)-D-leucinyl]amino]-1-[(2S)-4-methyl-2-[[(benzyloxycarbonyl)]amino]pentanoyl]-3-piperidinone

a.) (3RS,4RS)-4-[[N$^{\alpha}$-(benzyloxycarbonyl)-D-leucinyl]amino]-1-[(2S)-4-methyl-2-[[(benzyloxycarbonyl)]amino]pentanoyl]-3-piperidinol

To a solution of CBZ-leucine (7.97 g, 30 mmol) in $CH_2Cl_2$ (100 mL) was added 1,2,3,6-tetrahydropyridine (2.5 g, 30 mmol), EDC (6.9 g) and HOBT (4.06 g). The reaction was stirred until complete as indicated by TLC analysis. The reaction was diluted with ethyl acetate, washed with 2N HCl, sat'd $K_2CO_3$, water, brine, dried ($MgSO_4$), filtered and concentrated to give 9.39 g of the amide.

To a solution of the amide (9.39 g) in $CH_2Cl_2$ (250 mL) was added m-CPBA (19.61 g). The reaction was allowed to stir overnight whereupon it was concentrated, diluted with ether and washed sequentaillly with sat'd $K_2CO_3$ (5x's), water, brine, dried ($MgSO_4$) and concentrated to give 8.49 g of the epoxide as a clear oil.

To a solution of the epoxide (8.49 g) in $CH_3OH$:$H_2O$ (180 mL of an 8:1 solution) was added ammonium chloride (2.75 g) followed by sodium azide (7.96 g). This mixture was heated to 60°C for approximately 6 h. Workup as in Example 1(c) and column chromatography (2:1 hexane:ethyl acetate) of the residue gave 5.2 g of the azide.

To a solution of $SnCl_2$ dihydrate (432 mg) in methanol (10 mL) was added the azido alcohol (500 mg). The reaction was stirred overnight at room temperature whereupon it was concentrated, diluted with ethyl acetate and washed with 4N NaOH. The aqueous layer was washed with ethyl acetate. The combined organic layers were washed with water, brine, dried ($MgSO_4$), filtered and concentrated to give 235 mg of the amino alcohol.

To a solution of the above amino alcohol (150 mg, 0.41 mmol) was added CBZ-D-leucine (109 mg), EDC (95 mg) and HOBT (56 mg). The reaction was stirred until complete by TLC analysis. Workup gave 239.5 mg of the title compound: MS(ES+) 633.5 (M+Na).

b.) 4-[[N$^{\alpha}$-(benzyloxycarbonyl)-D-leucinyl]amino]-1-[(2S)-4-methyl-2-[[(benzyloxycarbonyl)]amino]pentanoyl]-3-piperidinone

Following the procedure of Example 3(b) except substituting the compound of Example 49(a), the title compound was prepared: MS(ES+) 609.1 ($MH^+$), 631.1 (M+Na).

Example 50

Preparation of 4-[[N$^{\alpha}$-(*tert*-butoxycarbonyl)-L-leucinyl]amino]-1-[(2S)-4-methyl-2-[[(benzyloxycarbonyl)]amino] pentanoyl]-3-piperidinone

a.) (3RS,4RS)-4-[[N$^{\alpha}$-(*tert*-butoxycarbonyl)-L-leucinyl]amino]-1-[(2S)-4-methyl-2-[[(benzyloxycarbonyl)]amino] pentanoyl]-3-piperidinol

Following the procedure of Example 49(a) except substituting BOC-leucine for CBZ-D-leucine, the title compound was prepared. This material was used directly in the following step.

b.) 4-[[N$^{\alpha}$-(*tert*-butoxycarbonyl)-L-leucinyl]amino]-1-[(2S)-4-methyl-2-[[(benzyloxycarbonyl)]amino]pentanoyl]-3-piperidinone

Following the procedure of Example 3(b) except substituting the compound of Example 50(a), the title compound was prepared: MS(ES+) 475.5 ($MH^+$-$CO_2$-t-Bu), 575.4 ($MH^+$), 597.5 (M+Na).

Example 51

Preparation of 4-[[N$^{\alpha}$-(benzyloxycarbonyl)-N$^{\varepsilon}$-(*tert*-butoxycarbonyl)-L-lysine]amino]-1-[(2S)-4-methyl-2-[(benzyloxycarbonyl)amino]pentanoyl]-3-piperidinone

a.) (3RS,4RS)-4-[[N$^{\alpha}$-(benzyloxycarbonyl)-N$^{\varepsilon}$-(*tert*-butoxycarbonyl)-L-lysine]amino]-1-[(2S)-4-methyl-2-[[(benzyloxycarbonyl)]amino]pentanoyl]-3-piperidinol

Following the procedure of Example 49(a) except substituting CBZ-Lys(Boc)-OH for CBZ-D-leucine, the title compound was prepared: MS(ES+) 748.5 (M+Na).

b.) 4-[[N$^{\alpha}$-(benzyloxycarbonyl)-N$^{\varepsilon}$-(*tert*-butoxycarbonyl)-L-lysine]amino]-1-[(2S)-4-methyl-2-[[(benzyloxycarbonyl)] amino]pentanoyl]-3-piperidinone

Following the procedure of Example 3(b) except substituting the compound of Example 51(a), the title compound was prepared: MS(ES) 724.7 ($MH^+$), 746.5 (M+Na).

Example 52

Preparation of 4-[[N$^{\alpha}$-(4-pyridinylmethoxycarbonyl)-L-leucinyl]amino]-1-*tert*-butoxycarbonyl-3-piperidinone

a.) (3RS,4RS)-4-[[N$^{\alpha}$-(4-pyridinylmethoxycarbonyl)-L-leucinyl]amino]-1-*tert*-butoxycarbonyl-3-piperidinol

To a solution the compound of Example 21 (d) (1.77g, 8.18 mmol) in DMF (50 mL) was added DIEA (2.9 mL, 16.6 mmol), HOBT (1.35 g, 9.99 mmol), N-(4-pyridylmethoxycarbonyl)-L-leucine (2.62 g, 9.84 mmol) and EDC (1.89 g, 9.87 mmol). The reaction was stirred for 16 hours whereupon it was concentrated and added to a rapidly stirred mixture of ethyl acetate (100 mL), 10% $Na_2CO_3$ (100 mL) and brine (100 mL). This mixture was stirred for 1 hour and the organic layer was separated and washed with 50% brine, brine dried ($Na_2SO_4$), filtered and concentrared. Column chromatography of the residue (5:95 $CH_3OH$:$CHCl_3$) gave 2.43 g of the title compound: MS(ES+) 465.5 ($MH^+$), 365.4 ($MH^+$-Boc).

b.) 4-[[N$^{\alpha}$-(4-pyridinylmethoxycarbonyl)-L-leucinyl]amino]-1-*tert*-butoxycarbonyl-3-piperidinone

Following the procedure of Example 3(b) except substituting the compound of Example 52(a), the title compound was prepared: MS(ES+) 463.5 ($MH^+$).

Example 53

Preparation of 4-[[N$^{\alpha}$-(4-pyridinylmethoxycarbonyl)-L-leucinyl]amino]-1-(4-methylpentanoyl)-3-piperidinone

a.) (3RS,4RS)-4-[[N$^{\alpha}$-(4-pyridinylmethoxycarbonyl)-L-leucinyl]amino]-3-piperidinol bis-hydrochloride

The compound from Example 52(a) (2.16 g, 4.5 mmol) was dissolved in 4N HCl/dioxane and stirred at room temperature for 1 hour. The mixture was concentrated and azeotroped with toluene to give the title compound: MS ($ES^+$) 365.4 (MH+).

b.) (3RS,4RS)-4-[[N$^{\alpha}$-(4-pyridinylmethoxycarbonyl)-L-leucinyl]amino]-1-(4-methylpentanoyl)-3-piperidinol

To a solution of the compound from Example 53(a) (220 mg, 0.50 mmol) in DMF (2.0 mL) was added DIEA (0.35 mL, 2.0 mmol), HOBT (82.9 mg, 0.61 mmol), 4-methylvaleric acid (0.08 mL, 0.60 mmol) and EDC (116.6 mg, 0.61 mmol). The reaction was stirred for 18 hours whereupon it was added to a rapidly stirred mixture of ethyl acetate (50 mL), 5% $Na_2CO_3$ (50 mL) and brine (50 mL). This mixture was stirred for 1 hour and the aqueous layer was washed with ethyl acetate. The combined organic layers were washed with 10% $Na_2CO_3$, brine, dried ($Na_2SO_4$), filtered and concentrated. Column chromatography of the residue (5:95 $CH_3OH$:$CHCl_3$) gave 138 mg of the title compound: MS (ES+) 463.5 ($MH^+$).

c.) 4-[[N$^{\alpha}$-(4-pyridinylmethoxycarbonyl)-L-leucinyl]amino]-1-(4-methylpentanoyl)-3-piperidinone

Following the procedure of Example 17(b) except substituting the compound of Example 53(a), the title compound was prepared: MS (ES) 461.4 (MH+), 493.5 (M+Na).

A second fraction of material with identical molecular weight was isolated from this reaction: MS(ES+) 461.4 (MH+).

Example 54

Preparation of 4-[[N$^{\alpha}$-(4-pyridinylmethoxycarbonyl)-L-leucinyl]amino]-1-[2-(benzyloxycarbonyl)]*iso*-butylamino] ethanoyl]-3-piperidinone

a.) (3RS,4RS)-4-[[N$^{\alpha}$-(4-pyridinylmethoxycarbonyl)-L-leucinyl]amino]-1-[2-(benzyloxycarbonyl)]*iso*-butylamino] ethanoyl]-3-piperidinol

Following the procedure of Example 53(b) except substituting N-iso-butyl-CBZ-glycine for 4-methylvaleric acid, the title compound was prepared: MS(ES+) 612.4 ($MH^+$).

b.) 4-[[N$^{\alpha}$-(4-pyridinylmethoxycarbonyl)-L-leucinyl]amino]-1-[2-(benzyloxycarbonyl)]*iso*-butylamino]ethanoyl]-3-piperidinone

Following the procedure of Example 17(b) except substituting the compound of Example 54(a), the title compound was prepared: MS(ES+) 610.5 ($MH^+$).

Example 55

Preparation of 4-[[N-2-(benzyloxycarbonyl)]*iso*-butylamino]ethanoyl]-1-[(2S)-4-methyl-2-[[(benzyloxycarbonyl)]amino] pentanoyl]-3-piperidinone

a.) (3RS,4RS)-4-[[N-2-(benzyloxycarbonyl)]*iso*-butylamino]ethanoyl]-1-[(2S)-4-methyl-2-[[(benzyloxycarbonyl)]amino] pentanoyl]-3-piperidinol

Following the procedure of Example 49(a) except substituting N-*iso*-butyl-N-CBZ-glycine for CBZ-D-leucine, the title compound was prepared: MS(ES+) 611.5 ($MH^+$), 633.5 (M+Na).

b.) 4-[[N-2-(benzyloxycarbonyl)]*iso*-butylamino]ethanoyl]-1-[(2S)-4-methyl-2-[[(benzyloxycarbonyl)]amino]pentanoyl]-3-piperidinone

Following the procedure of Example 17(b) except substituting the compound of Example 55(a), the title compound was prepared: MS (ES+) 609.5 ($MH^+$), 631.3 (M+Na).

Example 56

Preparation of 4-[[$N^\alpha$-(benzyloxycarbonyl)-L-leucinyl]amino]-1-(methanesulphonyl)-3-piperidinone

a.) (3RS,4RS)-4-[[$N^\alpha$-(benzyloxycarbonyl)-L-leucinyl]amino]-1-(methanesulphonyl)-3-piperidinol

The compound from Example 21(e) (368 mg, 0.79 mmol) was dissolved in 4N HCl/dioxane (10 mL). The rection was stirred at room temperature for ca. 30 minutes whereupon it was concentrated and azeotropically dried with toluene (2 x's) and left under high vacuum for 1 hour. The white solid was dissolved in $CH_2Cl_2$ (5.0 mL) and DIEA (0.41 mL, 2.4 mmol) was added. The reaction was cooled to 0°C. Methanesulphonyl chloride (0.073 mL, 0.94 mmol) was then added and the reaction was stirred at 0°C for 30 minutes and warmed to room temperature for 1.5 hours. The reaction was concentarted then dissolved in $CHCl_3$ (50 mL), washed with 1N HCl (2x25 mL), water, brine, dried ($MgSO_4$), filtered and concentrated to give 335 mg of a white solid: MS(ES+) 464.3 (M+Na).

b.) 4-[[$N^\alpha$-(benzyloxycarbonyl)-L-leucinyl]amino]-1-(methanesulphonyl)-3-piperidinone

Following the procedure of Example 3(b) except substituting the compound of Example 56(a), the title compound was prepared: MS(ES+) 440.3 ($MH^+$), 462.4 (M+Na).

Example 57

Preparation of 4-[[$N^\alpha$-(benzyloxycarbonyl)-L-leucinyl]amino]-1-(phenylsulphonyl)-3-piperidinone

a.) (3RS,4RS)-4-[[$N^\alpha$-(benzyloxycarbonyl)-L-leucinyl]amino]-1-(phenylsulphonyl)-3-piperidinol

To a 0°C solution of the compound from Example 21(f) (161.2 mg, 0.40 mmol) in $CH_2Cl_2$ (5.0 mL) was added DIEA (0.21 mL, 1.21 mmol) followed by phenylsulphonyl chloride (0.06 mL, 0.48 mmol). The reaction was stirred at 0°C for 30 minutes then warmed to room temperature for 2.5 hours. The reaction was concentrated and chromatographed (2.5:97.5 $CH_3OH$:$CHCl_3$) to give 146 mg of the title compound: MS(ES+) 504.4 ($MH^+$), 526.4 (M+Na).

b.) 4-[[$N^\alpha$-(benzyloxycarbonyl)-L-leucinyl]amino]-1-(phenylsulphonyl)-3-piperidinone

Following the procedure of Example 3(b) except substituting the compound of Example 57(a), the title compound was prepared: MS(ES+) 502.3 ($MH^+$), 524.3 (M+Na).

Example 58

Preparation of 4-[[$N^\alpha$-(4-pyridinylmethoxycarbonyl)-L-leucinyl]amino]-1-(8-quinolinesulphonyl)-3-pyrrolidinone

a.) (3RS,4RS)-4-[[$N^\alpha$-(4-pyridinylmethoxycarbonyl)-L-leucinyl]amino]-1-(8-quinolinesulphonyl)-3-pyrrolidinol

To a solution of the compound of Example 47(b) (500 mg, 1.18 mmol) in $CH_2Cl_2$ was added TEA (0.5 mL, 3.54 mmol) and 8-quinolinesulphonyl chloride (282 mg, 1.24 mmol). The reaction was stirred at room temperature until complete as indicated by TLC analysis. The reaction was diluted with ethyl acetate and washed with water, brine, dried ($Na_2SO_4$), concentrated. Column chromatography (100% ethyl acetate) of the residue gave 560 mg of the title compound: MS(ES+) 542 ($MH^+$).

b.) 4-[[$N^\alpha$-(4-pyridinylmethoxycarbonyl)-L-leucinyl]amino]-1-(8-quinolinesulphonyl)-3-pyrrolidinone

Following the procedure of Example 17(b) except substituting the compound of Example 58(c), the title compound was prepared: MS(ES+) 540 ($MH^+$).

Example 59

Preparation of 4-[[Nα-(4-pyridinylmethoxycarbonyl)-L-leucinyl]amino]-1-(2-pyridylsulphonyl)-3-pyrrolidinone

a.) (3RS,4RS)-4-[[Nα-(4-pyridinylmethoxycarbonyl)-L-leucinyl]amino]-1-(2-pyridylsulphonyl)-3-pyrrolidinone

Following the procedure of Example 58(a) except substituting 2-pyridylsulphonyl chloride for 8-quinolinesulphonyl chloride, the title compound was prepared: MS(ES+) 492 ($MH^+$).

b.) 4-[[Nα-(4-pyridinylmethoxycarbonyl)-L-leucinyl]amino]-1-(2-pyridylsulphonyl)-3-pyrrolidinone

Following the procedure of Example 17(b) except substituting the compound of Example 59(a), the title compound was prepared: MS (ES+) 490 ($MH^+$).

Example 60

Preparation of 4-[[Nα-(4-pyridinylmethoxycarbonyl)-L-leucinyl]amino]-1-[(2-propoxy)carbonyl]-3-pyrrolidinone

a.) (3RS,4RS)-4-[[Nα-(4-pyridinylmethoxycarbonyl)-L-leucinyl]amino]-1-[(2-propoxy)carbonyl]-3-pyrrolidinol

To a solution of the compound of Example 47(b) (383 mg, 0.91 mmol) in $CH_2Cl_2$ was added TEA (0.44 mL, 3.2 mmol) followed by isopropyl chloroformate (0.96 mL of a 1.0 molar solution in THF, 0.96 mmol). The reaction was allowed to stir until complete as indicated by TLC analysis. Workup and chromatography gave 180 mg of the title compound: MS(ES+) 437 ($MH^+$).

b.) 4-[[Nα-(4-pyridinylmethoxycarbonyl)-L-leucinyl]amino]-1-[(2-propoxy)carbonyl]-3-pyrrolidinone

Following the procedure of Example 17(b) except substituting the compound of Example 60(a), the title compound was prepared: MS(ES+) 435 ($MH^+$).

Example 61

Preparation of 4-[[Nα-(4-pyridinylmethoxycarbonyl)-L-leucinyl]amino]-1-[(3-methyl-1-propoxy)carbonyl]-3-pyrrolidinone

a.) (3RS,4RS)-4-[[Nα-(4-pyridinylmethoxycarbonyl)-L-leucinyl]amino]-1-[(3-methy-1-propoxy)carbonyl]-3-pyrrolidinol

Following the procedure of Example 60(a) except substituting isobutyl chloroformate for isopropyl chloroformate, the title compound was prepared: MS(ES+) 451 (MH+).

b.) 4-[[Nα-(4-pyridinylmethoxycarbonyl)-L-leucinyl]amino]-1-[(3-methyl-1-propoxy)carbonyl]-3-pyrrolidinone

Following the procedure of Example 17(b) except substituting the compound of Example 61(a), the title compound was prepared: MS(ES+) 449 (MH+).

Example 62

Preparation of 4-[[Nα-(benzyloxycarbonyl)-L-leucinyl]amino]-1-[(4-phenoxy)phenylsulphonyl]-3-pyrrolidinone

a.) (3RS,4RS)-4-[[Nα-(benzyloxycarbonyl)-L-leucinyl]amino]-1-[(4-phenoxy)phenylsulphonyl]-3-pyrrolidinol

To a solution of the compound of Example 1(f) (200 mg, 0.51 mmol) in $CH_2Cl_2$ (10 mL) was added N-methylmorpholine (0.22 mL, 2.04 mmol) and 4-phenoxyphenylsulphonyl chloride (201 mg, 0.76 mmol). The reaction was stirred at room temperature until complete as indicated by TLC analysis. The reaction was diluted with ethyl acetate and washed with water, brine, dried ($Na_2SO_4$) and concentrated. Column chromatography of the residue (1:1 hexanes: ethyl acetate ) gave 186 mg of the title compound: MS(ES+) 582.1 ($MH^+$), 604.1 (M+Na).

b.) 4-[[N$^{\alpha}$-(benzyloxycarbonyl)-L-leucinyl]amino]-1-[(4-phenoxy)phenylsulphonyl]-3-pyrrolidinone

Following the procedure of Example 3(b) except substituting the compound of Example 62(a), the title compound was prepared: MS(ES+) 580.2 (MH$^+$), 602.3 (M+Na).

Example 63

Preparation of 4-[[N$^{\alpha}$-(benzyloxycarbonyl)-L-leucinyl]amino]-1-[(4-phenoxy)phenylsulphonyl]-3-piperidinone

a.) (3RS,4RS)-4-[[N$^{\alpha}$-(benzyloxycarbonyl)-L-leucinyl]amino]-1-[(4-phenoxy)phenylsulphonyl]-3-piperidinol

To a solution of the compound of Example 21(f) (150 mg, 0.38 mmol) in $CH_2Cl_2$ (10 mL) was added N-methylmorpholine (0.21 mL, 1.94 mmol) and 4-phenoxyphenylsulphonyl chloride (135 mg, 0.50 mmol). The reaction was stirred at room temperature until complete as indicated by TLC analysis. The reaction was diluted with ethyl acetate and washed with water, brine, dried ($Na_2SO_4$) and concentrated. Column chromatography of the residue (1:2 hexanes: ethyl acetate ) gave 198 mg of the title compound: MS(ES+) 596.1 (MH$^+$), 618.2 (M+Na).

b.) 4-[[N$^{\alpha}$-(benzyloxycarbonyl)-L-leucinyl]amino]-1-[(4-phenoxy)phenylsulphonyl]-3-piperidinone

Following the procedure of Example 3(b) except substituting the compound of Example 63(a), the title compound was prepared: MS (ES$^+$) 594.2 (MH$^+$), 616.2 (M+Na).

Example 64

Preparation of 4-[[N$^{\alpha}$-(3,4-dichlorobenzoyl)-L-leucinyl]amino]-1-[(2S)-4-methyl-2-[[(benzyloxycarbonyl)]amino]pentyl]-3-pyrrolidinone

a.) (3RS,4RS)-4-[[N$^{\alpha}$-(3,4-dichlorobenzoyl)-L-leucinyl]amino]-1-[(2S)-4-methyl-2-[[(benzyloxycarbonyl)]amino] pentyl]-3-pyrrolidinol

Following the procedure of Example 46(j) except substituting 3,4-dichlorobenzoic acid for 3-isoquinolinecarboxylic acid, the title compound was produced: MS(ES+) 622 (MH$^+$).

b.) 4-[[N$^{\alpha}$-(3,4-dichlorobenzoyl)-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 64(a), the title compound was produced: MS(ES+) 619 (MH$^+$).

Example 65

Preparation of 4-[[N$^{\alpha}$-(6-quinolinecarbonyl)-L-leucinyl]amino]-1-[(2S)-4-methyl-2-[[(benzyloxycarbonyl)]amino]pentyl]-3-pyrrolidinone

a.) (3RS,4RS)-4-[[N$^{\alpha}$-(6-quinolinecarbonyl)-L-leucinyl]amino]-1-[(2S)-4-methyl-2-[[(benzyloxycarbonyl)]amino] pentyl]-3-pyrrolidinol

Following the procedure of Example 46(j) except substituting 6-quinolinecarboxylic acid for 3-isoquinolinecarboxylic acid, the title compound was produced: MS(ES+) 604 (MH$^+$).

b.) 4-[[N$^{\alpha}$-(6-quinolinecarbonyl)-L-leucinyl]amino]-1-[(2S)-4-methyl-2-[[(benzyloxycarbonyl)]amino]pentyl]-3-pyrrolidinone

Following the procedure of Example 46(k) except substituting the compund of Example 65(a), the title compound was produced: MS(ES+) 602 (MH$^+$).

Example 66

Preparation of 4-[(2-dibenzofuransulphonyl)amino]-1-[(2S)-4-methyl-2-[[(benzyloxycarbonyl)]amino]pentanoyl]-3-pyrrolidinone

a.) (3RS,4RS)-4-[(2-dibenzofuransulphonyl)amino]-1-*tert*-butoxycarbonyl-3-pyrrolidinol

To a solution of the compound of Example 1(d) (200 mg) in DMF (5.0 mL) was added N-methylmorpholine (0.11 mL) and 2-dibenzofuransulphonyl chloride (264 mg). The mixture was stirred for 5 hours whereupon it was diluted with ethyl acetate and washed with water, brine, dried ($Na_2SO_4$) and concentrared. Column chromatography of the residue (5% $CH_3OH$:$CHCl_3$) gave 490 mg of the title compound: MS(ES+) 433 ($MH^+$).

b.) (3RS,4RS)-4-[(2-dibenzofuransulphonyl)amino]-3-pyrrolidinol hydrochloride

The compound of Example 66(a) (490 mg) was dissolved in ethtyl acetate (20 mL) and cooled to 0°C. HCl (g) was bubbled through the mixture for approximately 10 minutes. The reaction was stirred at 0°C for 1 hour and warmed to room temperature for 10 minutes. The solvent was evaporated to give the title compound which was used directly in the following step with no further purification: MS(ES+) 333 ($MH^+$).

c.) (3RS,4RS)-4-[(2-dibenzofuransulphonyl)amino]-1-[(2S)-4-methyl-2-[[(benzyloxycarbonyl)]amino]pentanoyl]-3-pyrrolidinol

To a solution of the compound of Example 66(b) (210 mg, 0.57 mmol) in $CH_2Cl_2$ (50 mL) was added TEA (0.1 mL, 0.68 mmol) EDC (131 mg, 0.68 mmol), HOBT (92 mg, 0.68 mmol) and CBZ-leucine (131 mg, 0.57 mmol). The reaction was stirred at room temperature for 4 hours whereupon it was diluted with ethyl acetate and washed with 1N HCl, sat. $NaHCO_3$, water, brine, dried ($Na_2SO_4$), filtered and concentrated. Column chromatography of the residue (5% $CH_3OH$:$CH_2Cl_2$) gave 76 mg of the title compound: MS(ES+) 580 ($MH^+$).

d.) 4-[(2-dibenzofuransulphonyl)amino]-1-[(2S)-4-methyl-2-[[(benzyloxycarbonyl)]amino]pentanoyl]-3-pyrrolidinone

Following the procedure of Example 1(h) except substituting the compound from Example 66(c), the title compound was prepared: MS (ES+) 578 ($MH^+$).

Example 67

Preparation of 4-[(2-dibenzofuransulphonyl)amino]-1-[(2S)-4-methyl-2-[[(benzyloxycarbonyl)]methylamino] pentanoyl]-3-pyrrolidinone

a.) (3RS,4RS)-4-[(2-dibenzofuransulphonyl)amino]-1-[(2S)-4-methyl-2-[[(benzyloxycarbonyl)]methylamino] pentanoyl]-3-pyrrolidinol

Following the procedure of Example 66(c) except substituting N-methyl-CBZ-leucine for CBZ-leucine, the title compound was prepared: MS(ES+) 594 (M+Na).

b.) 4-[(2-dibenzofuransulphonyl)amino]-1-[(2S)-4-methyl-2-[[(benzyloxycarbonyl)]methylamino]pentanoyl]-3-pyrrolidinone

Following the procedure of Example 1(h) except substituting the compound of Example 67(a), the title compound was prepared: MS(ES+) 329 (M-(N-$CH_3$-CBZ-leucine)).

Example 68

Preparation of 4-[[$N^\alpha$-(benzyloxycarbonyl)-L-leucinyl]amino]-1-(4-methylpentyl)-3-piperidinone

a.) (3RS,4RS)-4-[[$N^\alpha$-(benzyloxycarbonyl)-L-leucinyl]amino]-1-(4-methylpentyl)-3-piperidinol

To a solution of the compound of Example 21(f) in $CH_2Cl_2$ (10 mL) was added TEA (0.43 mL) followed by 4-methylbutanal. The reaction was stirred at room temperature for 2 hours whereupon it was concentrated in vacuo

and allowed to dry under high vacuum for 1 hour. The residue was the dissolved in $CH_2Cl_2$ (10 mL) and sodium triacetoxyborohydride (1.22 g, 5.75 mmol) was added. The reaction was stirred at room temperature for 17 hours whereupon it was diluted with $CHCl_3$ and washed with water, brine, dried ($Na_2SO_4$) and concentrated. Column chromatography of the residue (5% $CH_3OH$:$CHCl_3$) gave 0.69 g of the title compound: MS(ES+) 448.4 ($MH^+$).

b.) 4-[[$N^{\alpha}$-(benzyloxycarbonyl)-L-leucinyl]amino]-1-(4-methylpentyl)-3-piperidinone

Following the procedure of Example 17(b) except substituting the compound of Example 68(a), the title compound was prepared: MS(ES+) 446.4 ($MH^+$).

Example 69

Preparation of 4-[[$N^{\alpha}$-(2-pyridylcarbonyl)-L-leucinyl]amino]-1-(4-methylpentyl)-3-piperidinone

a.) (3RS,4RS)-4-[(L-leucinyl)amino]-1-(4-methylpentyl)-3-piperidinol

To a solution of the compound of Example 68(a) in 5% formic acid:methanol (10 mL) was added palladium black (638 mg). The reaction was allowd to stir at room temperature for 4 hours whereupon it was filtered through a pad of celite to remove the catalyst. The pad of celite was rinsed sveral times with methanol. The methanol was concentrated to give an oil which was dissolved in ethyl acetate and washed with 10% $Na_2CO_3$, brine, dried ($Na_2SO_4$), filtered and concentrated to give 163 mg of an oil which was used directly in the following step with no further purification: MS(ES+) 314.4 ($MH^+$).

b.) (3RS,4RS)-4-[[$N^{\alpha}$-(2-pyridylcarbonyl)-L-leucinyl]amino]-1-(4-methylpentyl)-3-piperidinol

To a solution of the compound of Example 69(a) (159.3 mg, 0.51 mmol) in DMF (2.0 mL) was added picolinic acid (75.2 mg, 0.61 mmol), EDC (117 mg, 0.61 mmol and HOBT (82.6 mg, 0.61 mmol). The reaction was stirred for 48 hours at room temperature whereupon it was diluted with ethyl acetate and washed with 10% $Na_2CO_3$, brine, dried ($MgSO_4$), filtered and concentrated. Column chromatography of the residue (97:3 to 95:5 $CHCl_3$:$CH_3OH$) gave 149 mg of the title compound: MS(ES+) 419.3 ($MH^+$).

c.) 4-[[$N^{\alpha}$-(2-pyridylcarbonyl)-L-leucinyl]amino]-1-(4-methylpentyl)-3-piperidinone

Following the procedure of Example 17(b) except substituting the compound of Example 69(b), the title compound was prepared: MS(ES+) 417.3 ($MH^+$).

Example 70

Preparation of 4-[[$N^{\alpha}$-(3-chlorobenzoyl)-L-leucinyl]amino]-1-(4-methylpentyl)-3-piperidinone

a.) (3RS,4RS)-4-[[$N^{\alpha}$-(3-chlorobenzoyl)-L-leucinyl]amino]-1-(4-methylpentyl)-3-piperidinol

Following the procedure of Example 69(b) except substituting 3-chlorobenzoic acid for picolinic acid, the title compound was prepared: MS(ES+) 452.3 ($MH^+$).

b.) 4-[[$N^{\alpha}$-(3-chlorobenzoyl)-L-leucinyl]amino]-1-(4-methylpentyl)-3-piperidinone

Following the procedure of Example 17(b) except substituting the compound of Example 70(a), the title compound was prepared: MS (ES) 450.3 ($MH^+$).

Example 71

Preparation of 4-[[$N^{\alpha}$-(2-quinolinecarbonyl)-L-leucinyl]amino]-1-(4-methylpentyl)-3-piperidinone

a.) (3RS,4RS)-4-[[$N^{\alpha}$-(2-quinolinecarbonyl)-L-leucinyl]amino]-1-(4-methylpentyl)-3-piperidinol

Following the procedure of Example 69(b) except substituting 2-quinolinecarboxylic acid for picolinic acid, the title compound was prepared: MS(ES+) 469.4 ($MH^+$).

b.) 4-[[$N^{\alpha}$-(2-quinolinecarbonyl)-L-leucinyl]amino]-1-(4-methylpentyl)-3-piperidinone

Following the procedure of Example 17(b) except substituting the compound of Example 71(a), the title compound was prepared: MS (ES) 467.3 ($MH^+$).

Example 72

Preparation of 4-[[$N^{\alpha}$-(3,4-dichlorobenzoyl)-L-leucinyl]amino]-1-(4-methylpentyl)-3-piperidinone

a.) (3RS,4RS)-4-[[$N^{\alpha}$-(3,4-dichlorobenzoyl)-L-leucinyl]amino]-1-(4-methylpentyl)-3-piperidinol

Following the procedure of Example 69(b) except substituting 2,3-dichlorobenzoic acid for picolinic acid, the title compound was prepared: MS(ES+) 486.3 ($MH^+$).

b.) 4-[[$N^{\alpha}$-(3,4-dichlorobenzoyl)-L-leucinyl]amino]-1-(4-methylpentyl)-3-piperidinone

Following the procedure of Example 17(b) except substituting the compound of Example 72(a), the title compound was prepared: MS(ES+) 484.1 ($MH^+$).

Example 73

Preparation of 4-[[$N^{\alpha}$-(8-quinolinecarbonyl)-L-leucinyl]amino]-1-(4-methylpentyl)-3-piperidinone

a.) (3RS,4RS)-4-[[$N^{\alpha}$-(8-quinolinecarbonyl)-L-leucinyl]amino]-1-(4-methylpentyl)-3-piperidinol

Following the procedure of Example 69(b) except substituting 8-quinolinecarboxylic acid for picolinic acid, the title compound was prepared: MS(ES+) 469.4 ($MH^+$).

b.) 4-[[$N^{\alpha}$-(8-quinolinecarbonyl)-L-leucinyl]amino]-1-(4-methylpentyl)-3-piperidinone

Following the procedure of Example 17(b) except substituting the compound of Example 73(a), the title compound was prepared: MS(ES+) 467.3 ($MH^+$), 499.4 (M+Na).

Example 74

Preparation of 4-[[$N^{\alpha}$-(3-isoquinolinecarbonyl)-L-leucinyl]amino]-1-(4-methylpentyl)-3-piperidinone

a.) (3RS,4RS)-4-[[$N^{\alpha}$-(3-isoquinolinecarbonyl)-L-leucinyl]amino]-1-(4-methylpentyl)-3-piperidinol

Following the procedure of Example 69(b) except substituting 3-isoquinolinecarboxylic acid for picolinic acid, the title compound was prepared: MS(ES+) 469.4 ($MH^+$).

b.) 4-[[$N^{\alpha}$-(3-isoquinolinecarbonyl)-L-leucinyl]amino]-1-(4-methylpentyl)-3-piperidinone

Following the procedure of Example 17(b) except substituting the compound of Example 74(a), the title compound was prepared: MS(ES+) 467.3 ($MH^+$).

Example 75

Preparation of 4-[[$N^{\alpha}$-(2-pyridinylmethoxycarbonyl)-L-leucinyl]amino]-1-(4-methylpentyl)-3-piperidinone

a.) 4-(benzyloxycarbonyl)amino-1-*tert*-butoxycarbonyl-3-piperidinol

To a solution of the compound of amino alcohol of Example 21(d) (5.43 g, 25.1 mmol) in $CH_2Cl_2$ (75 mL) was added DIEA (6.5 mL, 37.3 mmol) followed by benzyl chloroformate (4.0 mL, 28.0 mmol). The reaction was stirred overnight whereupon it was concentrated and the residue was dissolved in $CHCl_3$ and washed with 5% $NaHCO_3$, water, 1N HCl, brine, dried ($Na_2SO_4$), filtered and concentrated. Column chromatography of the residue (40:60 ethyl acetate:hexanes) gave 2.30 g of the title compound: MS(ES+) 351.3 ($MH^+$), 373.3 (M+Na).

b.) 4-(benzyloxycarbonyl)amino-3-piperidinol hydrochloride

The compound of Example 75(a) (2.2 g, 6.3 mmol) was dissolved in 4N HCl/dioxane and stirred for 45 minutes. The reaction was then concentrated and azeotroped with toluene (3 x's) to afford 1.78 g of the title compound as a glassy yellow solid: MS (ES) 251.2 ($MH^+$).

c.) (3RS,4RS)-4-[(benzyloxycarbonyl)amino]-1-(4-methylpentyl)-3-piperidinol

To a solution of the compound of Example 75(b) (288.3 mg, 1.01 mmol) in $CH_2Cl_2$ (2.0 mL) was added TEA (0.17 mL, 1.22 mmol) and 4-methylbutanal (241.5 mg, 1.21 mmol, this material was approximately 50% pure). The reaction was stirred for 1.5 hours whereupon it was concentrated and placed under high vacuum for 1 hour. The residue was the dissolved in $CH_2Cl_2$ (3.0 mL) and sodium triacetoxyborohydride (467.4 mg, 2.21 mmol) was added. The reaction was stirred overnight at room temperature whereupon it was diluted with $CHCl_3$ and washed with 50% brine, brine, dried ($Na_2SO_4$) filtered and concentrated. Column chromatography of the residue (2.5:97.5 $CH_3OH$:$CHCl_3$) gave 128.7 mg of the title compound: MS(ES+) 335.3 ($MH^+$).

d.) (3RS,4RS)-4-amino-1-(4-methylpentyl)-3-piperidinol

To a 0°C solution of the compound of Example 75(c) (1.0 g, 2.99 mmol) in methanol (50 mL) was added palladium black (1.20 g). The mixture was stirred under a balloon of hydrogen for 2.5 hours whereupon it was filtered thru a pad of celite with methanol. The filtrate was concentrated to give 0.59 g of the title compound as a yellow oil: MS(ES+) 201.2 ($MH^+$).

e.) (3RS,4RS)-4-[[$N^\alpha$-(2-pyridinylmethoxycarbonyl)-L-leucinyl]amino]-1-(4-methylpentyl)-3-piperidinol

To a solution of the compound derived from Example 75(d) (178 mg, 0.90 mmol) in DMF (3.0 mL) was added EDC (207.2 mg, 1.08 mmol), HOBT (147.5 mg, 1.09 mmol) and N-(2-pyridylmethoxycarbonyl)-L-leucine (290.0 mg, 1.09 mmol). The reaction was stirred overnight whereupon it was poured rapidly into a stirred mixture of ethyl acetate (50 mL), 10% $Na_2CO_3$ and brine. This mixture was stirred for 1 hour and the organic layer as separated. The aqueous layer was washed with ethyl acetate (50 mL). The combined organic layers were washed with brine, dried ($Na_2SO_4$) filtered and concentrated. Column chromatography of the residue (5:95 $CH_3OH$:$CHCl_3$) gave 199.7 mg of the title compound: MS(ES+) 449.3 ($MH^+$).

f.) 4-[[$N^\alpha$-(2-pyridinylmethoxycarbonyl)-L-leucinyl]amino]-1-(4-methylpentyl)-3-piperidinone

Following the procedure of Example 17(b) except substituting the compound of Example 75(e), the title compound was prepared: MS (ES) 447.4 ($MH^+$).

Example 76

Preparation of 4-[[$N^\alpha$-(acetyl)-L-leucinyl]amino]-1-(4-methylpentyl)-3-piperidinone

a.) (3RS,4RS)-4-[[$N^\alpha$-(*tert*-butoxycarbonyl)-L-leucinyl]amino]-1-(4-methylpentyl)-3-piperidinol

To a solution of the compound of Example 75(d) (314 mg, 1.57 mmol) in DMF (7.0 mL) was added HOBT (256.7 mg, 1.90 mmol), BOC-leucine (473.2 mg, 1.90 mmol) and EDC (364.8 mg, 1.90 mmol). The reaction was stirred overnight at room temperature whereupon it was poured into a rapidly stirred mixture of ethyl acetate (50 mL), 10% $Na_2CO_3$ (50 mL) and brine (50 mL). This mixture was stirred for 30 minutes whereupon the aqueous layer was separated and washed with ethyl acetate (50 mL). The combined organic layers were washed with brine, dried ($Na_2SO_4$), filtered, and concentrated. Column chromatography of the residue (3:97 $CH_3OH$:$CHCl_3$) gave 434 mg of the title compound: MS(ES+) 414.5 ($MH^+$).

b.) (3RS,4RS)-4-[(L-leucinyl)amino]-1-(4-methylpentyl)-3-piperidinol hydrochloride

The compound from Example 76(a) (434 mg, 1.05 mmol) was dissolved in 4N HCl/dioxane (10 mL) and stirred at room temperature for approximately 30 minutes. The reaction was concentrated and azeotropically dried with toluene.. The amine salt (127.6 mg, 0.70 mmol) was then dissolved in $CH_2Cl_2$ (4.0 mL) and DIEA was added (0.27 mL, 1.54 mmol), This solution was then divided in half and used directly in the following procedure: MS (ES+) 314.4 ($MH^+$).

c.) (3RS,4RS)-4-[[N$^{\alpha}$-(acetyl)-L-leucinyl]amino]-1-(4-methylpentyl)-3-piperidinol

To a 0°C solution of the compound of Example 76(b) (0.35 mmol) was added acetic anhydride (0.04 mL). The reaction was stirred for 1.5 hours at 0°C whereupon it was diluted with $CHCl_3$ (50 mL) and washed with 5% $NaHCO_3$, brine, dried ($MgSO_4$), filtered and concentrated. Column chromatography of the residue (5:95 $CH_3OH:CHCl_3$) gave 51.3 mg of the title compound: MS(ES+) 356.5 ($MH^+$).

d.) 4-[[N$^{\alpha}$-(acetyl)-L-leucinyl]amino]-1-(4-methylpentyl)-3-piperidinone

Following the procedure of Example 17(b) except substituting the compound of Example 76(c), the title compound was prepared: MS(ES+) 354.4 ($MH^+$).

Example 77

Preparation of 4-[[N$^{\alpha}$-(p-trifluoromethylbenzenesulphonyl)-L-leucinyl]amino]-1-(4-methylpentyl)-3-piperidinone

a.) (3RS,4RS)-4-[[N$^{\alpha}$-(p-trifluoromethylbenzenesulphonyl)-L-leucinyl]amino]-1-(4-methylpentyl)-3-piperidinol

To a 0°C solution of the compound of Example 76(b) (0.35 mmol) in $CH_2Cl_2$ was added p-trifluoromethyl benzenesulphonyl chloride (105.8 mg, 0.43 mmol). The reaction was stirred for ca. 1.5 hours whereupon it was diluted with $CHCl_3$ (50 ml) and washed with 5% $NaHCO_3$, brine, dried ($MgSO_4$), filtered and concentrated. Column chromatography of the residue (5:95 $CH_3OH:CHCl_3$) gave 94.2 mg of the title compound: MS(ES+) 522.3 ($MH^+$).

b.) 4-[[N$^{\alpha}$-(p-trifluoromethylbenzenesulphonyl)-L-leucinyl]amino]-1-(4-methylpentyl)-3-piperidinone

Following the procedure of Example 17(b) except substituting the compound of Example 77(a), the title compound was prepared: MS(ES+) 520.2 ($MH^+$).

Example 78

Preparation of 4-[[N$^{\alpha}$-(6-quinolinecarbonyl)-L-leucinyl]amino]-1-(4-methylpentyl)-3-piperidinone

a.) (3RS,4RS)-4-[[N$^{\alpha}$-(6-quinolinecarbonyl)-L-leucinyl]amino]-1-(4-methylpentyl)-3-piperidinol

To a solution of the compound of example 76(b) (0.35 mmol) in DMF (2.0 mL) was added DIEA (0.13 mL), HOBT (58.2 mg), 6-quinolinecarboxylic acid (73.2 mg) and EDC (85.2 mg). The reaction was stirred at room temperature for 48 hours whereupon it was poured into a rapidly stirred mixture of ethyl acetate (50 mL), 10% $Na_2CO_3$ (50 mL) and brine (50 mL). This mixture was stirred for 30 minutes whereupon the aqueous layer was separated and washed with ethyl acetate (50 mL). The combined organic layers were washed with 10% $Na_2CO_3$, brine, dried ($MgSO_4$), filtered, and concentrated. Column chromatography of the residue (5:95 $CH_3OH:CHCl_3$) gave 91.8 mg of the title compound: MS(ES+) 469.4 ($MH^+$).

b.) 4-[[N$^{\alpha}$-(6-quinolinecarbonyl)-L-leucinyl]amino]-1-(4-methylpentyl)-3-piperidinone

Following the procedure of Example 17(b) except substituting the compound of Example 78(a), the title compound was prepared: MS(ES+) 467.4 ($MH^+$).

Example 79

Preparation of 4-[[2-(RS)-(3-biphenyl)-4-methyl]amino]pentanoyl]-1-(4-methylpentyl)-3-piperidinone

a.) (3RS,4RS)-4-[[2-(RS)-(3-biphenyl)-4-methyl]amino]pentanoyl]-1-(4-methylpentyl)-3-piperidinol

To a solution of the compound of example 75(d) (101.0 mg, 0.50 mmol) in DMF (2.0 mL) was added HOBT (82.5 mg, 0.61 mmol), 2-(3-biphenyl)-4-methylvaleric acid (161.8 mg, 0.60 mmol) and EDC (116.6 mg, 0.61 mmol). The reaction was stirred at room temperature for 17 hours whereupon it was poured into a rapidly stirred mixture of ethyl acetate (50 mL), 5% $NaHCO_3$ (50 mL) and brine (50 mL). This mixture was stirred for 30 minutes whereupon the aqueous layer was separated and washed with ethyl acetate (50 mL). The combined organic layers were washed with

brine, dried ($MgSO_4$), filtered, and concentrated. Column chromatography of the residue (2:98 $CH_3OH$:$CHCl_3$) gave 72.9 mg of the title compound: MS(ES+) 451.3 ($MH^+$).

b.) 4-[[2-(RS)-[(3-biphenyl)-4-methyl]amino]pentanoyl]-1-(4-methylpentyl)-3-piperidinone

Following the procedure of Example 17(b) except substituting the compound of Example 79(a), the title compound was prepared: MS(ES+) 449.4 ($MH^+$).

Example 80

Preparation of 4-[[$N^\alpha$-(benzyloxycarbony)-L-leucinyl]amino]-1-[2-[(benzyloxycarbonyl)methylamino]ethyl]-3-piperidinone

a.) (3RS,4RS)-4-[[$N^\alpha$-(benzyloxycarbonyl)-L-leucinyl]amino]-1-[2-[(benzyloxycarbonyl)methylamino]ethyl]-3-piperidinol

To a stirred suspension of the amine hydrochloride salt of Example 21(f) (2.04 g, 5.09 mmol) in $CH_2Cl_2$(10 mL) at room temperature was added triethylamine (836 uL, 6.0 mmol). A solution of N-[(benzyloxy)carbonyl]-N-methylaminoacetaldehyde (1.25 g, 6.0 mmol) in $CH_2Cl_2$ was added to the reaction mixture, which was stirred for 2h, then concentrated and stored under high vacuum for 2 h. The residue was dissolved in $CH_2Cl_2$ (15 mL), sodium triacetoxyborohydride (2.33 g, 11.0 mmol) was added, and the mixture was stirred overnight whereupon it was diluted with $CHCl_3$ and washed with $H_2O$ and brine. Column chromatography (silica gel, 3:97 MeOH: $CHCl_3$, then 5:95 MeOH: $CHCl_3$) gave the title compound which was used directly in the next step: MS($ES^+$) 555.2 (MH+).

b.) 4-[[$N^\alpha$-(benzyloxycarbonyl)-L-leucinyl]amino]-1-[2-[(benzyloxycarbonyl)methylamino]ethyl]-3-piperidinone

To a solution of the alcohol of Example 80(a) (305.9 mg, 0.55 mmol) in anhydrous DMSO (2 mL) under argon was added triethylamine (460 uL, 3.3 mmol) and $SO_3$•pyridine complex (266.1 mg, 1.7 mmol). The reaction was stirred at room temperature until TLC analysis indicated the complete consumption of starting material (1h) whereupon the mixture was diluted with $CHCl_3$ (100 mL) and washed with 1:1 brine : 5% $NaHCO_3$. The aqueous layer was washed with fresh $CHCl_3$ and the combined organic layers were washed with 5% $NaHCO_3$ and brine, then dried (MgSO4), filtered, and concentrated. Column chromatography of the residue (silica gel, $CHCl_3$, then 2:98 MeOH:$CHCl_3$) gave 131.4 mg of the title compound: MS(ES+) 553.2 ($MH^+$).

Example 81

Preparation of 4-[[$N^\alpha$-($\alpha$-toluenesulphonyl)-L-leucinyl]amino]-1-[3-(2-pyridyl)phenylacetyl)]-3-piperidinone

a.) (3RS,4RS)-4-[(L-leucinyl)amino]-1-[3-(2-pyridyl)phenylacetyl)]-3-piperidinol

To a 0°C solution of the compound of Example 33(d) in MeOH (30 mL) was added 10% Pd on carbon (1.50 g) under a blanket of argon. The mixture was stirred under an atmosphere of hydrogen, while warming to room temperature, until TLC analysis indicated the complete consumption of starting material (30 min). The reaction was filtered through a pad of celite, washed with MeOH and the filtrate was concentrated to give 389 mg of the title compound: MS(ES+) 425.2 ($MH^+$).

b.) (3RS,4RS)-4-[[$N^\alpha$-($\alpha$-toluenesulphonyl)-L-leucinyl]amino]-1-[3-(2-pyridyl)phenylacetyl)]-3-piperidinol

To a solution of the amine of Example 81(a) (0.23 mmol) in $CH_2Cl_2$ (2 mL) at 0°C was added N-methylmorpholine (31 uL, 0.28 mmol), and $\alpha$-toluenesulfonyl chloride (56.0 mg, 0.29 mmol). The reaction mixture was stirred for 2 h whereupon it was diluted with $CHCl_3$ (50 mL), washed with 10% $Na_2CO_3$ and brine, dried ($MgSO_4$), filtered and concentrated to give 129.3 mg of the title compound which was used in the following step without further purification: MS(ES+) 579.3 ($MH^+$).

c.) 4-[[$N^\alpha$-($\alpha$-toluenesulphonyl)-L-leucinyl]amino]-1-[3-(2-pyridyl)phenylacetyl)]-3-piperidinone

Following the procedure of Example 80(b), except substituting the compound of Example 81(b), the title compound was prepared: MS(ES+) 577.4 ($MH^+$).

Example 82

Preparation of 4-[[N$^{\alpha}$-(2-naphthylcarbonyl)-L-leucinyl]amino]-1-[3-(2-pyridyl)phenylacetyl)]-3-piperidinone

a.) (3RS,4RS)-4-[[N$^{\alpha}$-(2-naphthylcarbonyl)-L-leucinyl]amino]-1-[3-(2-pyridyl)phenylacetyl)]-3-piperidinone

Following the procedure of Example 81(b), except substituting 2-naphthoyl chloride for $\alpha$-toluenesulfonyl chloride, the title compound was prepared: MS (ES+) 579.3 (MH+).

b.) 4-[[N$^{\alpha}$-(2-naphthylcarbonyl)-L-leucinyl]amino]-1-[3-(2-pyridyl)phenylacetyl)]-3-piperidinone

Following the procedure of Example 80(b), except substituting the compound of Example 82 (a), the title compound was prepared and isolated as the higher $R_f$ component by column chromatography: MS (ES+) 577.3 (MH+). The lower $R_f$ diastereomer component was also isolated by column chromatography: MS (ES+) (MH+) 577.2

Example 83

Preparation of 4-[[N$^{\alpha}$-(benzensulphonyl)-L-leucinyl]amino]-1-[3-(2-pyridyl)phenylacetyl)]-3-piperidinone

a.) (3RS,4RS)-4-[[N$^{\alpha}$-(benzensulphonyl)-L-leucinyl]amino]-1-[3-(2-pyridyl)phenylacetyl)]-3-piperidinol

Following the procedure of Example 81(a), except substituting benzenesulfonyl chloride for $\alpha$-toluenesulfonyl chloride, the title compound was prepared: MS(ES+) 565.3 ($MH^+$).

b.) 4-[[N$^{\alpha}$-(benzensulphonyl)-L-leucinyl]amino]-1-[3-(2-pyridyl)phenylacetyl)]-3-piperidinone

Following the procedure of Example 80(b), except substituting the compound of Example 83(a), the title compound was prepared: MS(ES+) 563.4 ($MH^+$).

Example 84

Preparation of 4-[[N$^{\alpha}$-(3-isoquinolinecarbonyl)-L-leucinyl]amino]-1-[3-(2-pyridyl)phenylacetyl)]-3-piperidinone

a.) (3RS,4RS)-4-[[N$^{\alpha}$-(3-isoquinolinecarbonyl)-L-leucinyl]amino]-1-[3-(2-pyridyl)phenylacetyl)]-3-piperidinol

To a solution of the compound of 81(a) (0.23 mmol) in $CH_2Cl_2$ (2 mL) was added HOBT (37.6 mg, 0.28 mmol), 3-isoquinolinecarboxylic acid (48.7 mg, 0.28 mmol), and EDC (53.5 mg, 0.28 mmol). The reaction mixture was stirred at room temperature overnight whereupon it was poured into a rapidly-stirred mixture of EtOAc, 10% $Na_2CO_3$, and brine (50 mL each) and stirred for 30 min. The layers were separated, and the aqueous layer was washed with fresh EtOAc (50 mL), The combined organic layers were washed with 10% $Na_2CO_3$ and brine, dried ($MgSO_4$), filtered, and concentrated. Column chromatography (silica gel, 5:95 MeOH: EtOAc) gave 40.7 mg of the title compound: MS(ES+) 580.3 (MH+).

b) 4-[[N$^{\alpha}$-(3-isoquinolinecarbonyl)-L-leucinyl]amino]-1-[3-(2-pyridyl)phenylacetyl)]-3-piperidinone

Following the procedure of Example 80(b), except substituting the compound of Example 84 (a), the title compound was prepared: MS (ES+) 578.1 (MH+)

Example 85

Preparation of 4-[3-[(2-pyridyl)phenylacetyl)]amino]-1-[(2S)-4-methyl-2-[[(benzyloxycarbonyl)]amino]pentanoyl]-3-piperidinone

a.) (3RS,4RS)-4-[3-[(2-pyridyl)phenytacetyl)]amino]-1-(tert-butoxycarbonyl)-3-piperidinone

To a solution of the amino alcohol of Example 21(d) (434.1 mg, 2.0 mmol) in DMF (5 mL) was added HOBT (299.1 mg, 2.2 mmol), 3-(2-pyridyl)phenylacetic acid from Example 33(c) (471.4 mg, 2.2 mmol), and EDC (422.5 mg, 2.2 mmol). The reaction was stirred at room temperature overnight whereupon it was poured into a rapidly stirred

mixture of EtOAc, 10% $Na_2CO_3$, and brine (100 mL each) and stirred for 30 min. The layers were separated, and the aqueous layer was washed with fresh EtOAc (100 mL), The combined organic layers were washed with 10% $Na_2CO_3$ and brine, dried ($MgSO_4$), filtered and concentrated. Column chromatography (silica gel, EtOAc) gave 388 mg of the title compound: MS(ES+) 412.3 ($MH^+$).

b.) (3RS,4RS)-4-[3-[(2-pyridyl)phenylacetyl)]amino]-3-piperidinol bis-hydrochloride

To a solution of the compound of Example 85(a) was dissolved in 4 N HCl/dioxane (30 mL) and stirred at room temperature for 1.5 h, while monitoring gas evolution with a mineral oil bubbler. The reaction was concentrated and the residue was azeotropically dried to produce the title compound which was used directly in the following step: MS(ES+) 312.3 ($MH^+$).

c.) (3RS,4RS)-4-[3-[(2-pyridyl)phenylacetyl)]amino]-1-[(2S)-4-methyl-2-[[(benzyloxycarbonyl)]amino]pentanoyl]-3-piperidinol

To a solution of CBZ-Leucine (144.6 mg, 0.55 mmol) in DMF (4 mL) was added the compound of Example 85(b) (0.45 mmol), DIEA (173 uL, 0.99 mmol), HOBT (73.1 mg, 0.54 mmol), and EDC (106.1 mg, 0.55 mmol). The reaction was stirred at room temperature overnight whereupon it was poured into a rapidly stirred mixture of EtOAc, 10% $Na_2CO_3$, and brine (50 mL each) and stirred for 1 h. The layers were separated, and the aqueous layer was washed with fresh EtOAc (50 mL), The combined organic layers were washed with 10% $Na_2CO_3$ and brine, dried ($MgSO_4$), filtered and concentrated to give the title compound which was used directly in the following step without further purification: MS(ES+) 559.3 ($MH^+$).

d.) 4-[3-[(2-pyridyl)phenylacetyl)]amino]-1-[(2S)-4-methyl-2-([(benzyloxycarbonyl)]amino]pentanoyl]-3-piperidinone

Following the procedure of Example 80(b), except substituting the compound of Example 85(c), the title compound was prepared: MS(ES+) 557.3 ($MH^+$).

Example 86

Preparation of 4-[3-[(2-pyridyl)phenylacetyl)]amino]-1-[(2S)-4-methyl-2-[[2-(pyridinylmethoxycarbonyl)]amino] pentanoyl]-3-piperidinone

a.) (3RS,4RS)-4-[3-[(2-pyridyl)phenylacetyl)]amino]-1-[(2S)-4-methyl-2-[[(2-pyridinylmethoxycarbonyl)]amino] pentanoyl]-3-piperidinol

Following the procedure of Example 85(c), except substituting N-(2-pyridylmethoxycarbonyl)-L-leucine for CBZ-Leucine, the title compound was prepared: MS (ES+) 560.3 ($MH^+$).

b.) 4-[3-[(2-pyridyl)phenylacetyl)]amino]-1-[(2S)-4-methyl-2-[[2-(pyridinylmethoxycarbonyl)]amino]pentanoyl]-3-piperidinone

Following the procedure of Example 80(b), except substituting the compound of Example 86(a), the title compound was prepared: MS (ES+) 558.2 ($MH^+$).

Example 87

Preparation of 4-[[$N^{\alpha}$-(2-phenylacetyl)-L-leucinyl]amino]-1-[(2S)-4-methyl-2-[[(benzyloxycarbonyl)]amino]pentyl]-3-pyrrolidinone

a.) (3RS,4RS)-4-[[$N^{\alpha}$-(2-phenylacetyl)-L-leucinyl]amino]-1-[(2S)-4-methyl-2-[[(benzyloxycarbonyl)]amino]pentyl]-3-pyrrolidinol

Following the procedure of Example 46(j) except substituting phenylacetic acid for 3-isoquinolinecarboxylic acid gave the title compound: MS(ES+) 567 ($MH^+$).

b.) 4-[[N$^{\alpha}$-(2-phenylacetyl)-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 87(a), the title compound was produced: MS(ES+) 565 ($MH^+$).

Example 88

Preparation of 4-[[N$^{\alpha}$-(*tert*-butoxyoxycarbonyl)-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 46(h), the title compound was produced: MS(ES+) 547 ($MH^+$).

Example 89

Preparation of 4-[(L-leucinyl)amino]-1-[(2S)-4-methyl-2-[[(benzyloxycarbonyl)]amino]pentyl]-3-pyrrolidinone hydrochloride

Following the procedure of Example 46(i) except substituting the material of Example 88, the title compound was produced: MS(ES+) 447 ($MH^+$).

Example 90

Preparation of 4-[[N$^{\alpha}$-(2-quinolinecarbonyl)-L-leucinyl]amino]-1-[(2S)-4-methyl-2-[[(benzyloxycarbonyl)]amino]pentyl]-3-pyrrolidinone

a.) (3RS,4RS)-4-[[N$^{\alpha}$-(2-quinolinecarbonyl)-L-leucinyl]amino]-1-[(2S)-4-methyl-2-[[(benzyloxycarbonyl)]amino]pentyl]-3-pyrrolidinol

Following the procedure of Example 46(j) except substituting quinaldic acid for 3-isoquinolinecarboxylic acid, the title compound was produced: MS(ES+) 604 ($MH^+$).

b.) 4-[[N$^{\alpha}$-(2-quinolinecarbonyl)-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 90(a), the title compound was produced: MS(ES+) 602 ($MH^+$).

Example 91

Preparation of 4-[[N$^{\alpha}$-(piperonylcarbonyl)-L-leucinyl]amino]-1-[(2S)-4-methyl-2-[[(benzyloxycarbonyl)]amino]pentyl]-3-pyrrolidinone

a.) (3RS,4RS)-4-[[N$^{\alpha}$-(piperonylcarbonyl)-L-leucinyl]amino]-1-[(2S)-4-methyl-2-[[(benzyloxycarbonyl)]amino]pentyl]-3-pyrrolidinol

Following the procedure of Example 46(j) except substituting piperonylic acid for 3-isoquinolinecarboxylic acid, the title compound was produced: MS(ES+) 597 ($MH^+$).

b.) 4-[[N$^{\alpha}$-(piperonylcarbonyl)-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 91(a), the title compound was produced: MS(ES+) 595 ($MH^+$).

Example 92

Preparation of 4-[[N$^{\alpha}$-(4-fluorobenzoyl)-L-leucinyl]amino]-1-[(2S)-4-methyl-2-[[(benzyloxycarbonyl)]amino]pentyl]-3-pyrrolidinone

a.) (3RS,4RS)-4-[[N$^{\alpha}$-(4-fluorobenzoyl)-L-leucinyl]amino]-1-[(2S)-4-methyl-2-[[(benzyloxycarbonyl)]amino]pentyl]-3-pyrrolidinol

Following the procedure of Example 46(j) except substituting 4-fluorobenzoic acid for 3-isoquinolinecarboxylic acid, the title compound was produced: MS(ES+) 571 ($MH^+$).

b.) 4-[[N$^{\alpha}$-(4-fluorobenzoyl)-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 92(a), the title compound was produced: MS(ES+) 569 ($MH^+$).

Example 93

Preparation of 4-[[N$^{\alpha}$-(2-pyridylcarbonyl)-L-leucinyl]amino]-1-[(2S)-4-methyl-2-[[(benzyloxycarbonyl)]amino]pentyl]-3-pyrrolidinone

a.) (3RS,4RS)-4-[[N$^{\alpha}$-(2-pyridylcarbonyl)-L-leucinyl]amino]-1-[(2S)-4-methyl-2-[[(benzyloxycarbonyl)]amino]pentyl]-3-pyrrolidinol

Following the procedure of Example 46(j) except substituting picolinic acid for 3-isoquinolinecarboxylic acid, the title compound was produced: MS(ES+) 554 ($MH^+$).

b.) 4-[[N$^{\alpha}$-(2-pyridylcarbonyl)-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 93(a), the title compound was produced: MS(ES+) 552 ($MH^+$).

Example 94

Preparation of 4-[[N$^{\alpha}$-(2-nitro-$\alpha$-toluenesulphonyl)-L-leucinyl]amino]-1-[(2S)-4-methyl-2-[[(benzyloxycarbonyl)]amino]pentyl]-3-pyrrolidinone

a.) (3RS,4RS)-4-[[N$^{\alpha}$-(2-nitro-$\alpha$-toluenesulphonyl)-L-leucinyl]amino]-1-[(2S)-4-methyl-2-[[(benzyloxycarbonyl)]amino]pentyl]-3-pyrrolidinol

To a solution of Example 46(i) (250 mg, 0.48 mmol) in $CH_2Cl_2$ (10 mL) was added TEA (0.23 mL, 1.68 mmol) followed by 2-nitro-$\alpha$-toluenesulfonyl chloride (119 mg, 0.5 mmol). The reaction was stirred until complete by TLC analysis. Workup and column chromatography (10% $CH_3OH/CH_2Cl_2$) gave 95 mg of the title compound: MS(ES+) 648 ($MH^+$).

b.) 4-[[N$^{\alpha}$-(2-nitro-$\alpha$-toluenesulphonyl)-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 94(a), the title compound was produced: MS(ES+) 646 ($MH^+$).

Example 95

Preparation of 4-[[N$^{\alpha}$-(8-quinolinesulphonyl)-L-leucinyl]amino]-1-[(2S)-4-methyl-2-[[(benzyloxycarbonyl)]amino] pentyl]-3-pyrrolidinone

a.) (3RS,4RS)-4-[[N$^{\alpha}$-(8-quinolinesulphonyl)-L-leucinyl]amino]-1-[(2S)-4-methyl-2-[[(benzyloxycarbonyl)]amino] pentyl]-3-pyrrolidinol

To a solution of the compound of Example 46(i) (274 mg, 0.53 mmol) in $CH_2Cl_2$ (10 mL) was added TEA (0.26 mL, 1.84 mmol) followed by 8-quinolinesulfonyl chloride (125 mg, 0.55 mmol). The reaction was stirred until complete by TLC analysis. Workup and column chromatography (10% $CH_3OH/CH_2Cl_2$) gave 95 mg of the title compound: MS (ES+) 640 ($MH^+$).

b.) 4-[[N$^{\alpha}$-(8-quinolinesulphonyl)-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 95(a), the title compound was produced: MS(ES+) 638 ($MH^+$).

Example 96

Preparation of 4-[[N$^{\alpha}$-(2-naphthylcarbonyl)-L-leucinyl]aminomethyl]-1-[(2S)-4-methyl-2-[[(benzyloxycarbonyl)]amino] pentyl]-3-pyrrolidinone

a.) (3RS,4RS)-4-[[N$^{\alpha}$-(2-naphthylcarbonyl)-L-leucinyl]aminomethyl]-1-[(2S)-4-methyl-2-[[(benzyloxycarbonyl)]amino] pentyl]-3-pyrrolidinol

Following the procedure of Example 46(e-j) except substituting N-$CH_3$-N-BOC-L-leucine for BOC-L-leucine and naphthoic acid for 3-isoquinolinecarboxylic acid, the title compound was produced: MS(ES+) 617 ($MH^+$).

b.) 4-[[N$^{\alpha}$-(2-naphthylcarbonyl)-L-leucinyl]aminomethyl]-1-[(2S)-4-methyl-2-[[(benzyloxycarbonyl)]amino]pentyl]-3-pyrrolidinone

Following the procedure of example 46(k) except substituting the compound of Example 96(a), the title compound was produced: MS(ES+) 615 ($MH^+$).

Example 97

Preparation of 4-[[N$^{\alpha}$-(2-quinolinylcarbonyl)-L-leucinyl]aminomethyl]-1-[(2S)-4-methyl-2-[[(benzyloxycarbonyl)]amino] pentyl]-3-pyrrolidinone

a.) (3RS,4RS)-4-[[N$^{\alpha}$-(2-quinolinylcarbonyl)-L-leucinyl]aminomethyl]-1-[(2S)-4-methyl-2-[[(benzyloxycarbonyl)]amino] pentyl]-3-pyrrolidinol

Following the procedure of Example 46(e-j) except substituting N-$CH_3$-N-BOC-L-leucine for N-BOC-L-leucine and quinaidic acid for 3-isoquinolinecarboxylic acid, the title compound was produced: MS(ES+) 616 ($MH^+$).

b.) 4-[[N$^{\alpha}$-(2-quinolinylcarbanyl)-L-leucinyl]aminomethyl]-1-[(2S)-4-methyl-2-[[(benzyloxycarbonyl)]amino]pentyl]-3-pyrrolidinone

Following the procedure of Example 46(k) except substituting the compund of Example 97(a), the title compound was produced: MS(ES+) 614 ($MH^+$).

Example 98

Preparation of 4-[[N$^{\alpha}$-(phenylacetyl)-L-leucinyl]amino]-1-[(2S)-4-methyl-2-[[(benzyloxycarbonyl)]amino]pentyl]-3-piperidinone

a.) 1-benzyloxycarbonyl-1,2,3,6-tetrahydropyridine

To a solution of 1,2,3,6-tetrahydropyridine (5.4 g, 65 mmol) in $CH_2Cl_2$ (200 mL) at 0°C was added TEA (10 mL, 71.5 mmol) followed by benzyl chloroformate (9.8 mL, 68.3 mmol) in $CH_2Cl_2$ (50 mL) dropwise. The reaction was stirred at 0°C for 1h, room temperature for 1h whereupon it was diluted with $CH_2Cl_2$, washed with 1N HCl, water, brine, dried ($MgSO_4$) and concentrated. The residue was chromatographed (40:60 $CH_2Cl_2$:hexane) to give 8.0 g of title compound: MS(ES+) 218($MH^+$).

b.) 1-benzyloxycarbonyl-3,4-epoxy-piperidine

To a solution of the compound of Example 98(a) (8 g, 36.9 mmol) in $CH_2Cl_2$ (200 mL) was added m-CPBA (19 g, 111 mmol). The reaction was stirred at room temperature overnight whereupon it was concentrated and filtered with petroleum ether. The orangic layer was washed with saturated $K_2CO_3$ (3 times), water, brine, dried ($MgSO_4$) and concentrated to give a clear colorless oil which was used directly in the next step. MS(ES+) 256(M+Na).

c.) 1-benzyloxycarbonyl-3-hydroxy-4-azido-piperidine

To a solution of the compound from Example 98(b) (8.6 g, 36.9 mmol) in methanol: water (200 mL of an 8:1 solution) was added ammonium chloride (4.0 g, 73.8 mmol) and sodium azide (4.8 g, 73.8 mmol). The reaction was heated at 50°C for 3h whereupon it was concentrated, diluted with ethyl acetate and washed sequentially with pH 4 buffer, saturated $NaHCO_3$, water and brine. The organic layer was dried ($MgSO_4$), filtered and concentrated to give the title compound: $^1H$ NMR (400 MHz, $CDCl_3$) 7.35(m, 5H), 5.1(s, 2H), 4.2(m, 1H), 3.4(m, 1H), 2.3-2.9 (m, 6H).

d.) 1-benzyloxycarbonyl-3-hydroxy-4-amino-piperidine

To a solution of the compound of Example 98(c) (10 g, 36.9 mmol) in $CH_3OH$ (200 mL) was added triethylamine (15.4 mL, 110.7 mmol) followed by 1,3-propanethiol (11.0 mL, 10.7 mmol). The reaction was stirred at room temperature overnight whereupon it was concentrated and purified by column chromatography (20:80 methanol: ethyl acetate) to give 3 g of the title compound: MS(ES+) 251 ($MH^+$).

e.) (3RS,4RS)-4-[[N$^{\alpha}$-(*tert*-butoxycarbonyl)-L-leucinyl]amino]-1-benzyloxycarbonyl-3-piperidinol

To a solution of the compound of Example 98(d) (3.0 g, 12 mmol) in $CH_2Cl_2$ (300 mL) was added BOC-L-leucine (3.1 g, 12.6 mmol), HOBT (1.7 g, 12.6 mmol) and EDC (2.8 g, 15 mmol). The reaction was stirred at room temperature overnight whereupon it was diluted with $CH_2Cl_2$ and washed 0.5 N HCl, sat'd $NaHCO_3$, water and brine. The organic layer was dried ($MgSO_4$), filtered and concentrated. Column chromatography of the residue (5:95 MeOH: $CH_2Cl_2$) gave 4.8 g of the title compound: MS(ES+) 464 ($MH^+$).

f.) (3RS,4RS)-4-[[N$^{\alpha}$-(*tert*-butoxycarbonyl)-L-leucinyl]amino]-3-piperidinol

To a solution of the compound of Example 98(e) (3 g, 6.47mmol) in methanol:ethyl acetate (100 mL of a 1:2 solution) was added 10% Pd/C. The mixture shaken with a Parr hydrogenator at approximately 45 psi for 2h. The reaction was filtered through a pad of celite with $CH_2Cl_2$ and concentrated to give 2 g of the title compound: MS(ES+) 330 ($MH^+$).

h.) (3RS,4RS)-4-[[N$^{\alpha}$-(*tert*-butoxycarbonyl)-L-leucinyl]amino]-1-[(2S)-4-methyl-2-[[(benzyloxycarbonyl)]amino]pentyl]-3-piperidinol

To a solution of the compound of Example 98(f) (1.6 g, 4.86 mmol) in $CH_2Cl_2$ (200 mL) was added CBZ-leucinal (1.8 g, 7.29 mmol). The reaction was allowed to stir at room temperature for 0.5 h whereupon sodium triacetoxyborohydride (2.1 g, 9.72 mmol) was added. The reaction was stirred at room temperature for 2h whereupon it was diluted with ethyl acetate, washed with sat'd $NaHCO_3$, brine, dried ($Na_2SO_4$) , filtered and concentrated. Column chromatography of the the residue (5:95 methanol:$CH_2Cl_2$) gave 2 g of the title compound: MS(ES+) 563 ($MH^+$).

i.) (3RS,4RS)-4-[(L-leucinyl)amino]-1-[(2S)-4-methyl-2-[[(benzyloxycarbonyl)]amino]pentyl]-3-piperidinol hydrochloride

To a solution of the compound of Example 98(h) (2 g, 3.6 mmol) in methanol (50 mL) was added 4M HCl in dioxane (50 mL). The reaction was stirred at room overnight whereupon it was concentrated to give 2.2 g of the title compound: MS(ES+) 463 ($MH^+$).

j.) (3RS,4RS)-4-[[$N^\alpha$-(phenylacetyl)-L-leucinyl]amino]-1-[(2S)-4-methyl-2-[[(benzyloxycarbonyl)]amino]pentyl]-3-piperidinol

To a solution of the compound of Example 98(i) (337 mg, 0.63 mmol) in $CH_2Cl_2$ (10 mL) was added TEA (0.22 mL, 1.58 mmol) followed by phenylacetic acid (90 mg, 0.66 mmol), EDC (151 mg, 0.78 mmol) and HOBT (89 mg, 0.66 mmol). The reaction was stirred until complete by TLC analysis. Workup and column chromatography (5% $CH_3OH$/$CH_2Cl_2$) gave 273 mg of the title compound: MS(ES+) 581 ($MH^+$).

k.) 4-[[$N^\alpha$-(phenylacetyl)-L-leucinyl]amino]-1-[(2S)-4-methyl-2-[[(benzyloxycarbonyl)]amino]pentyl]-3-piperidinone

To a solution of the compound of Example 98(j) (270 mg, 0.46 mmol) in DMSO (2.5 mL) was added TEA (0.39 mL, 2.8 mmol) and sulphur trioxide pyridine complex (223 mg, 1.4 mmol). The reaction was stirred at room temperature for 1h whereupon it was partitioned between ethyl acetate and sat'd $NaHCO_3$. The organic layer was washed with brine, dried ($Na_2SO_4$), concentrated and the residue chromatographed (5% $CH_3OH$/$CH_2Cl_2$) to give 220 mg of the title compound: MS(ES+) 579 ($MH^+$).

Example 99

Preparation of 4-[[$N^\alpha$-(4-pyridinylmethoxycarbonyl)-L-leucinyl]amino]-1-[(2S)-4-methyl-2-[[(benzyloxycarbonyl)]amino]pentyl]-3-piperidinone

a.) (3RS,4RS)-4-[[$N^\alpha$-(4-pyrdinylmethoxycarbonyl)-L-leucinyl]amino]-1-*tert*-butoxycarbonyl-3-piperidinol

To a solution of the amino alcohol of Example 21(d) (320 mg, 1.48 mmol) in $CH_2Cl_2$ (20 mL) was added N-(4-pyridylmethoxycarbonyl)-L-leucine (415 mg, 1.85 mmol), HOBT (210 mg, 1.55mmol) and EDC (355mg, 1.85mmol). The reaction was allowed to stir at room temperature overnight whereupon it was diluted with $CH_2Cl_2$ and washed 0.5N HCl, Sat'd $NaHCO_3$, water and brine. The organic was dried ($MgSO_4$), filtered and concentrated. Column chromatography of the residue (10:90 MeOH:$CH_2Cl_2$) gave 573 mg of the title compound: MS(ES+) 465 (MH+).

b.) (3RS,4RS)-4-[[$N^\alpha$-(4-pyrdinylmethoxycarbonyl)-L-leucinyl]amino]-3-piperidinol bis-hydrochloride

To the compound of Example 99(a) (570 mg, 1.22 mmol) in methanol (10 mL) was added 4M HCl in dioxane (10 mL). The reaction was stirred at room overnight whereupon it was concentrated to give 536 mg of the title compound: MS(ES+) 365 ($MH^+$).

c.) (3RS,4RS)-4-[[$N^\alpha$-(4-pyridinylmethoxycarbonyl)-L-leucinyl]amino]-1-[(2S)-4-methyl-2-[[(benzyloxycarbonyl)]amino]pentyl]-3-piperidinol

Following the procedure of Example 46(h) except substituting the compound of Example 99(b), title compound was produced: MS(ES+) 598($MH^+$).

d.) 4-[[$N^\alpha$-(4-pyridinylmethoxycarbonyl)-L-leucinyl]amino]-1-[(2S)-4-methyl-2-[[(benzyloxycarbonyl)]amino]pentyl]-3-piperidinone

Following the procedure of Example 46(k) except substituting the compound of Example 99(c), the title compound was produced: MS(ES+) 596 ($MH^+$).

Example 100

Preparation of 4-[[N$^{\alpha}$-(4-pyridinylmethoxycarbonyl)-L-leucinyl]amino]-1-[(2R)-4-methyl-2-[[(benzyloxycarbonyl)]amino]pentyl]-3-piperidinone

a.) (3RS,4RS)-4-[[N$^{\alpha}$-(4-pyridinylmethoxycarbonyl)-L-leucinyl]amino]-1-[(2R)-4-methyl-2-[[(benzyloxycarbonyl)]amino]pentyl]-3-piperidinol

Following the procedure of Example 99(c) except substituting CBZ-D-leucinal for CBZ-L-leucinal, the title compound was produced: MS(ES+) 598 ($MH^+$).

b.) 4-[[N$^{\alpha}$-(4-pyridinylmethoxycarbonyl)-L-leucinyl]amino]-1-[(2R)-4-methyl-2-[[(benzyloxycarbonyl)]amino]pentyl]-3-piperidinone

Following the procedure of Example 46(k) except substituting the compound of Example 100(a), the title compound was produced: MS(ES+) 596 ($MH^+$).

Example 101

Preparation of 4-[[N$^{\alpha}$-(phenylacetyl)-L-leucinyl]amino]-1-[(2R)-4-methyl-2-[[(benzyloxycarbonyl)]amino]pentyl]-3-piperidinone

a.) (3RS,4RS)-4-[[N$^{\alpha}$-(phenylacetyl)-L-leucinyl]amino]-1-[(2R)-4-methyl-2-[[(benzyloxycarbonyl)]amino]pentyl]-3-piperidinol

Following the procedure of Example 98(h-j) except substituting CBZ-D-leucinal for CBZ-L-leucinal, the title compound was produced: MS(ES+) 581 ($MH^+$).

b.) 4-[[N$^{\alpha}$-(phenylacetyl)-L-leucinyl]amino]-1-[(2R)-4-methyl-2-[[(benzyloxycarbonyl)]amino]pentyl]-3-piperidinone

Following the procedure of Example 46(k) except substituting the compound of Example 101(a), the title compound was produced: MS(ES+) 579 ($MH^+$).

Example 102

Preparation of 4-[[N$^{\alpha}$-(4-imidazoleacetyl)-L-leucinyl]amino]-1-[(2R)-4-methyl-2-[[(benzyloxycarbonyl)]amino]pentyl]-3-piperidinone

a.) (3RS,4RS)-4-[[N$^{\alpha}$-(4-imidazoleacetyl)-L-leucinyl]amino]-1-[(2R)-4-methyl-2-[[(benzyloxycarbonyl)]amino]pentyl]-3-piperidinol

Following the procedure of Example 98(h-j) except substituting CBZ-D-leucinal for CBZ-L-leucinal and 4-imidazoleacetic acid for phenylacetic acid, the title compound was produced: MS(ES+) 571 ($MH^+$).

b.) 4-[[N$^{\alpha}$-(4-imidazoleacetyl)-L-leucinyl]amino]-1-[(2R)-4-methyl-2-[[(benzyloxycarbonyl)]amino]pentyl]-3-piperidinone

Following the procedure of Example 98(k) except substituting the compound of Example 102(a), the title compound was produced: MS(ES+) 570 ($MH^+$).

Example 103

Preparation of 4-[[N$^{\alpha}$-(4-imidazoleacetyl)-L-leucinyl]amino]-1-[(2S)-4-methyl-2-[[(benzyloxycarbonyl)]amino]pentyl]-3-piperidinone

a.) (3RS,4RS)-4-[[N$^{\alpha}$-(4-imidazoleacetyl)-L-leucinyl]amino]-1-[(2S)-4-methyl-2-[[(benzyloxycarbonyl)]amino]pentyl]-3-piperidinol

Following the procedure of Example 98(h-j) except substituting 4-imidazoleacetic acid for phenylacetic acid, the title compound was produced: MS(ES+) 571 ($MH^+$).

b.) 4-[[N$^{\alpha}$-(4-imidazoleacetyl)-L-leucinyl]amino]-1-[(2S)-4-methyl-2-[[(benzyloxycarbonyl)]amino]pentyl]-3-piperidinone

Following the procedure of Example 98(k) except substituting the compound of Example 103(a), the title compound was produced: MS(ES+) 570 ($MH^+$).

Example 104

Preparation of 4-[[N$^{\alpha}$-(4-pyridinylcarbonyl)-L-leucinyl]amino]-1-[(2S)-4-methyl-2-[[(benzyloxycarbonyl)]amino]pentyl]-3-piperidinone

a.) (3RS,4RS)-4-[[N$^{\alpha}$-(4-pyridinylcarbonyl)-L-leucinyl]amino]-1-[(2S)-4-methyl-2-[[(benzyloxycarbonyl)]amino]pentyl]-3-piperidinol

Following the procedure of Example 98(h-j) except substituting isonicotinic acid for phenylacetic acid, the title compound was prepared: MS(ES+) 568 (MH+).

b.) 4-[[N$^{\alpha}$-(4-pyridinylcarbonyl)-L-leucinyl]amino]-1-[(2S)-4-methyl-2-[[(benzyloxycarbonyl)]amino]pentyl]-3-piperidinone

Following the procedure of Example 98(k) except substituting the compound of Example 104(a), the title compound was produced: MS(ES+) 566 ($MH^+$).

Example 105

Preparation of 4-[[N$^{\alpha}$-(*tert*-butoxycarbonyl)-L-leucinyl]amino]-1-(benzyloxycarbonyl)-3-piperidinone

Following the procedure of Example 98(k) except substituting the material of Example 98(e), the title compound was produced: MS (ES) 462 ($MH^+$).

Example 106

Preparation of 4-[[N$^{\alpha}$-(8-quinolinesulphonyl)-L-leucinyl]amino]-1-(benzyloxycarbonyl)-3-piperidinone

a.) (3RS,4RS)-4-[(L-leucinyl)amino]-1-(benzyloxycarbonyl)-3-piperidinol hydrochloride

To the material of Example of 98(e) (1.5 g, 3.2 mmol) in methanol (10 mL) was added 4M HCl in dioxane (10 mL). The reaction was stirred at room overnight whereupon it was concentrated to give 1g of the title compound: MS (ES+) 364 ($MH^+$).

b.) (3RS,4RS)-4-[[N$^{\alpha}$-(8-quinolinesulphonyl)-L-leucinyl]amino]-1-(benzyloxycarbonyl)-3-piperidinol

To a solution of the compound of Example 106(a) (250 mg, 0.63 mmol) in $CH_2Cl_2$ (10 mL) was added TEA (0.22 mL, 1.58 mmol) followed by 8-quinolinesulfonyl chloride (150 mg, 0.66 mmol). The reaction was stirred until complete by TLC analysis. Workup and column chromatography (5% $CH_3OH/CH_2Cl_2$) gave 250 mg of the title compound: MS(ES+) 555 ($MH^+$).

c.) 4-[[N$^{\alpha}$-(8-quinolinesulphonyl)-L-leucinyl]amino]-1-(benzyloxycarbonyl)-3-piperidinone

Following the procedure of Example 98(k) except substituting the compound of Example 106(b), the title compound was produced: MS(ES+) 553 ($MH^+$).

Example 107

Preparation of 4-[[N$^{\alpha}$-(4-pyridinylacetyl)-L-leucinyl]amino]-1-(benzyloxycarbonyl)-3-piperidinone

a.) (3RS,4RS)-4-[[N$^{\alpha}$-(4-pyridinylacetyl)-L-leucinyl]amino]-1-(benzyloxycarbonyl)-3-piperidinol

To a solution of the material of Example 106(a) (250 mg, 0.63 mmol) in $CH_2Cl_2$ (10 mL) was added TEA (0.31 mL, 2.2 mmol) followed by 4-pyridylacetic acid hydrochloride (89 mg, 0.66 mmol), EDC (151mg, 0.78 mmol) and HOBT (89 mg, 0.66 mmol). The reaction was stirred until complete by TLC analysis. Workup and column chromatography (10% $CH_3OH/CH_2Cl_2$) gave 213 mg of the title compound: MS(ES+) 483 ($MH^+$).

b.) 4-[[N$^{\alpha}$-(4-pyridinylacetyl)-L-leucinyl]amino]-1-(benzyloxycarbonyl)-3-piperidinone

Following the procedure of Example 46(k) except substituting the material of Example 107(a), the title compound was produced: MS(ES+) 481 ($MH^+$).

Example 108

Preparation of 4-[[N$^{\alpha}$-(4-imidazoleacetyl)-L-leucinyl]amino]-1-(benzyloxycarbonyl)-3-pineridinone

a.) (3RS,4RS)-4-[[N$^{\alpha}$-(4-imidazoleacetyl)-L-leucinyl]amino]-1-(benzyloxycarbonyl)-3-piperidinol

To a solution of the material of Example 106(a) (250 mg, 0.63 mmol) in $CH_2Cl_2$ (10 mL) was added TEA (0.22 mL, 1.58 mmol) followed by 4-imidazoleacetic acid (107 mg, 0.66 mmol), EDC (151mg, 0.78 mmol) and HOBT (89 mg, 0.66 mmol). The reaction was stirred until complete by TLC analysis. Workup and column chromatography (10% $CH_3OH/CH_2Cl_2$) gave 213 mg of the title compound: MS(ES+) 472 ($MH^+$).

b.) 4-[[N$^{\alpha}$-(4-imidazoleacetyl)-L-leucinyl]amino]-1-(benzyloxycarbonyl)-3-piperidinone

Following the procedure of Example 46(k) except substituting the compound of Example 108(a), the title compound was produced: MS(ES+) 470 ($MH^+$).

Example 109

Preparation of 4-[[N$^{\alpha}$-(4-pyridinylcarbonyl)-L-leucinyl]amino]-1-(benzyloxycarbonyl)-3-piperidinone

a.) (3RS,4RS)-4-[[N$^{\alpha}$-(4-pyridinylcarbonyl)-L-leucinyl]amino]-1-(benzyloxycarbonyl)-3-piperidinol

To a solution of the material of Example 106(a) (100 mg, 0.25 mmol) in $CH_2Cl_2$ (10mL) was added TEA (0.05 mL, 0.38 mmol) followed by isonicotinic acid (33 mg, 0.26 mmol), EDC (60 mg, 0.31 mmol) and HOBT (35 mg, 0.26 mmol). The reaction was stirred until complete by TLC analysis. Workup and column chromatography (10% $CH_3OH/CH_2Cl_2$) gave 100 mg of the title compound: MS(ES+) 469 ($MH^+$).

b.) 4-[[N$^{\alpha}$-(4-pyridinylcarbonyl)-L-leucinyl]amino]-1-(benzyloxycarbonyl)-3-piperidinone

Following the procedure of Example 46(k) except substituting the material of Example 109(a), the title compound was produced: MS(ES+) 467 (MH+)

Example 110

Preparation of 1-benzyl-4-[[N$^{\alpha}$-(3-isoquinolinylcarbonyl)-L-leucinyl]amino]-3-pyrrolidinone

a.) (3RS,4RS)-1-benzyl-4-[[N'-(*tert*-butoxyoxycarbonyl)-L-leucinyl]amino]-3-pyrrolidinol

To a solution of compound of Example 46(f) (2 g, 6.34 mmol) in $CH_2Cl_2$ (100 mL) was added benzylaldehyde (0.82 mL, 7.6 mmol). The reaction was allowed to stir at room temperature for 0.5h whereupon sodium triacetoxyborohydride (3.36 g, 15.9 mmol) was added. The reaction was stirred at room temperature for 2 h whereupon it was diluted with ethyl acetate and washed with sat'd $NaHCO_3$, brine, dried ($Na_2SO_4$), filtered and concentrated. Column chromatography of the the residue (5:95 methanol: $CH_2Cl_2$) gave 2 g of the title compound: MS(ES+) 406 ($MH^+$).

b.) (3RS,4RS)-1-benzyl-4-[(L-leucinyl)amino]-3-pyrrolidinol bis-hydrochloride

To a solution of the compound of Example 110(a) (2 g, 4.9 mmol) in methanol (20 mL) was added 4M HCl in dioxane (20 mL). The reaction was stirred at room overnight whereupon it was concentrated to give 1.4 g of the title compound: MS(ES+) 306 ($MH^+$).

c.) (3RS,4RS)-1-benzyl-4-[[N$^{\alpha}$-(3-isoquinolinylcarbonyl)-L-leucinyl]amino]-3-pyrrolidinol

To a solution of the compound of Example 110(b) (250 mg, 0.66 mmol) in $CH_2Cl_2$ (10 mL) was added TEA (0.23 mL, 1.65 mmol) followed by 3-isoquinolinecarboxylic acid (132 mg, 0.69 mmol), EDC (158 mg, 0.82 mmol) and HOBT (94 mg, 0.69 mmol). The reaction was stirred until complete by TLC analysis. Workup and column chromatography (5% $CH_3OH/CH_2Cl_2$) gave 180 mg of the title compound: MS(ES+) 461 ($MH^+$).

d.) 1-benzyl-4-[[N$^{\alpha}$-(3-isoquinolinylcarbonyl)-L-leucinyl]amino]-3-pyrrolidinone

Following the procedure of Example 46(k) except substituting the compound of Example 110(c), the title compound was produced: MS(ES+) 459 ($MH^+$).

Example 111

Preparation of 1-benzyl-4-[[N$^{\alpha}$-(3,4-dichlorobenzoyl)-L-leucinyl]amino]-3-pyrrolidinone

a.) (3RS,4RS)-1-benzyl-4-[[N$^{\alpha}$-(3,4-dichlorobenzoyl)-L-leucinyl]amino]-3-pyrrolidinol

Following the procedure of Example 110(c) except substituting 3,4-dichlorobenzoic acid for 3-isoquinolinecarboxylic acid, the title compound was produced: MS(ES+) 478 ($MH^+$).

b.) 1-benzyl-4-[[N$^{\alpha}$-(3,4-dichlorobenzoyl)-L-leucinyl]amino]-3-pyrrolidinone

Following the procedure of Example 46(k) except substituting the compound of Example 111(a), the title compound was produced: MS(ES+) 476 ($MH^+$).

Example 112

Preparation of 1-benzyl-4-[[N$^{\alpha}$-(2-naphthylcarbonyl)-L-leucinyl]aminomethyl]-3-pyrrolidinone

a.) (3RS,4RS)-4-[[N$^{\alpha}$-(*tert*-butoxyoxycarbonyl)-L-leucinyl]aminomethyl]-3-pyrrolidinol

Following the procedure of Example 46(e) except substituting N-$CH_3$-BOC-L-leucine for BOC-L-leucine, the title compound was produced: MS(ES+) 464 (MH+).

b.) (3RS,4RS)-1-benzyl-4-[(L-leucinyl)aminomethyl]-3-pyrrolidinol

Following the procedure of Example 46(f) except substituting the compound of Example 112(a), the title compound was produced: MS(ES+) 330 ($MH^+$).

c.) (3RS,4RS)-1-benzyl-4-[[$N^{\alpha}$-(2-naphthylcarbonyl)-L-leucinyl]aminomethyl]-3-pyrrolidinol

Following the procedure of Example 110(a-c) except substituting the compound of Example 112(b) and substituting 2-naphthoic acid for 3-isoquinolinecarboxylic acid, the title compound was produced: MS(ES+) 474 ($MH^+$).

d.) 1-benzyl-4-[[$N^{\alpha}$-(2-naphthylcarbonyl)-L-leucinyl]aminomethyl]-3-pyrrolidinone

Following the procedure of Example 46(k) except substituting the compound of Example 112(c), the title compound was produced: MS(ES+) 472 ($MH^+$).

Example 113

Preparation of 1-benzyl-4-[[$N^{\alpha}$-(2-quinolinylcarbonyl)-L-leucinyl]aminomethyl]-3-pyrrolidinone

a.) (3RS,4RS)-1-benzyl-4-[[$N^{\alpha}$-(2-quinolinylcarbonyl)-L-leucinyl]aminomethyl]-3-pyrrolidinol

Following the procedure of Example 112(c) except substituting quinaldic acid for naphthoic acid, the title compound was produced: MS(ES+) 475 ($MH^+$).

b.) 1-benzyl-4-[[$N^{\alpha}$-(2-quinolinylcarbonyl)-L-leucinyl]aminomethyl]-3-pyrrolidinone

Following the procedure of Example 46(k) except substituting the compound of Example 113(b), the title compound was produced: MS(ES+) 473 ($MH^+$).

Example 114

Preparation of 1-benzyl-4-[[$N^{\alpha}$-(2-quinolinylcarbonyl)-L-leucinyl]amino]-3-pyrrolidinone

a.) (3RS,4RS)-1-benzyl-4-[[$N^{\alpha}$-(2-quinolinylcarbonyl)-L-leucinyl]amino]-3-pyrrolidinol

Following the procedure of Example 110(c) except substituting quinaldic acid for 3-isoquinolinecarboxylic acid, the title compound was produced: MS(ES+) 461 ($MH^+$),

b.) 1-benzyl-4-[[$N^{\alpha}$-(2-quinolinylcarbonyl)-L-leucinyl]amino]-3-pyrrolidinone

Following the procedure of Example 46(k) except substituting the compound of Example 114(a), the title compound was produced: MS(ES+) 459 ($MH^+$).

Example 115

Preparation of 1-benzyl-4-[[$N^{\alpha}$-(piperonylcarbonyl)-L-leucinyl]amino]-3-pyrrolidinone

a.) (3RS,4RS)-1-benzyl-4-[[$N^{\alpha}$-(piperonylcarbonyl)-L-leucinyl]amino]-3-pyrrolidinol

Following the procedure of Example 110(c) except substituting piperonylic acid for 3-isoquinoline carboxylic acid, the title compound was produced: MS(ES+) 454 ($MH^+$).

b.) 1-benzyl-4-[[$N^{\alpha}$-(piperonylcarbonyl)-L-leucinyl]amino]-3-pyrrolidinone

Following the procedure of Example 46(k) except substituting the compund of Example 115(a), the title compound was produced: MS(ES+) 452 ($MH^+$).

Example 116

Preparation of 1-benzyl-4-[[Nα-(4-fluorobenzoyl)-L-leucinyl]amino]-3-pyrrolidinone

a.) (3RS,4RS)-1-benzyl-4-[[Nα-(4-fluorobenzoyl)-L-leucinyl]amino]-3-pyrrolidinol

Following the procedure of Example 110(c) except substituting 4-fluorobenzoic acid for 3-isoquinolinecarboxylic acid, the title compound was produced: MS(ES+) 428 ($MH^+$).

b.) 1-benzyl-4-[[Nα-(4-fluorobenzoyl)-L-leucinyl]amino]-3-pyrrolidinone

Following the procedure of Example 46(k) except substituting the compound of Example 116(a), the title compound was produced: MS(ES+) 426 (MH+).

Example 117

Preparation of 1-benzyl-4-[[Nα-(6-hydroxy-2-naphthylcarbonyl)-L-leucinyl]amino]-3-pyrrolidinone

a.) (3RS,4RS)-1-benzyl-4-[[Nα-(6-hydroxy-2-naphthylcarbonyl)-L-leucinyl]amino]-3-pyrrolidinol

Followed the procedure of Example 110(c) except substituting 6-hydroxy-2-naphthoic acid for 3-isoquinolinecarboxylic acid, the title compound was produced: MS(ES+) 476 ($MH^+$).

b.) 1-benzyl-4-[[Nα-(6-hydroxy-2-naphthylcarbonyl)-L-leucinyl]amino]-3-pyrrolidinone

Following the procedure of Example 46(k) except substituting the compound of Example 117(a), the title compound was produced: MS(ES+) 474 (MH+).

Example 118

Preparation of 1-benzyl-4-[[Nα-(2-naphthylcarbonyl)-L-leucinyl]amino]-3-pyrrolidinone

a.) (3RS,4RS)-1-benzyl-4-[[Nα-(2-naphthylcarbonyl)-L-leucinyl]amino]-3-pyrrolidinol

Following the procedure of Example 110(c) except substituting 2-naphthoic acid for 3-isoquinolinecarboxylic acid, the title compound was produced: MS(ES+) 460 ($MH^+$).

b.) 1-benzyl-4-[[Nα-(2-naphthylcarbonyl)-L-leucinyl]amino]-3-pyrrolidinone

Following the procedure of Example 46(k) except substituting the compound of Example 118(a), the title compound was produced: MS(ES+) 458 ($MH^+$).

Example 119

Preparation of 1-benzyl-4-[[Nα-(6-quinolinylcarbonyl)-L-leucinyl]amino]-3-pyrrolidinone

a.) (3RS,4RS)-1-benzyl-4-[[Nα-(6-quinolinylcarbonyl)-L-leucinyl]amino]-3-pyrrolidinol

Following the procedure of Example 110(c) except substituting 6-quinolinecarboxylic acid for 3-isoquinolinecarboxylic acid, the title compound was produced: MS(ES+) 461 ($MH^+$).

b.) 1-benzyl-4-[[Nα-(6-quinolinylcarbonyl)-L-leucinyl]amino]-3-pyrrolidinone

Following the procedure of Example 46(k) except substituting the compound of Example 119(a), the title compound was produced: MS(ES+) 459 ($MH^+$).

Example 120

Preparation of 1-benzyl-4-[[N$^{\alpha}$-(4-imidazoleacetyl)-L-leucinyl]amino]-3-pyrrolidinone

a.) (3RS,4RS)-1-benzyl-4-[[N$^{\alpha}$-(4-imidazoleacetyl)-L-leucinyl]amino]-3-pyrrolidinol

Following the procedure of Example 110(c) except substituting 4-imidazoleacetic acid hydrochloride for 3-isoquinolinecarboxylic acid, the title compound was produced: MS(ES+) 414 ($MH^+$).

b.) 1-benzyl-4-[[N$^{\alpha}$-(4-imidazoleacetyl)-L-leucinyl]amino]-3-pyrrolidinone

Following the procedure of Example 46(k) except substituting the compound of Example 120(a), the title compound was produced: MS(ES) 412 ($MH^+$).

Example 121

Preparation of 1-benzyl-4-[[N$^{\alpha}$-(4-pyridinylcarbonyl)-L-leucinyl]amino]-3-pyrrolidinone

a.) (3RS,4RS)-1-benzyl-4-[[N$^{\alpha}$-(4-pyridinylcarbonyl)-L-leucinyl]amino]-3-pyrrolidinol

Following the procedure of Example 110(c) except substituting isonicotinic acid for 3-isoquinolinecarboxylic acid, the title compound was produced: MS(ES+) 411 ($MH^+$).

b.) 1-benzyl-4-[[N$^{\alpha}$-(4-pyridinylcarbonyl)-L-leucinyl]amino]-3-pyrrolidinone

Following the procedure of Example 46(k) except substituting the compound of Example 121(a), the title compound was produced: MS(ES+) 409 ($MH^+$).

Example 122

Preparation of 4-[[N$^{\alpha}$-(*tert*-butoxycarbonyl)-L-leucinyl]amino]-1-benzyloxycarbonyl-3-pyrrolidinone

Following the procedure of Example 46(k) except sustituting the compound of Example 46(e), the title compound was produced: MS(ES) 448 ($MH^+$).

Example 123

Preparation of 4-[[N$^{\alpha}$-(4-pyridinylmethoxy)carbonyl]-L-leucinyl]amino]-1-[(2R)-4-methyl-2-[[(benzyloxycarbonyl)]amino]pentyl]-3-pyrrolidinone

a.) (3RS,4RS)-4-[[N$^{\alpha}$-(4-pyridinylmethoxy)carbonyl]-L-leucinyl]amino]-1-[(2R)-4-methyl-2-[[(benzyloxycarbonyl)]amino]pentyl]-3-pyrrolidinol

Following the procedure of Example 46(h) except substituting the compound of Example 47(b) and also substituting CBZ-D-leucinal for CBZ-leucinal, the title compound was produced: MS(ES) 584 ($MH^+$).

b.) 4-[[N$^{\alpha}$-(4-pyridinylmethoxy)carbonyl]-L-leucinyl]amino]-1-[(2R)-4-methyl-2-[[(benzyloxycarbonyl)]amino]pentyl]-3-pyrrolidinone

Following the procedure of Example 46(k) except substituting the compound of Example 123(a), the title compound was produced: MS(ES+) 582 ($MH^+$).

Example 124

Preparation of 4-[[N$^{\alpha}$-(4-pyridinylmethoxy)carbonyl]-L-leucinyl]amino]-1-[(2S)-4-methyl-2-[[(*tert*-butoxycarbonyl)]amino]pentyl]-3-pyrrolidinone

a.) (3RS,4RS)-4-[[N$^{\alpha}$-(4-pyridinylmethoxy)carbonyl]-L-leucinyl]amino]-1-[(2S)-4-methyl-2-[[(*tert*-butoxycarbonyl)]amino]pentyl]-3-pyrrolidinol

Following the procedure of Example 123(a) except substituting BOC-L-leucinal for CBZ-D-leucinal, the title compound was produced: MS(ES) 550 ($MH^+$).

b.) 4-[[N$^{\alpha}$-(4-pyridinylmethoxy)carbonyl]-L-leucinyl]amino]-1-[(2S)-4-methyl-2-[[(*tert*-butoxycarbonyl)]amino]pentyl]-3-pyrrolidinone

Following the procedure of Example 46(k) except substituting the compound of Example 124(a), the title compound was produced: MS(ES+) 548 ($MH^+$).

Example 125

Preparation of 4-[[N$^{\alpha}$-(4-pyridinylmethoxy)carbonyl]-L-leucinyl]amino]-1-(2S)-4-methyl-2-(amino)pentyl]-3-pyrrolidinone bis-hydrochloride

Following the procedure of Example 46(i) except sustituting the material of Example 124(b), the title compound was produced: MS(ES+) 448 ($MH^+$).

Example 126

Preparation of 4-[[N$^{\alpha}$-(2-methylpropoxy)carbonyl]-L-leucinyl]amino]-1-[(2S)-4-methyl-2-[[(benzyloxycarbonyl)]amino]pentyl]-3-pyrrolidinone

a.) (3RS,4RS)-4-[[N$^{\alpha}$-(2-methylpropoxy)carbonyl]-L-leucinyl]amino]-1-[(2S)-4-methyl-2-[[(benzyloxycarbonyl)]amino]pentyl]-3-pyrrolidinol

To a solution of the compound of Example 46(i) (274 mg, 0.53 mmol) in $CH_2Cl_2$ (10 mL) was added TEA (0.26 mL, 1.84 mmol) followed by isobutyl chloroformate (0.075 mL, 0.55 mmol). The reaction was stirred until complete by TLC analysis. Workup and column chromatography (10% $CH_3OH/CH_2Cl_2$) gave 78 mg of the title compound: MS(ES+) 549 ($MH^+$).

b.) 4-[[N$^{\alpha}$-(2-methylpropoxy)carbonyl]-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 126(a), the title compound was produced: MS(ES+) 547 ($MH^+$).

Example 127

Preparation of 4-[[N$^{\alpha}$-(methylamino)thiocarbonyl]-L-leucinyl]amino]-1-[(2S)-4-methyl-2-[[(benzyloxycarbonyl)]amino]pentyl]-3-pyrrolidinone

a.) (3RS,4RS)-4-[[N$^{\alpha}$-(methylamino)thiocarbonyl]-L-leucinyl]amino]-1-[(2S)-4-methyl-2-[[(benzyloxycarbonyl)]amino]pentyl]-3-pyrrolidinol

To a solution of the compound of Example 46(i) (250 mg, 0.48 mmol) in $CH_2Cl_2$ was added TEA (0.14 mL, 1 mmol) followed by methyl isothiocyanate (0.03 mL, 0.5 mmol). The reaction was stirred until complete by TLC analysis. Workup and column chromatography (10% $CH_3OH/CH_2Cl_2$) gave 59 mg of the title compound: MS(ES+) 522 ($MH^+$).

b.) 4-[[N$^{\alpha}$-(methylamino)thiocarbonyl]-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 127(a), the title compound was produced: MS(ES+) 520 ($MH^+$).

Example 128

Preparation of 4-[[N$^{\alpha}$-(phenylmethylamino)carbonyl]-L-leucinyl]amino]-1-[(2S)-4-methyl-2-[[(benzyloxycarbonyl)]amino]pentyl]-3-pyrrolidinone

a.) (3RS,4RS)-4-[[N$^{\alpha}$-(phenylmethylamino)carbonyl]-L-leucinyl]amino]-1-[(2S)-4-methyl-2-[[(benzyloxycarbonyl)]amino]pentyl]-3-pyrrolidinol

To a solution of the compound of Example 46(i) (250 mg, 0.48 mmol) in $CH_2Cl_2$ (10 mL) was added TEA (0.14 mL, 1 mmol) followed by benzyl isocyanate (0.06 mL, 0.5 mmol). The reaction was stirred until complete by TLC analysis. Workup and column chromatography (10% $CH_3OH/CH_2Cl_2$) gave 59 mg of the title compound: MS(ES+) 582 ($MH^+$).

b.) 4-[[N$^{\alpha}$-(phenylmethylamino)carbonyl]-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 128(a), the title compound was produced: MS(ES+) 580 ($MH^+$).

Example 129

Preparation of 4-[[N$^{\alpha}$-(3,4-dichlorophenylamino)carbonyl]-L-leucinyl]aminol-1-[(2S)-4-methyl-2-[[(benzyloxycarbonyl)]amino]pentyl]-3-pyrrolidinone

a.) (3RS,4RS)-4-[[N$^{\alpha}$-(3,4-dichlorophenylamino)carbonyl]-L-leucinyl]amino]-1-[(2S)-4-methyl-2-[[(benzyloxycarbonyl)]amino]pentyl]-3-pyrrolidinol

To a solution of the compound of Example 46(i) (250 mg, 0.48 mmol) in $CH_2Cl_2$ (10 mL) was added TEA (0.14 mL, 1 mmol) followed by 3,4-dichlorophenyl isocyanate (95 mg, 0.5 mmol). The reaction was stirred until complete by TLC analysis. Workup and column chromatography (10% $CH_3OH/CH_2Cl_2$) gave 240 mg of the title compound: MS (ES+) 636 ($MH^+$).

b.) 4-[[N$^{\alpha}$-(3,4-dichlorophenylamino)carbonyl]-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 129(a), the title compound was produced: MS(ES+) 634 ($MH^+$).

Example 130

Preparation of 1-benzyl-4-[[N$^{\alpha}$-(3,4-dichlorophenylamino)-L-leucinyl]amino]-3-pyrrolidinone

a.) (3RS,4RS)-1-benzyl-4-[[N$^{\alpha}$-(3,4-dichlorophenylamino)-L-leucinyl]amino]-3-pyrrolidinol

To a solution of the compound of Example 110(b) (250 mg, 0.66 mmol) in $CH_2Cl_2$ (10 mL) was added TEA (0.19 mL, 1.3 mmol) followed by 3,4-dichlorophenyl isocyanate (130 mg, 0.69 mmol). The reaction was stirred until complete by TLC analysis. Workup and column chromatography (10% $CH_3OH/CH_2Cl_2$) gave 240 mg of the title compound: MS(ES+) 493 ($MH^+$).

b.) 1-benzyl-4-[[N$^{\alpha}$-(3,4-dichlorophenylamino)-L-leucinyl]amino]-3-pyrrolidinone

Following the procedure of Example 46(k) except substituting the compound of Example 130(a), the title

compound was produced: MS (ES) 491 ($MH^+$).

Example 131

Preparation of 4-[[$N^\alpha$-(1,2,3,4-tetrahydro-6-quinolinecarbonyl)-L-leucinyl]amino]-1-[(2S)-4-methyl-2-[[(p-toluenesulphonyl)]amino]pentyl]-3-pyrrolidinone

a.) (3RS,4RS)-4-[[$N^\alpha$-(1,2,3,4-tetrahydro-6-quinolinecarbonyl)-L-leucinyl]amino]-1-[(2S)-4-methyl-2-aminopentyl]-3-pyrrolidinol

To a solution of the compound of Example 65(a) (2.2 g, 3.6 mmol) in methanol:ethyl acetate (200 mL of a 1:2 solution) was added 10% Pd on carbon. The mixture was shaken on a Parr hydrogenator for 2h at approximately 40 psi. The reaction was filtered through a pad of celite with $CH_2Cl_2$ and concentrated to give 1.73 g of the title compound: MS(ES+) 474 ($MH^+$).

b.) (3RS,4RS)-4-[[$N^\alpha$-(1,2,3,4-tetrahydro-6-quinolinecarbonyl)-L-leucinyl]amino]-1-[(2S)-4-methyl-2-[(p-toluenesulphonyl)amino]pentyl]-3-pyrrolidinol

To a solution of the material from Example 131(a) (300 mg, 0.63 mmol) in $CH_2Cl_2$ (20 mL) was added TEA (0.1 mL, 0.69 mmol) followed by p-toluenesulfonyl chloride (127 mg, 0.66 mmol). The reaction was stirred until complete by TLC analysis. Workup and column chromatography (5% $CH_3OH/CH_2Cl_2$) gave 260 mg of the title compound: MS (ES+) 628 ($MH^+$).

c.) 4-[[$N^\alpha$-(1,2,3,4-tetrahydro-6-quinolinecarbonyl)-L-leucinyl]amino]-1-[(2S)-4-methyl-2-[(p-toluenesulphonyl)amino]pentyl]-3-pyrrolidinone

Following the procedure of Example 46(k) except substituting the compound of Example 131(b), the title compound was produced: MS(ES+) 626 ($MH^+$).

Example 132

Preparation of 4-[[$N^\alpha$-(1,2,3,4-tetrahydro-6-quinolinecarbonyl)-L-leucinyl]amino]-1-[(2S)-4-methyl-2-[[(acetyl)]amino]pentyl]-3-pyrrolidinone

a.) (3RS,4RS)-4-[[$N^\alpha$-(1,2,3,4-tetrahydro-6-quinolinecarbonyl)-L-leucinyl]amino]-1-[(2S)-4-methyl-2-[(acetyl)amino]pentyl]-3-pyrrolidinol

Following the procedure of Example 131(b) except substituting acetyl chloride for p-toluenesulfonyl chloride, the title compound was prepared: MS(ES+) 516 ($MH^+$).

b.) 4-[[$N^\alpha$-(1,2,3,4-tetrahydro-6-quinolinecarbonyl)-L-leucinyl]amino]-1-[(2S)-4-methyl-2-[(acetyl)amino]pentyl]-3-pyrrolidinone

Following the procedure of Example 46(k) except substituting the compound of Example 132(a), the title compound was produced: MS(ES+) 514 ($MH^+$).

Example 133

Preparation of 4-[[$N^\alpha$-(4-fluorobenzoyl)-L-leucinyl]amino]-1-[(2S)-4-methyl-2-[(acetyl)amino]pentyl]-3-pyrrolidinone

a.) (3RS,4RS)-4-[[$N^\alpha$-(4-fluorobenzoyl)-L-leucinyl]amino]-1-[(2S)-4-methyl-2-aminopentyl]-3-pyrrolidinol

To a solution of the compound of Example 92(a) (1.9 g, 3.3mmol) in methanol:ethyl acetate (200 mL of a 1:2 solution) was added 10% Pd on carbon. The mixture was shaken on a Parr hydrogenator for 2h at approximately 45 psi. The reaction was filtered through a pad of celite with $CH_2Cl_2$ and concentrated to give 1.2 g of the title compound: MS(ES+) 437 ($MH^+$).

b.) (3RS,4RS)-4-[[N$^{\alpha}$-(4-fluorobenzoyl)-L-leucinyl]amino]-1-[(2S)-4-methyl-2-[(acetyl)amino]pentyl]-3-pyrrolidinol

To a solution of the material of Example 133(a) (250 mg, 0.57 mmol) in $CH_2Cl_2$ (20 mL) was added TEA (0.1 mL, 0.63 mmol) followed by acetyl chloride (0.04 mL, 0.6 mmol). The reaction was stirred until complete by TLC analysis. Workup and column chromatography (10% $CH_3OH/CH_2Cl_2$) gave 190 mg of the title compound: MS(ES+) 479 ($MH^+$).

c.) 4-[[N$^{\alpha}$-(4-fluorobenzoyl)-L-leucinyl]amino]-1-[(2S)-4-methyl-2-[(acetyl)amino]pentyl]-3-pyrrolidinone

Following the procedure of Example 46(k) except substituting the material from Example 133(b), the title compound was produced: MS(ES+) 477 ($MH^+$).

Example 134

Preparation of 4-[[N$^{\alpha}$-(4-fluorobenzoyl)-L-leucinyl]amino]-1-[(2S)-4-methyl-2-[(p-toluenesulphonyl)amino]pentyl]-3-pyrrolidinone

a.) (3RS,4RS)-4-[[N$^{\alpha}$-(4-fluorobenzoyl)-L-leucinyl]amino]-1-[(2S)-4-methyl-2-[(p-toluenesulphonyl)amino]pentyl]-3-pyrrolidinol

Following the procedure of Example 133(b) except substituting p-toluenesulfonyl chloride for acetyl chloride, the title compound was prepared: MS(ES+) 591 ($MH^+$).

b.) 4-[[N$^{\alpha}$-(4-fluorobenzoyl)-L-leucinyl]amino]-1-[(2S)-4-methyl-2-[(p-toluenesulphonyl)amino]pentyl]-3-pyrrolidinone

Following the procedure of Example 46(k) except substituting the material from Example 134(a), the title compound was produced: MS(ES+) 589 ($MH^+$).

Example 135

Preparation of 4-[[N$^{\alpha}$-(4-fluorobenzoyl)-L-leucinyl]amino]-1-[(2S)-4-methyl-2-[(methanesulphonyl)amino]pentyl]-3-pyrrolidinone

a.) (3RS,4RS)-4-[[N$^{\alpha}$-(4-fluorobenzoyl)-L-leucinyl]amino]-1-[(2S)-4-methyl-2-[(methanesulphonyl)amino]pentyl]-3-pyrrolidinone

Following the procedure of Example 133(b) except substituting methanesulfonyl chloride for acetyl chloride, the title compound was prepared: MS(ES+) 515 ($MH^+$).

b.) 4-[[N$^{\alpha}$-(4-fluorobenzoyl)-L-leucinyl]amino]-1-[(2S)-4-methyl-2-[(methanesulphonyl)amino]pentyl]-3-pyrrolidinone

Following the procedure of Example 46(k) except substituting the material from Example 135(a), the title compound was produced: MS(ES+) 513 ($MH^+$).

Example 136

Preparation of 4-[[N$^{\alpha}$-(4-fluorobenzoyl)-L-leucinyl]amino]-1-[(2S)-4-methyl-2-[($\alpha$-toluenesulphonyl)amino]pentyl]-3-pyrrolidinone

a.) (3RS,4RS)-4-[[N$^{\alpha}$-(4-fluorobenzoyl)-L-leucinyl]amino]-1-[(2S)-4-methyl-2-[($\alpha$-toluenesulphonyl)amino]pentyl]-3-pyrrolidinone

Following the procedure of Example 133(b) except substituting $\alpha$-toluenesulfonyl chloride for acetyl chloride the title compound was prepared: MS(ES+) 591 ($MH^+$).

b.) 4-[[N$^{\alpha}$-(4-fluorobenzoyl)-L-leucinyl]amino]-1-[(2S)-4-methyl-2-[($\alpha$-toluenesulphonyl)amino]pentyl]-3-pyrrolidinone

Following the procedure of Example 46(k) except substituting the material from Example 135(a), the title compound was produced: MS(ES+) 589 ($MH^+$).

Example 137

Preparation of 1-(2-phenethyl)-4-[[N$^{\alpha}$-(4-fluorobenzoyl)-L-leucinyl]amino]-3-pyrrolidinone

a.) (3RS,4RS)-1-(2-phenethyl)-4-[[N$^{\alpha}$-(*tert*-butoxycarbonyl)-L-leucinyl]amino]-3-pyrrolidinol

Following the procedure of Example 110(a) except substituting phenylacetaldehyde for benzylaldehyde, the title compound was produced: MS(ES+) 420 (MH$^+$).

b.) (3RS,4RS)-1-(2-phenethyl)-4-[(L-leucinyl)amino]-3-pyrrolidinol hydrochloride

Following the procedure of Example I 10(b) except substituting the compound of Example 137(a), the title compound was produced: MS(ES+) 320 (MH$^+$).

c.) (3RS,4RS)-1-(2-phenethyl)-4-[[N$^{\alpha}$-(4-fluorobenzoyl)-L-leucinyl]amino]-3-pyrrolidinol

Following the procedure of Example 110(c) except substituting 4-fluorobenzoic acid for 3-isoquinolinecarboxylic acid, the title compound was produced: MS(ES+) 442 (MH$^+$).

d.) 1-(2-phenethyl)-4-[[N$^{\alpha}$-(4-fluorobenzoyl)-L-leucinyl]amino]-3-pyrrolidinone

Following the procedure of Example 46(k) except substituting the compound of Example 137(c), the title compound was produced: MS(ES+) 440 (MH$^+$).

Example 138

Preparation of 1-(2-phenethyl)-4-[[N$^{\alpha}$-(2-quinolinylcarbonyl)-L-leucinyl]amino]-3-pyrrolidinone

a.) (3RS,4RS)-1-(2-phenethyl)-4-[[N$^{\alpha}$-(2-quinolinylcarbonyl)-L-leucinyl]amino]-3-pyrrolidinol

Following the procedure of Example 137(c) except substituting quinaldic acid for 4-fluorobenzoic acid, the title compound was prepared: MS(ES+) 475 (MH$^+$).

b.) 1-(2-phenethyl)-4-[[N$^{\alpha}$-(2-quinolinylcarbonyl)-L-leucinyl]amino]-3-pyrrolidinone

Following the procedure of Example 46(k) except substituting the material of Example 138(a), the title compound was produced: MS(ES+) 473 (MH$^+$).

Example 139

Preparation of 1-(2-phenethyl)-4-[[N$^{\alpha}$-(2-naphthylcarbonyl)-L-leucinyl]amino]-3-pyrrolidinone

a.) (3RS,4RS)-1-(2-phenethyl)-4-[[N$^{\alpha}$-(2-naphthylcarbonyl)-L-leucinyl]amino]-3-pyrrolidinol

Following the procedure of Example 137(c) except substituting 2-naphthoic acid for 4-fluorobenzoic acid, the title compound was produced: MS(ES+) 474 (MH$^+$).

b.) 1-(2-phenethyl)-4-[[N$^{\alpha}$-(2-naphthylcarbonyl)-L-leucinyl]amino]-3-pyrrolidinone

Following the procedure of Example 46(k) except substituting the material of Example 139(a), the title compound was produced: MS(ES+) 472 (MH$^+$).

Example 140

Preparation of 1-(2-phenethyl)-4-[[$N^{\alpha}$-($\alpha$-toluenesulphonyl)-L-leucinyl]amino]-3-pyrrolidinone

a.) (3RS,4RS)-1-(2-phenethyl)-4-[[$N^{\alpha}$-($\alpha$-toluenesulphonyl)-L-leucinyl]amino]-3-pyrrolidinol

Following the procedure of Example 131(b) except substituting the compound of Example 137(b) and $\alpha$-toluenesulfonyl chloride for p-toluenesulfonyl chloride, the title compound was produced: MS(ES+) 474 ($MH^+$).

b.) 1-(2-phenethyl)-4-[[$N^{\alpha}$-($\alpha$-toluenesulphonyl)-L-leucinyl]amino]-3-pyrrolidinone

Following the procedure of Example 46(k) except substituting the material of Example 140(a), the title compound was produced: MS(ES+) 472 ($MH^+$).

Example 141

Preparation of 1-(2-phenethyl)-4-[[$N^{\alpha}$-(2-nitro-$\alpha$-toluenesulphonyl)-L-leucinyl]amino]-3-pyrrolidinone

a.) (3RS,4RS)-1-(2-phenethyl)-4-[[$N^{\alpha}$-(2-nitro-$\alpha$-toluenesulphonyl)-L-leucinyl]amino]-3-pyrrolidinol

Following the procedure of Example 140(a) except substituting 2-nitro-$\alpha$-toluenesulfonyl chloride for $\alpha$-toluenesulphonyl chloride, the title compound was produced: MS(ES+) 519 (MH+).

b.) 1-(2-phenethyl)-4-[[$N^{\alpha}$-(2-nitro-$\alpha$-toluenesulphonyl)-L-leucinyl]amino]-3-pyrrolidinone

Following the procedure of Example 46(k) except substituting the material of Example 141(a), the title compound was produced: MS(ES+) 517 ($MH^+$).

Example 142

Preparation of 4-[[$N^{\alpha}$-(2-phenylacetyl)-L-leucinyl]amino]-1-[(2S)-4-methyl-2-[[(4-pyridinylcarbonyl)]amino]pentyl]-3-pyrrolidinone

a.) (3RS,4RS)-4-[[$N^{\alpha}$-(2-phenylacetyl)-L-leucinyl]amino]-1-[(2S)-4-methyl-2-(amino)pentyl]-3-pyrrolidinol

To a solution of the compound of Example 87(a) (1g, 1.76 mmol) in methanol:ethyl acetate (200 mL of a 1:2 solution) was added 10% Pd on carbon. The mixture was placed on a Parr hydrogenator for 2h at approximately 45 psi. The reaction was filtered through a pad of celite with $CH_2Cl_2$ and concentrated to give 740 mg of the title compound: MS(ES+) 433 ($MH^+$).

b.) (3RS,4RS)-4-[[$N^{\alpha}$-(2-phenylacetyl)-L-leucinyl]amino]-1-[(2S)-4-methyl-2-[[(4-pyridinylcarbonyl)]amino]pentyl]-3-pyrrolidinol

To a solution of the compound of Example 142(a) (247 mg, 0.57 mmol) in $CH_2Cl_2$ (10 mL) was added isonicotinic acid (74 mg, 0.6 mmol) followed by EDC (137 mg, 0.7 mmol) and HOBT (81 mg, 0.6 mmol). The reaction was stirred until complete by TLC analysis. Workup and column chromatography (5% $CH_3OH/CH_2Cl_2$) gave 180 mg of the title compound: MS(ES+) 538 ($MH^+$).

c.) 4-[[$N^{\alpha}$-(2-phenylacetyl)-L-leucinyl]amino]-1-[(2S)-4-methyl-2-[[(4-pyridinylcarbonyl)]amino]pentyl]-3-pyrrolidinone

Following the procedure of Example 46(k) except substituting the compound of Example 142(b), the title compound was produced: MS(ES+) 536 ($MH^+$).

Example 143

a.) (3RS,4RS)-4-[[N$^{\alpha}$-(2-phenylacetyl)-L-leucinyl]amino]-1-[(2S)-4-methyl-2-[[(p-toluenesulphonyl)]amino]pentyl]-3-pyrrolidinol

To a solution of the compound of Example 142(a) (247 mg, 0.57 mmol) in $CH_2Cl_2$ (10 mL) was added TEA (0.1 mL, 0.7 mmol) followed by p-toluenesulfonyl chloride (114 mg, 0.6 mmol). The reaction was stirred until complete by TLC analysis. Workup and column chromatography (5% $CH_3OH/CH_2Cl_2$) gave 300 mg of the title compound: MS (ES+) 587 ($MH^+$).

b.) 4-[[N$^{\alpha}$-(2-phenylacetyl)-L-leucinyl]amino]-1-[(2S)-4-methyl-2-[[(p-toluenesulphonyl)]amino]pentyl]-3-pyrrolidinone

Following the procedure of Example 46(k) except substituting the compound of Example 143(a), the title compound was produced: MS(ES+) 585 ($MH^+$).

Example 144

Preparation of 4-[[N$^{\alpha}$-(2-phenylacetyl)-L-leucinyl]amino]-1-[(2S)-4-methyl-2-[[(4-imidazoleacetyl)]amino]pentyl]-3-pyrrolidinone

a.) (3RS,4RS)-4-[[N$^{\alpha}$-(2-phenylacetyl)-L-leucinyl]amino]-1-[(2S)-4-methyl-2-[[(4-imidazoleacetyl)]amino]pentyl]-3-pyrrolidinol

Following the procedure of Example 142(b) except substituting 4-imidazoleacetic acid for isonicotinic acid, the title compound was produced: MS(ES+) 541 ($MH^+$).

b.) 4-[[N$^{\alpha}$-(2-phenylacetyl)-L-leucinyl]amino]-1-[(2S)-4-methyl-2-[[(4-imidazoleacetyl)]amino]pentyl]-3-pyrrolidinone

Following the procedure of Example 46(k) except substituting the compound of Example 144(a), the title compound was produced: MS(ES+) 539 ($MH^+$).

Example 145

Preparation of 4-[(4-phenoxybenzoyl)amino]-1-[(2S)-4-methyl-2-[[(benzyloxycarbonyl)]amino]pentanoyl]-3-pyrrolidinone

a.) (3RS,4RS)-4-[(4-phenoxybenzoyl)amino]-1-[(2S)-4-methyl-2-[[(benzyloxycarbonyl)]amino]pentanoyl]-3-pyrrolidinol

Following the procedure of Example 1(e-g) except substituting 4-phenoxybenzoic acid for CBZ-leucine in step 1(e), the title compound was produced: MS(ES+) 546.3 ($MH^+$), 568.2 (M+Na)

b.) 4-[(4-phenoxybenzoyl)amino]-1-[(2S)-4-methyl-2-[[(benzyloxycarbonyl)]amino]pentanoyl]-3-pyrrolidinone

Following the procedure of Example 1(h) except substituing the compound of Example 145(a), the title compound was produced: MS(ES+) 544.2 ($MH^+$).

Example 146

Preparation of 1-benzyl-4-[[N$^{\alpha}$-[(4-pyridinylmethoxy)carbonyl]-L-leucinyl]amino]-3-pyrrolidinone

a.) (3RS,4RS)-1-benzyl-4-[[N$^{\alpha}$-[(4-pyridinylmethoxy)carbonyl]-L-leucinyl]amino]-3-pyrrolidinol

To a solution of the compound of Example 47(b) (383 mg, 0.91 mmol) in $CH_2Cl_2$ (20 mL) was added TEA (0.25 mL, 1.81 mmol) followed by benzylaldehyde (0.11 mL, 1.1 mmol). The reaction was allowed to stir at room temperature for 0.5 h whereupon sodium triacetoxyborohydride (423 mg, 2 mmol) was added. The reaction was stirred at room temperature for 2 h whereupon it was diluted with ethyl acetate and washed with sat'd $NaHCO_3$, brine, dried ($Na_2SO_4$), filtered and concentrated. Column chromatography of the the residue (5:95 methanol:$CH_2CL_2$) gave 210

mg of the title compound: MS(ES+) 441 ($MH^+$).

b.) 1-benzyl-4-[[$N^{\alpha}$-[(4-pyridinylmethoxy)carbonyl]-L-leucinyl]amino]-3-pyrrolidinone

Following the procedure of Example 46(k) except substituting the compound of Example 146(a), the title compound was produced: MS(ES+) 439 ($MH^+$).

Example 147

Preparation of 1-(2-naphthylmethyl)-4-[[$N^{\alpha}$-(4-pyridinylmethoxycarbonyl)-L-leucinyl]amino]-3-pyrrolidinone

a.) (3RS,4RS)-1-(2-naphthylmethyl)-4-[[$N^{\alpha}$-(4-pyridinylmethoxycarbonyl)-L-leucinyl]amino]-3-pyrrolidinol

Following the procedure of Example 146(a) except substituting 2-naphthaldehyde for benzylaldehyde, the title compound was produced: MS(ES+) 491 ($MH^+$).

b.) 1-(2-naphthylmethyl)-4-[[$N^{\alpha}$-(4-pyridinylmethoxycarbonyl)-L-leucinyl]amino]-3-pyrrolidinone

Following the procedure of Example 46(k) except substituting the compound of Example 147(a), the title compound was produced: MS(ES+) 489 ($MH^+$).

Example 148

Preparation of 1-(3-cyanobenzyl)-4-[[$N^{\alpha}$-(4-pyridinylmethoxycarbonyl)-L-leucinyl]amino]-3-pyrrolidinone

a.) (3RS,4RS)-1-(3-cyanobenzyl)-4-[[$N^{\alpha}$-(4-pyridinylmethoxycarbonyl)-L-leucinyl]amino]-3-pyrrolidinol

Following the procedure of Example 146(a) except substituting 3-cyanobenzaldehyde for benzylaldehyde, the title compound was produced: MS(ES+) 466 ($MH^+$).

b.) 1-(3-cyanobenzyl)-4-[[$N^{\alpha}$-(4-pyridinylmethoxycarbonyl)-L-leucinyl]amino]-3-pyrrolidinone

Following the procedure of Example 46(k) except substituting the compound of Example 148(a), the title compound was produced: MS(ES+) 464 ($MH^+$).

Example 149

Preparation of 1-(3-amidobenzyl)-4-[[$N^{\alpha}$-(4-pyridinylmethoxycarbonyl)-L-leucinyl]amino]-3-pyrrolidinone

a.) (3RS,4RS)-1-(3-amidobenzyl)-4-[[$N^{\alpha}$-(4-pyridinylmethoxycarbonyl)-L-leucinyl]amino]-3-pyrrolidinol

To a solution of the compound of Example 148(a) (160 mg, 0.34 mmol) in DMSO (4 mL) was added $H_2O_2$ (0.5 mL) followed by 29 mg of $K_2CO_3$. The reaction was stirred at room temperature of I h whereupon is was diluted with ethyl acetate and washed with water, brine, dried ($Na_2SO_4$) and concentrated to give the title compound: MS(ES+) 484 ($MH^+$).

b.) 1-(3-amidobenzyl)-4-[[$N^{\alpha}$-(4-pyridinylmethoxycarbonyl)-L-leucinyl]amino]-3-pyrrolidinone

Following the procedure of Example 46(k) except substituting the compound of Example 149(a), the title compound was produced: MS(ES+) 482 ($MH^+$).

Example 150

Preparation of 1-(3-nitrobenzyl)-4-[[$N^{\alpha}$-(4-pyridinylmethoxycarbonyl)-L-leucinyl]amino]-3-pyrrolidinone

a.) (3RS,4RS)-1-(3-nitrobenzyl)-4-[[$N^{\alpha}$-(4-pyridinylmethoxycarbonyl)-L-leucinyl]amino]-3-pyrrolidinol

Following the procedure of Example 146(a) except substituting 3-nitrobenzaldehyde for benzylaldehyde, the

title compound was produced: MS(ES+) 486 ($MH^+$).

b.) 1-(3-nitrobenzyl)-4-[[$N^\alpha$-(4-pyridinylmethoxycarbonyl)-L-leucinyl]amino]-3-pyrrolidinone

Following the procedure of example 46(k) except substituting the compound of Example 150(a), the title compound was produced: MS (ES) 484 ($MH^+$).

Example 151

Preparation of 1-(2-nitrobenzyl)-4-[[$N^\alpha$-(4-pyridinylmethoxycarbonyl)-L-leucinyl]amino]-3-pyrrolidinone

a.) (3RS,4RS)-1-(2-nitrobenzyl)-4-[[$N^\alpha$-(4-pyridinylmethoxycarbonyl)-L-leucinyl]amino]-3-pyrrolidinol

Following the procedure of Example 146(a) except substituting 2-nitrobenzaldehyde for benzylaldehyde, the title compound was produced: MS(ES+) 486 ($MH^+$).

b.) 1-(2-nitrobenzyl)-4-[[$N^\alpha$-(4-pyridinylmethoxycarbonyl)-L-leucinyl]amino]-3-pyrrolidinone

Following the procedure of Example 46(k) except substituting the compound of Example 151(a), the title compound was produced: MS(ES+) 484 ($MH^+$).

Example 152

Preparation of 1-(4-cyanobenzyl)-4-[[$N^\alpha$-(4-pyridinylmethoycarbonyl)-L-leucinyl]amino]-3-pyrrolidinone

a.) (3RS,4RS)-1-(4-cyanobenzyl)-4-[[$N^\alpha$-(4-pyridinylmethoxycarbonyl)-L-leucinyl]amino]-3-pyrrolidinol

Following the procedure of Example 146(a) except substituting 4-cyanobenzaldehyde for benzylaldehyde, the title compound was produced: MS(ES+) 466 ($MH^+$).

b.) 1-(4-cyanobenzyl)-4-[[$N^\alpha$-(4-pyridinylmethoxycarbonyl)-L-leucinyl]amino]-3-pyrrolidinone

Following the procedure of Example 46(k) except substituting the compound of Example 152(a), the title compound was produced: MS(ES+) 464 ($MH^+$).

Example 153

Preparation of 1-(4-bromobenzyl)-4-[[$N^\alpha$-(4-pyridinylmethoxycarbonyl)-L-leucinyl]amino]-3-pyrrolidinone

a.) (3RS,4RS)-1-(4-bromobenzyl)-4-[[$N^\alpha$-(4-pyridinylmethoxycarbonyl)-L-leucinyl]amino]-3-pyrrolidinol

Following the procedure of Example 146(a) except substituting 4-bromobenzaldehyde for benzylaldehyde, the title compound was prepared: MS(ES+) 520 ($MH^+$).

b.) 1-(4-bromobenzyl)-4-[[$N^\alpha$-(4-pyridinylmethoxycarbonyl)-L-leucinyl]amino]-3-pyrrolidinone

Following the procedure of Example 46(k) except substituting the compound of Example 153(a), the title compound was produced: MS(ES+) 518 ($MH^+$).

Example 154

Preparation of 1-phenethyl-4-[[$N^\alpha$-(4-pyridinylmethoxycarbonyl)-L-leucinyl]amino]-3-pyrrolidinone

a.) (3RS,4RS)-1-phenethyl-4-[[$N^\alpha$-(4-pyridinylmethoxycarbonyl)-L-leucinyl]amino]-3-pyrrolidinol

Following the procedure of Example 146(a) except substituting phenylacetaldehyde for benzylaldehyde, the title compound was produced: MS(ES+) 455 ($MH^+$).

b.) 1-phenethyl-4-[[$N^{\alpha}$-(4-pyridinylmethoxycarbonyl)-L-leucinyl]amino]-3-pyrrolidinone

Following the procedure of Example 46(k) except substituting the compound of Example 154(a), the title compound was produced: MS(ES+) 453 ($MH^+$).

Example 155

Preparation of 1-(3-aminobenzyl)-4-[[$N^{\alpha}$-(4-pyridinylmethoxycarbonyl)-L-leucinyl]amino]-3-pyrrolidinone

To the compound of Example 150(b) (20 mg, 0,04 mmol) in ethanol (2 mL) was added $SnCl_2$ (20 mg, 0.1 mmol) followed by $Na_2CO_3$ (8 mg, 0.08 mmol). The reaction was stirred at room temperature overnight whereupon is was diluted with ethyl acetate and washed with sat'd $NaHCO_3$, water, brine, dried ($Na_2SO_4$) and concentrated to give the title compound: MS(ES+) 454 ($MH^+$).

Example 156

Preparation of 1-(3-benzyloxybenzyl)-4-[[$N^{\alpha}$-(4-pyridinylmethoxycarbonyl)-L-leucinyl]amino]-3-pyrrolidinone

a.) (3RS,4RS)-1-(3-benzyloxybenzyl)-4-[[$N^{\alpha}$-(4-pyridinylmethoxycarbonyl)-L-leucinyl]amino]-3-pyrrolidinol

Following the procedure of Example 146(a) except substituting 3-benzyloxybenzaldehyde for benzylaldehyde, the title compound was produced: MS(ES+) 547 ($MH^+$).

b.) 1-(3-benzyloxybenzyl)-4-[[$N^{\alpha}$-(4-pyridinylmethoxycarbonyl)-L-leucinyl]amino]-3-pyrrolidinone

Following the procedure of Example 46(k) except substituting the compound of Example 156(a), the title compound was produced: MS(ES) 545 ($MH^+$).

Example 157

Preparation of 1-(3-hydoxybenzyl)-4-[[$N^{\alpha}$-(4-pyridinylmethoxycarbonyl)-L-leucinyl]amino]-3-pyrrolidinone

a.) (3RS,4RS)-1-(3-hydroxybenzyl)-4-[[$N^{\alpha}$-(4-pyridinylmethoxycarbonyl)-L-leucinyt]amino]-3-pyrrolidinol

Following the procedure of Example 146(a) except substituting 3-hydroxybenzaldehyde for benzylaldehyde, the title compound was produced: MS(ES+) 457 ($MH^+$).

b.) 1-(3-hydroxybenzyl)-4-[[$N^{\alpha}$-(4-pyridinylmethoxycarbonyl)-L-leucinyl]amino]-3-pyrrolidinone

Following the procedure of Example 46(k) except substituting the compound of Example 157(a), the title compound was produced: MS(ES+) 455 ($MH^+$).

Example 158

Preparation of 1-ethyl-4-[[$N^{\alpha}$-(4-pyridinylmethoxycarbonyl)-L-leucinyl]amino]-3-pyrrolidinone

a.) (3RS,4RS)-1-ethyl-4-[[$N^{\alpha}$-(4-pyridinylmethoxycarbonyl)-L-leucinyl]amino]-3-pyrrolidinol

To a solution of the compound of Example 47(b) (300 mg, 0.71 mmol) in DMF (10 mL) was added bromoethane (0.06 mL, 0.85 mmol), $Na_2CO_3$ (393 mg, 2.84 mmol) and a catalytic amount of KI. The reaction was stirred at room overnight whereupon it was diluted with ethyl acetate, washed with sat'd $NaHCO_3$, brine, dried ($Na_2SO_4$), concentrated and chromatographed (20%$CH_3OH$:$CH_2Cl_2$) to give 120 mg of the title compound: MS(ES+) 379 (MH+).

b.) 1-ethyl-4-[[$N^{\alpha}$-(4-pyridinylmethoxycarbonyl)-L-leucinyl]amino]-3-pyrrolidinone

Following the procedure of Example 46(k) except substituting the compound of Example 158(a), the title compound was produced: MS(ES+) 377 ($MH^+$).

Example 159

Preparation of 1-cyclopropylmethyl-4-[[N$^{\alpha}$-(4-pyridinylmethoxycarbonyl)-L-leucinyl]amino]-3-pyrrolidinone

a.) (3RS,4RS)-1-cyclopropylmethyl-4-[[N$^{\alpha}$-(4-pyridinylmethoxycarbonyl)-L-leucinyl]amino]-3-pyrrolidinol

To a solution of the compound of Example 47(b) (300 mg, 0.71 mmol) in DMF (10 mL) was added bromomethylcyclopropane (0.08 mL, 0.85 mmol), $Na_2CO_3$ (393 mg, 2.84 mmol) and a catalytic amount of KI. The reaction was stirred at room overnight whereupon it was diluted with ethyl acetate, washed with sat'd $NaHCO_3$, brine, dried ($Na_2SO_4$), concentrated and chromatographed (20%$CH_3OH$:$CH_2Cl_2$) to give 120 mg of the title compound: MS(ES+) 405 (MH+).

b.) 1-cyclopropylmethyl-4-[[N$^{\alpha}$-(4-pyridinylmethoxycarbonyl)-L-leucinyl]amino]-3-pyrrolidinone

Following the procedure of Example 46(k) except substituting the compound of Example 159(a), the title compound was produced: MS(ES+) 403 ($MH^+$).

Example 160

Preparation of 1-(2-N,N-dimethylaminoethyl)-4-[[N$^{\alpha}$-(4-pyridinylmethoxycarbonyl)-L-leucinyl]amino]-3-pyrrolidinone

a.) (3RS,4RS)-1-(2-N,N-dimethylaminoethyl)-4-[[N$^{\alpha}$-(4-pyridinylmethoxycarbonyl)-L-leucinyl]amino]-3-pyrrolidinol

To a solution of the compound of Example 47(b) (383 mg, 0.91 mmol) in ethanol (10 mL) was added 2-dimethylaminoethyl chloride hydrochloride (158 mg, 1.1 mmol), $Na_2CO_3$ (250 mg, 1.81 mmol) and a catalytic amount of KI. The reaction was refluxed for 6 h whereupon it was diluted with ethyl acetate, washed with sat' $NaHCO_3$, brine, dried ($Na_2SO_4$), concentrated and chromatographed (25% $CH_3OH$:$CH_2Cl_2$) to give 150 mg of the title compound: MS(ES+) 422 ($MH^+$).

b.) 1-(2-N,N-dimethylaminoethyl)-4-[[N$^{\alpha}$-(4-pyridinylmethoxycarbonyl)-L-leucinyl]amino]-3-pyrrolidinone

Following the procedure of Example 46(k) except substituting the compound of Example 160(a), the title compound was produced: MS(ES+) 420 ($MH^+$).

Example 161

Preparation of 1-(2-morpholinoethyl)-4-[[N$^{\alpha}$-(4-pyridinylmethoxycarbonyl)-L-leucinyl]amino]-3-pyrrolidinone

a.) (3RS,4RS)-1-(2-morpholinoethyl)-4-[[N$^{\alpha}$-(4-pyridinylmethoxycarbonyl)-L-leucinyl]amino]-3-pyrrolidinonol

To a solution of the compound of Example 47(b) (400 mg, 0.95 mmol) in ethanol (10 mL) was added N-(2-chloroethyl)morpholine hydrochloride (194 mg, 1.1 mmol), $Na_2CO_3$ (525 mg, 3.8 mmol) and a catalytic amount of KI. The reaction was refluxed for 6h whereupon it was diluted with ethyl acetate, washed with sat' $NaHCO_3$, brine, dried ($Na_2SO_4$), concentrated and chromatographed (10% $CH_3OH$:$CH_2Cl_2$) to give 80 mg of the title compound: MS(ES+) 464 ($MH^+$).

b.) 1-(2-morpholinoethyl)-4-[(N$^{\alpha}$-(4-pyridinylmethoxycarbonyl)-L-leucinyl]amino]-3-pyrrolidinone

Following the procedure of Example 46(k) except substituting the compound of Example 161(a), the title compound was produced: MS(ES+) 462 ($MH^+$).

Example 162

Preparation of 1-(2-bromobenzyl)-4-[[N$^{\alpha}$-(2-pyridinylmethoxycarbonyl)-L-leucinyl]amino]-3-pyrrolidinonone

a.) (3RS,4RS)-4-[[N$^{\alpha}$-(2-pyridinylmethoxycarbonyl)-L-leucinyl]amino]-3-pyrrolidinol bis-hydrochloride

Following the procedure of Example 47(a-b) except substituting N-(2-pyridylmethoxycarbonyl)-L-leucine for N-(4-pyridylmethoxycarbonyl)-L-leucine, the title compound was prepared: MS(ES+) 351 ($MH^+$).

b.) (3RS,4RS)-1-(2-bromobenzyl)-4-[[N$^{\alpha}$-(2-pyridinylmethoxycarbonyl)-L-leucinyl]amino]-3-pyrrolidinonol

To a solution of the compound of Example 162(b) (200 mg, 0.47 mmol) in $CH_2Cl_2$ (20 mL) was added TEA (0.08 mL, 0.57 mmol) followed by 3-bromobenzylaldehyde (0.07 mL, 0.57 mmol). The reaction was allowed to stir at room temperature for 0.5 h whereupon sodium triacetoxyborohydride (120 mg, 0.57 mmol) was added. The reaction was stirred at room temperature for 2 h whereupon it was diluted with ethyl acetate and washed with sat'd $NaHCO_3$, brine, dried ($Na_2SO_4$), filtered and concentrated. Column chromatography of the the residue (5:95 methanol: $CH_2Cl_2$) gave 210 mg of the title compound: MS(ES+) 521 ($MH^+$),

c.) 1-(2-bromobenzyl)-4-[[N$^{\alpha}$-(2-pyridinylmethoxycarbonyl)-L-leucinyl]amino]-3-pyrrolidinonone

Following the procedure of Example 46(k) except substituting the compound of Example 162(c), the title compound was produced: MS(ES+) 519 ($MH^+$).

Example 163

Preparation of 4-[[N$^{\alpha}$-(4-pyrdinylmethoxy)carbonyl)-L-leucinyl]amino]-1-[(2S)-4-methyl-2-[[(benzyloxycarbonyl)]amino]pentyl]-3-pyrrolidinone

a.) (3RS,4RS)-4-[[N$^{\alpha}$-(4-pyrdinylmethoxy)carbonyl)-L-leucinyl]amino]-1-[(2S)-4-methyl-2-[[(benzyloxycarbonyl)]amino]pentyl]-3-pyrrolidinol

Following the procedure of Example 146(a) except substituting CBZ-leucinal for benzylaldehyde, the title compound was prepared: MS(ES+) 584 ($MH^+$).

b.) 4-[[N$^{\alpha}$-(4-pyrdinylmethoxy)carbonyl)-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 163(a), the title compound was produced: MS(ES+) 582 ($MH^+$).

Example 164

Preparation of 4-[[N$^{\alpha}$-(4-pyrdinylmethoxy)carbonyl)-L-leucinyl]amino]-1-[(2S)-4-methyl-2-[[(benzyloxycarbonyl)]aminomethyl]pentyl]-3-pyrrolidinone

a.) (3RS,4RS)-4-[[N$^{\alpha}$-(4-pyrdinylmethoxy)carbonyl)-L-leucinyl]amino]-1-[(2S)-4-methyl-2-[[(benzyloxycarbonyl)]aminomethyl]pentyl]-3-pyrrolidinol

Following the procedure of Example 146(a) except substituting N-methyl-CBZ-leucinal for benzylaldehyde, the title compound was produced: MS(ES+) 598 ($MH^+$).

b.) 4-[[N$^{\alpha}$-(4-pyrdinylmethoxy)carbonyl)-L-leucinyl]amino]-1-[(2S)-4-methyl-2-[[(benzyloxycarbonyl)]aminomethyl]pentyl]-3-pyrrolidinone

Following the procedure of Example 46(k) except substituting the compound of Example 164(a), the title compound was produced: MS(ES+) 596 ($MH^+$).

Example 165

Preparation of 4-[[N$^{\alpha}$-[(2-pyridinylmethoxy)carbonyl]-L-leucinyl]amino]-1-[(2S)-4-methyl-2-[[(benzyloxycarbonyl)]amino]pentyl]-3-pyrrolidinone

a.) (3RS,4RS)-4-[[N$^{\alpha}$-[(2-pyridinylmethoxy)carbonyl]-L-leucinyl]amino]-1-[(2S)-4-methyl-2-[[(benzyloxycarbonyl)]amino]pentyl]-3-pyrrolidinol

Following the procedure of Example 46(h) except substituting the compound of Example 162(a), the title compound was produced: MS(ES+) 584 ($MH^+$).

b.) 4-[[N$^{\alpha}$-[(2-pyridinylmethoxy)carbonyl]-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 165(a), the title compound was produced: MS(ES+) 582 ($MH^+$).

Example 166

Preparation of 4-[[N$^{\alpha}$-[(3-pyridinylmethoxy)carbonyl]-L-leucinyl]amino]-1-[(2S)-4-methyl-2-[[(benzyloxycarbonyl)]amino]pentyl]-3-pyrrolidinone

a.) (3RS,4RS)-4-[[N$^{\alpha}$-(3-pyridinylmethoxycarbonyl)-L-leucinyl]amino]-3-pyrrolidinol bis-hydrochloride

Following the procedure of Example 162(a) except substituting N-(3-pyridylmethoxycarbonyl)-L-leucine for N-(2-pyridylmethoxycarbonyl)-L-leucine, the title compound was prepared: MS(ES+) 351 ($MH^+$).

b.) (3RS,4RS)-4-[[N$^{\alpha}$-[(3-pyridinylmethoxy)carbonyl]-L-leucinyl]amino]-1-[(2S)-4-methyl-2-[[(benzyloxycarbonyl)]amino]pentyl]-3-pyrrolidinol

Following the procedure of Example 165(a) except substituting the compound of Example 166(a), the title compound was produced: MS(ES+) 584 ($MH^+$).

c.) 4-[[N$^{\alpha}$-[(3-pyridinylmethoxy)carbonyl]-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 166(b), the title compound was produced: MS(ES+) 582 ($MH^+$).

Example 167

Preparation of 4-[[N$^{\alpha}$-(benzyloxycarbonyl)-L-leucinyl]amino]-1-[(2S)-4-methyl-2-[[(benzyloxycarbonyl)]amino]pentyl]-3-pyrrolidinone

a.) (3RS,4RS)-4-[(N$^{\alpha}$-(benzyloxycarbonyl)-L-leucinyl]amino]-1-[(2S)-4-methyl-2-[[(benzyloxycarbonyl)]amino]pentyl]-3-pyrrolidinol

Following the procedure of Example 46(h) except substituting the compound of Example 1(f), the title compound was prepared: MS(ES+) 583 ($MH^+$).

b.) 4-[[N$^{\alpha}$-(benzyloxycarbonyl)-L-leucinyl]amino]-1-[(2S)-4-methyl-2-[[(benzyloxycarbonyl)]amino]pentyl]-3-pyrrolidinone

Following the procedure of Example 46(k) except substituting the compound of Example 167(a), the title compound was produced: MS(ES+) 581 ($MH^+$).

Example 168

Preparation of 4-[[N$^{\alpha}$-(4-pyridinylmethoxycarbonyl)-L-leucinyl]aminomethyl]-1-[(2S)-4-methyl-2-[[(benzyloxycarbonyl)]amino]pentyl]-3-pyrrolidinone

a.) 1-*tert*-butoxycarbonyl-*trans*-3-aminomethyl-4-hydroxypyrrolidine

A solution of the compound of Example 1(b) (2.3 g, 12.4 mmol) in methylamine (10 mL) was stirred at room temperature for 48 h whereupon it was concentrated, diluted with ethyl acetate and washed saturated $NaHCO_3$, water and brine. The organic layer was dried ($MgSO_4$), filtered and concentrated. Column chromatography of the residue (20% $CH_3OH$:ethyl acetate) gave 424 mg of the title compound: MS(ES) 116 ($MH^+$-Boc).

b.) (3RS,4RS)-4-[[N$^{\alpha}$-(4-pyridinylmethoxycarbonyl)-L-leucinyl]aminomethyl]-1-*tert*-butoxycarbonyl-3-pyrrolidinol

To a solution of the compound of Example 170(a) (420 mg, 1.94 mmol) in $CH_2Cl_2$ (20 mL) was added N-(4-pyridylmethoxycarbonyl)-L-leucine (545 mg, 2.04 mmol), HOBT (276 mg, 2.04 mmol) and EDC (446 mg, 2.33 mmol). The reaction was allowed to stir at room temperature overnight whereupon it was diluted with $CH_2Cl_2$ and washed 0.5N HCl, sat'd $NaHCO_3$, water and brine. The organic layer was dried ($MgSO_4$), filtered and concentrated. Column chromatography of the residue (5:95 MeOH:$CH_2Cl_2$) gave 600 mg of the title compound: MS(ES+) 465 ($MH^+$).

c.) (3RS,4RS)-4-[[N$^{\alpha}$-(4-pyridinylmethoxycarbonyl)-L-leucinyl]aminomethyl]-3-pyrrolidinol bis-hydrochloride

To a solution of the compound of Example 170(b) (600 mg, 1.3 mmol) in methanol (10 mL) was added 4M HCl in dioxane (10 mL). The reaction was stirred at room overnight whereupon it was concentrated to give 608 mg of the title compound: MS(ES+) 365 ($MH^+$).

d.) (3RS,4RS)-4-[[N$^{\alpha}$-(4-pyridinylmethoxycarbonyl)-L-leucinyl]aminomethyl]-1-[(2S)-4-methyl-2-[[(benzyloxycarbonyl)]amino]pentyl]-3-pyrrolidinol

To a solution of the compound of Example 170(c) (325 mg, 0.74 mmol) in $CH_2Cl_2$ (10 mL) was added TEA (0.21 mL, 1.49 mmol) followed by CBZ-leucinal (405 mg, 1.63 mmol). The reaction was allowed to stir at room temperature for 0.5 h whereupon sodium triacetoxyborohydride (392 mg, 1.85 mmol) was added. The reaction was stirred at room temperature for 2 h whereupon it was diluted with ethyl acetate and washed with sat'd $NaHCO_3$, brine, dried ($Na_2SO_4$), filtered and concentrated. Column chromatography of the the residue (5:95 methanol: $CH_2Cl_2$) gave 120 mg of the title compound: MS(ES+) 598 ($MH^+$).

e.) 4-[[N$^{\alpha}$-(4-pyridinylmethoxycarbonyl)-L-leucinyl]aminomethyl]-1-[(2S)-4-methyl-2-[[(benzyloxycarbonyl)]amino]pentyl]-3-pyrrolidinone

Following the procedure of Example 46(k) except substituting the compound of Example 170(d), the title compound was produced: MS(ES+) 596 ($MH^+$).

Example 169

Preparation of 1-benzyl-4-[[N$^{\alpha}$-(2-nitro-$\alpha$-toluenesulphonyl)-L-leucinyl]amino]-3-pyrrolidinone

a.) (3RS,4RS)-1-benzyl-4-[[N$^{\alpha}$-(2-nitro-$\alpha$-toluenesulphonyl)-L-leucinyl]amino]-3-pyrrolidinol

To a solution of the compound of Example 110(b) (500 mg, 1.32 mmol) in $CH_2Cl_2$ (20 mL) was added TEA (0.64 mL, 4.62 mmol) followed by 2-nitro-$\alpha$-toluenesulfonyl chloride (327 mg, 1.38 mmol). The reaction was stirred until complete by TLC analysis. Workup and column chromatography (10% $CH_3OH/CH_2Cl_2$) gave 100 mg of the title compound: MS(ES+) 505 ($MH^+$).

b.) 1-benzyl-4-[[N$^{\alpha}$-(2-nitro-$\alpha$-toluenesulphonyl)-L-leucinyl]amino]-3-pyrrolidinone

Following the procedure of Example 46(k) except substituting the compound of Example 171(a), the title compound was produced: MS (ES) 503 ($MH^+$).

Example 170

Preparation of 1-benzyl-4-[[N$^{\alpha}$-(phenylsulphonyl)-L-leucinyl]amino]-3-pyrrolidinone

a.) (3RS,4RS)-1-benzyl-4-[[N$^{\alpha}$-(phenylsulphonyl)-L-leucinyl]amino]-3-pyrrolidinol

Following the procedure of Example 169(a) except substituting benzenesulfonyl chloride for 2-nitro-$\alpha$-toluenesulfonyl chloride, the title compound was prepared: MS(ES+) 446 ($MH^+$).

b.) 1-benzyl-4-[[N$^{\alpha}$-(phenylsulphonyl)-L-leucinyl]amino]-3-pyrrolidinone

Following the procedure of Example 46(k) except substituting the compound of Example 172(a), the title

compound was produced: MS(ES+) 444 ($MH^+$).

Example 171

Preparation of 1-benzyl-4-[[$N^{\alpha}$-($\alpha$-toluenesulphonyl)-L-leucinyl]amino]-3-pyrrolidinone

a.) (3RS,4RS)-1-benzyl-4-[[$N^{\alpha}$-($\alpha$-toluenesulphonyl)-L-leucinyl]amino]-3-pyrrolidinol

Following the procedure of Example 169(a) except substituting $\alpha$-toluenesulfonyl chloride for 2-nitro-a-toluenesulfonyl chloride, the title compound was prepared: MS(ES+) 460 ($MH^+$).

b.) 1-benzyl-4-[[$N^{\alpha}$-($\alpha$-toluenesulphonyl)-L-leucinyl]amino]-3-pyrrolidinone

Following the procedure of Example 46(k) except substituting the compound of Example 173(a), the title compound was produced: MS(ES+) 458 ($MH^+$).

Example 172

Preparation of 1-benzyl-4-[[$N^{\alpha}$-(2-naphthylsulphonyl)-L-leucinyl]amino]-3-pyrrolidinone

a.) (3RS,4RS)-1-benzyl-4-[[$N^{\alpha}$-(2-naphthylsulphonyl)-L-leucinyl]amino]-3-pyrrolidinol

Following the procedure of Example 169(a) except substituting 2-naphthalenesulfonyl chloride for 2-nitro-$\alpha$-toluenesulfonyl chloride, the title compound was produced: MS(ES+) 496 ($MH^+$).

b.) 1-benzyl-4-[[$N^{\alpha}$-(2-naphthylsulphonyl)-L-leucinyl]amino]-3-pyrrolidinone

Following the procedure of Example 46(k) except substituting the compound of Example 174(a), the title compound was produced: MS(ES+) 494 ($MH^+$).

Example 173

Preparation of 1-benzyl-4-[[$N^{\alpha}$-(2-naphthylcarbonyl)-L-leucinyl]amino]-3-piperidinone

a.) (3RS,4RS)-1-benzyl-4-[[$N^{\alpha}$-(L-leucinyl)amino]-3-piperidinol hydrochloride

Following the procedure of Example 98(h-i) except substituting benzaldehyde for CBZ-leucinal, the title compound was prepared: MS(ES+) 320.3 (MH+).

b.) (3RS,4RS)-1-benzyl-4-[[$N^{\alpha}$-(2-naphthylcarbonyl)-L-leucinyl]amino]-3-piperidinol

Following the procedure of Example 98(j) except substituting the 2-naphthoic acid for phenylacetic acid, the title compound was produced: MS(ES+) 474.1 ($MH^+$).

c.) 1-benzyl-4-[[$N^{\alpha}$-(2-naphthylcarbonyl)-L-leucinyl]amino]-3-piperidinone

Following the procedure of Example 98(k) except substituting the compound of Example 176(c), the title compound was produced: MS(ES+) 472.3 ($MH^+$).

Example 174

Preparation of 1-benzyl-4-[[$N^{\alpha}$-(2-quinolinylcarbonyl)-L-leucinyl]amino]-3-piperidinone

a.) (3RS,4RS)-1-benzyl-4-[[$N^{\alpha}$-(2-quinolinylcarbonyl)-L-leucinyl]amino]-3-piperidinol

Following the procedure of Example 173(b) except substituting quinaldic acid for 2-naphthoic acid , the title compound was produced: MS(ES+) 475.3 ($MH^+$).

b.) 1-benzyl-4-[[Nα-(2-quinolinylcarbonyl)-L-leucinyl]amino]-3-piperidinone

Following the procedure of Example 98(k) except substituting the compound of Example 177(a), the title compound was produced: MS(ES+) 473.3 ($MH^+$).

Example 175

Preparation of 1-benzyl-4-[[Nα-(2-naphthylsulphonyl)-L-leucinyl]amino]-3-piperidinone

a.) (3RS,4RS)-1-benzyl-4-[[Nα-(2-naphthylsulphonyl)-L-leucinyl]amino]-3-piperidinol

Following the procedure of Example 172(a) except substituting the compound of Example 173(a), the title compound was produced: MS(ES+) 510.3 (MH+).

b.) 1-benzyl-4-[[Nα-(2-naphthylsulphonyl)-L-leucinyl]amino]-3-piperidinone

Following the procedure of Example 98(k) except substituting the compound of Example 178(a), the title compound was produced: 508 MS(ES+) ($MH^+$).

Example 176

Preparation of 4-[[Nα-(benzyloxycarbonyl)-L-leucinyl]aminomethyl]-1-[(2S)-4-methyl-2-[[(benzyloxycarbonyl)]aminomethyl]pentanoyl]-3-pyrrolidinone

a.) (3RS,4RS)-4-[[Nα-(benzyloxycarbonyl)-L-leucinyl]aminomethyl]-1-[(2S)-4-methyl-2-[[(benzyloxycarbonyl)]aminomethyl]pentanoyl]-3-pyrrolidinol

Following the procedure of Example 1(e-g) except substituting N-methyl-CBZ-leucine for CBZ-leucine in steps 1(e) and 1(g), the title compound was produced: MS(ES+) 625.3 (M+H), 647. (M+Na).

b.) 4-[[Nα-(benzyloxycarbonyl)-L-leucinyl]aminomethyl]-1-[(2S)-4-methyl-2-[[(benzyloxycarbonyl)]aminomethyl]pentanoyl]-3-pyrrolidinone

Following the procedure of Example 3(b) except substituting the compound of Example 176 the title compound was produced: MS(ES+) 623.4 (M+H), 645.4 (M+Na).

Examples 177-198

The following compounds were prepared using processes analogous to those detailed in Examples 1-176.

| Example | Name |
|---|---|
| **177** | (3RS,4RS)-4-[(2S)-4-methyl-2-[(benzyloxycarbonyl)amino]pentyl]-1-[(2S)-4-methyl-2-[[(benzyloxycarbonyl)]amino]pentanoyl]-3-pyrrolidinone |
| | 4-[(2S)-4-methyl-2-[(benzyloxycarbonyl)amino]pentyl]-1-[(2S)-4-methyl-2-[[(benzyloxycarbonyl)]amino]pentanoyl]-3-pyrrolidinone |
| **178** | (3RS,4RS)-4-[[Nα-(4-fluorobenzoyl)-L-leucinyl]amino]-1-[2-[(α-toluenesulphonyl)amino]ethyl]-3-pyrrolidinol |
| | 4-[[Nα-(4-fluorobenzoyl)-L-leucinyl]amino]-1-[2-[(α-toluenesulphonyl)amino]ethyl]-3-pyrrolidinone |

(continued)

| Example | Name |
|---|---|
| **179** | (3RS,4RS)-4-[[N$^{\alpha}$-(4-fluorobenzoyl)-L-leucinyl]amino]-1-benzoyl-3-pyrrolidinol |
| | 4-[[N$^{\alpha}$-(4-fluorobenzoyl)-L-leucinyl]amino]-1-benzoyl-3-pyrrolidinone |
| **180** | (3RS,4RS)-4-[[N$^{\alpha}$-(piperonylcarbonyl)-L-leucinyl]amino]-1-benzoyl-3-pyrrolidinol |
| | 4-[[N$^{\alpha}$-(piperonylcarbonyl)-L-leucinyl]amino]-1-benzoyl-3-pyrrolidinone |
| **181** | (3RS,4RS)-4-[[N$^{\alpha}$-(4-fluorobenzoyl)-L-leucinyl]amino]-1-[2-[(4-fluorobenzoyl)amino]ethyl]-3-pyrrolidinol |
| | 4-[[N$^{\alpha}$-(4-fluorobenzoyl)-L-leucinyl]amino]-1-[2-[(4-fluorobenzoyl)amino]ethyl]-3-pyrrolidinone |
| **182** | (3RS,4RS)-4-[[N$^{\alpha}$-(*tert*-butoxycarbonyl)-L-leucinyl]amino]-1-benzoyl-3-pyrrolidinol |
| | 4-[[N$^{\alpha}$-(*tert*-butoxycarbonyl)-L-leucinyl]amino]-1-benzoyl-3-pyrrolidinone |
| **183** | (3RS,4RS)-4-[[N$^{\alpha}$-(4-fluorobenzoyl)-L-leucinyl]amino]-1-[(2S)-4-methyl-2-[(4-fluorobenzoyl)amino]pentyl]-3-pyrrolidinol |
| | 4-[[N$^{\alpha}$-(4-fluorobenzoyl)-L-leucinyl]amino]-1-[(2S)-4-methyl-2-[(4-fluorobenzoyl)amino]pentyl]-3-pyrrolidinone |
| **184** | 4-[[N$^{\alpha}$-(4-carboxybenzoyl)-L-leucinyl]amino]-1-[(2S)-4-methyl-2-[(4-fluorobenzoyl)amino]pentyl]-3-pyrrolidinone lithium salt |
| **185** | 1-benzyl-4-[[N$^{\alpha}$-(4-carboxybenzoyl)-L-leucinyl]amino]-3-pyrrolidinone lithium salt |
| **186** | (3RS,4RS)-1-benzyl-4-[[N$^{\alpha}$-(4-carboxymethyl)benzoyl)-L-leucinyl]amino]-3-pyrrolidinol |
| | 1-benzyl-4-[[N$^{\alpha}$-(4-carboxymethyl)benzoyl)-L-leucinyl]amino]-3-pyrrolidinone |
| **187** | (3RS,4RS)-4-[[N$^{\alpha}$-[(4-carboxymethyl)benzoyl]-L-leucinyl]amino]-1-[(2S)-4-methyl-2-[[(4-fluorobenzoyl)amino]pentyl]-3-pyrrolidinol |
| | 4-[[N$^{\alpha}$-[(4-carboxymethyl)benzoyl]-L-leucinyl]amino]-1-[(2S)-4-methyl-2-[[(4-fluorobenzoyl)amino]pentyl]-3-pyrrolidinone |
| **188** | (3RS,4RS)-1-phenethyl-4-[[N$^{\alpha}$-(2-amino-$\alpha$-toluenesulphonyl)-L-leucinyl]; 3-pyrrolidinol |
| | 1-phenethyl-4-[[N$^{\alpha}$-(2-amino-$\alpha$-toluenesulphonyl)-L-leucinyl]amino]-3-pyrrolidinone |
| **189** | (3RS,4RS)-4-[[N$^{\alpha}$-(2-naphthylcarbonyl)-L-leucinyl]amino]-1-benzoyl-3-piperidinol |
| | 4-[[N$^{\alpha}$-(2-naphthylcarbonyl)-L-leucinyl]amino]-1-benzoyl-3-piperidinone |

(continued)

| Example | Name |
|---|---|
| **190** | (3RS,4RS)-4-[[N$^{\alpha}$-(2-quinolinecarbonyl)-L-leucinyl]amino]-1-benzoyl-3-piperidinol |
| | 4-[[N$^{\alpha}$-(2-quinolinecarbonyl)-L-leucinyl]amino]-1-benzoyl-3-piperidinone |
| **191** | (3RS,4RS)-4-[[N$^{\alpha}$-(3-isoquinolinecarbonyl)-L-leucinyl]amino]-1-benzoyl-3-piperidinol |
| | 4-[[N$^{\alpha}$-(3-isoquinolinecarbonyl)-L-leucinyl]amino]-1-benzoyl-3-piperidinone |
| **192** | (3RS,4RS)-4-[[(2S)-4-methyl-2-(benzyl)oxy]pentanoyl]-1-[(2S)-4-methyl-2-[[(benzyloxycarbonyl)] amino]pentanoyl]-3-piperidinol |
| | 4-[[(2S)-4-methyl-2-(benzyl)oxy]pentanoyl]-1-[(2S)-4-methyl-2-[[(benzyloxycarbonyl)]amino] pentanoyl]-3-piperidinone |
| **193** | (3RS,4RS)-4-[[3-(2-pyridyl)phenylacetyl)]amino]-1-[3-(2-pyridyl)phenyla piperidinol |
| | 4-[[3-(2-pyridyl)phenylacetyl)]amino]-1-[3-(2-pyhdyl)phenylacetyl)]-3-pil |
| **194** | (3RS,4RS)-4-[[N$^{\alpha}$-(p-trifluoromethanephenylsulphonyl)-L-leucinyl]amino]-1-[3-(2-pyridyl) phenylacetyl)]-3-piperidinol |
| | 4-[[N$^{\alpha}$-(p-trifluoromethanephenylsulphonyl)-L-leucinyl]amino]-1-[3-(2-pyridyl)phenylacetyl)]-3-piperidinone |
| **195** | (3RS,4RS)-4-[[N$^{\alpha}$-(2-naphthylsulphonyl)-L-leucinyl]amino]-1-[3-(2-pyridyl)phenylacetyl)]-3-piperidinol |
| | 4-[[N$^{\alpha}$-(2-naphthylsulphonyl)-L-leucinyl]amino]-1-[3-(2-pyridyl)phenylacetyl)]-3-piperidinone |
| **196** | (3RS,4RS)-4-[[N$^{\alpha}$-(3,4-dichlorophenylsulphonyl)-L-leucinyl]aminol-1-[3-(2-pyridyl)phenylacetyl)]-3-piperidinol |
| | 4-[[N$^{\alpha}$-(3,4-dichlorophenylsulphonyl)-L-leucinyl]amino]-1-[3-(2-pyridyl)phenylacetyl)]-3-piperidinone |
| **197** | (3RS,4RS)-4-[[N$^{\alpha}$-(methanesulphonyl)-L-leucinyl]amino]-1-[3-(2-pyridyl)phenylacetyl)]-3-piperidinol |
| | 4-[[N$^{\alpha}$-(methanesulphonyl)-L-leucinyl]amino]-1-[3-(2-pyridyl)phenylacetyl)]-3-piperidinone |
| **198** | (3RS,4RS)-4-[[N$^{\alpha}$-(4-fluorophenylsulphonyl)-L-leucinyl]amino]-1-[3-(2-pyridyl)phenylacetyl)]-3-piperidinol |
| | 4-[[N$^{\alpha}$-(4-fluorophenylsulphonyl)-no]-1-[3-(2-pyridyl)phenylacetyl)]-3-piperidinone |

The above specification and Examples fully disclose how to make and use the compounds of the present

invention. However, the present invention is not limited to the particular embodiments described hereinabove, but includes all modifications thereof within the scope of the following claims.

## Claims

**1.** A compound according to formula (I):

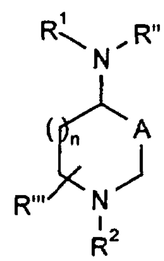

(I)

wherein:

A is C(O) or CH(OH);
$R^1$ is

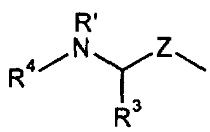
,
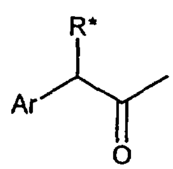
,
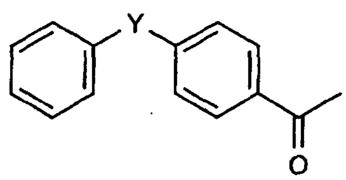
,

or

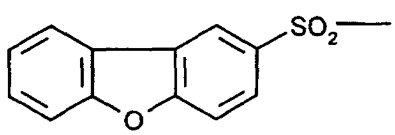
;

$R^2$ is H, $C_{1-6}$alkyl optionally substituted independently by one or two halogens, SR, OR, $N(R)_2$, $C(O)N(R)_2$, carbamyl or $C_{1-4}$alkyl, $C_{3-6}$cycloalkyl-$C_{0-6}$alkyl, Ar-$C_{0-6}$alkyl, Het-$C_{0-6}$alkyl, $R^5C(O)$-, $R^5C(S)$-, $R^5SO_2$-, $R^5OC(O)$-, $R^5R'NC(O)$-, $R^5RNC(S)$-, adamantyl-C(O)-, or

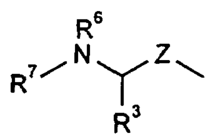
;

R" is H, $C_{1-6}$alkyl optionally substituted independently by one or two halogens, SR, OR, $N(R)_2$, $C(O)N(R)_2$, carbamyl or $C_{1-4}$alkyl, Ar-$C_{0-6}$alkyl, or Het-$C_{0-6}$alkyl;
R"' is H, $C_{1-6}$alkyl optionally substituted independently by one or two halogens, SR, OR, $N(R)_2$, $C(O)N(R)_2$, carbamyl or $C_{1-4}$alkyl, $C_{3-6}$cycloalkyl-$C_{0-6}$alkyl, Ar-$C_{0-6}$alkyl, or Het-$C_{0-6}$alkyl;
each $R^3$ independently is H, $C_{2-6}$alkenyl, $C_{2-6}$alkynyl, Het, Ar or $C_{1-6}$alkyl optionally substituted by OR', SR', $NR'_2$, $R'NC(O)OR^5$, $CO_2R'$, $CO_2NR'_2$, $N(C=NH)NH_2$, Het or Ar;

$R^4$ is H, $C_{1-6}$alkyl optionally substituted independently by one or two halogens, SR, OR, $N(R)_2$, $C(O)N(R)_2$, carbamyl or $C_{1-4}$alkyl, $C_{3-6}$cycloalkyl-$C_{0-6}$alkyl, Ar-$C_{0-6}$alkyl, Het-$C_{0-6}$alkyl, $R^5C(O)$-, $R^5C(S)$-, $R^5SO_2$-, $R^5OC(O)$-, $R^5R'NC(O)$-, $R^5R'NC(S)$-, R'HNCH(R')C(O)-, or $R^5OC(O)NR'CH(R')C(O)$-;

each $R^5$ independently is $C_{3-6}$cycloalkyl-$C_{0-6}$alkyl, Ar-$C_{0-6}$alkyl, Het-$C_{0-6}$alkyl, Ar-$C_{0-6}$alkoxy, Het-$C_{0-6}$alkoxy, or $C_{1-6}$alkyl optionally substituted by OR', SR', $NR'_2$, $R'NC(O)OR^5$, $CO_2R'$, $CO_2NR'_2$, $N(C{=}NH)NH_2$, Het or Ar;

$R^6$ is H, $C_{1-6}$alkyl optionally substituted independently by one or two halogens, SR, OR, $N(R)_2$, $C(O)N(R)_2$, carbamyl or $C_{1-4}$alkyl, Ar-$C_{0-6}$alkyl, or Het-$C_{0-6}$alkyl and $R^7$ is H, $C_{1-6}$alkyl optionally substituted independently by one or two halogens, SR, OR, $N(R)_2$, $C(O)N(R)_2$, carbamyl or $C_{1-4}$alkyl, $C_{3-6}$cycloalkyl-$C_{0-6}$alkyl, Ar-$C_{0-6}$alkyl, Het-$C_{0-6}$alkyl, $R^5C(O)$-, $R^5C(S)$-, $R^5SO_2$-, $R^5OC(O)$-, $R^5R'NC(O)$-, $R^5R'NC(S)$-, R'HNCH(R')C(O)-, or $R^5OC(O)NR'CH(R')C(O)$-; or $R^6$ and $R^7$ are connected to form a pyrrolidine, a piperidine, or a morpholine ring;

each R' independently is H, $C_{1-6}$alkyl, Ar-$C_{0-6}$alkyl, or Het-$C_{0-6}$alkyl;

R* is H, $C_{1-6}$alkyl, $C_{3-6}$cycloalkyl-$C_{0-6}$alkyl, Ar-$C_{0-6}$alkyl, or Het-$C_{0-6}$alkyl;

R is H or $C_{1-6}$alkyl;

Y is a single bond or O;

each Z independently is CO or $CH_2$;

n is 0, 1, or 2;

$C_{3-6}$cycloalkyl is optionally substituted independently by one or two halogens, SR, OR, $N(R)_2$, $C(O)N(R)_2$, carbamyl or $C_{1-4}$alkyl, where R is H or $C_{1-6}$alkyl;

Ar means unsubstituted phenyl or naphthyl; or phenyl or naphthyl substituted by one or more of Ph-$C_{0-6}$alkyl, Het-$C_{0-6}$alkyl, $C_{1-6}$alkoxy, Ph-$C_{0-6}$alkoxy, Het-$C_{0-6}$alkoxy, OH, $(CH_2)_{1-6}NR^aR^a$, $O(CH_2)_{1-6}NR^aR^a$; or phenyl or naphthyl substituted by one to three moieties selected from $C_{1-4}$alkyl, $OR^a$, $N(R^a)_2$, $SR^a$, $CF_3$, $NO_2$, CN, $CO_2R^a$, $CON(R^a)$, F, Cl, Br and I, or substituted by a methylenedioxy group; and

Het represents a stable 5- to 7-membered monocyclic or a stable 7- to 10-membered bicyclic heterocyclic ring, which is either saturated or unsaturated, and which consists of carbon atoms and from one to four heteroatoms selected from the group consisting of N, O and S, and wherein the N and S heteroatoms may optionally be oxidized, and the N heteroatom may optionally be quaternized, and including any bicyclic group in which any of the above-defined heterocyclic rings is fused to a benzene ring; wherein the Het group may be optionally substituted with one or two moieties selected from $C_{1-4}$alkyl, $OR^a$, $N(R^a)_2$, $SR^a$, $CF_3$, $NO_2$, CN, $CO_2R^a$, $CON(R^a)$, F, Cl, Br and I, and wherein each $R^a$ independently is H, $C_{1-6}$alkyl, Ar-$C_{0-6}$alkyl, or Het-$C_{0-6}$alkyl;

or a pharmaceutically acceptable salt thereof.

2. A compound according to claim 1 wherein $R^1$ is

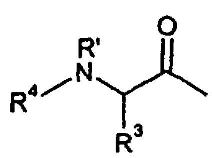

.

3. A compound according to claim 1 or claim 2 wherein $R^4$ is $R^5C(O)$-, $R^5SO_2$-, or $R^5OC(O)$-.

4. A compound according to any one of claims 1 to 3 wherein $R^5$ in said $R^1$ group is Ar-$C_{0-6}$alkyl or Het-$C_{0-6}$alkyl.

5. A compound according to any one of claims 1 to 4 wherein $R^5$ in said $R^1$ group is phenyl or benzyl which are unsubstituted or substituted by one or two of the group consisting of Cl, Br, F, $CF_3$, $C_{1-4}$alkyl, OH, $C_{1-4}$alkoxy, CN, $CONH_2$, $NH_2$, or $NO_2$, or substituted by methylenedioxy; or

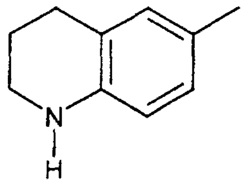 , 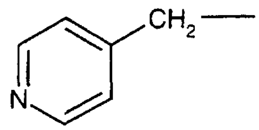 ,

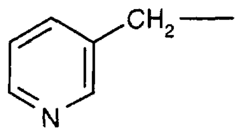 , 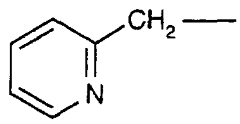 ,

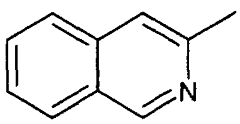 , 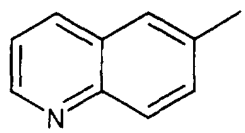 ,

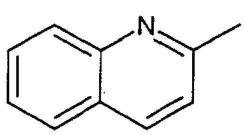 , 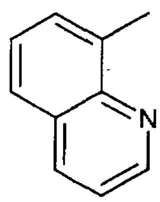 ,

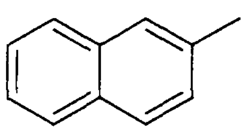 , or 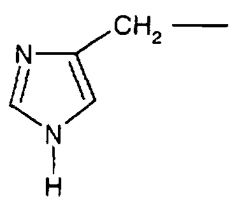

6. A compound according to claim 1 or claim 2 wherein, in said $R^1$ group, R' is H or $CH_3$ and $R^3$ is i-butyl.

7. A compound according to claim 1 wherein $R^1$ is

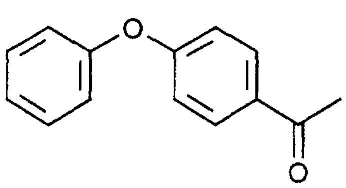 or 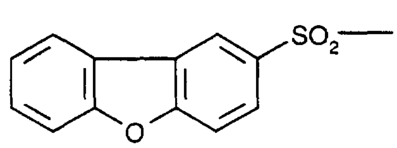 .

**8.** A compound according to claim 1 wherein $R^2$ is

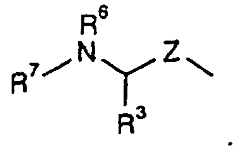

**9.** A compound according to claim 1 or claim 8 wherein $R^7$ is $R^5OC(O)$-.

**10.** A compound according to any one of claim 1, claim 8 or claim 9 wherein $R^5$ in said $R^7$ group is Ar-$C_{0-6}$alkyl or Het-$C_{0-6}$alkyl.

**11.** A compound according to any one of claim 1 or claims 8 to 10 wherein $R^5$ in said $R^7$ group is phenyl or benzyl which are unsubstituted or substituted by one or two of the group consisting of Cl, Br, F, $CF_3$, $C_{1-4}$alkyl, OH, $C_{1-4}$alkoxy, CN, $CONH_2$, $NH_2$, or $NO_2$, or substituted by methylenedioxy; or 2-, 3-, or 4-pyridyl-$CH_2$-.

**12.** A compound according to claim 1 wherein $R^2$ is

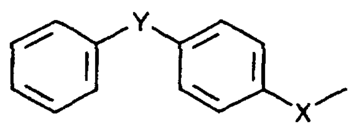

in which X is CO, $SO_2$, or $CH_2$-CO and Y is a single bond or O.

**13.** A compound according to claim 1 or claim 8 wherein, in said $R^2$ group, $R^6$ is H or $CH_3$ and $R^3$ is i-butyl.

**14.** A compound according to claim 1 wherein $R^2$ is Ar-$C_{0-6}$alkyl, Het-$C_{0-6}$alkyl,$C_{3-6}$cycloalkyl-$C_{0-6}$alkyl, $R^5C(O)$-, $R^5C(S)$-, $R^5SO_2$-, $R^5OC(O)$-, $R^5R'NC(O)$-, $R^5R'NC(S)$-, R'HNCH(R')C(O)-, $R^5OC(O)NR'CH(R')C(O)$-, or adamantyl-C(O)-.

**15.** A compound according to claim 1 wherein A is C(O).

**16.** A compound according to claim 1 which is:

(3RS,4RS)-4-[[$N^{\alpha}$-(benzyloxycarbonyl)-L-leucinyl]amino]-1-[(2S)-4-methyl-2-[[(benzyloxycarbonyl)]amino]pentanoyl]-3-pyrrolidinol;
4-[[$N^{\alpha}$-(benzyloxycarbonyl)-L-leucinyl]amino]-1-[(2S)-4-methyl-2-[[(benzyloxycarbonyl)]amino]pentanoyl]-3-pyrrolidinone;
4-[[$N^{\alpha}$-(benzyloxycarbonyl)-L-leucinyl]amino]-1-[4-(phenoxybenzamide)]-3-pyrrolidinone;
(3RS,4RS)-4-[[$N^{\alpha}$-(benzyloxycarbonyl)-L-leucinyl]amino]-1-[4-(biphenylethanoyl)]-3-pyrrolidinol;
4-[[$N^{\alpha}$-(benzyloxycarbonyl)-L-leucinyl]amino]-1-[4-(biphenylethanoyl)]-3-pyrrolidinone;
(3RS,4RS)-4-[[$N^{\alpha}$-(benzyloxycarbonyl)-L-leucinyl]amino]-1-[(2S)-4-methyl-2-[[(benzyloxycarbonyl)]aminomethyl]pentanoyl]-3-pyrrolidinol;
4-[[$N^{\alpha}$-(benzyloxycarbonyl)-L-leucinyl]amino]-1-[(2S)-4-methyl-2-[[(benzyloxycarbonyl)]aminomethyl]pentanoyl]-3-pyrrolidinone;
(3RS,4RS)-4-[[$N^{\alpha}$-(benzyloxycarbonyl)-L-leucinyl]amino]-1-[(2S)-4-methyl-2-[[(tert-butoxycarbonyl)]aminomethyl]pentanoyl]-3-pyrrolidinol;
4-[[$N^{\alpha}$-(benzyloxycarbonyl)-L-leucinyl]amino]-1-[(2S)-4-methyl-2-[[(tert-butoxycarbonyl)]aminomethyl]pentanoyl]-3-pyrrolidinone;
4-[[$N^{\alpha}$-(benzyloxycarbonyl)-L-leucinyl]amino]-1-[(2S)-4-methyl-2-(aminomethyl)pentanoyl]-3-pyrrolidinone;
4-[[$N^{\alpha}$-(benzyloxycarbonyl)-L-leucinyl]amino]-1-tert-butoxycarbonyl-3-pyrrolidinone;
4-[[$N^{\alpha}$-(benzyloxycarbonyl)-L-leucinyl]amino]-3-pyrrolidinone;
4-[[$N^{\alpha}$-(benzyloxycarbonyl)-L-leucinyl]amino]-1-[(2S)-4-methyl-2-[[(N-tert-butoxycarbonyl)ethanoyl]aminomethyl]pentanoyl]-3-pyrrolidinone;
4-[[$N^{\alpha}$-(benzyloxycarbonyl)-L-leucinyl]amino]-1-[(2S)-4-methyl-2-[(ethanoyl)aminomethyl]pentanoyl]-3-pyrrolidinone;
(3RS,4RS)-4-[[$N^{\alpha}$-(benzyloxycarbonyl)-L-leucinyl]amino]-1-[(2S)-4-methyl-2-[[(tert-butoxycarbonyl)]amino]pentanoyl]-3-pyrrolidinol;
4-[[$N^{\alpha}$-(benzyloxycarbonyl)-L-leucinyl]amino]-1-[(2S)-4-methyl-2-[[(tert-butoxycarbonyl)]amino]pentanoyl]-3-pyrrolidinone;
(3RS,4RS)-4-[[$N^{\alpha}$-(benzyloxycarbonyl)-L-leucinyl]amino]-1-[(2R)-4-methyl-2-[[(benzyloxycarbonyl)]amino]pentanoyl]-3-pyrrolidinol;
4-[[$N^{\alpha}$-(benzyloxycarbonyl)-L-leucinyl]amino]-1-[(2R)-4-methyl-2-[[(benzyloxycarbonyl)]amino]pentanoyl]-3-pyrrolidinone;
(3RS,4RS)-4-[[$N^{\alpha}$-(benzyloxycarbonyl)-L-leucinyl]amino]-1-[2-[(benzyloxycarbonyl)amino]ethanoyl]-3-pyrrolidinol;
4-[[[$N^{\alpha}$-(benzyloxycarbonyl)-L-leucinyl]amino]-1-[2-[(benzyloxycarbonyl)amino]ethanoyl]-3-pyrrolidinone;
(3RS,4RS)-4-[[$N^{\alpha}$-(benzyloxycarbonyl)-L-leucinyl]amino]-1-[(2S)-3-tert-butoxy-[[(benzyloxycarbonyl)]amino]propanoyl]-3-pyrrolidinol;
(3RS,4RS)-4-[[$N^{\alpha}$-(benzyloxycarbonyl)-L-leucinyl]amino]-1-[(2S)-2-[[(benzyloxycarbonyl)]amino]propanoyl]-3-pyrrolidinol;
4-[[$N^{\alpha}$-(benzyloxycarbony)-L-leucinyl]amino]-1-[(2S)-2-[[(benzyloxycarbonyl)]amino]propanoyl]-3-pyrrolidinone;
(3RS,4RS)-4-[[$N^{\alpha}$-(benzyloxycarbonyl)-L-leucinyl]amino]-1-[cyclohexanepropanoyl]-3-pyrrolidinol;
4-[[$N^{\alpha}$-(benzyloxycarbonyl)-L-leucinyl]amino]-1-[cyclohexanepropanoyl]-3-pyrrolidinone;
(3RS,4RS)-4-[[$N^{\alpha}$-(benzyloxycarbonyl)-L-leucinyl]amino]-1-[(2S)-4-methyl-2-[[(4-pyridinylmethoxycarbonyl)]amino]pentanoyl]-3-pyrrolidinol;
4-[[$N^{\alpha}$-(benzyloxycarbonyl)-L-leucinyl]amino]-1-[(2S)-4-methyl-2-[[(4-pyridinylmethoxycarbonyl)]amino]pentanoyl]-3-pyrrolidinone;
(3RS,4RS)-4-[[$N^{\alpha}$-(benzyloxycarbonyl)-L-leucinyl]amino]-1-[(2S)-4-methyl-2-[[(2-pyridinylmethoxycarbonyl)]amino]pentanoyl]-3-pyrrolidinol;
4-[[$N^{\alpha}$-(benzyloxycarbonyl)-L-leucinyl]amino]-1-[(2S)-4-methyl-2-[[(2-pyridinylmethoxycarbonyl)]amino]pentanoyl]-3-pyrrolidinone;
(3RS,4RS)-4-[[$N^{a}$-(benzyloxycarbonyl)-L-leucinyl]amino]-1-[(2S)-4-methyl-2-[[(3-pyridinylmethoxycarbonyl)]amino]pentanoyl]-3-pyrrolidinol;
4-[[$N^{\alpha}$-(benzyloxycarbonyl)-L-leucinyl]amino]-1-[(2S)-4-methyl-2-[[(3-pyridinylmethoxycarbonyl)]amino]pentanoyl]-3-pyrrolidinone;
(3RS,4RS)-4-[[$N^{\alpha}$-(benzyloxycarbonyl)-L-leucinyl]amino]-1-(2-pyridylcarbonyl)-3-pyrrolidinol;
4-[[$N^{\alpha}$-(benzyloxycarbonyl)-L-leucinyl]amino]-1-(2-pyridylcarbonyl)-3-pyrrolidinone;
(3RS,4RS)-4-[[$N^{\alpha}$-(benzyloxycarbonyl)-L-leucinyl]amino]-1-[(2S)-4-methyl-2-[[(benzyloxycarbonyl)]amino]pentanoyl]-3-piperidinol;
4-[[$N^{\alpha}$-(benzyloxycarbonyl)-L-leucinyl]amino]-1-[(2S)-4-methyl-2-[[(benzyloxycarbonyl)]amino]pentanoyl]-3-piperidinone;
(3RS,4RS)-4-[[$N^{\alpha}$-(benzyloxycarbonyl)-L-leucinyl]amino]-1-[4-(biphenyl)ethanoyl]- 3-piperidinol;
4-[[$N^{\alpha}$-(benzyloxycarbonyl)-L-leucinyl]amino]-1-[4-(biphenyl)ethanoyl]-3-piperidinone;
(3RS,4RS)-4-[[$N^{\alpha}$-(benzyloxycarbonyl)-L-leucinyl]amino]-1-[(2S)-4-methyl-2-[[(benzyloxycarbonyl)]aminomethyl]pentanoyl]-3-piperidinol;
4-[[$N^{\alpha}$-(benzyloxycarbonyl)-L-leucinyl]amino]-1-[(2S)-4-methyl-2-[[(benzyloxycarbonyl)]aminomethyl]pentanoyl]-3-piperidinone;
4-[[$N^{\alpha}$-(benzyloxycarbonyl)-L-leucinyl]amino]-1-tert-butoxycarbonyl-3-piperidinone;
(3RS,4RS)-4-[[$N^{\alpha}$-(benzyloxycarbonyl)-L-leucinyl]amino]-1-[2-[[(benzyloxycarbonyl)]iso-butylamino]ethanoyl]-3-piperidinol;
4-[[$N^{\alpha}$-(benzyloxycarbonyl)-L-leucinyl]amino]-1-[2-[[(benzyloxycarbonyl)]iso-butylamino]ethanoyl]-3-piperidinone;
(3RS,4RS)-4-[[$N^{\alpha}$-(benzyloxycarbonyl)-L-leucinyl]amino]-1-[2-[(tert-butoxycarbonyl)amino]ethanoyl]-3-piperidinol;
4-[[$N^{\alpha}$-(benzyloxycarbonyl)-L-leucinyl]amino]-1-[2-[(tert-butoxycarbonyl)amino]ethanoyl]-3-piperidinone;
4-[[$N^{\alpha}$-(benzyloxycarbonyl)-L-leucinyl]amino]-1-[2-(amino)ethanoyl]-3-piperidinone;
(3RS,4RS)-4-[[$N^{\alpha}$-(benzyloxycarbonyl)-L-leucinyl]amino]-1-(4-methylpentanoyl)-3-piperidinol;
4-[[$N^{\alpha}$-(benzyloxycarbonyl)-L-leucinyl]amino]-1-(4-methylpentanoyl)-3-piperidinone;
(3RS,4RS)-4-[[$N^{\alpha}$-(benzyloxycarbonyl)-L-leucinyl]amino]-1-(benzoyl)-3-piperidinol;

4-[[N$^{\alpha}$-(benzyloxycarbonyl)-L-leucinyl]amino]-1-(benzoyl)-3-piperidinone;
(3RS,4RS)-4-[[N$^{\alpha}$-(benzyloxycarbonyl)-L-leucinyl]amino]-1-(acetyl)-3-piperidinol;
4-[[N$^{\alpha}$-(benzyloxycarbonyl)-L-leucinyl]amino]-1-(acetyl)-3-piperidinone;
(3RS,4RS)-4-[[N$^{\alpha}$-(benzyloxycarbonyl)-L-leucinyl]amino]-1-(2-pyridoxyacetyl)-3-piperidinol;
4-[[N$^{\alpha}$-(benzyloxycarbonyl)-L-leucinyl]amino]-1-(2-pyridoxyacetyl)-3-piperidinone;
(3RS,4RS)-4-[[N$^{\alpha}$-(benzyloxycarbonyl)-L-leucinyl]amino]-1-[2-[(benzyloxycarbonyl)methylamino]ethanoyl]-3-piperidinol;
4-[[N$^{\alpha}$-(benzyloxycarbonyl)-L-leucinyl]amino]-1-[2-[(benzyloxycarbonyl)methylamino]ethanoyl]-3-piperidinone;
(3RS,4RS)-4-[[N$^{\alpha}$-(benzyloxycarbonyl)-L-leucinyl]amino]-1-[3-(2-pyridyl)phenylacetyl)]-3-piperidinol;
4-[[N$^{\alpha}$-(benzyloxycarbonyl)-L-leucinyl]amino]-1-(3-(2-pyridyl)phenylacetyl)]-3-piperidinone;
(3RS,4RS)-4-[[N$^{\alpha}$-(benzyloxycarbonyl)-L-leucinyl]amino]-1-[2-[(benzyloxycarbonyl)methylamino]ethanoyl]-3-pyrrolidinol;
4-[[N$^{\alpha}$-(benzyloxycarbonyl)-L-leucinyl]amino]-1-[2-[(benzyloxycarbonyl)methylamino]ethanoyl]-3-pyrrolidinone;
(3RS,4RS)-4-[[N$^{\alpha}$-(benzyloxycarbonyl)-L-leucinyl]amino]-1-[2-(phenoxy)ethanoyl]-3-pyrrolidinol;
4-[[N$^{\alpha}$-(benzyloxycarbonyl)-L-leucinyl]amino]-1-[2-(phenoxy)ethanoyl]-3-pyrrolidinone;
(3RS,4RS)-4-[[N$^{\alpha}$-(4-pyridinylmethoxycarbonyl)-L-leucinyl]amino]-1-[(2-phenyl)ethanoyl]-3-pyrrolidinol;
4-[[N$^{\alpha}$-(4-pycidinylmethoxycarbonyl)-L-leucinyl]amino]-1-[(2-phenyl)ethanoyl]-3-pyrrolidinone;
(3RS,4RS)-4-[[N$^{\alpha}$-(4-pyridinylmethoxycarbonyl)-L-leucinyl]amino]-1-ethanonyl-3-pyrrolidinol;
4-[[N$^{\alpha}$-(4-pyridinylmethoxycarbonyl)-L-leucinyl]amino]-1-ethanoyl-3-pyrrolidinone;
(3RS,4RS)-4-[[N$^{\alpha}$-(4-pyridinylmethoxycarbonyl)-L-leucinyl)amino]-1-(4-cyanobenzoyl)-3-pyrrolidinol;
4-[[N$^{\alpha}$-(4-pyridinylmethoxycarbonyl)-L-leucinyl]amino]-1-(4-cyanobenzoyl)-3-pyrrolidinone;
(3RS,4RS)-4-[[N$^{\alpha}$-(4-pyridinylmethoxycarbonyl)-L-leucinyl]amino]-1-tert-butoxycarbonyl-3-pyrrolidinol;
4-[[N$^{\alpha}$-(4-pyridinylmethoxycarbonyl)-L-leucinyl]amino]-1-tert-butoxycarbonyl-3-pyrrolidinone;
(3RS,4RS)-4-[[N$^{\alpha}$-(3-pyridinylmethoxycarbonyl)-L-leucinyl]amino]-1-tert-butoxycarbonyl-3-pyrrolidinol;
4-[[N$^{\alpha}$-(3-pyridinylmethoxycarbonyl)-L-leucinyl]amino]-1-tert-butoxycarbonyl-3-pyrrolidinone;
4-[[N$^{\alpha}$-(3-pyridinylmethoxycarbonyl)-L-leucinyl]amino]-3-pyrrolidinone;
4-[[N$^{\alpha}$-(4-pyridinylmethoxycarbonyl)-L-leucinyl]amino]-3-pyrrolidinone;
(3RS,4RS)-4-[[N$^{\alpha}$-(2-pyridinylmethoxycarbonyl)-L-leucinyl]amino]-1-[(2S)-4-methyl-2-[[(benzyloxycarbonyl)]aminomethyl]pentanoyl]-3-pyrrolidinone;
4-[[N$^{\alpha}$-(2-pyridinylmethoxycarbonyl)-L-leucinyl]amino]-1-[(2S)-4-methyl-2-[[(benzyloxycarbonyl)]aminomethyl]pentanoyl]-3-pyrrolidinone;
(3RS,4RS)-4-[[N$^{\alpha}$-(4-pyridinylmethoxycarbonyl)-L-leucinyl]amino]-1-[(2S)-4-methyl-2-[[(4-pyridinylmethoxycarbonyl)]amino]pentanoyl]-3-pyrrolidinol;
4-[[N$^{\alpha}$-(4-pyridinylmethoxycarbonyl)-L-leucinyl]amino]-1-[(2S)-4-methyl-2-[[(4-pyridinylmethoxycarbonyl)]amino]pentanoyl]-3-pyrrolidinone;
(3RS,4RS)-4-[[N$^{\alpha}$-(4-pyridinylmethoxycarbonyl)-L-leucinyl]amino]-1-[(2S)-4-methyl-2-[[(benzyloxycarbonyl)]aminomethyl]pentanoyl]-3-pyrrolidinol;
4-[[N$^{\alpha}$-(4-pyridinylmethoxycarbonyl)-L-leucinyl]amino]-1-[(2S)-4-methyl-2-[[(benzyloxycarbonyl)]aminomethyl]pentanoyl]-3-pyrrolidinone;
(3RS,4RS)-4-[[N$^{\alpha}$-(3-isoquinolinecarbonyl)-L-leucinyl]amino]-1-[(2S)-4-methyl-2-[[(benzyloxycarbonyl)]amino]pentyl]-3-pyrrolidinol;
4-[[N$^{\alpha}$-(3-isoquinolinecarbonyl)-L-leucinyl]amino]-1-[(2S)-4-methyl-2-[[(benzyloxycarbonyl)]amino]pentyl]-3-pyrrolidinone;
(3RS,4RS)-4-[[N$^{\alpha}$-(4-pyridinylmethoxycarbonyl)-L-leucinyl]amino]-1-[1-(adamantyl)carbonyl]-3-pyrrolidinol;
4-[[N$^{\alpha}$-(4-pyridinylmethoxycarbonyl)-L-leucinyl]amino]-1-[1-(adamantyl)carbonyl]-3-pyrrolidinone;
(3RS,4RS)-4-[[N$^{\alpha}$-(benzyloxycarbonyl)-L-leucinyl]amino]-1-(4-methylpentanoyl)-3-pyrrolidinol;
4-[[N$^{\alpha}$-(benzyloxycarbonyl)-L-leucinyl]amino]-1-(4-methyl-pentanoyl)-3-pyrrolidinone;
(3RS,4RS)-4-[[N$^{\alpha}$-(benzyloxycarbonyl)-D-leucinyl]amino]-1-[(2S)-4-methyl-2-[[(benzyloxycarbonyl)]amino]pentanoyl]-3-piperidinol;
4-[[N$^{\alpha}$-(benzyloxycarbonyl)-D-leucinyl]amino]-1-[(2S)-4-methyl-2-[[(benzyloxycarbonyl)]amino]pentanoyl]-3-piperidinone;
(3RS,4RS)-4-[[N$^{\alpha}$-(tert-butoxycarbonyl)-L-leucinyl]amino]-1-[(2S)-4-methyl-2-[[(benzyloxycarbonyl)]amino]pentanoyl]-3-piperidinol;
4-[[N$^{\alpha}$-(tert-butoxycarbonyl)-L-leucinyl]amino]-1-[(2S)-4-methyl-2-[[(benzyloxycarbonyl)]amino]pentanoyl]-3-piperidinone;
(3RS,4RS)-4-[[N$^{\alpha}$-(benzyloxycarbonyl)-N$^{\varepsilon}$-(tert-butoxycarbonyl)-L-lysine]amino]-1-[(2S)-4-methyl-2-[[(benzyloxycarbonyl)]amino]pentanoyl]-3-piperidinol;
4-[[N$^{\alpha}$-(benzyloxycarbonyl)-N$^{\varepsilon}$-(tert-butoxycarbonyl)-L-lysine]amino]-1-[(2S)-4-methyl-2-[[(benzyloxycarbonyl)]amino]pentanoyl]-3-piperidinone;
(3RS,4RS)-4-[[N$^{\alpha}$-(4-pyridinylmethoxycarbonyl)-L-leucinyl]amino]-1-tert-butoxycarbonyl-3-piperidinol;
4-[[N$^{\alpha}$-(4-pyridinylmethoxycarbonyl)-L-leucinyl]amino]-1-tert-butoxycarbonyl-3-piperidinone;
(3RS,4RS)-4-[[N$^{\alpha}$-(4-pyridinylmethoxycarbonyl)-L-leucinyl]amino]-1-(4-methylpentanoyl)-3-piperidinol;
4-[[N$^{\alpha}$-(4-pyridinylmethoxycarbonyl)-L-leucinyl]amino]-1-(4-methylpentanoyl)-3-piperidinone;
(3RS,4RS)-4-[[N$^{\alpha}$-(4-pyridinylmethoxycarbonyl)-L-leucinyl]amino]-1-[2-(benzyloxycarbonyl)]iso-butylamino]ethanoyl]-3-piperidinol;
4-[[N$^{\alpha}$-(4-pyridinylmethoxycarbonyl)-L-leucinyl]amino]-1-[2-(benzyloxycarbonyl)]iso-butylamino]ethanoyl]-3-piperidinone;
(3RS,4RS)-4-[[[N-2-(benzyloxycarbonyl)]iso-butylamino]ethanoyl]-1-[(2S)-4-methyl-2-[[(benzyloxycarbonyl)]amino]pentanoyl]-3-piperidinol;
4-[[[N-2-(benzyloxycarbonyl)]iso-butylamino]ethanoyl]-1-[(2S)-4-methyl-2-[[(benzyloxycarbonyl)]amino]pentanoyl]-3-piperidinone;
(3RS,4RS)-4-[[N$^{\alpha}$-(benzyloxycarbonyl)-L-leucinyl]amino]-1-(methanesulphonyl)-3-piperidinol;
4-[[N$^{\alpha}$-(benzyloxycarbonyl)-L-leucinyl]amino]-1-(methanesulphonyl)-3-piperidinone;
(3RS,4RS)-4-[[N$^{\alpha}$-(benzyloxycarbonyl)-L-leucinyl]amino]-1-(phenylsulphonyl)-3-piperidinol;
4-[[N$^{\alpha}$-(benzyloxycarbonyl)-L-leucinyl]amino]-1-(phenylsulphonyl)-3-piperidinone;
(3RS,4RS)-4-[[N$^{\alpha}$-(4-pyridinylmethoxycarbonyl)-L-leucinyl]amino]-1-(8-quinolinesulphonyl)-3-pyrrolidinol;
4-[[N$^{\alpha}$-(4-pyridinylmethoxycarbonyl)-L-leucinyl]amino]-1-(8-quinolinesulphonyl)-3-pyrrolidinone;
(3RS,4RS)-4-[[N$^{\alpha}$-(4-pyridinylmethoxycarbonyl)-L-leucinyl]amino]-1-(2-pyridylsulphonyl)-3-pyrrolidinone;
4-[[N$^{\alpha}$-(4-pyridinylmethoxycarbonyl)-L-leucinyl]amino]-1-(2-pyridylsulphonyl)-3-pyrrolidinone;
(3RS,4RS)-4-[[N$^{\alpha}$-(4-pyridinylmethoxycarbonyl)-L-leucinyl]amino]-1-[(2-propoxy)carbonyl]-3-pyrrolidinol;
4-[[N$^{\alpha}$-(4-pyridinylmethoxycarbonyl)-L-leucinyl]amino]-1-[(2-propoxy)carbonyl]-3-pyrrolidinone;
(3RS,4RS)-4-[[N$^{\alpha}$-(4-pyridinylmethoxycarbonyl)-L-leucinyl]amino]-1-[(3-methyl-1-propoxy)carbonyl]-3-pyrrolidinol;
4-[[N$^{\alpha}$-(4-pyridinylmethoxycarbonyl)-L-leucinyl]amino]-1-[(3-methyl-1-propoxy)carbonyl]-3-pyrrolidinone;
(3RS,4RS)-4-[[N$^{\alpha}$-(benzyloxycarbonyl)-L-leucinyl]amino]-1-[(4-phenoxy)phenylsulphonyl]-3-pyrrolidinol;
4-[[N$^{\alpha}$-(benzyloxycarbonyl)-L-leucinyl]amino]-1-[(4-phenoxy)phenylsulphonyl]-3-pyrrolidinone;
(3RS,4RS)-4-[[N$^{\alpha}$-(benzyloxycarbonyl)-L-leucinyl]amino]-1-[(4-phenoxy)phenylsulphonyl]-3-piperidinol;
4-[[N$^{\alpha}$-(benzyloxycarbonyl)-L-leucinyl]amino]-1-[(4-phenoxy)phenylsulphonyl]-3-piperidinone;
(3RS,4RS)-4-[[N$^{\alpha}$-(3,4-dichlorobenzoyl)-L-leucinyl]amino]-1-[(2S)-4-methyl-2-[[(benzyloxycarbonyl)]amino]pentyl]-3-pyrrolidinol;
4-[[N$^{\alpha}$-(3,4-dichlorobenzoyl)-L-leucinyl]amino]-1-[(2S)-4-methyl-2-[[(benzyloxycarbonyl)]amino]pentyl]-3-pyrrolidinone;
(3RS,4RS)-4-[[N$^{\alpha}$-(6-quinolinecarbonyl)-L-leucinyl]amino]-1-[(2S)-4-methyl-2-[[(benzyloxycarbonyl)]amino]pentyl]-3-pyrrolidinol;
4-[[N$^{\alpha}$-(6-quinolinecarbonyl)-L-leucinyl]amino]-1-[(2S)-4-methyl-2-[[(benzyloxycarbonyl)]amino]pentyl]-3-pyrrolidinone;
(3RS,4RS)-4-[(2-dibenzofuransulphonyl)amino]-1-[(2S)-4-methyl-2-[[(benzyloxycarbonyl)]amino]pentanoyl]-3-pyrrolidinol;
4-[(2-dibenzofuransulphonyl)amino]-1-[(2S)-4-methyl-2-[[(benzyloxycarbonyl)]amino]pentanoyl]-3-pyrrolidinone;
(3RS,4RS)-4-[(2-dibenzofuransulphonyl)amino]-1-[(2S)-4-methyl-2-[[(benzyloxycarbonyl)]methylamino]pentanoyl]-3-pyrrolidinol;
4-[(2-dibenzofuransulphonyl)amino]-1-[(2S)-4-methyl-2-[[(benzyloxycarbonyl)]methylamino]pentanoyl]-3-pyrrolidinone;
(3RS,4RS)-4-[[N$^{\alpha}$-(benzyloxycarbonyl)-L-leucinyl]amino]-1-(4-methylpentyl)-3-piperidinol;
4-[[N$^{\alpha}$-(benzyloxycarbonyl)-L-leucinyl]amino]-1-(4-methylpentyl)-3-piperidinone;
(3RS,4RS)-4-[[N$^{\alpha}$-(2-pyridylcarbonyl)-L-leucinyl]amino]-1-(4-methylpentyl)-3-piperidinol;
4-[[N$^{\alpha}$-(2-pyridylcarbonyl)-L-leucinyl]amino]-1-(4-methylpentyl)-3-piperidinone;
(3RS,4RS)-4-[[N$^{\alpha}$-(3-chlorobenzoyl)-L-leucinyl]amino]-1-(4-methylpentyl)-3-piperidinol;
4-[[N$^{\alpha}$-(3-chlorobenzoyl)-L-leucinyl]amino]-1-(4-methylpentyl)-3-piperidinone;
(3RS,4RS)-4-[[N$^{\alpha}$-(2-quinolinecarbonyl)-L-leucinyl]amino]-1-(4-methylpentyl)-3-piperidinol;
4-[[N$^{\alpha}$-(2-quinolinecarbonyl)-L-leucinyl]amino]-1-(4-methylpentyl)-3-piperidinone;
(3RS,4RS)-4-[[N$^{\alpha}$-(3,4-dichlorobenzoyl)-L-leucinyl]amino]-1-(4-methylpentyl)-3-piperidinol;
4-[[N$^{\alpha}$-(3,4-dichlorobenzoyl)-L-leucinyl]amino]-1-(4-methylpentyl)-3-piperidinone;

(3RS,4RS)-4-[[N$^{\alpha}$-(8-quinolinecarbonyl)-L-leucinyl]amino]-1-(4-methylpentyl)-3-piperidinol;
4-[[N$^{\alpha}$-(8-quinolinecarbonyl)-L-leucinyl]amino]-1-(4-methylpentyl)-3-piperidinone;
(3RS,4RS)-4-[[N$^{\alpha}$-(3-isoquinolinecarbonyl)-L-leucinyl]amino]-1-(4-methylpentyl)-3-piperidinol;
4-[[N$^{\alpha}$-(3-isoquinolinecarbonyl)-L-leucinyl]amino]-1-(4-methylpentyl)-3-piperidinone;
(3RS,4RS)-4-[[N$^{\alpha}$-(2-pyridinylmethoxycarbonyl)-L-leucinyl]amino]-1-(4-methylpentyl)-3-piperidinol;
4-[[N$^{\alpha}$-(2-pyridinylmethoxycarbonyl)-L-leucinyl]amino]-1-(4-methylpentyl)-3-piperidinone;
(3RS,4RS)-4-[[N$^{\alpha}$-(acetyl)-L-leucinyl]amino]-1-(4-methylpentyl)-3-piperidinol;
4-[[N$^{\alpha}$-(acetyl)-L-leucinyl]amino]-1-(4-methylpentyl)-3-piperidinone;
(3RS,4RS)-4-[[N$^{\alpha}$-(p-trifluoromethylbenzenesulphonyl)-L-leucinyl]amino]-1-(4-methylpentyl)-3-piperidinol;
4-[[N$^{\alpha}$-(p-trifluoromethylbenzenesulphonyl)-L-leucinyl]amino]-1-(4-methylpentyl)-3-piperidinone;
(3RS,4RS)-4-[[N$^{\alpha}$-(6-quinolinecarbonyl)-L-leucinyl]amino]-1-(4-methylpentyl)-3-piperidinol;
4-[[N$^{\alpha}$-(6-quinolinecarbonyl)-L-leucinyl]amino]-1-(4-methylpentyl)-3-piperidinone;
(3RS,4RS)-4-[[2-(RS)-(3-biphenyl)-4-methyl]amino]pentanoyl]-1-(4-methylpentyl)-3-piperidinol;
4-[[2-(RS)-[(3-biphenyl)-4-methyl]amino]pentanoyl]-1-(4-methylpentyl)-3-piperidinone;
(3RS,4RS)-4-[[N$^{\alpha}$-(benzyloxycarbonyl)-L-leucinyl]amino]-1-[2-[(benzyloxycarbonyl)methylamino]ethyl]-3-piperidinol;
4-[[N$^{\alpha}$-(benzyloxycarbonyl)-L-leucinyl]amino]-1-[2-[(benzyloxycarbonyl)methylamino]ethyl]-3-piperidinone;
(3RS,4RS)-4-[[N$^{\alpha}$-($\alpha$-toluenesulphonyl)-L-leucinyl]amino]-1-[3-(2-pyridyl)phenylacetyl)]-3-piperidinol;
4-[[N$^{\alpha}$-($\alpha$-toluenesulphonyl)-L-leucinyl]amino]-1-[3-(2-pyridyl)phenylacetyl)]-3-piperidinone;
(3RS,4RS)-4-[[N$^{\alpha}$-(2-naphthylcarbonyl)-L-leucinyl]amino]-1-[3-(2-pyridyl)phenylacetyl)]-3-piperidinone;
4-[[N$^{\alpha}$-(2-naphthylcarbonyl)-L-leucinyl]amino]-1-[3-(2-pyridyl)phenylacetyl)]-3-piperidinone;
(3RS,4RS)-4-[(N$^{\alpha}$-(benzenesulphonyl)-L-leucinyl]amino]-1-[3-(2-pyridyl)phenylacetyl)]-3-piperidinol;
4-[[N$^{\alpha}$-(benzenesulphonyl)-L-leucinyl]amino]-1-[3-(2-pyridyl)phenylacetyl)]-3-piperidinone;
(3RS,4RS)-4-[[N$^{\alpha}$-(3-isoquinolinecarbonyl)-L-leucinyl]amino]-1-[3-(2-pyridyl)phenylacetyl)]-3-piperidinol;
4-[[N$^{\alpha}$-(3-isoquinolinecarbonyl)-L-leucinyl]amino]-1-[3-(2-pyridyl)phenylacetyl)]-3-piperidinone;
(3RS,4RS)-4-[3-[(2-pyridyl)phenylacetyl)]amino]-1-[(2S)-4-methyl-2-[[(benzyloxycarbonyl)]amino]pentanoyl]-3-piperidinol;
4-[3-[(2-pyridyl)phenylacetyl)]amino]-1-[(2S)-4-methyl-2-[[(benzyloxycarbonyl)]amino]pentanoyl]-3-piperidinone;
(3RS,4RS)-4-[3-[(2-pyridyl)phenylacetyl)]amino]-1-[(2S)-4-methyl-2-[[(2-pyridinylmethoxycarbonyl)]amino]pentanoyl]-3-piperidinol;
4-[3-[(2-pyridyl)phenylacetyl)]aminol-1-[(2S)-4-methyl-2-[[2-(pyridinylmethoxycarbonyl)]amino]pentanoyl]-3-piperidinone;
(3RS,4RS)-4-[[N$^{\alpha}$-(2-phenylacetyl)-L-leucinyl]amino]-1-[(2S)-4-methyl-2-[[(benzyloxycarbonyl)]amino]pentyl]-3-pyrrolidinol;
4-[[N$^{\alpha}$-(2-phenylacetyl)-L-leucinyl]amino]-1-[(2S)-4-methyl-2-[[(benzyloxycarbonyl)]amino]pentyl]-3-pyrrolidinone;
4-[[N$^{\alpha}$-(tert-butoxycarbonyl)-L-leucinyl]amino]-1-[(2S)-4-methyl-2-[[(benzyloxycarbonyl)]amino]pentyl]-3-pyrrolidinone;
4-[(L-leucinyl)amino]-1-[(2S)-4-methyl-2-[[(benzyloxycarbonyl)lamino]pentyl]-3-pyrrolidinone;
(3RS,4RS)-4-[[N$^{\alpha}$-(2-quinolinecarbonyl)-L-leucinyl]amino]-1-[(2S)-4-methyl-2-[[(benzyloxycarbonyl)]amino]pentyl]-3-pyrrolidinol;
4-[[N$^{\alpha}$-(2-quinolinecarbonyl)-L-leucinyl]amino]-1-[(2S)-4-methyl-2-[[(benzyloxycarbonyl)]amino]pentyl]-3-pyrrolidinone;
(3RS,4RS)-4-[[N$^{\alpha}$-(piperonylcarbonyl)-L-leucinyl]amino]-1-[(2S)-4-methyl-2-[[(benzyloxycarbonyl)]amino]pentyl]-3-pyrrolidinol;
4-[[N$^{\alpha}$-(piperonylcarbonyl)-L-leucinyl]amino]-1-[(2S)-4-methyl-2-[[(benzyloxycarbonyl)]amino]pentyl]-3-pyrrolidinone;
(3RS,4RS)-4-[[N$^{\alpha}$-(4-fluorobenzoyl)-L-leucinyl]amino]-1-[(2S)-4-methyl-2-[[(benzyloxycarbonyl)]amino]pentyl]-3-pyrrolidinol;
4-[[N$^{\alpha}$-(4-fluorobenzoyl)-L-leucinyl]amino]-1-[(2S)-4-methyl-2-[[(benzyloxycarbonyl)]amino]pentyl]-3-pyrrolidinone;
(3RS,4RS)-4-[[N$^{\alpha}$-(2-pyridylcarbonyl)-L-leucinyl]amino]-1-[(2S)-4-methyl-2-[[(benzyloxycarbonyl)]amino]pentyl]-3-pyrrolidinol;
4-[[N$^{\alpha}$-(2-pyridylcarbonyl)-L-leucinyl]amino]-1-[(2S)-4-methyl-2-[[(benzyloxycarbonyl)]amino]pentyl]-3-pyrrolidinone;
(3RS,4RS)-4-[[N$^{\alpha}$-(2-nitro-$\alpha$-toluenesulphonyl)-L-leucinyl]amino]-1-[(2S)-4-methyl-2-[[(benzyloxycarbonyl)]amino]pentyl]-3-pyrrolidinol;

4-[[N$^{\alpha}$-(2-nitro-$\alpha$-toluenesulphonyl)-L-leucinyl]amino]-1-[(2S)-4-methyl-2-[[(benzyloxycarbonyl)]amino]pentyl]-3-pyrrolidinone;
(3RS,4RS)-4-[[N$^{\alpha}$-(8-quinolinesulphonyl)-L-leucinyl]amino]-1-[(2S)-4-methyl-2-[[(benzyloxycarbonyl)]amino]pentyl]-3-pyrrolidinol;
4-[[N$^{\alpha}$-(8-quinolinesulphonyl)-L-leucinyl]amino]-1-[(2S)-4-methyl-2-[[(benzyloxycarbonyl)]amino]pentyl]-3-pyrrolidinone;
(3RS,4RS)-4-[[N$^{\alpha}$-(2-naphthylcarbonyl)-L-leucinyl]aminomethyl]-1-[(2S)-4-methyl-2-[[(benzyloxycarbonyl)]amino]pentyl]-3-pyrrolidinol;
4-[[N$^{\alpha}$-(2-naphthylcarbonyl)-L-leucinyl]aminomethyl]-1-[(2S)-4-methyl-2-[[(benzyloxycarbonyl)]amino]pentyl]-3-pyrrolidinone;
(3RS,4RS)-4-[[N$^{\alpha}$-(2-quinolinylcarbonyl)-L-leucinyl]aminomethyl]-1-[(2S)-4-methyl-2-[[(benzyloxycarbonyl)]amino]pentyl]-3-pyrrolidinol;
4-[[N$^{\alpha}$-(2-quinolinylcarbonyl)-L-leucinyl]aminomethyl]-1-[(2S)-4-methyl-2-[[(benzyloxycarbonyl)]amino]pentyl]-3-pyrrolidinone;
(3RS,4RS)-4-[[N$^{\alpha}$-(phenylacetyl)-L-leucinyl]amino]-1-[(2S)-4-methyl-2-[[(benzyloxycarbonyl)]amino]pentyl]-3-piperidinol;
4-[[N$^{\alpha}$-(phenylacetyl)-L-leucinyl]amino]-1-[(2S)-4-methyl-2-[[(benzyloxycarbonyl)]amino]pentyl]-3-piperidinone;
(3RS,4RS)-4-[[N$^{\alpha}$-(4-pyridinylmethoxycarbonyl)-L-leucinyl]amino]-1-[(2S)-4-methyl-2-[[(benzyloxycarbonyl)]amino]pentyl]-3-piperidinol;
4-[[N$^{\alpha}$-(4-pyridinylmethoxycarbonyl)-L-leucinyl]amino]-1-[(2S)-4-methyl-2-[[(benzyloxycarbonyl)]amino]pentyl]-3-piperidinone;
(3RS,4RS)-4-[[N$^{\alpha}$-(4-pyridinylmethoxycarbonyl)-L-leucinyl]amino]-1-[(2R)-4-methyl-2-[[(benzyloxycarbonyl)]amino]pentyl]-3-piperidinol;
4-[[N$^{\alpha}$-(4-pyridinylmethoxycarbonyl)-L-leucinyl]amino]-1-[(2R)-4-methyl-2-[[(benzyloxycarbonyl)]amino]pentyl]-3-piperidinone;
(3RS,4RS)-4-[[N$^{\alpha}$-(phenylacetyl)-L-leucinyl]amino]-1-[(2R)-4-methyl-2-[[(benzyloxycarbonyl)]amino]pentyl]-3-piperidinol;
4-[[N$^{\alpha}$-(phenylacetyl)-L-leucinyl]amino]-1-[(2R)-4-methyl-2-[[(benzyloxycarbonyl)]amino]pentyl]-3-piperidinone;
(3RS,4RS)-4-[[N$^{\alpha}$-(4-imidazoleacetyl)-L-leucinyl]amino]-1-[(2R)-4-methyl-2-[[(benzyloxycarbonyl)]amino]pentyl]-3-piperidinol;
4-[[N$^{\alpha}$-(4-imidazoleacetyl)-L-leucinyl]amino]-1-[(2R)-4-methyl-2-[[(benzyloxycarbonyl)]amino]pentyl]-3-piperidinone;
(3RS,4RS)-4-[[N$^{\alpha}$-(4-imidazoleacetyl)-L-leucinyl]amino]-1-[(2S)-4-methyl-2-[[(benzyloxycarbonyl)]amino]pentyl]-3-piperidinol;
4-[[N$^{\alpha}$-(4-imidazoleacetyl)-L-leucinyl]amino]-1-[(2S)-4-methyl-2-[[(benzyloxycarbonyl)]amino]pentyl]-3-piperidinone;
(3RS,4RS)-4-[[N$^{\alpha}$-(4-pyridinylcarbonyl)-L-leucinyl]amino]-1-[(2S)-4-methyl-2-[[(benzyloxycarbonyl)]amino]pentyl]-3-piperidinol;
4-[[N$^{\alpha}$-(4-pyridinylcarbonyl)-L-leucinyl]amino]-1-[(2S)-4-methyl-2-[[(benzyloxycarbonyl)]amino]pentyl]-3-piperidinone;
4-[[N$^{\alpha}$-(tert-butoxycarbonyl)-L-leucinyl]amino]-1-(benzyloxycarbonyl)-3-piperidinone;
(3RS,4RS)-4-[[N$^{\alpha}$-(8-quinolinesulphonyl)-L-leucinyl]amino]-1-(benzyloxycarbonyl)-3-piperidinol;
4-[[N$^{\alpha}$-(8-quinolinesulphonyl)-L-leucinyl]amino]-1-(benzyloxycarbonyl)-3-piperidinone;
(3RS,4RS)-4-[[N$^{\alpha}$-(4-pyridinylacetyl)-L-leucinyl]amino]-1-(benzyloxycarbonyl)-3-piperidinol;
4-[[N$^{\alpha}$-(4-pyridinylacetyl)-L-leucinyl]amino]-1-(benzyloxycarbonyl)-3-piperidinone;
(3RS,4RS)-4-[[N$^{\alpha}$-(4-imidazoleacetyl)-L-leucinyl]amino]-1-(benzyloxycarbonyl)-3-piperidinol;
4-[[N$^{\alpha}$-(4-imidazoleacetyl)-L-leucinyl]amino]-1-(benzyloxycarbonyl)-3-piperidinone;
(3RS,4RS)-4-[[N$^{\alpha}$-(4-pyridinylcarbonyl)-L-leucinyl]amino]-1-(benzyloxycarbonyl)-3-piperidinol;
4-[[N$^{\alpha}$-(4-pyridinylcarbonyl)-L-leucinyl]amino]-1-(benzyloxycarbonyl)-3-piperidinone;
(3RS,4RS)-1-benzyl-4-[[N$^{\alpha}$-(3-isoquinolinylcarbonyl)-L-leucinyl]amino]-3-pyrrolidinol;
1-benzyl-4-[[N$^{\alpha}$-(3-isoquinolinylcarbonyl)-L-leucinyl]amino]-3-pyrrolidinone;
(3RS,4RS)-1-benzyl-4-[[N$^{\alpha}$-(3,4-dichlorobenzoyl)-L-leucinyl]amino]-3-pyrrolidinol;
1-benzyl-4-[[N$^{\alpha}$-(3,4-dichlorobenzoyl)-L-leucinyl]amino]-3-pyrrolidinone;
(3RS,4RS)-1-benzyl-4-[[N$^{\alpha}$-(2-naphthylcarbonyl)-L-leucinyl]aminomethyl]-3-pyrrolidinol;
1-benzyl-4-[[N$^{\alpha}$-(2-naphthylcarbonyl)-L-leucinyl]aminomethyl]-3-pyrrolidinone;
(3RS,4RS)-1-benzyl-4-[[N$^{\alpha}$-(2-quinolinylcarbonyl)-L-leucinyl]aminomethyl]-3-pyrrolidinol;

1-benzyl-4-[[N$^{\alpha}$-(2-quinolinylcarbonyl)-L-leucinyl]aminomethyl]-3-pyrrolidinone;
(3RS,4RS)-1-benzyl-4-[[N$^{\alpha}$-(2-quinolinylcarbonyl)-L-leucinyl]amino]-3-pyrrolidinol;
1-benzyl-4-[[N$^{\alpha}$-(2-quinolinylcarbonyl)-L-leucinyl]amino]-3-pyrrolidinone;
(3RS,4RS)-1-benzyl-4-[[N$^{\alpha}$-(piperonylcarbonyl)-L-leucinyl]amino]-3-pyrrolidinol;
1-benzyl-4-[[N$^{\alpha}$-(piperonylcarbonyl)-L-leucinyl]amino]-3-pyrrolidinone;
(3RS,4RS)-1-benzyl-4-[[N$^{\alpha}$-(4-fluorobenzoyl)-L-leucinyl]amino]-3-pyrrolidinol;
1-benzyl-4-[[N$^{\alpha}$-(4-fluorobenzoyl)-L-leucinyl]amino]-3-pyrrolidinone;
(3RS,4RS)-1-benzyl-4-[[N$^{\alpha}$-(6-hydroxy-2-naphthylcarbonyl)-L-leucinyl]amino]-3-pyrrolidinol;
1-benzyl-4-[[N$^{\alpha}$-(6-hydroxy-2-naphthylcarbonyl)-L-leucinyl]amino]-3-pyrrolidinone;
(3RS,4RS)-1-benzyl-4-[[N$^{\alpha}$-(2-naphthylcarbonyl)-L-leucinyl]amino]-3-pyrrolidinol;
1-benzyl-4-[[N$^{\alpha}$-(2-naphthylcarbonyl)-L-leucinyl]amino]-3-pyrrolidinone;
(3RS,4RS)-1-benzyl-4-[[N$^{\alpha}$-(6-quinolinylcarbonyl)-L-leucinyl]amino]-3-pyrrolidinol;
1-benzyl-4-[[N$^{\alpha}$-(6-quinolinylcarbonyl)-L-leucinyl]amino]-3-pyrrolidinone;
(3RS,4RS)-1-benzyl-4-[[N$^{\alpha}$-(4-imidazoleacetyl)-L-leucinyl]amino]-3-pyrrolidinol;
1-benzyl-4-[[N$^{\alpha}$-(4-imidazoleacetyl)-L-leucinyl]amino]-3-pyrrolidinone;
(3RS,4RS)-1-benzyl-4-[[N$^{\alpha}$-(4-pyridinylcarbonyl)-L-leucinyl]amino]-3-pyrrolidinol;
1-benzyl-4-[[N$^{\alpha}$-(4-pyridinylcarbonyl)-L-leucinyl]amino]-3-pyrrolidinone;
4-[[N$^{\alpha}$-(tert-butoxycarbonyl)-L-leucinyl]amino]-1-benzyloxycarbonyl-3-pyrrolidinone;
(3RS,4RS)-4-[[N$^{\alpha}$-(4-pyridinylmethoxy)carbonyl]-L-leucinyl]amino]-1-[(2R)-4-methyl-2-[[(benzyloxycarbonyl)]amino]pentyl]-3-pyrrolidinol,
4-[[N$^{\alpha}$-(4-pyridinylmethoxy)carbonyl]-L-leucinyl]amino]-1-[(2R)-4-methyl-2-[[(benzyloxycarbonyl)]amino]pentyl]-3-pyrrolidinone;
(3RS,4RS)-4-[[N$^{\alpha}$-(4-pyridinylmethoxy)carbonyl]-L-leucinyl]amino]-1-[(2S)-4-methyl-2-[[(tert-butoxycarbonyl)]amino]pentyl]-3-pyrrolidinone;
4-[[N$^{\alpha}$-(4-pyridinylmethoxy)carbonyl]-L-leucinyl]amino]-1-[(2S)-4-methyl-2-[[(tert-butoxycarbonyl)]amino]pentyl]-3-pyrrolidinone;
4-[[N$^{\alpha}$-(4-pyridinylmethoxy)carbonyl]-L-leucinyl]amino]-1-[(2S)-4-methyl-2-(amino)pentyl]-3-pyrrolidinone;
(3RS,4RS)-4-[[N$^{\alpha}$-(2-methylpropoxy)carbonyl]-L-leucinyl]amino]-1-[(2S)-4-methyl-2-[[(benzyloxycarbonyl)]amino]pentyl]-3-pyrrolidinol;
4-[[N$^{\alpha}$-(2-methylpropoxy)carbonyl]-L-leucinyl]amino]-1-[(2S)-4-methyl-2-[[(benzyloxycarbonyl)]amino]pentyl]-3-pyrrolidinone;
(3RS,4RS)-4-[[N$^{\alpha}$-(methylamino)thiocarbonyl]-L-leucinyl]amino]-1-[(2S)-4-methyl-2-[[(benzyloxycarbonyl)]amino]pentyl]-3-pyrrolidinol;
4-[[N$^{\alpha}$-(methylamino)thiocarbonyl]-L-leucinyl]amino]-1-[(2S)-4-methyl-2-[[(benzyloxycarbonyl)]amino]pentyl]-3-pyrrolidinone;
(3RS,4RS)-4-[[N$^{\alpha}$-(phenylmethylamino)carbonyl]-L-leucinyl]amino]-1-[(2S)-4-methyl-2-[[(benzyloxycarbonyl)]amino]pentyl]-3-pyrrolidinol;
4-[[N$^{\alpha}$-(phenylmethylamino)carbonyl]-L-leucinyl]amino]-1-[(2S)-4-methyl-2-[[(benzyloxycarbonyl)]amino]pentyl]-3-pyrrolidinone;
(3RS,4RS)-4-[[N$^{\alpha}$-(3,4-dichlorophenylamino)carbonyl]-L-leucinyl]amino]-1-[(2S)-4-methyl-2-[[(benzyloxycarbonyl)]amino]pentyl]-3-pyrrolidinol;
4-[[N$^{\alpha}$-(3,4-dichlorophenylamino)carbonyl]-L-leucinyl]amino]-1-[(2S)-4-methyl-2-[[(benzyloxycarbonyl)]amino]pentyl]-3-pyrrolidinone;
(3RS,4RS)-1-benzyl-4-[[N$^{\alpha}$-(3,4-dichlorophenylamino)-L-leucinyl]amino]-3-pyrrolidinol;
1-benzyl-4-[[N$^{\alpha}$-(3,4-dichlorophenylamino)-L-leucinyl]amino]-3-pyrrolidinone;
(3RS,4RS)-4-[[N$^{\alpha}$-(1,2,3,4-tetrahydro-6-quinolinecarbonyl)-L-leucinyl]amino]-1-[(2S)-4-methyl-2-[(p-toluenesulphonyl)amino]pentyl]-3-pyrrolidinol;
4-[[N$^{\alpha}$-(1,2,3,4-tetrahydro-6-quinolinecarbonyl)-L-leucinyl]amino]-1-[(2S)-4-methyl-2-[(p-toluenesulphonyl)amino]pentyl]-3-pyrrolidinone;
(3RS,4RS)-4-[[N$^{\alpha}$-(1,2,3,4-tetrahydro-6-quinolinecarbonyl)-L-leucinyl]amino]-1-[(2S)-4-methyl-2-[(acetyl)amino]pentyl]-3-pyrrolidinol;
4-[[[N$^{\alpha}$-(1,2,3,4-tetrahydro-6-quinolinecarbonyl)-L-leucinyl]amino]-1-[(2S)-4-methyl-2-[(acetyl)amino]pentyl]-3-pyrrolidinone;
(3RS,4RS)-4-[[N$^{\alpha}$-(4-fluorobenzoyl)-L-leucinyl]amino]-1-[(2S)-4-methyl-2-[(acetyl)amino]pentyl]-3-pyrrolidinol;
4-[[[N$^{\alpha}$-(4-fluorobenzoyl)-L-leucinyl]amino]-1-[(2S)-4-methyl-2-[(acetyl)amino]pentyl]-3-pyrrolidinone;
(3RS,4RS)-4-[[N$^{\alpha}$-(4-fluorobenzoyl)-L-leucinyl]amino]-1-[(2S)-4-methyl-2-[(p-toluenesulphonyl)amino]pentyl]-3-pyrrolidinol;

4-[[$N^{\alpha}$-(4-fluorobenzoyl)-L-leucinyl]amino]-1-[(2S)-4-methyl-2-[(p-toluenesulphonyl)amino]pentyl]-3-pyrrolidinone;
(3RS,4RS)-4-[[$N^{\alpha}$-(4-fluorobenzoyl)-L-leucinyl]amino]-1-[(2S)-4-methyl-2-[(methanesulphonyl)amino]pentyl]-3-pyrrolidinone;
4-[[$N^{\alpha}$-(4-fluorobenzoyl)-L-leucinyl]amino]-1-[(2S)-4-methyl-2-[(methanesulphonyl)amino]pentyl]-3-pyrrolidinone;
(3RS,4RS)-4-[[$N^{\alpha}$-(4-fluorobenzoyl)-L-leucinyl]amino]-1-[(2S)-4-methyl-2-[($\alpha$-toluenesulphonyl)amino]pentyl]-3-pyrrolidinone;
4-[[$N^{\alpha}$-(4-fluorobenzoyl)-L-leucinyl]amino]-1-[(2S)-4-methyl-2-[($\alpha$-toluenesulphonyl)amino]pentyl]-3-pyrrolidinone;
(3RS,4RS)-1-(2-phenethyl)-4-[[$N^{\alpha}$-(4-fluorobenzoyl)-L-leucinyl]amino]-3-pyrrolidinol;
1-(2-phenethyl)-4-[[$N^{\alpha}$-(4-fluorobenzoyl)-L-leucinyl]amino]-3-pyrrolidinone;
(3RS,4RS)-1-(2-phenethyl)-4-[[$N^{\alpha}$-(2-quinolinylcarbonyl)-L-leucinyl]amino]-3-pyrrolidinol;
1-(2-phenethyl)-4-[[$N^{\alpha}$-(2-quinolinylcarbonyl)-L-leucinyl]amino]-3-pyrrolidinone;
(3RS,4RS)-1-(2-phenethyl)-4-[[$N^{\alpha}$-(2-naphthylcarbonyl)-L-leucinyl]amino]-3-pyrrolidinol;
1-(2-phenethyl)-4-[[$N^{\alpha}$-(2-naphthylcarbonyl)-L-leucinyl]amino]-3-pyrrolidinone;
(3RS,4RS)-1-(2-phenethyl)-4-[[$N^{\alpha}$-($\alpha$-toluenesulphonyl)-L-leucinyl]amino]-3-pyrrolidinol;
1-(2-phenethyl)-4-[[$N^{\alpha}$-($\alpha$-toluenesulphonyl)-L-leucinyl]amino]-3-pyrrolidinone;
(3RS,4RS)-1-(2-phenethyl)-4-[[$N^{\alpha}$-(2-nitro-$\alpha$-toluenesulphonyl)-L-leucinyl]amino]-3-pyrrolidinol;
1-(2-phenethyl)-4-[[$N^{\alpha}$-(2-nitro-$\alpha$-toluenesulphonyl)-L-leucinyl]amino]-3-pyrrolidinone;
(3RS,4RS)-4-[[$N^{\alpha}$-(2-phenylacetyl)-L-leucinyl]amino]-1-[(2S)-4-methyl-2-[[(4-pyridinylcarbonyl)]amino]pentyl]-3-pyrrolidinol;
4-[[$N^{\alpha}$-(2-phenylacetyl)-L-leucinyl]amino]-1-[(2S)-4-methyl-2-[[(4-pyridinylcarbonyl)]amino]pentyl]-3-pyrrolidinone;
(3RS,4RS)-4-[[$N^{\alpha}$-(2-phenylacetyl)-L-leucinyl]amino]-1-[(2S)-4-methyl-2-[[(p-toluenesulphonyl)]amino]pentyl]-3-pyrrolidinol;
4-[[$N^{\alpha}$-(2-phenylacetyl)-L-leucinyl]amino]-1-[(2S)-4-methyl-2-[[(p-toluenesulphonyl)]amino]pentyl]-3-pyrrolidinone;
(3RS,4RS)-4-[[$N^{\alpha}$-(2-phenylacetyl)-L-leucinyl]amino]-1-[(2S)-4-methyl-2-[[(4-imidazoleacetyl)]amino]pentyl]-3-pyrrolidinol;
4-[[$N^{\alpha}$-(2-phenylacetyl)-L-leucinyl]amino]-1-[(2S)-4-methyl-2-[[(4-imidazoleacetyl)]amino]pentyl]-3-pyrrolidinone;
(3RS,4RS)-4-[(4-phenoxybenzoyl)amino]-1-[(2S)-4-methyl-2-[[(benzyloxycarbonyl)]amino]pentanoyl)-3-pyrrolidinoylcarbonyl)-L-leucinyl]amino]-3-pyrrolidinol;
1-(3-nitrobenzyl)-4-[[$N^{\alpha}$-(4-pyridinylmethoxycarbonyl)-L-leucinyl]amino]-3-pyrrolidinone;
(3RS,4RS)-1-(2-nitrobenzyl)-4-[[$N^{\alpha}$-(4-pyridinylmethoxycarbonyl)-L-leucinyl]amino]-3-pyrrolidinol;
1-(2-nitrobenzyl)-4-[[$N^{\alpha}$-(4-pyridinylmethoxycarbonyl)-L-leucinyl]amino]-3-pyrrolidinone;
(3RS,4RS)-1-(4-cyanobenzyl)-4-[[$N^{\alpha}$-(4-pyridinylmethoxycarbonyl)-L-leucinyl]amino]-3-pyrrolidinol;
1-(4-cyanobenzyl)-4-[[$N^{\alpha}$-(4-pyridinylmethoxycarbonyl)-L-leucinyl]amino]-3-pyrrolidinone;
(3RS,4RS)-1-(4-bromobenzyl)-4-[[$N^{\alpha}$-(4-pyridinylmethoxycarbonyl)-L-leucinyl]amino]-3-pyrrolidinol;
1-(4-bromobenzyl)-4-[[$N^{\alpha}$-(4-pyridinylmethoxycarbonyl)-L-leucinyl]amino]-3-pyrrolidinone;
(3RS,4RS)-1-phenethyl-4-[[$N^{\alpha}$-(4-pyridinylmethoxycarbonyl)-L-leucinyl]amino]-3-pyrrolidinol;
1-phenethyl-4-[[$N^{\alpha}$-(4-pyridinylmethoxycarbonyl)-L-leucinyl]amino]-3-pyrrolidinone;
1-(3-aminobenzyl)-4-[[$N^{\alpha}$-(4-pyridinylmethoxycarbonyl)-L-leucinyl]amino]-3-pyrrolidinone;
(3RS,4RS)-1-(3-benzyloxybenzyl)-4-[[$N^{\alpha}$-(4-pyridinylmethoxycarbonyl)-L-leucinyl]amino]-3-pyrrolidinol;
1-(3-benzyloxybenzyl)-4-[[$N^{\alpha}$-(4-pyridinylmethoxycarbonyl)-L-leucinyl]amino]-3-pyrrolidinone;
(3RS,4RS)-1-(3-hydroxybenzyl)-4-[[$N^{\alpha}$-(4-pyridinylmethoxycarbonyl)-L-leucinyl]amino]-3-pyrrolidinol;
1-(3-hydroxybenzyl)-4-[[$N^{\alpha}$-(4-pyridinylmethoxycarbonyl)-L-leucinyl]amino]-3-pyrrolidinone;
(3RS,4RS)-1-ethyl-4-[[$N^{\alpha}$-(4-pyridinylmethoxycarbonyl)-L-leucinyl]amino]-3-pyrrolidinol;
1-ethyl-4-[[$N^{\alpha}$-(4-pyridinylmethoxycarbonyl)-L-leucinyl]amino]-3-pyrrolidinone;
(3RS,4RS)-1-cyclopropylmethyl-4-[[$N^{\alpha}$-(4-pyridinylmethoxycarbonyl)-L-leucinyl]amino]-3-pyrrolidinol;
1-cyclopropylmethyl-4-[[$N^{\alpha}$-(4-pyridinylmethoxycarbonyl)-L-leucinyl]amino]-3-pyrrolidinone;
(3RS,4RS)-1-(2-N,N-dimethylaminoethyl)-4-[[$N^{\alpha}$-(4-pyridinylmethoxycarbonyl)-L-leucinyl]amino]-3-pyrrolidinol;
1-(2-N,N-dimethylaminoethyl)-4-[[$N^{\alpha}$-(4-pyridinylmethoxycarbonyl)-L-leucinyl]amino]-3-pyrrolidinone;
(3RS,4RS)-1-(2-morpholinoethyl)-4-[[$N^{\alpha}$-(4-pyridinylmethoxycarbonyl)-L-leucinyl]amino]-3-pyrrolidinol;
1-(2-morpholinoethyl)-4-[[$N^{\alpha}$-(4-pyridinylmethoxycarbonyl)-L-leucinyl]amino]-3-pyrrolidinone;
(3RS,4RS)-1-(2-bromobenzyl)-4-[[$N^{\alpha}$-(2-pyridinylmethoxycarbonyl)-L-leucinyl]amino]-3-pyrrolidinol;

1-(2-bromobenzyl)-4-[[N$^{\alpha}$-(2-pyridinylmethoxycarbonyl)-L-leucinyl]amino]-3-pyrrolidinone;
(3RS,4RS)-4-[[N$^{\alpha}$-(4-pyridinylmethoxy)carbonyl)-L-leucinyl]amino]-1-[(2S)-4-methyl-2-[[(benzyloxycarbonyl)]amino]pentyl]-3-pyrrolidinol;
4-[[N$^{\alpha}$-(4-pyridinylmethoxy)carbonyl)-L-leucinyl]amino]-1-[(2S)-4-methyl-2-[[(benzyloxycarbonyl)]amino]pentyl]-3-pyrrolidinone;
(3RS,4RS)-4-[[N$^{\alpha}$-(4-pyridinylmethoxy)carbonyl)-L-leucinyl]amino]-1-[(2S)-4-methyl-2-[[(benzyloxycarbonyl)]aminomethyl]pentyl]-3-pyrrolidinol;
4-[[N$^{\alpha}$-(4-pyridinylmethoxy)carbonyl-L-leucinyl]amino]-1-[(2S)-4-methyl-2-[[(benzyloxycarbonyl)]aminomethyl]pentyl]-3-pyrrolidinone;
(3RS,4RS)-4-[[N$^{\alpha}$-[(2-pyridinylmethoxy)carbonyl]-L-leucinyl]amino]-1-[(2S)-4-methyl-2-[[(benzyloxycarbonyl)]amino]pentyl]-3-pyrrolidinol;
4-[[N$^{\alpha}$-[(2-pyridinylmethoxy)carbonyl]-L-leucinyl]amino]-1-[(2S)-4-methyl-2-[[(benzyloxycarbonyl)]amino]pentyl]-3-pyrrolidinone;
(3RS,4RS)-4-[[N$^{\alpha}$-[(3-pyridinylmethoxy)carbonyl]-L-leucinyl]amino]-1-[(2S)-4-methyl-2-[[(benzyloxycarbonyl)]amino]pentyl]-3-pyrrolidinol;
4-[[N$^{\alpha}$-[(3-pyridinylmethoxy)carbonyl)-L-leucinyl]amino]-1-[(2S)-4-methyl-2-[[(benzyloxycarbonyl)]amino]pentyl]-3-pyrrolidinone;
(3RS,4RS)-4-[[N$^{\alpha}$-(benzyloxycarbonyl)-L-leucinyl]amino]-1-[(2S)-4-methyl-2-[[(benzyloxycarbonyl)]amino]pentyl]-3-pyrrolidinol;
4-[[N$^{\alpha}$-(benzyloxycarbonyl)-L-leucinyl]amino]-1-[(2S)-4-methyl-2-[[(benzyloxycarbonyl)]amino]pentyl]-3-pyrrolidinone;
(3RS,4RS)-4-[[N$^{\alpha}$-(4-pyridinylmethoxycarbonyl)-L-leucinyl]aminomethyl]-1-[(2S)-4-methyl-2-[[(benzyloxycarbonyl)]amino]pentyl]-3-pyrrolidinol;
4-[[N$^{\alpha}$-(4-pyridinylmethoxycarbonyl)-L-leucinyl]aminomethyl]-1-[(2S)-4-methyl-2-[[(benzyloxycarbonyl)]amino]pentyl]-3-pyrrolidinone;
(3RS,4RS)-1-benzyl-4-[[N$^{\alpha}$-(2-nitro-$\alpha$-toluenesulphonyl)-L-leucinyl]amino]-3-pyrrolidinol;
1-benzyl-4-[[N$^{\alpha}$-(2-nitro-$\alpha$-toluenesulphonyl)-L-leucinyl]amino]-3-pyrrolidinone;
(3RS,4RS)-1-benzyl-4-[[N$^{\alpha}$-(phenylsulphonyl)-L-leucinyl]amino]-3-pyrrolidinol;
1-benzyl-4-[[N$^{\alpha}$-(phenylsulphonyl)-L-leucinyl]amino]-3-pyrrolidinone;
(3RS,4RS)-1-benzyl-4-[[N$^{\alpha}$-($\alpha$-toluenesulphonyl)-L-leucinyl]amino]-3-pyrrolidinol;
1-benzyl-4-[[N$^{\alpha}$-($\alpha$-toluenesulphonyl)-L-leucinyl]amino]-3-pyrrolidinone;
(3RS,4RS)-1-benzyl-4-[[N$^{\alpha}$-(2-naphthylsulphonyl)-L-leucinyl]amino]-3-pyrrolidinol;
1-benzyl-4-[[N$^{\alpha}$-(2-naphthylsulphonyl)-L-leucinyl]amino]-3-pyrrolidinone
(3RS,4RS)-1-benzyl-4-[[N$^{\alpha}$-(2-naphthylcarbonyl)-L-leucinyl]amino]-3-piperidinol;
1-benzyl-4-[[N$^{\alpha}$-(2-naphthylcarbonyl)-L-leucinyl]amino]-3-piperidinone
(3RS,4RS)-1-benzyl-4-[[N$^{\alpha}$-(2-quinolinylcarbonyl)-L-leucinyl]amino]-3-piperidinol;
1-benzyl-4-[[N$^{\alpha}$-(2-quinolinylcarbonyl)-L-leucinyl]amino]-3-piperidinone;
(3RS,4RS)-1-benzyl-4-[[N$^{\alpha}$-(2-naphthylsulphonyl-L-leucinyl]amino]-3-piperidinol;
1-benzyl-4-[[N$^{\alpha}$-(2-naphthylsulphonyl)-L-leucinyl]amino]-3-piperidinone;
(3RS,4RS)-4-[[N$^{\alpha}$-(benzyloxycarbonyl)-L-leucinyl]aminomethyl]-1-[(2S)-4-methyl-2-[[(benzyloxycarbonyl)]aminomethyl]pentanoyl]-3-pyrrolidinol;
4-[[N$^{\alpha}$-(benzyloxycarbonyl)-L-leucinyl]aminomethyl]-1-[(2S)-4-methyl-2-[[(benzyloxycarbonyl)]aminomethyl]pentanoyl]-3-pyrrolidinone;
(3RS,4RS)-4-[(2S)-4-methyl-2-[(benzyloxycarbonyl)amino]pentyl]-1-[(2S)-4-methyl-2-[[(benzyloxycarbonyl)]amino]pentanoyl]-3-pyrrolidinol;
4-[(2S)-4-methyl-2-[(benzyloxycarbonyl)amino]pentyl]-1-[(2S)-4-methyl-2-[[(benzyloxycarbonyl)]amino]pentanoyl]-3-pyrrolidinone;
(3RS,4RS)-4-[[N$^{\alpha}$-(4-fluorobenzoyl)-L-leucinyl]amino]-1-[2-[($\alpha$-toluenesulphonyl)amino]ethyl]-3-pyrrolidinol;
4-[[N$^{\alpha}$-(4-fluorobenzoyl)-L-leucinyl]amino]-1-[2-[($\alpha$-toluenesulphonyl)amino]ethyl]-3-pyrrolidinone;
(3RS,4RS)-4-[[N$^{\alpha}$-(4-fluorobenzoyl)-L-leucinyl]amino]-1-benzoyl-3-pyrrolidinol;
4-[[N$^{\alpha}$-(4-fluorobenzoyl)-L-leucinyl]amino]-1-benzoyl-3-pyrrolidinone;
(3RS,4RS)-4-[[N$^{\alpha}$-(piperonylcarbonyl)-L-leucinyl]amino]-1-benzoyl-3-pyrrolidinol;
4-[[N$^{\alpha}$-(piperonylcarbonyl)-L-leucinyl]amino]-1-benzoyl-3-pyrrolidinone;
(3RS,4RS)-4-[[N$^{\alpha}$-(4-fluorobenzoyl)-L-leucinyl]amino]-1-[2-[(4-fluorobenzoyl)amino]ethyl]-3-pyrrolidinol;
4-[[N$^{\alpha}$-(4-fluorobenzoyl)-L-leucinyl]amino]-1-[2-[(4-fluorobenzoyl)amino]ethyl]-3-pyrrolidinone;
(3RS,4RS)-4-[[N$^{\alpha}$-(*tert*-butoxycarbonyl)-L-leucinyl]amino]-1-benzoyl-3-pyrrolidinol;
4-[[N$^{\alpha}$-(*tert*-butoxycarbonyl)-L-leucinyl]amino]-1-benzoyl-3-pyrrolidinone;
(3RS,4RS)-4-[[N$^{\alpha}$-(4-fluorobenzoyl)-L-leucinyl]amino]-1-[(2S)-4-methyl-2-[(4-fluorobenzoyl)amino]pentyl]-

3-pyrrolidinol;
4-[[Nα-(4-fluorobenzoyl)-L-leucinyl]amino]-1-[(2S)-4-methyl-2-[(4-fluorobenzoyl)amino]pentyl]-3-pyrrolidinone;
4-[[Nα-(4-carboxybenzoyl)-L-leucinyl]amino]-1-[(2S)-4-methyl-2-[(4-fluorobenzoyl)amino]pentyl]-3-pyrrolidinone;
1-benzyl-4-[[Nα-(4-carboxybenzoyl)-L-leucinyl]amino]-3-pyrrolidinone;
(3RS,4RS)-1-benzyl-4-[[Nα-(4-carboxymethyl)benzoyl)]-L-leucinyl]amino]-3-pyrrolidinol;
1-benzyl-4-[[Nα-(4-carboxymethyl)benzoyl)]L-leucinyl]amino]-3-pyrrolidinone;
(3RS,4RS)-4-[[Nα-[(4-carboxymethyl)benzoyl]-L-leucinyl]amino]-1-[(2S)-4-methyl-2-[[(4-fluorobenzoyl)amino]pentyl]-3-pyrrolidinol;
4-[[Nα-[(4-carboxymethyl)benzoyl]-L-leucinyl]amino]-1-[(2S)-4-methyl-2-[[(4-fluorobenzoyl)amino]pentyl]-3-pyrrolidinone;
(3RS,4RS)-1-phenethyl-4-[[Nα-(2-amino-α-toluenesulphonyl)-L-leucinyl]amino]-3-pyrrolidinol;
1-phenethyl-4-[[Nα-(2-amino-α-toluenesulphonyl)-L-leucinyl]amino]-3-pyrrolidinone;
(3RS,4RS)-4-[[Nα-(2-naphthylcarbonyl)-L-leucinyl]amino]-1-benzoyl-3-piperidinol;
4-[[Nα-(2-naphthylcarbonyl)-L-leucinyl]amino]-1-benzoyl-3-piperidinone;
(3RS,4RS)-4-[[Nα-(2-quinolinecarbonyl)-L-leucinyl]amino]-1-benzoyl-3-piperidinol;
4-[[Nα-(2-quinolinecarbonyl)-L-leucinyl]amino]-1-benzoyl-3-piperidinone;
(3RS,4RS)-4-[[Nα-(3-isoquinolinecarbonyl)-L-leucinyl]amino]-1-benzoyl-3-piperidinol;
4-[[Nα-(3-isoquinolinecarbonyl)-L-leucinyl]amino]-1-benzoyl-3-piperidinone;
(3RS,4RS)-4-[[(2S)-4-methyl-2-(benzyl)oxy]pentanoyl]-1-[(2S)-4-methyl-2-[[(benzyloxycarbonyl)]amino]pentanoyl]-3-piperidinol;
4-[[(2S)-4-methyl-2-(benzyl)oxy]pentanoyl]-1-[(2S)-4-methyl-2-[[(benzyloxycarbonyl)]amino]pentanoyl]-3-piperidinone;
(3RS,4RS)-4-[[3-(2-pyridyl)phenylacetyl]amino]-1-[3-(2-pyridyl)phenylacetyl]-3-piperidinol;
4-[[3-(2-pyridyl)phenylacetyl]amino]-1-[3-(2-pyridyl)phenylacetyl]-3-piperidinone;
(3RS,4RS)-4-[[Nα-(p-trifluoromethanephenylsulphonyl)-L-leucinyl]amino]-1-[3-(2-pyridyl)phenylacetyl]-3-piperidinol;
4-[[Nα-(p-trifluoromethanephenylsulphonyl)-L-leucinyl]amino]-1-[3-(2-pyridyl)phenylacetyl]-3-piperidinone;
(3RS,4RS)-4-[[Nα-(2-naphthylsulphonyl)-L-leucinyl]amino]-1-[3-(2-pyridyl)phenylacetyl]-3-piperidinol;
4-[[Nα-(2-naphthylsulphonyl)-L-leucinyl]amino]-1-[3-(2-pyridyl)phenylacetyl]-3-piperidinone;
(3RS,4RS)-4-[[Nα-(3,4-dichlorophenylsulphonyl)-L-leucinyl]amino]-1-[3-(2-pyridyl)phenylacetyl]-3-piperidinol;
4-[[Nα-(3,4-dichlorophenylsulphonyl)-L-leucinyl]amino]-1-[3-(2-pyridyl)phenylacetyl]-3-piperidinone;
(3RS,4RS)-4-[[Nα-(methanesulphonyl)-L-leucinyl]amino]-1-[3-(2-pyridyl)phenylacetyl)]-3-piperidinol;
4-[[Nα-(methanesulphonyl)-L-leucinyl]amino]-1-[3-(2-pyridyl)phenylacetyl]-3-piperidinone;
(3RS,4RS)-4-[[Nα-(4-fluorophenylsulphonyl)-L-leucinyl]amino]-1-[3-(2-pyridyl)phenylacetyl]-3-piperidinol; or
4-[[Nα-(4-fluorophenylsulphonyl)-L-leucinyl]amino]-1-[3-(2-pyridyl)phenylacetyl]-3-piperidinone;

or a pharmaceutically acceptable salt thereof.

**17.** A compound according to any one of claims 1 to 16 for use in therapy.

**18.** A pharmaceutical composition comprising a compound according to any one of claims 1 to 16 and a pharmaceutically acceptable carrier. <

**19.** A compound according to any one of claims 1 to 16 for use in treating diseases in which inhibition of a cysteine protease is a factor.

**20.** The use according to claim 19 wherein the cysteine protease is cathepsin K.

**21.** A compound according to any one of claims 1 to 16 for use in inhibiting bone loss.

**22.** A compound according to any one of claims 1 to 16 for use in treating osteoporosis.

**23.** A compound according to any one of claims 1 to 16 for use in treating gingival or periodontal disease.

**24.** A compound according to any one of claims 1 to 16 for use in treating a disease **characterized by** excessive

cartilage or matrix degradation.

**25.** The use according to claim 24 wherein said disease is osteoarthritis or rheumatoid arthritis.

**26.** The use of a compound of the formula (I) as defined in any one of claims 1 to 16 in the manufacture of a medicament for the treatment of diseases in which inhibition of a cysteine protease is a factor.

**27.** A process for preparing a compound of the formula (I) as defined in any one of claims 1 to 16, which process comprises:

(A) for compounds in which A is CH(OH):

(i) reacting a compound of the formula (III):

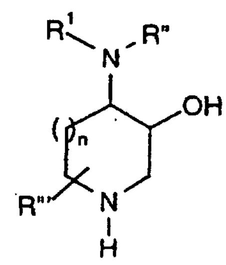

(III)

or a salt thereof,

wherein $R^1$, R", R"' and n are as defined in formula (I) of claim 1, with any reactive functional groups protected, with:

(a) $R^5C(O)Cl$, in which $R^5$ is as defined in formula (I) of claim 1; or
(b) $R^5C(O)OH$, in which $R^5$ is as defined in formula (I) of claim 1, in the presence of EDC and HOBT; or
(c) $R^5C(O)H$, in which $R^5$ is as defined in formula (I) of claim 1, followed by reduction; or
(d) $R^5OC(O)Cl$, in which $R^5$ is as defined in formula (I) of claim 1, in the presence of base; or
(e) $R^5SO_2Cl$, in which $R^5$ is as defined in formula (I) of claim 1, in the presence of base; or
(f)

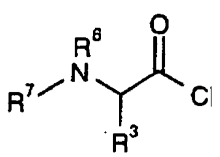

wherein $R^3$, $R^6$ and $R^7$ are as defined in formula (I) of claim 1; or
(g) adamantyl-C(O)Cl;

(ii) reacting a compound of the formual (IV):

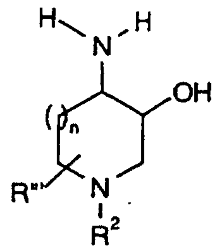

(IV)

wherein $R^2$, R''' and n are as defined in formula (I) of claim 1, with any reactive functional groups protected, with

(a)

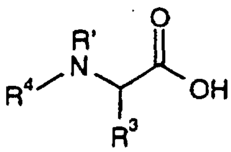

,

in which $R^3$, $R^4$ and R' are as defined in formula (I) of claim 1, in the presence of EDC and HOBT; or

(b)

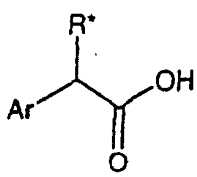

,

in which R* is as defined in formula (I) of claim 1, in the presence of EDC and HOBT; or

(c)

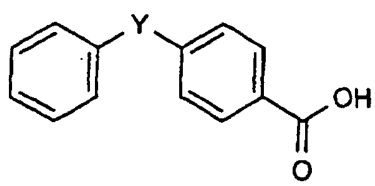

,

in which Y is as defined in formula (I) of claim 1, in the presence of EDC and HOBT; or

(d)

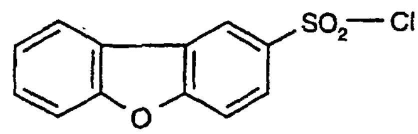

;

(iii) reacting a compound of the formula (V):

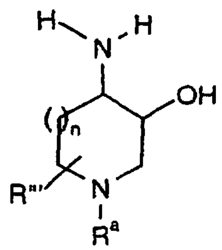

(V)

wherein R''' and n are as defined in formula (I) of claim 1, with any reactive functional groups protected, and $R^a$ is $C_{1-6}$alkyl, $C_{3-6}$cycloalkyl-$C_{0-6}$alkyl, Ar-$C_{0-6}$alkyl, or Het-$C_{0-6}$alkyl with:

(a)

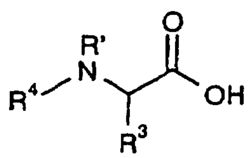

,

in which $R^3$, $R^4$ and R' are as defined in formula (I) of claim 1, in the presence of EDC and HOBT; or

(b)

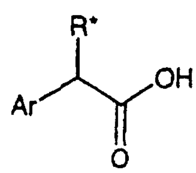

,

in which R* is as defined in formula (I) of claim 1, in the presence of EDC and HOBT; or

(c)

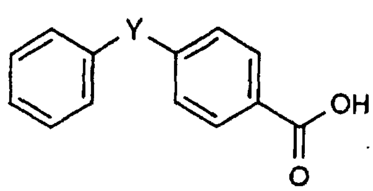

,

in which Y is as defined in formula (I) of claim 1, in the presence of EDC and HOBT; or

(d)

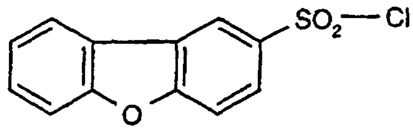

;

(B) for compounds in which A is C(O):

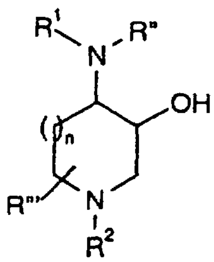

(VI)

(i) reacting a compound of the formula (VI):

wherein $R^1$, $R^2$, R", R"' and n are as defined in formula (I) of claim 1, with any reactive functional groups protected, with an oxidizing agent;

and thereafter removing any protecting groups and optionally forming a pharmaceutically acceptable salt.

**Patentansprüche**

**1.** Verbindung der Formel (I):

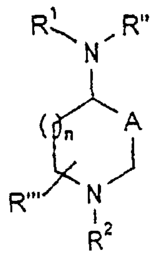

(I)

wobei:

A C(O) oder CH(OH) ist;
$R^1$

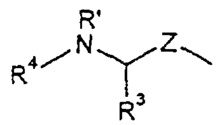, 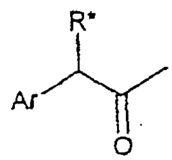, 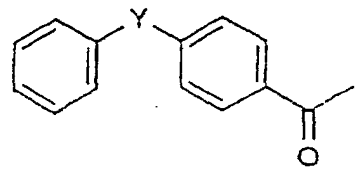,

oder

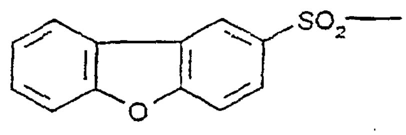;

ist
$R^2$ H, ein gegebenenfalls unabhängig durch ein oder zwei Halogenatome, einen Rest SR, OR, $N(R)_2$, C(O)N$(R)_2$, einen Carbamoyl- oder $C_{1-4}$-Alkylrest substituierter $C_{1-6}$-Alkylrest, ein $C_{3-6}$-Cycloalkyl-$C_{0-6}$-alkyl-, Ar-$C_{0-6}$-alkyl-, Het-$C_{0-6}$-alkylrest, ein Rest $R^5$C(O)-, $R^5$C(S)-, $R^5SO_2$-, $R^5$OC(O)-, $R^5$R'NC(O)-, $R^5$R'NC(S)-, Adamantyl-C(O)-oder

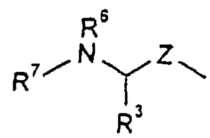;

ist;
R" H, ein gegebenenfalls unabhängig durch ein oder zwei Halogenatome, einen Rest SR, OR, $N(R)_2$, C(O)N$(R)_2$, einen Carbamoyl- oder $C_{1-4}$-Alkylrest substituierter $C_{1-6}$-Alkylrest, ein Ar-$C_{0-6}$-alkyl- oder Het-$C_{0-6}$-alkylrest ist;
R"' H, ein gegebenenfalls unabhängig durch ein oder zwei Halogenatome, einen Rest SR, OR, $N(R)_2$, C(O)N$(R)_2$, einen Carbamoyl- oder $C_{1-4}$-Alkylrest substituierter $C_{1-6}$-Alkylrest, ein $C_{3-6}$-Cycloalkyl-$C_{0-6}$-alkyl-, Ar-$C_{0-6}$-alkyl- oder Het-$C_{0-6}$-alkylrest ist;
$R^3$ jeweils unabhängig H, ein $C_{2-6}$-Alkenyl-, $C_{2-6}$-Alkinyl-, Het-, Ar- oder $C_{1-6}$-Alkylrest ist, welcher gegebenenfalls durch einen Rest OR', SR', $NR'_2$, R'NC(O)$OR^5$, $CO_2R'$, $CO_2NR'_2$, N(C=NH)$NH_2$, Het oder Ar substituiert ist;
$R^4$ H, ein gegebenenfalls unabhängig durch ein oder zwei Halogenatome, einen Rest SR, OR, $N(R)_2$, C(O)N$(R)_2$, einen Carbamoyl- oder $C_{1-4}$-Alkylrest substituierter $C_{1-6}$-Alkylrest, ein $C_{3-6}$-Cycloalkyl-$C_{0-6}$-alkyl-, Ar-$C_{0-6}$-alkyl-, Het-$C_{0-6}$-alkylrest, ein Rest $R^5$C(O)-, $R^5$C(S)-, $R^5SO_2$-, $R^5$OC(O)-, $R^5$R'NC(O)-, $R^5$R'NC(S)-, R'HNCH(R')C(O)-oder $R^5$OC(O)NR'CH(R')C(O)- ist;
$R^5$ jeweils unabhängig ein $C_{3-6}$-Cycloalkyl-$C_{0-6}$-alkyl-, Ar-$C_{0-6}$-alkyl-, Het-$C_{0-6}$-alkyl-, Ar-$C_{0-6}$-alkoxy-, Het-$C_{0-6}$-alkoxy- oder $C_{1-6}$-Alkylrest ist, welcher gegebenenfalls durch einen Rest OR', SR', $NR'_2$, R'NC(O)$OR_5$, $CO_2R'$, $CO_2NR'_2$, N(C=NH)$NH_2$, Het oder Ar substituiert ist;
$R^6$ H, ein gegebenenfalls unabhängig durch ein oder zwei Halogenatome, einen Rest SR, OR, $N(R)_2$, C(O)N$(R)_2$, einen Carbamoyl- oder $C_{1-4}$-Alkylrest substituierter $C_{1-6}$-Alkylrest, ein Ar-$C_{0-6}$-alkyl- oder Het-$C_{0-6}$-alkylrest ist und $R^7$ H, ein gegebenenfalls unabhängig durch ein oder zwei Halogenatome, einen Rest SR, OR, $N(R)_2$, C(O)N$(R)_2$, einen Carbamoyl- oder $C_{1-4}$-Alkylrest substituierter $C_{1-6}$-Alkylrest, ein $C_{3-6}$-Cycloalkyl-$C_{0-6}$-alkyl-, Ar-$C_{0-6}$-alkyl-, Het-$C_{0-6}$-alkylrest, ein Rest $R^5$C(O)-, $R^5$C(S)-, $R^5SO_2$-, $R^5$OC(O)-, $R^5$R'NC(O)-, $R^5$R'NC(S)-, R'HNCH(R')C(O)- oder $R^5$OC(O)NR'CH(R')C(O)- ist; oder $R^6$ und $R^7$ verbunden sind, um einen Pyrrolidin-, einen Piperidin- oder einen Morpholinring zu bilden;
R' jeweils unabhängig H, ein $C_{1-6}$-Alkyl-, Ar-$C_{0-6}$-alkyl- oder Het-$C_{0-6}$-alkylrest ist;
R* H, ein $C_{1-6}$-Alkyl-, $C_{3-6}$-Cycloalkyl-$C_{0-6}$-alkyl-, Ar-$C_{0-6}$-alkyl- oder Het-$C_{0-6}$-alkylrest ist;
R H oder ein $C_{1-6}$-Alkylrest ist;

Y eine Einfachbindung oder O ist;
Z jeweils unabhängig CO oder $CH_2$ ist;
n 0, 1 oder 2 ist;
$C_{3-6}$-Cycloalkyl gegebenenfalls unabhängig durch ein oder zwei Halogenatome, einen Rest SR, OR, $N(R)_2$, $C(O)N(R)_2$, einen Carbamoyl- oder $C_{1-4}$-Alkylrest substituiert ist, wobei R H oder ein $C_{1-6}$-Alkylrest ist;
Ar einen unsubstituierten Phenyl- oder Naphthylrest, oder einen durch einen oder mehrere von Ph-$C_{0-6}$-alkyl-, Het-$C_{0-6}$-alkyl-, $C_{1-6}$-Alkoxy-, Ph-$C_{0-6}$-alkoxy-, Het-$C_{0-6}$alkoxy-, OH-, $(CH_2)_{1-6}NR^aR^a$-, $O(CH_2)_{1-6}NR^aR^a$-Resten substituierten Phenyl- oder Naphthylrest, oder einen durch eine bis drei Einheiten, ausgewählt aus einem $C_{1-4}$-Alkylrest, einem Rest $OR^a$, $N(R^a)_2$, $SR^a$, $CF_3$, $NO_2$, CN, $CO_2R^a$, $CON(R^a)$, F, Cl, Br und I, oder einen durch einen Methylendioxyrest substituierten Phenyl- oder Naphthylrest bedeutet; und
Het einen stabilen, 5- bis 7-gliedrigen, monocyclischen oder einen stabilen, 7- bis 10-gliedrigen, bicyclischen, heterocyclischen Ring bedeutet, welcher entweder gesättigt oder ungesättigt ist, und welcher aus Kohlenstoffatomen und einem bis vier Heteroatomen, ausgewählt aus N, O und S, besteht, und wobei die N- und S-Heteroatome gegebenenfalls oxidiert sein können und die N-Heteroatome gegebenenfalls quartemisiert sein können, einschließlich aller bicyclischen Reste, in welchen einer der vorstehend definierten heterocyclischen Ringe an einen Benzolring kondensiert ist, wobei der Het-Rest gegebenenfalls durch eine oder zwei Einheiten, ausgewählt aus einem $C_{1-4}$-Alkylrest, einem Rest $OR^a$, $N(R^a)_2$, $SR^a$, $CF_3$, $NO_2$, CN, $CO_2R^a$, CON ($R^a$), F, Cl, Br und I, substituiert sein kann, und wobei $R^a$ jeweils unabhängig H, ein $C_{1-6}$-Alkyl-, Ar-$C_{0-6}$-alkyl- oder Het-$C_{0-6}$-alkylrest ist; oder ein pharmazeutisch verträgliches Salz davon.

**2.** Verbindung gemäß Anspruch 1, wobei $R^1$

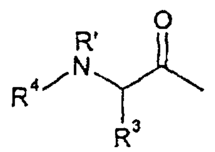

ist.

**3.** Verbindung gemäß Anspruch 1 oder Anspruch 2, wobei $R^4$ ein Rest $R^5C(O)$-, $R^5SO_2$oder $R^5OC(O)$- ist.

**4.** Verbindung gemäß einem der Ansprüche 1 bis 3, wobei $R^5$ in dem Rest $R^1$ ein Ar-$C_{0-6}$-alkyl- oder Het-$C_{0-6}$-alkylrest ist.

**5.** Verbindung gemäß einem der Ansprüche 1 bis 4, wobei $R^5$ in dem Rest $R^1$ ein Phenyloder Benzylrest ist, welche unsubstituiert oder durch einen oder zwei Reste, ausgewählt aus Cl, Br, F, einer Gruppe $CF_3$, einem $C_{1-4}$-Alkylrest, einer Gruppe OH, einem $C_{1-4}$-Alkoxyrest, einer Gruppe CN, $CONH_2$, $NH_2$, oder $NO_2$, substituiert sind, oder durch einen Methylendioxyrest substituiert sind; oder

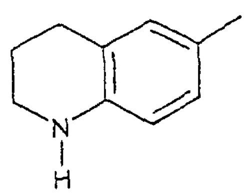
,
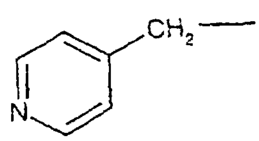
,

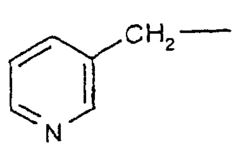
,
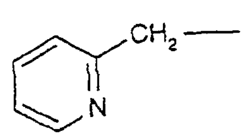
,

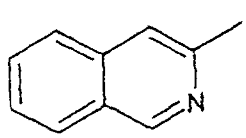 , 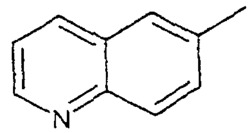 ,

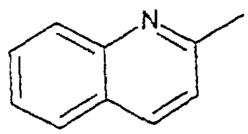 , 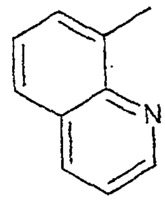 ,

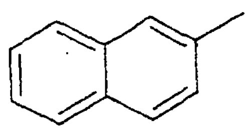 , oder 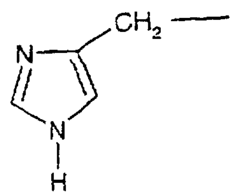 .

ist.

**6.** Verbindung gemäß Anspruch 1 oder Anspruch 2, wobei R' in dem Rest $R^1$ H oder eine Gruppe $CH_3$ ist und $R^3$ eine *i*-Butylgruppe ist.

**7.** Verbindung gemäß Anspruch 1, wobei $R^1$

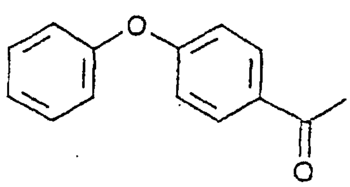 oder 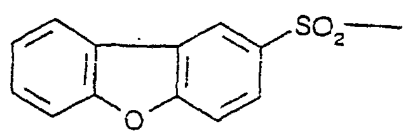

ist.

**8.** Verbindung gemäß Anspruch 1, wobei $R^2$

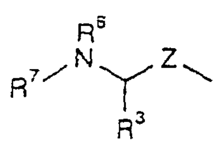 .

ist.

**9.** Verbindung gemäß Anspruch 1 oder Anspruch 8, wobei $R^7$ ein Rest $R^5OC(O)$- ist.

**10.** Verbindung gemäß einem der Ansprüche 1, 8 oder 9, wobei $R^5$ in dem Rest $R^7$ ein Ar-$C_{0-6}$-alkyl- oder Het-$C_{0-6}$-

alkylrest ist.

**11.** Verbindung gemäß einem der Ansprüche 1 oder 8 bis 10, wobei $R^5$ in dem Rest $R^7$ ein Phenyl- oder Benzylrest ist, welche unsubstituiert oder durch einen oder zwei Reste aus Cl, Br, F, einer Gruppe $CF_3$, einem $C_{1-4}$-Alkylrest, einer Gruppe OH, einem $C_{1-4}$-Alkoxyrest, einer Gruppe CN, $CONH_2$, $NH_2$, oder $NO_2$, substituiert sind, oder durch einen Methylendioxyrest substituiert sind; oder eine 2-, 3- oder 4-Pyridyl-$CH_2$-Gruppe ist.

**12.** Verbindung gemäß Anspruch 1, wobei $R^2$

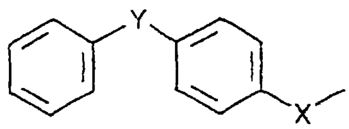

ist, wobei X CO, $SO_2$ oder $CH_2$-CO ist und Y eine Einfachbindung oder O ist.

**13.** Verbindung gemäß Anspruch 1 oder Anspruch 8, wobei $R^6$ in dem Rest $R^2$ H oder eine Gruppe $CH_3$ ist und $R_3$ eine *i*-Butylgruppe ist.

**14.** Verbindung gemäß Anspruch 1, wobei $R^2$ ein Ar-$C_{0-6}$-alkyl-, Het-$C_{0-6}$-alkyl-, $C_{3-6}$-Cycloalkyl-$C_{0-6}$-alkylrest, ein Rest $R^5C(O)$-, $R^5C(S)$-, $R^5SO_2$-, $R^5OC(O)$-, $R^5R'NC(O)$-, $R^5R'NC(S)$-, R'HNCH(R')C(O)-, $R^5OC(O)NR'CH(R')C(O)$- oder Adamantyl-C(O)-ist.

**15.** Verbindung gemäß Anspruch 1, wobei A C(O) ist.

**16.** Verbindung gemäß Anspruch 1, nämlich:

(3RS,4RS)-4-[[$N^{\alpha}$-(Benzyloxycarbonyl)-L-leucinyl]amino]-1-[(2S)-4-methyl-2-[[(benzyloxycarbonyl)]amino] pentanoyl]-3-pyrrolidinol;
4-[[$N^{\alpha}$-(Benzyloxycarbonyl)-L-leucinyl]amino]-1-[(2S)-4-methyl-2-[[(benzyloxycarbonyl)]amino]pentanoyl]-3-pyrrolidinon;
4-[[$N^{\alpha}$-(Benzyloxycarbonyl)-L-leucinyl]amino]-1-[4-(phenoxybenzamid)]-3-pyrrolidinon;
(3RS,4RS)-4-[[$N^{\alpha}$-(Benzyloxycarbonyl)-L-leucinyl]amino]-1-[4-(biphenylethanoyl)]-3-pyrrolidinol;
4-[[$N^{\alpha}$-(Benzyloxycarbonyl)-L-leucinyl]amino]-1-[4-(biphenylethanoyl)]-3-pyrrolidinon;
(3RS,4RS)-4-[[$N^{\alpha}$-(Benzyloxycarbonyl)-L-leucinyl]amino]-1-[(2S)-4-methyl-2-[[(benzyloxycarbonyl)]aminomethyl]pentanoyl]-3-pyrrolidinol;
4-[[$N^{\alpha}$-(Benzyloxycarbonyl)-L-leucinyl]amino]-1-[(2S)-4-methyl-2-[[(benzyloxycarbonyl)]aminomethyl]pentanoyl]-3-pyrrolidinon;
(3RS,4RS)-4-[[$N^{\alpha}$-(Benzyloxycarbonyl)-L-leucinyl]amino]-1-[(2S)-4-methyl-2-[[(*tert*butoxyoxycarbonyl)]aminomethyl]pentanoyl]-3-pyrrolidinol;
4-[[$N^{\alpha}$-(Benzyloxycarbonyl)-L-leucinyl]amino]-1-[(2S)-4-methyl-2-[[(*tert*butoxyoxycarbonyl)]aminomethyl] pentanoyl]-3-pyrrolidinonon;
4-[[$N^{\alpha}$-(Benzyloxycarbonyl)-L-leucinyl]amino]-1-[(2S)-4-methyl-2-(aminomethyl)pentanoyl]-3-pyrrolidinon;
4-[[$N^{\alpha}$-(Benzyloxycarbonyl)-L-leucinyl]amino]-1-*tert*-butoxycarbonyl-3-pyrrolidinon;
4-[[$N^{\alpha}$-(Benzyloxycarbonyl)-L-leucinyl]amino]-3-pyrrolidinon;
4-[[$N^{\alpha}$-(Benzyloxycarbonyl)-L-leucinyl]amino]-1-[(2S)-4-methyl-2-[[(N-*tert*butoxycarbonyl)ethanoyl]aminomethyl]pentanoyl]-3-pyrrolidinon;
4-[[$N^{\alpha}$-(Benzyloxycarbonyl)-L-leucinyl]amino]-1-[(2S)-4-methyl-2-[(ethanoyl)aminomethyl]pentanoyl]-3-pyrrolidinon;
(3RS,4RS)-4-[[$N^{\alpha}$-(Benzyloxycarbonyl)-L-leucinyl]amino]-1-[(2S)-4-methyl-2-[[(*tert*butoxycarbonyl)]amino] pentanoyl]-3-pyrrolidinol;
4-[[$N^{\alpha}$-(Benzyloxycarbonyl)-L-leucinyl]amino]-1-[(2S)-4-methyl-2-[[(*tert*butoxycarbonyl)]amino]pentanoyl]-3-pyrrolidinon;
(3RS,4RS)-4-[[$N^{\alpha}$-(Benzyloxycarbonyl)-L-leucinyl]amino]-1-[(2R)-4-methyl-2-[[(benzyloxycarbonyl)]amino] pentanoyl]-3-pyrrolidinol;

4-[[N$^{\alpha}$-(Benzyloxycarbonyl)-L-leucinyl]amino)-1-[(2R)-4-methyl-2-[[(benzyloxycarbonyl)]amino]pentanoyl]-3-pyrrolidinon;
(3RS,4RS)-4-[[N$^{\alpha}$-(Benzyloxycarbonyl)-L-leucinyl]amino]-1-[2-[(benzyloxycarbonyl)amino]ethanoyl]-3-pyrrolidinonol;
4-[[[N$^{\alpha}$-(Benzyloxycarbonyl)-L-leucinyl]amino]-1-[2-[(benzyloxycarbonyl)amino]ethanoyl]-3-pyrrolidinonon;
(3RS,4RS)-4-[[N$^{\alpha}$-(Benzyloxycarbonyl)-L-leucinyl]amino]-1-[(2S)-3-*tert*-butoxy-[[(benzyloxycarbonyl)]amino] propanoyl]-3-pyrrolidinol;
(3RS,4RS)-4-[[N$^{\alpha}$-(Benzyloxycarbonyl)-L-leucinyl]amino]-1-[(2S)-2-[[(benzyloxycarbonyl)]amino]propanoyl]-3-pyrrolidinol;
4-[[N$^{\alpha}$-(Benzyloxycarbonyl)-L-leucinyl]amino]-1-[(2S)-2-[[(benzyloxycarbonyl)]amino]propanoyl]-3-pyrrolidinon;
(3RS,4RS)-4-[[N$^{\alpha}$-(Benzyloxycarbonyl)-L-leucinyl]amino]-1-[cyclohexanpropanoyl]-3-pyrrolidinol;
4-[[N$^{\alpha}$-(Benzyloxycarbonyl)-L-leucinyl]amino]-1-[cyclohexanpropanoyl]-3-pyrrolidinon;
(3RS,4RS)-4-[[N$^{\alpha}$-(Benzyloxycarbonyl)-L-leucinyl]amino]-1-[(2S)-4-methyl-2-[[(4-pyridinylmethoxycarbonyl)] amino]pentanoyl]-3-pyrrolidinol;
4-[[N$^{\alpha}$-(Benzyloxycarbonyl)-L-leucinyl]amino]-1-[(2S)-4-methyl-2-[[(4-pyridinylmethoxycarbonyl)]amino]pentanoyl]-3-pyrrolidinon;
(3RS,4RS)-4-[[N$^{\alpha}$-(Benzyloxycarbonyl)-L-leucinyl]amino]-1-[(2S)-4-methyl-2-[[(2-pyridinylmethoxycarbonyl)] amino]pentanoyl]-3-pyrrolidinol;
4-[[N$^{\alpha}$-(Benzyloxycarbonyl)-L-leucinyl]amino]-1-[(2S)-4-methyl-2-[[(2-pyridinylmethoxycarbonyl)]amino]pentanoyl]-3-pyrrolidinon;
(3RS,4RS)-4-[[N$^{\alpha}$-(Benzyloxycarbonyl)-L-leucinyl]amino]-1-[(2S)-4-methyl-2-[[(3-pyridinylmethoxycarbonyl)] amino]pentanoyl]-3-pyrrolidinol;
4-[[N$^{\alpha}$-(Benzyloxycarbonyl)-L-leucinyl]amino]-1-[(2S)-4-methyl-2-[[(3-pyridinylmethoxycarbonyl)]amino]pentanoyl]-3-pyrrolidinon;
(3RS,4RS)-4-[[N$^{\alpha}$-(Benzyloxycarbonyl)-L-leucinyl]amino]-1-(2-pyridylcarbonyl)-3-pyrrolidinol;
4-[[N$^{\alpha}$-(Benzyloxycarbonyl)-L-leucinyl]amino]-1-(2-pyridylcarbonyl)-3-pyrrolidinon;
(3RS,4RS)-4-[[N$^{\alpha}$-(Benzyloxycarbonyl)-L-leucinyl]amino]-1-[(2S)-4-methyl-2-[[(benzyloxycarbonyl)]amino] pentanoyl]-3-piperidinol;
4-[[N$^{\alpha}$-(Benzyloxycarbonyl)-L-leucinyl]amino]-1-[(2S)-4-methyl-2-[[(benzyloxycarbonyl)]amino]pentanoyl]-3-piperidinon;
(3RS,4RS)-4-[[N$^{\alpha}$-(Benzyloxycarbonyl)-L-leucinyl]amino]-1-[4-(biphenyl)ethanoyl]-3-piperidinol;
4-[[N$^{\alpha}$-(Benzyloxycarbonyl)-L-leucinyl]amino]-1-[4-(biphenyl)ethanoyl]-3-piperidinon;
(3RS,4RS)-4-[[N$^{\alpha}$-(Benzyloxycarbonyl)-L-leucinyl]amino]-1-[(2S)-4-methyl-2-[[(benzyloxycarbonyl)]aminomethyl]pentanoyl]-3-piperidinol;
4-[[N$^{\alpha}$-(Benzyloxycarbonyl)-L-leucinyl]amino]-1-[(2S)-4-methyl-2-[[(benzyloxycarbonyl)]aminomethyl]pentanoyl]-3-piperidinon;
4-[[N$^{\alpha}$-(Benzyloxycarbonyl)-L-leucinyl]amino]-1-*tert*-butoxycarbonyl-3-piperidinon;
(3RS,4RS)-4-[[N$^{\alpha}$-(Benzyloxycarbonyl)-L-leucinyl]amino]-1-[2-[[(benzyloxycarbonyl)]*iso*-butylamino]ethanoyl]-3-piperidinol;
4-[[N$^{\alpha}$-(Benzyloxycarbonyl)-L-leucinyl]amino]-1-[2-[[(benzyloxycarbonyl)]*iso*butylamino]ethanoyl]-3-piperidinon;
(3RS,4RS)-4-[[N$^{\alpha}$-(Benzyloxycarbonyl)-L-leucinyl]amino]-1-[2-[(*tert*butoxycarbonyl)amino]ethanoyl]-3-piperidinol;
4-[[N$^{\alpha}$-(Benzyloxycarbonyl)-L-leucinyl]amino]-1-[2-[(*tert*butoxycarbonyl)amino]ethanoyl]-3-piperidinon;
4-[[N$^{\alpha}$-(Benzyloxycarbonyl)-L-leucinyl]amino]-1-[2-(amino)ethanoyl]-3-piperidinon;
(3RS,4RS)-4-[[N$^{\alpha}$-(Benzyloxycarbonyl)-L-leucinyl]amino]-1-(4-methylpentanoyl)-3-piperidinol;
4-[[N$^{\alpha}$-(Benzyloxycarbonyl)-L-leucinyl]amino]-1-(4-methylpentanoyl)-3-piperidinon;
(3RS,4RS)-4-[[N$^{\alpha}$-(Benzyloxycarbonyl)-L-leucinyl]amino]-1-(benzoyl)-3-piperidinol;
4-[[N$^{\alpha}$-(Benzyloxycarbonyl)-L-leucinyl]amino]-1-(benzoyl)-3-piperidinon;
(3RS,4RS)-4-[[N$^{\alpha}$-(Benzyloxycarbonyl)-L-leucinyl]amino]-1-(acetyl)-3-piperidinol;
4-[[N$^{\alpha}$-(Benzyloxycarbonyl)-L-leucinyl]amino]-1-(acetyl)-3-piperidinon;
(3RS,4RS)-4-[[N$^{\alpha}$-(Benzyloxycarbonyl)-L-leucinyl]amino]-1-(2-pyridoxyacetyl)-3-piperidinol;
4-[[N$^{\alpha}$-(Benzyloxycarbonyl)-L-leucinyl]amino]-1-(2-pyridoxyacetyl)-3-piperidinon;
(3RS,4RS)-4-[[N$^{\alpha}$-(Benzyloxycarbonyl)-L-leucinyl]amino]-1-(2-[(benzyloxycarbonyl)methylamino]ethanoyl]-3-piperidinol;
4-[[N$^{\alpha}$-(Benzyloxycarbonyl)-L-leucinyl]amino]-1-[2-[(benzyloxycarbonyl)methylamino]ethanoyl]-3-piperidinon;

(3RS,4RS)-4-[[N$^{\alpha}$-(Benzyloxycarbonyl)-L-leucinyl]amino]-1-[3-(2-pyridyl)phenylacetyl)]-3-piperidinol;
4-[[N$^{\alpha}$-(Benzyloxycarbonyl)-L-leucinyl]amino]-1-[3-(2-pyridyl)phenylacetyl)]-3-piperidinon;
(3RS,4RS)-4-[[N$^{\alpha}$-(Benzyloxycarbonyl)-L-leucinyl]amino]-1-[2-[(benzyloxycarbonyl)methylamino]ethanoyl]-3-pyrrolidinol;
4-[[N$^{\alpha}$-(Benzyloxycarbonyl)-L-leucinyl]amino]-1-[2-[(benzyloxycarbonyl)methylamino]ethanoyl]-3-pyrrolidinon;
(3RS,4RS)-4-[[N$^{\alpha}$-(Benzyloxycarbonyl)-L-leucinyl]amino]-1-[2-(phenoxy)ethanoyl]-3-pyrrolidinol;
4-[[N$^{\alpha}$-(Benzyloxycarbonyl)-L-leucinyl]amino]-1-[2-(phenoxy)ethanoyl]-3-pyrrolidinon;
(3RS,4RS)-4-[[N$^{\alpha}$-(4-Pyridinylmethoxycarbonyl)-L-leucinyl]amino]-1-[(2-phenyl)ethanoyl]-3-pyrrolidinol;
4-[[N$^{\alpha}$-(4-Pyridinylmethoxycarbonyl)-L-leucinyl]amino]-1-[(2-phenyl)ethanoyl]-3-pyrrolidinon;
(3RS,4RS)-4-[(N$^{\alpha}$-(4-Pyridinylmethoxycarbonyl)-L-leucinyl]amino]-1-ethanoyl-3-pyrrolidinol;
4-[[N$^{\alpha}$-(4-Pyridinylmethoxycarbonyl)-L-leucinyl]amino]-1-ethanoyl-3-pyrrolidinon;
(3RS,4RS)-4-[[N$^{\alpha}$-(4-Pyridinylmethoxycarbonyl)-L-leucinyl]amino]-1-(4-cyanobenzoyl)-3-pyrrolidinol;
4-[[N$^{\alpha}$-(4-Pyridinylmethoxycarbonyl)-L-leucinyl]amino]-1-(4-cyanobenzoyl)-3-pyrrolidinon;
(3RS,4RS)-4-[[N$^{\alpha}$-(4-Pyridinylmethoxycarbonyl)-L-leucinyl]amino]-1-*tert*butoxycarbonyl-3-pyrrolidinol;
4-[[N$^{\alpha}$-(4-Pyridinylmethoxycarbonyl)-L-leucinyl]amino]-1-*tert*-butoxycarbonyl-3-pyrrolidinon;
(3RS,4RS)-4-[[N$^{\alpha}$-(3-Pyridinylmethoxycarbonyl)-L-leucinyl]amino]-1-*tert*butoxycarbonyl-3-pyrrolidinol;
4-[[N$^{\alpha}$-(3-Pyridinylmethoxycarbonyl)-L-leucinyl]amino]-1-*tert*-butoxycarbonyl-3-pyrrolidinon;
4-[[N$^{\alpha}$-(3-Pyridinylmethoxycarbonyl)-L-leucinyl]amino]-3-pyrrolidinon;
4-[[N$^{\alpha}$-(4-Pyridinylmethoxycarbonyl)-L-leucinyl]amino]-3-pyrrolidinon;
(3RS,4RS)-4-[[N$^{\alpha}$-(2-Pyridinylmethoxycarbonyl)-L-leucinyl]amino]-1-[(2S)-4-methyl-2-[[(benzyloxycarbonyl)]aminomethyl]pentanoyl]-3-pyrrolidinon;
4-[[N$^{\alpha}$-(2-Pyridinylmethoxycarbonyl)-L-leucinyl]amino]-1-[(2S)-4-methyl-2-[[(benzyloxycarbonyl)]aminomethyl]pentanoyl]-3-pyrrolidinon;
(3RS,4RS)-4-[[N$^{\alpha}$-(4-Pyridinylmethoxycarbonyl)-L-leucinyl]amino]-1-[(2S)-4-methyl-2-[[(4-pyridinylmethoxycarbonyl)]amino]pentanoyl]-3-pyrrolidinol;
4-[[N$^{\alpha}$-(4-Pyridinylmethoxycarbonyl)-L-leucinyl]amino]-1-[(2S)-4-methyl-2-[[4-pyridinylmethoxycarbonyl)]amino]pentanoyl]-3-pyrrolidinon;
(3RS,4RS)-4-[[N$^{\alpha}$-(4-Pyridinylmethoxycarbonyl)-L-leucinyl]amino]-1-[(2S)-4-methyl-2-[[(benzyloxycarbonyl)]aminomethyl]pentanoyl]-3-pyrrolidinol;
4-[[N$^{\alpha}$-(4-Pyridinylmethoxycarbonyl)-L-leucinyl]amino]-1-[(2S)-4-methyl-2-[[(benzyloxycarbonyl)]aminomethyl]pentanoyl]-3-pyrrolidinon;
(3RS,4RS)-4-[[N$^{\alpha}$-(3-Isochinolincarbonyl)-L-leucinyl]amino]-1-[(2S)-4-methyl-2-[[(benzyloxycarbonyl)]amino]pentyl]-3-pyrrolidinol;
4-[[N$^{\alpha}$-(3-Isochinolincarbonyl)-L-leucinyl]amino]-1-[(2S)-4-methyl-2-[[(benzyloxycarbonyl)]amino]pentyl]-3-pyrrolidinon;
(3RS,4RS)-4-[[N$^{\alpha}$-(4-Pyridinylmethoxycarbonyl)-L-leucinyl]amino]-1-[1-(adamantyl)carbonyl)-3-pyrrolidinol;
4-[[N$^{\alpha}$-(4-Pyridinylmethoxycarbonyl)-L-leucinyl]amino]-1-[1-(adamantyl)carbonyl]-3-pyrrolidinon;
(3RS,4RS)-4-[[N$^{\alpha}$-(Benzyloxycarbonyl)-L-leucinyl]amino]-1-(4-methyl-pentanoyl)-3-pyrrolidinol;
4-[[N$^{\alpha}$-(Benzyloxycarbonyl)-L-leucinyl]amino]-1-(4-methyl-pentanoyl)-3-pyrrolidinon;
(3RS,4RS)-4-[[N$^{\alpha}$-(Benzyloxycarbonyl)-D-leucinyl]amino]-1-[(2S)-4-methyl-2-[[(benzyloxycarbonyl)]amino]pentanoyl]-3-piperidinol;
4-[[N$^{\alpha}$-(Benzyloxycarbonyl)-D-leucinyl]amino]-1-[(2S)-4-methyl-2-[[(benzyloxycarbonyl)]amino]pentanoyl]-3-piperidinon;
(3RS,4RS)-4-[[N$^{\alpha}$-(*tert*-Butoxycarbonyl)-L-leucinyl]amino]-1-[(2S)-4-methyl-2-[[(benzyloxycarbonyl)]amino]pentanoyl]-3-piperidinol;
4-[[N$^{\alpha}$-(*tert*-Butoxycarbonyl)-L-leucinyl]amino]-1-[(2S)-4-methyl-2-[[(benzyloxycarbonyl)]amino]pentanoyl]-3-piperidinon;
(3RS,4RS)-4-[[N$^{\alpha}$-(Benzyloxycarbonyl)-N$^{\varepsilon}$-(*tert*-butoxycarbonyl)-L-lysin]amino]-1-[(2S)-4-methyl-2-[[(benzyloxycarbonyl)amino]pentanoyl]-3-piperidinol;
4-[[N$^{\alpha}$-(Benzyloxycarbonyl)-N$^{\varepsilon}$-(*tert*-butoxycarbonyl)-L-lysin]amino]-1-[(2S)-4-methyl-2-[[(benzyloxycarbonyl)]amino]pentanoyl]-3-piperidinon;
(3RS,4RS)-4-[[N$^{\alpha}$-(4-Pyridinylmethoxycarbonyl)-L-leucinyl]amino]-1-*tert*butoxycarbonyl-3-piperidinol;
4-[[N$^{\alpha}$-(4-Pyridinylmethoxycarbonyl)-L-leucinyl]amino]-1-*tert*-butoxycarbonyl-3-piperidinon;
(3RS,4RS)-4-[[N$^{\alpha}$-(4-Pyridinylmethoxycarbonyl)-L-leucinyl]amino]-1-(4-methylpentanoyl)-3-piperidinol;
4-[[N$^{\alpha}$-(4-Pyridinylmethoxycarbonyl)-L-leucinyl]amino]-1-(4-methylpentanoyl)-3-piperidinon;
(3RS,4RS)-4-[[N$^{\alpha}$-(4-Pyridinylmethoxycarbonyl)-L-leucinyl]amino]-1-[2-(benzyloxycarbonyl)]*iso*-butylamino]ethanoyl]-3-piperidinol;

4-[[N$^{\alpha}$-(4-pyridinylmethoxycarbonyl)-L-leucinyl]amino]-1-[2-(benzyloxycarbonyl)]*iso*butylamino]ethanoyl]-3-piperidinon;
(3RS,4RS)-4-[[N-2-(Benzyloxycarbonyl)*iso*-butylamino]ethanoyl]-1-[(2S)-4-methyl-2-[[(benzyloxycarbonyl)]amino]pentanoyl]-3-piperidinol;
4-[[N-2-(Benzyloxycarbonyl)*iso*-butylamino]ethanoyl]-1-[(2S)-4-methyl-2-[[(benzyloxycarbonyl)]amino]pentanoyl]-3-piperidinon;
(3RS,4RS)-4-[[N$^{\alpha}$-(Benzyloxycarbonyl)-L-leucinyl]amino]-1-(methansulfonyl)-3-piperidinol;
4-[[N$^{\alpha}$-(Benzyloxycarbonyl)-L-leucinyl]amino]-1-(methansulfonyl)-3-piperidinon;
(3RS,4RS)-4-[[N$^{\alpha}$-(Benzyloxycarbonyl)-L-leucinyl]amino]-1-(phenylsulfonyl)-3-piperidinol;
4-[[N$^{\alpha}$-(Benzyloxycarbonyl)-L-leucinyl]amino]-1-(phenylsulfonyl)-3-piperidinon;
(3RS,4RS)-4-[[N$^{\alpha}$-(4-Pyridinylmethoxycarbonyl)-L-leucinyl]amino]-1-(8-chinolinsulfonyl)-3-pyrrolidinol;
4-[[N$^{\alpha}$-(4-Pyridinylmethoxycarbonyl)-L-leucinyl]amino]-1-(8-chinolinsulfonyl)-3-pyrrolidinon;
(3RS,4RS)-4-[[N$^{\alpha}$-(4-Pyridinylmethoxycarbonyl)-L-leucinyl]amino]-1-(2-pyridylsulfonyl)-3-pyrrolidinon;
4-[[N$^{\alpha}$-(4-Pyridinylmethoxycarbonyl)-L-leucinyl]amino]-1-(2-pyridylsulfonyl)-3-pyrrolidinon;
(3RS,4RS)-4-[[N$^{\alpha}$-(4-Pyridinylmethoxycarbonyl)-L-leucinyl]amino]-1-[(2-propoxy)carbonyl]-3-pyrrolidinol;
4-[[N$^{\alpha}$-(4-Pyridinylmethoxycarbonyl)-L-leucinyl]amino]-1-[(2-propoxy)carbonyl]-3-pyrrolidinon;
(3RS,4RS)-4-[[N$^{\alpha}$-(4-Pyridinylmethoxycarbonyl)-L-leucinyl]amino]-1-[(3-methyl-1-propoxy)carbonyl]-3-pyrrolidinol;
4-[[N$^{\alpha}$-(4-Pyridinylmethoxycarbonyl)-L-leucinyl]amino]-1-[(3-methyl-1-propoxy)carbonyl]-3-pyrrolidinon;
(3RS,4RS)-4-[[N$^{\alpha}$-(Benzyloxycarbonyl)-L-leucinyl]amino]-1-[(4-phenoxy)phenylsulfonyl]-3-pyrrolidinol;
4-[[N$^{\alpha}$-(Benzyloxycarbonyl)-L-leucinyl]amino]-1-[(4-phenoxy)phenylsulfonyl]-3-pyrrolidinon;
(3RS,4RS)-4-[[N$^{\alpha}$-(Benzyloxycarbonyl)-L-leucinyl]amino]-1-[(4-phenoxy)phenylsulfonyl]-3-piperidinol;
4-[[N$^{\alpha}$-(Benzyloxycarbonyl)-L-leucinyl]amino]-1-[(4-phenoxy)phenylsulfonyl]-3-piperidinon;
(3RS,4RS)-4-[[N$^{\alpha}$-(3,4-Dichlorbenzoyl)-L-leucinyl]amino]-1-[(2S)-4-methyl-2-[[(benzyloxycarbonyl)]amino]pentyl]-3-pyrrolidinol;
4-[[N$^{\alpha}$-(3,4-Dichlorbenzoyl)-L-leucinyl]amino]-1-[(2S)-4-methyl-2-[[(benzyloxycarbonyl)]amino]pentyl]-3-pyrrolidinon;
(3RS,4RS)-4-[[N$^{\alpha}$-(6-Chinolincarbonyl)-L-leucinyl]amino]-1-[(2S)-4-methyl-2-[[(benzyloxycarbonyl)]amino]pentyl]-3-pyrrolidinol;
4-[[N$^{\alpha}$-(6-Chinolincarbonyl)-L-leucinyl]amino]-1-[(2S)-4-methyl-2-[[(benzyloxycarbonyl)]amino]pentyl]-3-pyrrolidinon;
(3RS,4RS)-4-[(2-Dibenzofuransulfonyl)amino]-1-[(2S)-4-methyl-2-[[(benzyloxycarbonyl)]amino]pentanoyl]-3-pyrrolidinol;
4-[(2-Dibenzofuransulfonyl)amino]-1-[(2S)-4-methyl-2-[[(benzyloxycarbonyl)]amino]pentanoyl]-3-pyrrolidinon;
(3RS,4RS)-4-[(2-Dibenzofuransulfonyl)amino]-1-[(2S)-4-methyl-2-[[(benzyloxycarbonyl)]methylamino]pentanoyl]-3-pyrrolidinol;
4-[(2-Dibenzofuransulfonyl)amino]-1-[(2S)-4-methyl-2-[[(benzyloxycarbonyl)]methylamino]pentanoyl]-3-pyrrolidinon;
(3RS,4RS)-4-[[N$^{\alpha}$-(Benzyloxycarbonyl)-L-leucinyl]amino]-1-(4-methylpentyl)-3-piperidinol;
4-[[N$^{\alpha}$-(Benzyloxycarbonyl)-L-leucinyl]amino]-1-(4-methylpentyl)-3-piperidinon;
(3RS,4RS)-4-[[N$^{\alpha}$-(2-Pyridylcarbonyl)-L-leucinyl]amino]-1-(4-methylpentyl)-3-piperidinol;
4-[[N$^{\alpha}$-(2-Pyridylcarbonyl)-L-leucinyl]amino]-1-(4-methylpentyl)-3-piperidinon;
(3RS,4RS)-4-[[N$^{\alpha}$-(3-Chlorbenzoyl)-L-leucinyl]amino]-1-(4-methylpentyl)-3-piperidinol;
4-[[N$^{\alpha}$-(3-Chlorbenzoyl)-L-leucinyl]amino]-1-(4-methylpentyl)-3-piperidinon;
(3RS,4RS)-4-[[N$^{\alpha}$-(2-Chinolincarbonyl)-L-leucinyl]amino]-1-(4-methylpentyl)-3-piperidinol;
4-[[N$^{\alpha}$-(2-Chinolincarbonyl)-L-leucinyl]amino]-1-(4-methylpentyl)-3-piperidinon;
(3RS,4RS)-4-[[N$^{\alpha}$-(3,4-Dichlorbenzoyl)-L-leucinyl]amino]-1-(4-methylpentyl)-3-piperidinol;
4-[[N$^{\alpha}$-(3,4-Dichlorbenzoyl)-L-leucinyl]amino]-1-(4-methylpentyl)-3-piperidinon;
(3RS,4RS)-4-[[N$^{\alpha}$-(8-Chinolincarbonyl)-L-leucinyl]amino]-1-(4-methylpentyl)-3-piperidinol;
4-[[N$^{\alpha}$-(8-Chinolincarbonyl)-L-leucinyl]amino]-1-(4-methylpentyl)-3-piperidinon;
(3RS,4RS)-4-[[N$^{\alpha}$-(3-Isochinolincarbonyl)-L-leucinyl]amino]-1-(4-methylpentyl)-3-piperidinol;
4-[[N$^{\alpha}$-(3-Isochinolincarbonyl)-L-leucinyl]amino]-1-(4-methylpentyl)-3-piperidinon(3RS,4RS)-4-[[N$^{\alpha}$-(2-Pyridinylmethoxycarbonyl)-L-leucinyl]amino]-1-(4-methylpentyl)-3-piperidinol;
4-[[N$^{\alpha}$-(2-Pyridinylmethoxycarbonyl)-L-leucinyl]amino]-1-(4-methylpentyl)-3-piperidinon;
(3RS,4RS)-4-[[N$^{\alpha}$-(Acetyl)-L-leucinyl]amino]-1-(4-methylpentyl)-3-piperidinol;
4-[[N$^{\alpha}$-(Acetyl)-L-leucinyl]amino]-1-(4-methylpentyl)-3-piperidinon;
(3RS,4RS)-4-[[N$^{\alpha}$-(*p*-Trifluormethylbenzolsulfonyl)-L-leucinyl]amino]-1-(4-methylpentyl)-3-piperidinol;

4-[[N$^{\alpha}$-(*p*-Trifluormethylbenzolsulfonyl)-L-leucinyl]amino]-1-(4-methylpentyl)-3-piperidinon;
(3RS,4RS)-4-[[N$^{\alpha}$-(6-Chinolincarbonyl)-L-leucinyl]amino]-1-(4-methylpentyl)-3-piperidinol;
4-[[N$^{\alpha}$-(6-Chinolincarbonyl)-L-leucinyl]amino]-1-(4-methylpentyl)-3-piperidinon;
(3RS,4RS)-4-[[2-(RS)-(3-Biphenyl)-4-methyl]amino]pentanoyl]-1-(4-methylpentyl)-3-piperidinol;
4-[[2-(RS)-[(3-Biphenyl)-4-methyl]amino]pentanoyl]-1-(4-methylpentyl)-3-piperidinon;
(3RS,4RS)-4-[[N$^{\alpha}$-(Benzyloxycarbonyl)-L-leucinyl]amino]-1-[2-[(benzyloxycarbonyl)methylamino]ethyl]-3-piperidinol;
4-[[N$^{\alpha}$-(Benzyloxycarbonyl)-L-leucinyl]amino]-1-[2-[(benzyloxycarbonyl)methylamino]ethyl]-3-piperidinon;
(3RS,4RS)-4-[[N$^{\alpha}$-(a-Toluolsulfonyl)-L-leucinyl]amino]-1-[3-(2-pyridyl)phenylacetyl)]-3-piperidinol;
4-[[N$^{\alpha}$-(a-Toluolsulfonyl)-L-leucinyl]amino]-1-[3-(2-pyridyl)phenylacetyl)]-3-piperidinon;
(3RS,4RS)-4-[[N$^{\alpha}$-(2-Naphthylcarbonyl)-L-leucinyl]amino]-1-[3-(2-pyridyl)phenylacetyl)]-3-piperidinon;
4-[[N$^{\alpha}$-(2-Naphthylcarbonyl)-L-leucinyl]amino]-1-[3-(2-pyridyl)phenylacetyl)]-3-piperidinon;
(3RS,4RS)-4-[[N$^{\alpha}$-(Benzolsulfonyl)-L-leucinyl]amino]-1-[3-(2-pyridyl)phenylacetyl)]-3-piperidinol;
4-[[N$^{\alpha}$-(Benzolsulfonyl)-L-leucinyl]amino]-1-[3-(2-pyridyl)phenylacetyl)]-3-piperidinon;
(3RS,4RS)-4-[[N$^{\alpha}$-(3-Isochinolincarbonyl)-L-leucinyl]amino]-1-[3-(2-pyridyl)phenylacetyl)]-3-piperidinol;
4-[[N$^{\alpha}$-(3-Isochinolincarbonyl)-L-leucinyl]amino]-1-[3-(2-pyridyl)phenylacetyl)]-3-piperidinon;
(3RS,4RS)-4-[3-[(2-Pyridyl)phenylacetyl)]amino]-1-[(2S)-4-methyl-2-[[(benzyloxycarbonyl)]amino]pentanoyl]-3-piperidinol;
4-[3-[(2-Pyridyl)phenylacetyl)]amino]-1-[(2S)-4-methyl-2-[[(benzyloxycarbonyl)]amino]pentanoyl]-3-piperidinon;
(3RS,4RS)-4-[3-[(2-Pyridyl)phenylacetyl)]amino]-1-[(2S)-4-methyl-2-[[(2-pyridinylmethoxycarbonyl)]amino] pentanoyl]-3-piperidinol;
4-[3-[(2-Pyridyl)phenylacetyl)]amino]-1-[(2S)-4-methyl-2-[[2-(pyridinylmethoxycarbonyl)]amino]pentanoyl]-3-piperidinon;
(3RS,4RS)-4-[[N$^{\alpha}$-(2-Phenylacetyl)-L-leucinyl]amino]-1-[(2S)-4-methyl-2-[[(benzyloxycarbonyl)]amino]pentyl]-3-pyrrolidinol;
4-[[N$^{\alpha}$-(2-Phenylacetyl)-L-leucinyl]amino]-1-[(2S)-4-methyl-2-[[(benzyloxycarbonyl)]amino]pentyl]-3-pyrrolidinon;
4-[[N$^{\alpha}$-(*tert*-Butoxyoxycarbonyl)-L-leucinyl]amino]-1-[(2S)-4-methyl-2-[[(benzyloxycarbonyl)]amino]pentyl]-3-pyrrolidinon;
4-[(L-Leucinyl)amino]-1-[(2S)-4-methyl-2-[[(benzyloxycarbonyl)]amino]pentyl]-3-pyrrolidinon;
(3RS,4RS)-4-[[N$^{\alpha}$-(2-Chinolincarbonyl)-L-leucinyl]amino]-1-[(2S)-4-methyl-2-[[(benzyloxycarbonyl)]amino] pentyl]-3- pyrrolidinol;
4-[[N$^{\alpha}$-(2-Chinolincarbonyl)-L-leucinyl]amino]-1-[(2S)-4-methyl-2-[[(benzyloxycarbonyl)]amino]pentyl]-3- pyrrolidinon;
(3RS,4RS)-4-[[N$^{\alpha}$-(Piperonylcarbonyl)-L-leucinyl]amino]-1-[(2S)-4-methyl-2-[[(benzyloxycarbonyl)]amino] pentyl]-3- pyrrolidinol;
4-[[N$^{\alpha}$-(Piperonylcarbonyl)-L-leucinyl]amino]-1-[(2S)-4-methyl-2-[[(benzyloxycarbonyl)]amino]pentyl]-3- pyrrolidinon;
(3RS,4RS)-4-[[N$^{\alpha}$-(4-Fluorbenzoyl)-L-leucinyl]amino]-1-[(2S)-4-methyl-2-[[(benzyloxycarbonyl)]amino]pentyl]-3- pyrrolidinol;
4-[[N$^{\alpha}$-(4-Fluorbenzoyl)-L-leucinyl]amino]-1-[(2S)-4-methyl-2-[[(benzyloxycarbonyl)]amino]pentyl]-3- pyrrolidinon;
(3RS,4RS)-4-[[N$^{\alpha}$-(2-Pyridylcarbonyl)-L-leucinyl]amino]-1-[(2S)-4-methyl-2-[[(benzyloxycarbonyl)]amino] pentyl]-3-pyrrolidinol;
4-[[N$^{\alpha}$-(2-Pyridylcarbonyl)-L-leucinyl]amino]-1-[(2S)-4-methyl-2-[[(benzyloxycarbonyl)]amino]pentyl]-3-pyrrolidinon;
(3RS,4RS)-4-[[N$^{\alpha}$-(2-Nitro-a-toluolsulfonyl)-L-leucinyl]amino]-1-[(2S)-4-methyl-2-[[(benzyloxycarbonyl)]amino]pentyl]-3-pyrrolidinol;
4-[[N$^{\alpha}$-(2-Nitro-a-toluolsulfonyl)-L-leucinyl]amino]-1-[(2S)-4-methyl-2-[[(benzyloxycarbonyl)]amino]pentyl]-3-pyrrolidinon;
(3RS,4RS)-4-[[N$^{\alpha}$-(8-Chinolinsulfonyl)-L-leucinyl]amino]-1-[(2S)-4-methyl-2-[[(benzyloxycarbonyl)]amino] pentyl]-3-pyrrolidinol;
4-[[N$^{\alpha}$-(8-Chinolinsulfonyl)-L-leucinyl]amino]-1-[(2S)-4-methyl-2-[[(benzyloxycarbonyl)]amino]pentyl]-3-pyrrolidinon;
(3RS,4RS)-4-[[N$^{\alpha}$-(2-Naphthylcarbonyl)-L-leucinyl]aminomethyl]-1-[(2S)-4-methyl-2-[[(benzyloxycarbonyl)] amino]pentyl]-3-pyrrolidinol;
4-[[N$^{\alpha}$-(2-Naphthylcarbonyl)-L-leucinyl]aminomethyl]-1-[(2S)-4-methyl-2-[[(benzyloxycarbonyl)]amino]pentyl]-3-pyrrolidinon;
(3RS,4RS)-4-[[$N^{\alpha}$-(2-Chinolinylcarbonyl)-L-leucinyl]aminomethyl]-1-[(2S)-4-methyl-2-[[(benzyloxycarbonyl)]amino]pentyl]-3-pyrrolidinol;
4-[[$N^{\alpha}$-(2-Chinolinylcarbonyl)-L-leucinyl]aminomethyl]-1-[(2S)-4-methyl-2-[[(benzyloxycarbonyl)]amino]pentyl]-3-pyrrolidinon;
(3RS,4RS)-4-[[$N^{\alpha}$-(Phenylacetyl)-L-leucinyl]amino]-1-[(2S)-4-methyl-2-[[(benzyloxycarbonyl)]amino]pentyl]-3-piperidinol;
4-[[$N^{\alpha}$-(Phenylacetyl)-L-leucinyl]amino]-1-[(2S)-4-methyl-2-[[(benzyloxycarbonyl)]amino]pentyl]-3-piperidinon;
(3RS,4RS)-4-[[$N^{\alpha}$-(4-Pyridinylmethoxycarbonyl)-L-leucinyl]amino]-1-[(2S)-4-methyl-2-[[(benzyloxycarbonyl)]amino]pentyl]-3-piperidinol;
4-[[$N^{\alpha}$-(4-Pyridinylmethoxycarbonyl)-L-leucinyl]amino]-1-[(2S)-4-methyl-2-[[(benzyloxycarbonyl)]amino]pentyl]-3-piperidinon;
(3RS,4RS)-4-[[$N^{\alpha}$-(4-Pyridinylmethoxycarbonyl)-L-leucinyl]amino]-1-[(2R)-4-methyl-2-[[(benzyloxycarbonyl)]amino]pentyl]-3-piperidinol;
4-[[$N^{\alpha}$-(4-Pyridinylmethoxycarbonyl)-L-leucinyl]amino]-1-[(2R)-4-methyl-2-[[(benzyloxycarbonyl)]amino]pentyl]-3-piperidinon;
(3RS,4RS)-4-[[$N^{\alpha}$-(Phenylacetyl)-L-leucinyl]amino]-1-[(2R)-4-methyl-2-[[(benzyloxycarbonyl)]amino]pentyl]-3-piperidinol;
4-[[$N^{\alpha}$-(Phenylacetyl)-L-leucinyl]aminol-1-[(2R)-4-methyl-2-[[(benzyloxycarbonyl)]amino]pentyl]-3-piperidinon;
(3RS,4RS)-4-[[$N^{\alpha}$-(4-Imidazolacetyl)-L-leucinyl]amino]-1-[(2R)-4-methyl-2-[[(benzyloxycarbonyl)]amino]pentyl]-3-piperidinol;
4-[[$N^{\alpha}$-(4-Imidazolacetyl)-L-leucinyl]amino]-1-[(2R)-4-methyl-2-[[(benzyloxycarbonyl)]amino]pentyl]-3-piperidinon;
(3RS,4RS)-4-[[$N^{\alpha}$-(4-Imidazolacetyl)-L-leucinyl]amino]-1-[(2S)-4-methyl-2-[[(benzyloxycarbonyl)]amino]pentyl]-3-piperidinol;
4-[[$N^{\alpha}$-(4-Imidazolacetyl)-L-leucinyl]amino]-1-[(2S)-4-methyl-2-[[(benzyloxycarbonyl)]amino]pentyl]-3-piperidinon;
(3RS,4RS)-4-[[$N^{\alpha}$-(4-Pyridinylcarbonyl)-L-leucinyl]amino]-1-[(2S)-4-methyl-2-[[(benzyloxycarbonyl)]amino]pentyl]-3-piperidinol;
4-[[$N^{\alpha}$-(4-Pyridinylcarbonyl)-L-leucinyl]amino]-1-[(2S)-4-methyl-2-[[(benzyloxycarbonyl)]amino]pentyl]-3-piperidinon;
4-[[$N^{\alpha}$-(*tert*-Butoxycarbonyl)-L-leucinyl]amino]-1-(benzyloxycarbonyl)-3-piperidinon(3RS,4RS)-4-[[$N^{\alpha}$-(8-Chinolinsulfonyl)-L-leucinyl]amino]-1-(benzyloxycarbonyl)-3-piperidinol;
4-[[$N^{\alpha}$-(8-Chinolinsulfonyl)-L-leucinyl]amino]-1-(benzyloxycarbonyl)-3-piperidinon;(3RS,4RS)-4-[[$N^{\alpha}$-(4-Pyridinylacetyl)-L-leucinyl]amino]-1-(benzyloxycarbonyl)-3-piperidinol;
4-[[$N^{\alpha}$-(4-Pyridinylacetyl)-L-leucinyl]amino]-1-(benzyloxycarbonyl)-3-piperidinon;
(3RS,4RS)-4-[[$N^{\alpha}$-(4-Imidazolacetyl)-L-leucinyl]amino]-1-(benzyloxycarbonyl)-3-piperidinol;
4-[[$N^{\alpha}$-(4-Imidazolacetyl)-L-leucinyl]amino]-1-(benzyloxycarbonyl)-3-piperidinon;
(3RS,4RS)-4-[[$N^{\alpha}$-(4-Pyridinylcarbonyl)-L-leucinyl]amino]-1-(benzyloxycarbonyl)-3-piperidinol;
4-[[$N^{\alpha}$-(4-Pyridinylcarbonyl)-L-leucinyl]amino]-1-(benzyloxycarbonyl)-3-piperidinon; (3RS,4RS)-1-Benzyl-4-[[$N^{\alpha}$-(3-isochinolinylcarbonyl)-L-leucinyl]amino]-3-pyrrolidinol;
1-Benzyl-4-[[$N^{\alpha}$-(3-isochinolinylcarbonyl)-L-leucinyl]amino)-3-pyrrolidinon;
(3RS,4RS)-1-Benzyl-4-[[$N^{\alpha}$-(3,4-dichlorbenzoyl)-L-leucinyl]amino]-3-pyrrolidinol;
1-Benzyl-4-[[$N^{\alpha}$-(3,4-dichlorbenzoyl)-L-leucinyl]amino]-3-pyrrolidinon;
(3RS,4RS)-1-Benzyl-4-[[$N^{\alpha}$-(2-naphthylcarbonyl)-L-leucinyl]aminomethyl]-3-pyrrolidinol;
1-Benzyl-4-[[$N^{\alpha}$-(2-naphthylcarbonyl)-L-leucinyl]aminomethyl]-3-pyrrolidinon;
(3RS,4RS)-1-Benzyl-4-[[$N^{\alpha}$-(2-chinolinylcarbonyl)-L-leucinyl]aminomethyl]-3-pyrrolidinol;
1-Benzyl-4-[[$N^{\alpha}$-(2-chinolinylcarbonyl)-L-leucinyl]aminomethyl]-3-pyrrolidinon;
(3RS,4RS)-1-Benzyl-4-[[$N^{\alpha}$-(2-chinolinylcarbonyl)-L-leucinyl]amino]-3-pyrrolidinol;
1-Benzyl-4-[[$N^{\alpha}$-(2-chinolinylcarbonyl)-L-leucinyl]amino]-3-pyrrolidinon;
(3RS,4RS)-1-Benzyl-4-[[$N^{\alpha}$-(piperonylcarbonyl)-L-leucinyl]amino]-3-pyrrolidinol;
1-Benzyl-4-[[$N^{\alpha}$-(piperonylcarbonyl)-L-leucinyl]amino]-3-pyrrolidinon;
(3RS,4RS)-1-Benzyl-4-[[$N^{\alpha}$-(4-fluorbenzoyl)-L-leucinyl]amino]-3-pyrrolidinol;
1-Benzyl-4-[[$N^{\alpha}$-(4-fluorbenzonyl)-L-leucinyl]amino]-3-pyrrolidinon;
(3RS,4RS)-1-Benzyl-4-[[$N^{\alpha}$-(6-hydroxy-2-naphthylcarbonyl)-L-leucinyl]amino]-3-pyrrolidinol;
1-Benzyl-4-[[$N^{\alpha}$-(6-hydroxy-2-naphthylcarbonyl)-L-leucinyl]amino]-3-pyrrolidinon;

(3RS,4RS)-1-Benzyl-4-[[N$^{\alpha}$-(2-naphthylcarbonyl)-L-leucinyl]amino]-3-pyrrolidinol;
1-Benzyl-4-[[N$^{\alpha}$-(2-naphthylcarbonyl)-L-leucinyl]amino]-3-pyrrolidinon;
(3RS,4RS)-1-Benzyl-4-[[N$^{\alpha}$-(6-chinolinylcarbonyl)-L-leucinyl]amino]-3-pyrrolidinol;
1-Benzyl-4-[[N$^{\alpha}$-(6-chinolinylcarbonyl)-L-leucinyl]amino]-3-pyrrolidinon;
(3RS,4RS)-1-Benzyl-4-[[N$^{\alpha}$-(4-imidazolacetyl)-L-leucinyl]amino]-3-pyrrolidinol;
1-Benzyl-4-[[N$^{\alpha}$-(4-imidazolacetyl)-L-leucinyl]amino]-3-pyrrolidinon;
(3RS,4RS)-1-Benzyl-4-[[N$^{\alpha}$-(4-pyridinylcarbonyl)-L-leucinyl]amino]-3-pyrrolidinol;
1-Benzyl-4-[[N$^{\alpha}$-(4-pyridinylcarbonyl)-L-leucinyl]amino]-3-pyrrolidinon;
4-[[N$^{\alpha}$-(*tert*-Butoxycarbonyl)-L-leucinyl]amino]-1-benzyloxycarbonyl-3-pyrrolidinon;
(3RS,4RS)-4-[[N$^{\alpha}$-(4-Pyridinylmethoxy)carbonyl]-L-leucinyl]amino]-1-[(2R)-4-methyl-2-[[(benzyloxycarbonyl)]amino]pentyl]-3-pyrrolidinol;
4-[[N$^{\alpha}$-(4-Pyridinylmethoxy)carbonyl]-L-leucinyl]amino]-1-[(2R)-4-methyl-2-[[(benzyloxycarbonyl)]amino]pentyl]-3-pyrrolidinon;
(3RS,4RS)-4-[[N$^{\alpha}$-(4-Pyridinylmethoxy)carbonyl]-L-leucinyl]amino]-1-[(2S)-4-methyl-2-[[(*tert*-butoxycarbonyl)]amino]pentyl]-3-pyrrolidinon;
4-[[N$^{\alpha}$-(4-Pyridinylmethoxy)carbonyl]-L-leucinyl]amino]-1-[(2S)-4-methyl-2-[[(*tert*butoxycarbonyl)]amino]pentyl]-3-pyrrolidinon;
4-[[N$^{\alpha}$-(4-Pyridinylmethoxy)carbonyl]-L-leucinyl]amino]-1-[(2S)-4-methyl-2-(amino)pentyl]-3-pyrrolidinon;
(3RS,4RS)-4-[[N$^{\alpha}$-(2-Methylpropoxy)carbonyl]-L-leucinyl]amino]-1-[(2S)-4-methyl-2-[[(benzyloxycarbonyl)]amino]pentyl]-3-pyrrolidinol;
4-[[N$^{\alpha}$-(2-Methylpropoxy)carbonyl]-L-leucinyl]amino]-1-[(2S)-4-methyl-2-[[(benzyloxycarbonyl)]amino]pentyl]-3-pyrrolidinon;
(3RS,4RS)-4-[[N$^{\alpha}$-(Methylamino)thiocarbonyl]-L-leucinyl]amino]-1-[(2S)-4-methyl-2-[[(benzyloxycarbonyl)]amino]pentyl]-3-pyrrolidinol;
4-[[N$^{\alpha}$-(Methylamino)thiocarbonyl]-L-leucinyl]amino]-1-[(2S)-4-methyl-2-[[(benzyloxycarbonyl)]amino]pentyl]-3-pyrrolidinon;
(3RS,4RS)-4-[[N$^{\alpha}$-(Phenylmethylamino)carbonyl]-L-leucinyl]amino]-1-[(2S)-4-methyl-2-[[(benzyloxycarbonyl)]amino)pentyl]-3-pyrrolidinol;
4-[[N$^{\alpha}$-(Phenylmethylamino)carbonyl]-L-leucinyl]amino]-1-[(2S)-4-methyl-2-[[(benzyloxycarbonyl)]amino]pentyl]-3-pyrrolidinon;
(3RS,4RS)-4-[[N$^{\alpha}$-(3,4-Dichlorphenylamino)carbonyl]-L-leucinyl]amino]-1-[(2S)-4-methyl-2-[[(benzyloxycarbonyl)]amino]pentyl]-3-pyrrolidinol;
4-[[N$^{\alpha}$-(3,4-Dichlorphenylamino)carbonyl]-L-leucinyl]amino]-1-[(2S)-4-methyl-2-[[(benzyloxycarbonyl)]amino]pentyl]-3-pyrrolidinon;
(3RS,4RS)-1-Benzyl-4-[[N$^{\alpha}$-(3,4-dichlorphenylamino)-L-leucinyl]amino-3-pyrolidinol; 1-Benzyl-4-[[N$^{\alpha}$-(3,4-dichlorphenylamino)-L-leucinyl]amino]-3-pyrrolidinon;
(3RS,4RS)-4-[[N$^{\alpha}$-(1,2,3,4-Tetrahydro-6-chinolincarbonyl)-L-leucinyl]amino]-1-[(2S)-4-methyl-2-[[(*p*-toluolsulfonyl)]amino]pentyl]-3-pyrrolidinol;
4-[[N$^{\alpha}$-(1,2,3,4-Tetrahydro-6-chinolincarbonyl)-L-leucinyl]amino]-1-[(2S)-4-methyl-2-[[(*p*-toluolsulfonyl)]amino]pentyl]-3-pyrrolidinon;
(3RS,4RS)-4-[[N$^{\alpha}$-(1,2,3,4-Tetrahydro-6-chinolincarbonyl)-L-leucinyl]amino]-1-[(2S)-4-methyl-2-[(acetyl)amino]pentyl]-3-pyrrolidinol;
4-[[N$^{\alpha}$-(1,2,3,4-Tetrahydro-6-chinolincarbonyl)-L-leucinyl]amino]-1-[(2S)-4-methyl-2-[(acetyl)amino]pentyl]-3-pyrrolidinon;
(3RS,4RS)-4-[[N$^{\alpha}$-(4-Fluorbenzoyl)-L-leucinyl]amino]-1-[(2S)-4-methyl-2-[(acetyl)amino]pentyl]-3-pyrrolidinol;
4-[[N$^{\alpha}$-(4-Fluorbenzoyl)-L-leucinyl]amino]-1-[(2S)-4-methyl-2-[(acetyl)amino]pentyl]-3-pyrrolidinon;
(3RS,4RS)-4-[[N$^{\alpha}$-(4-Fluorbenzoyl)-L-leucinyl]amino]-1-[(2S)-4-methyl-2-[(*p*toluolsulfonyl)amino]pentyl]-3-pyrrolidinol;
4-[[N$^{\alpha}$-(4-Fluorbenzoyl)-L-leucinyl]amino]-1-[(2S)-4-methyl-2-[(*p*toluolsulfonyl)amino]pentyl]-3-pyrrolidinon;
(3RS,4RS)-4-[[N$^{\alpha}$-(4-Fluorbenzoyl)-L-leucinyl]amino]-1-[(2S)-4-methyl-2-[(methansulfonyl)amino]pentyl]-3-pyrrolidinon;
4-[[N$^{\alpha}$-(4-Fluorbenzoyl)-L-leucinyl]amino]-1-[(2S)-4-methyl-2-[(methansulfonyl)amino]pentyl]-3-pyrrolidinon;
(3RS,4RS)-4-[[N$^{\alpha}$-(4-Fluorbenzoyl)-L-leucinyl]amino]-1-[(2S)-4-methyl-2-[(atoluolsulfonyl)amino]pentyl]-3-pyrrolidinon;
4-[[N$^{\alpha}$-(4-Fluorbenzoyl)-L-leucinyl]amino]-1-[(2S)-4-methyl-2-[(atoluolsulfonyl)amino]pentyl]-3-pyrrolidinon;
(3RS,4RS)-1-(2-Phenethyl)-4-[[N$^{\alpha}$-(4-fluorbenzoyl)-L-leucinyl]amino]-3-pyrrolidinol;
1-(2-Phenethyl)-4-[[N$^{\alpha}$-(4-fluorbenzoyl)-L-leucinyl]amino]-3-pyrrolidinon;

(3RS,4RS)-1-(2-Phenethyl)-4-[[N$^{\alpha}$-(2-chinolinylcarbonyl)-L-leucinyl]amino]-3-pyrrolidinol;
1-(2-Phenethyl)-4-[[N$^{\alpha}$-(2-chinolinylcarbonyl)-L-leucinyl]amino]-3-pyrrolidinon;
(3RS,4RS)-1-(2-Phenethyl)-4-[[N$^{\alpha}$-(2-naphthylcarbonyl)-L-leucinyl]amino]-3-pyrrolidinol;
1-(2-Phenethyl)-4-[[N$^{\alpha}$-(2-naphthylcarbonyl)-L-leucinyl]amino]-3-pyrrolidinon;
(3RS,4RS)-1-(2-Phenethyl)-4-[[N$^{\alpha}$-($\alpha$-toluolsulfonyl)-L-leucinyl]amino]-3-pyrrolidinol;
1-(2-Phenethyl)-4-[[N$^{\alpha}$-($\alpha$-toluolsulfonyl)-L-leucinyl]amino]-3-pyrrolidinon;
(3RS,4RS)-1-(2-Phenethyl)-4-[[N$^{\alpha}$-(2-nitro-$\alpha$-toluolsulfonyl)-L-leucinyl]amino]-3-pyrrolidinol;
1-(2-Phenethyl)-4-[[N$^{\alpha}$-(2-nitro-$\alpha$-toluolsulfonyl)-L-leucinyl]amino]-3-pyrrolidinon;
(3RS,4RS)-4-[[N$^{\alpha}$-(2-Phenylacetyl)-L-leucinyl]amino]-1-[(2S)-4-methyl-2-[[(4-pyridinylcarbonyl)]amino]pentyl]-3-pyrrolidinol;
4-[[N$^{\alpha}$-(2-Phenylacetyl)-L-leucinyl]amino]-1-[(2S)-4-methyl-2-[[(4-pyridinylcarbonyl)]amino]pentyl]-3-pyrrolidinon;
(3RS,4RS)-4-[[N$^{\alpha}$-(2-Phenylacetyl)-L-leucinyl]amino]-1-[(2S)-4-methyl-2-[[(*p*toluolsulfonyl)]amino]pentyl]-3-pyrrolidinol;
4-[[N$^{\alpha}$-(2-Phenylacetyl)-L-leucinyl]amino]-1-[(2S)-4-methyl-2-[[(*p*toluolsulfonyl)]amino]pentyl]-3-pyrrolidinon;
(3RS,4RS)-4-[[(N$^{\alpha}$-(2-Phenylacetyl)-L-leucinyl]amino]-1-[(2S)-4-methyl-2-[[(4-imidazolacetyl)]amino]pentyl]-3-pyrrolidinol;
4-[[N$^{\alpha}$-(2-Phenylacetyl)-L-leucinyl]amino]-1-[(2S)-4-methyl-2-[[(4-imidazolacetyl)]amino]pentyl]-3-pyrrolidinon;
(3RS,4RS)-4-[(4-Phenoxybenzoyl)amino]-1-[(2S)-4-methyl-2-[[(benzyloxycarbonyl)]amino]pentanoyl]-3-pyrrolidinoylcarbonyl)-L-leucinyl]amino]-3-pyrrolidinol;
1-(3-Nitrobenzyl)-4-[[N$^{\alpha}$-(4-pyridinylmethoxycarbonyl)-L-leucinyl]amino]-3-pyrrolidinon;
(3RS,4RS)-1-(2-Nitrobenzyl)-4-[[N$^{\alpha}$-(4-pyridinylmethoxycarbonyl)-L-leucinyl]amino]-3-pyrrolidinol;
1-(2-Nitrobenzyl)-4-[[N$^{\alpha}$-(4-pyridinylmethoxycarbonyl)-L-leucinyl]amino]-3-pyrrolidinon;
(3RS,4RS)-1-(4-Cyanobenzyl)-4-[[N$^{\alpha}$-(4-pyridinylmethoxycarbonyl)-Lleucinyl]amino]-3-pyrrolidinol;
1-(4-Cyanobenzyl)-4-[[N$^{\alpha}$-(4-pyridinylmethoxycarbonyl)-L-leucinyl]amino]-3-pyrrolidinon;
(3RS,4RS)-1-(4-Brombenzyl)-4-[[N$^{\alpha}$-(4-pyridinylmethoxycarbonyl)-L-leucinyl]amino]-3-pyrrolidinol;
1-(4-Brombenzyl)-4-[[N$^{\alpha}$-(4-pyridinylmethoxycarbonyl)-L-leucinyl]amino]-3-pyrrolidinon;
(3RS,4RS)-1-Phenethyl-4-[[N$^{\alpha}$-(4-pyridinylmethoxycarbonyl)-L-leucinyl]amino]-3-pyrrolidinol;
1-Phenethyl-4-[[N$^{\alpha}$-(4-pyridinylmethoxycarbonyl)-L-leucinyl]amino]-3-pyrrolidinon;
1-(3-Aminobenzyl)-4-[[N$^{\alpha}$-(4-pyridinylmethoxycarbonyl)-L-leucinyl]amino]-3-pyrrolidinon;
(3RS,4RS)-1-(3-Benzyloxybenzyl)-4-[[N$^{\alpha}$-(4-pyridinylmethoxycarbonyl)-Lleucinyl]amino]-3-pyrrolidinol;
1-(3-Benzyloxybenzyl)-4-[[N$^{\alpha}$-(4-pyridinylmethoxycarbonyl)-L-leucinyl]amino]-3-pyrrolidinon;
(3RS,4RS)-1-(3-Hydroxybenzyl)-4-[[N$^{\alpha}$-(4-pyridinylmethoxycarbonyl)-Lleucinyl]amino]-3-pyrrolidinol;
1-(3-Hydroxybenzyl)-4-[[N$^{\alpha}$-(4-pyridinylmethoxycarbonyl)-L-leucinyl]amino]-3-pyrrolidinon;
(3RS,4RS)-1-Ethyl-4-[[N$^{\alpha}$-(4-pyridinylmethoxycarbonyl)-L-leucinyl]amino]-3-pyrrolidinol;
1-Ethyl-4-[[N$^{\alpha}$-(4-pyridinylmethoxycarbonyl)-L-leucinyl]amino]-3-pyrrolidinon;
(3RS,4RS)-1-Cyclopropylmethyl-4-[[N$^{\alpha}$-(4-pyridinylmethoxycarbonyl)-Lleucinyl]amino]-3-pyrrolidinol;
1-Cyclopropylmethyl-4-[[N$^{\alpha}$-(4-pyridinylmethoxycarbonyl)-L-leucinyl]amino]-3-pyrrolidinon;
(3RS,4RS)-1-(2-*N*,*N*-Dimethylaminoethyl)-4-[[N$^{\alpha}$-(4-pyridinylmethoxycarbonyl)-Lleucinyl]amino]-3-pyrrolidinol;
1-(2-*N*,*N*-Dimethylaminoethyl)-4-[[N$^{\alpha}$-(4-pyridinylmethoxycarbonyl)-Lleucinyl]amino]-3-pyrrolidinon;
(3RS,4RS)-1-(2-Morpholinoethyl)-4-[[N$^{\alpha}$-(4-pyridinylmethoxycarbonyl)-Lleucinyl]amino]-3-pyrrolidinonol;
1-(2-Morpholinoethyl)-4-[[N$^{\alpha}$-(4-pyridinylmethoxycarbonyl)-L-leucinyl]amino]-3-pyrrolidinon;
(3RS,4RS)-1-(2-Brombenzyl)-4-[[N$^{\alpha}$-(2-pyridinylmethoxycarbonyl)-L-leucinyl]amino]-3-pyrrolidinonol;
1-(2-Brombenzyl)-4-[[N$^{\alpha}$-(2-pyridinylmethoxycarbonyl)-L-leucinyl]amino]-3-pyrrolidinonon;
(3RS,4RS)-4-[[N$^{\alpha}$-(4-Pyridinylmethoxy)carbonyl)-L-leucinyl]amino]-1-[(2S)-4-methyl-2-[[(benzyloxycarbonyl)]amino]pentyl]-3-pyrrolidinol;
4-[[N$^{\alpha}$-(4-Pyridinylmethoxy)carbonyl)-L-leucinyl]amino]-1-[(2S)-4-methyl-2-[[(benzyloxycarbonyl)]amino]pentyl]-3-pyrrolidinon;
(3RS,4RS)-4-[[N$^{\alpha}$-(4-Pyridinylmethoxy)carbonyl)-L-leucinyl]amino]-1-[(2S)-4-methyl-2-[[(benzyloxycarbonyl)]aminomethyl]pentyl]-3-pyrrolidinol;
4-[[N$^{\alpha}$-(4-Pyridinylmethoxy)carbonyl)-L-leucinyl]amino]-1-[(2S)-4-methyl-2-[[(benzyloxycarbonyl)]aminomethyl]pentyl]-3-pyrrolidinon;
(3RS,4RS)-4-[[N$^{\alpha}$-(2-Pyridinylmethoxy)carbonyl)-L-leucinyl]amino]-1-[(2S)-4-methyl-2-[[(benzyloxycarbonyl)]amino]pentyl]-3-pyrrolidinol;
4-[[N$^{\alpha}$-(2-Pyridinylmethoxy)carbonyl]-L-leucinyl]amino]-1-[(2S)-4-methyl-2-[[(benzyloxycarbonyl)]amino]pentyl]-3-pyrrolidinon;

(3RS,4RS)-4-[[$N^{\alpha}$-[(3-Pyridinylmethoxy)carbonyl]-L-leucinyl]amino]-1-[(2S)-4-methyl-2-[[(benzyloxycarbonyl)]amino]pentyl]-3-pyrrolidinol;
4-[[$N^{\alpha}$-(3-Pyridinylmethoxy)carbonyl]-L-leucinyl]amino]-1-[(2S)-4-methyl-2-[[(benzyloxycarbonyl)]amino]pentyl]-3-pyrrolidinon;
(3RS,4RS)-4-[[$N^{\alpha}$-(Benzyloxycarbonyl)-L-leucinyl]amino]-1-[(2S)-4-methyl-2-[[(benzyloxycarbonyl)]amino] pentyl]-3-pyrrolidinol;
4-[[$N^{\alpha}$-(Benzyloxycarbonyl)-L-leucinyl]amino]-1-[(2S)-4-methyl-2-[[(benzyloxycarbonyl)]amino]pentyl]-3-pyrrolidinon;
(3RS,4RS)-4-[[$N^{\alpha}$-(4-Pyridinylmethoxycarbonyl)-L-leucinyl]aminomethyl]-1-[(2S)-4-methyl-2-[[(benzyloxycarbonyl)]amino]pentyl]-3-pyrrolidinol;
4-[[$N^{\alpha}$-(4-Pyridinylmethoxycarbonyl)-L-leucinyl]aminomethyl]-1-[(2S)-4-methyl-2-[[(benzyloxycarbonyl)]amino]pentyl]-3-pyrrolidinon;
(3RS,4RS)-1-Benzyl-4-[[$N^{\alpha}$-(2-nitro-a-toluolsulfonyl)-L-leucinyl]amino]-3-pyrrolidinol;
1-Benzyl-4-[[$N^{\alpha}$-(2-nitro-$\alpha$-toluolsulfonyl)-L-leucinyl]amino]-3-pyrrolidinon;
(3RS,4RS)-1-Benzyl-4-[[$N^{\alpha}$-(phenylsulfonyl)-L-leucinyl]amino]-3-pyrrolidinol;
1-Benzyl-4-[[$N^{\alpha}$-(phenylsulfonyl)-L-leucinyl]amino]-3-pyrrolidinon;
(3RS,4RS)-1-Benzyl-4-[[$N^{\alpha}$-(a-toluolsulfonyl)-L-leucinyl]amino]-3-pyrrolidinol;
1-Benzyl-4-[[$N^{\alpha}$-(a-toluolsulfonyl)-L-leucinyl]amino]-3-pyrrolidinon;
(3RS,4RS)-1-Benzyl-4-[[$N^{\alpha}$-(2-naphthylsulfonyl)-L-leucinyl]amino]-3-pyrrolidinol;
1-Benzyl-4-[[$N^{\alpha}$-(2-naphthylsulfonyl)-L-leucinyl]amino]-3-pyrrolidinon;
(3RS,4RS)-1-Benzyl-4-[[$N^{\alpha}$-(2-naphthylcarbonyl)-L-leucinyl]amino]-3-piperidinol;
1-Benzyl-4-[[$N^{\alpha}$-(2-naphthylcarbonyl)-L-leucinyl]amino]-3-piperidinon;
(3RS,4RS)-1-Benzyl-4-[[$N^{\alpha}$-(2-chinolinylcarbonyl)-L-leucinyl]amino]-3-piperidinol;
1-Benzyl-4-[[$N^{\alpha}$-(2-chinolinylcarbonyl)-L-leucinyl]amino]-3-piperidinon;
(3RS,4RS)-1-Benzyl-4-[[$N^{\alpha}$-(2-naphthylsulfonyl]amino]-3-piperidinol;
1-Benzyl-4-[[$N^{\alpha}$-(2-naphthylsulfonyl)-L-leucinyl]amino]-3-piperidinon;
(3RS,4RS)-4-[[$N^{\alpha}$-(Benzyloxycarbonyl)-L-leucinyl]aminomethyl]-1-[(2S)-4-methyl-2-[[(benzyloxycarbonyl)] aminomethyl]pentanoyl]-3-pyrrolidinol;
4-[[$N^{\alpha}$-(Benzyloxycarbonyl)-L-leucinyl]aminomethyl]-1-[(2S)-4-methyl-2-[[(benzyloxycarbonyl)]aminomethyl] pentanoyl]-3-pyrrolidinon;
(3RS,4RS)-4-[(2S)-4-Methyl-2-[(benzyloxycarbonyl)amino]pentyl]-1-[(2S)-4-methyl-2-[[(benzyloxycarbonyl)] amino]pentanoyl]-3-pyrrolidinol;
4-[(2S)-4-Methyl-2-[(benzyloxycarbonyl)amino]pentyl]-1-[(2S)-4-methyl-2-[[(benzyloxycarbonyl)]amino]pentanoyl]-3-pyrrolidinon;
(3RS,4RS)-4-[[$N^{\alpha}$-(4-Fluorbenzoyl)-L-leucinyl]amino]-1-[2-[($\alpha$-toluolsulfonyl)amino]ethyl]-3-pyrrolidinol;
4-[[$N^{\alpha}$-(4-Fluorbenzoyl)-L-leucinyl]amino]-1-[2-[($\alpha$-toluolsulfonyl)amino]ethyl]-3-pyrrolidinon;
(3RS,4RS)-4-[[$N^{\alpha}$-(4-Fluorbenzoyl)-L-leucinyl]amino]-1-benzoyl-3-pyrrolidinol;
4-[[$N^{\alpha}$-(4-Fluorbenzoyl)-L-leucinyl]amino]-1-benzoyl-3-pyrrolidinon;
(3RS,4RS)-4-[[$N^{\alpha}$-(Piperonylcarbonyl)-L-leucinyl]amino]-1-benzoyl-3-pyrrolidinol;
4-[[$N^{\alpha}$-(Piperonylcarbonyl)-L-leucinyl]amino]-1-benzoyl-3-pyrrolidinon;
(3RS,4RS)-4-[[$N^{\alpha}$-(4-Fluorbenzoyl)-L-leucinyl]amino]-1-[2-[(4-fluorbenzoyl)amino]ethyl]-3-pyrrolidinol;
4-[[$N^{\alpha}$-(4-Fluorbenzoyl)-L-leucinyl]amino]-1-[2-[(4-fluorbenzoyl)amino]ethyl]-3-pyrrolidinon;
(3RS,4RS)-4-[[$N^{\alpha}$-(*tert*-Butoxycarbonyl)-L-leucinyl]amino]-1-benzoyl-3-pyrrolidinol;
4-[[$N^{\alpha}$-(*tert*-Butoxycarbonyl)-L-leucinyl]amino]-1-benzoyl-3-pyrrolidinon;
(3RS,4RS)-4-[[$N^{\alpha}$-(4-Fluorbenzoyl)-L-leucinyl]amino]-1-[(2S)-4-methyl-2-[(4-fluorbenzoyl)amino]pentyl]-3-pyrrolidinol;
4-[[$N^{\alpha}$-(4-Fluorbenzoyl)-L-leucinyl]amino]-1-[(2S)-4-methyl-2-[(4-fluorbenzoyl)amino]pentyl]-3-pyrrolidinon;
4-[[$N^{\alpha}$-(4-Carboxybenzoyl)-L-leucinyl]amino]-1-[(2S)-4-methyl-2-[(4-fluorbenzoyl)amino]pentyl]-3-pyrrolidinon;
1-Benzyl-4-[[$N^{\alpha}$-(4-carboxybenzoyl)-L-leucinyl]amino]-3-pyrrolidinon;
(3RS,4RS)-1-Benzyl-4-[[$N^{\alpha}$-(4-carboxymethyl)benzoyl)-L-leucinyl]amino]-3-pyrrolidinol;
1-Benzyl-4-[[$N^{\alpha}$-(4-carboxymethyl)benzoyl)-L-leucinyl]amino]-3-pyrrolidinon;
(3RS,4RS)-4-[[$N^{\alpha}$-[(4-Carboxymethyl)benzoyl]-L-leucinyl]amino]-1-[(2S)-4-methyl-2-[[(4-fluorbenzoyl)amino] pentyl]-3-pyrrolidinol;
4-[[$N^{\alpha}$-[(4-Carboxymethyl)benzoyl]-L-leucinyl]amino]-1-[(2S)-4-methyl-2-[[(4-fluorbenzoyl)amino]pentyl]-3-pyrrolidinon;
(3RS,4RS)-1-Phenethyl-4-[[$N^{\alpha}$-(2-amino-$\alpha$-toluolsulfonyl)-L-leucinyl]amino]-3-pyrrolidinol;
1-Phenethyl-4-[[$N^{\alpha}$-(2-amino-$\alpha$-toluolsulfonyl)-L-leucinyl]amino]-3-pyrrolidinon;

(3RS,4RS)-4-[[Nα-(2-Naphthylcarbonyl)-L-leucinyl]amino]-1-benzoyl-3-piperidinol;
4-[[Nα-(2-Naphthylcarbonyl)-L-leucinyl]amino]-1-benzoyl-3-piperidinon;
(3RS,4RS)-4-[[Nα-(2-Chinolincarbonyl)-L-leucinyl]amino]-1-benzoyl-3-piperidinol;
4-[[Nα-(2-Chinolincarbonyl)-L-leucinyl]amino]-1-benzoyl-3-piperidinon;
(3RS,4RS)-4-[[Nα-(3-Isochinolincarbonyl)-L-leucinyl]amino]-1-benzoyl-3-piperidinol;
4-[[Nα-(3-Isochinolincarbonyl)-L-leucinyl]amino]-1-benzoyl-3-piperidinon;
(3RS,4RS)-4-[[(2S)-4-Methyl-2-(benzyl)oxy]pentanoyl]-1-[(2S)-4-methyl-2-[[(benzyloxycarbonyl)]amino]pentanoyl]-3-piperidinol;
4-[[(2S)-4-Methyl-2-(benzyl)oxy]pentanoyl]-1-[(2S)-4-methyl-2-[[(benzyloxycarbonyl)]amino]pentanoyl]-3-piperidinon;
(3RS,4RS)-4-[[3-(2-Pyridyl)phenylacetyl]amino]-1-[3-(2-pyridyl)phenylacetyl]-3-piperidinol;
4-[[3-(2-Pyridyl)phenylacetyl]amino]-1-[3-(2-pyridyl)phenylacetyl]-3-piperidinon;
(3RS,4RS)-4-[[Nα-(*p*-Trifluormethanphenylsulfonyl)-L-leucinyl]amino]-1-[3-(2-pyridyl)phenylacetyl]-3-piperidinol;
4-[[Nα-(*p*-Trifluormethanphenylsulfonyl)-L-leucinyl]amino]-1-[3-(2-pyridyl)phenylacetyl]-3-piperidinon;
(3RS,4RS)-4-[[Nα-(2-Naphthylsulfonyl)-L-leucinyl]amino]-1-[3-(2-pyridyl)phenylacetyl]-3-piperidinol;
4-[[Nα-(2-Naphthylsulfonyl)-L-leucinyl]amino]-1-[3-(2-pyridyl)phenylacetyl]-3-piperidinon;
(3RS,4RS)-4-[[Nα-(3,4-Dichlorphenylsulfonyl)-L-leucinyl]amino]-1-[3-(2-pyridyl)phenylacetyl]-3-piperidinol;
4-[[Nα-(3,4-Dichlorphenylsulfonyl)-L-leucinyl]amino]-1-[3-(2-pyridyl)phenylacetyl]-3-piperidinon;
(3RS,4RS)-4-[[Nα-(Methansulfonyl)-L-leucinyl]amino]-1-[3-(2-pyridyl)phenylacetyl]-3-piperidinol;
4-[[Nα-(Methansulfonyl)-L-leucinyl]amino]-1-[3-(2-pyridyl)phenylacetyl]-3-piperidinon;
(3RS,4RS)-4-[[Nα-(Fluorphenylsulfonyl)-L-leucinyl]amino]-1-[3-(2-pyridyl)phenylacetyl]-3-piperidinol; oder
4-[[Nα-(Fluorphenylsulfonyl)-L-leucinyl]amino]-1-[3-(2-pyridyl)phenylacetyl]-3-piperidinon;

oder ein pharmazeutisch verträgliches Salz davon.

**17.** Verbindung nach einem der Ansprüche 1 bis 16 zur Verwendung in der Therapie.

**18.** Arzneimittel, umfassend eine Verbindung nach einem der Ansprüche 1 bis 16 und einen pharmazeutisch verträglichen Träger.

**19.** Verbindung nach einem der Ansprüche 1 bis 16 zur Verwendung bei der Behandlung von Erkrankungen, bei welchen die Hemmung einer Cysteinprotease ein Faktor ist.

**20.** Verwendung gemäß Anspruch 19, wobei die Cysteinprotease Kathepsin K ist.

**21.** Verbindung nach einem der Ansprüche 1 bis 16 zur Verwendung bei der Hemmung von Knochenschwund.

**22.** Verbindung nach einem der Ansprüche 1 bis 16 zur Verwendung bei der Behandlung von Osteoporose.

**23.** Verbindung nach einem der Ansprüche 1 bis 16 zur Verwendung bei der Behandlung einer Zahnfleisch- oder Periodontalerkrankung.

**24.** Verbindung nach einem der Ansprüche 1 bis 16 zur Verwendung bei der Behandlung einer Erkrankung, welche durch übermäßigen Knorpel- oder Matrixabbau gekennzeichnet ist.

**25.** Verwendung gemäß Anspruch 24, wobei die Erkrankung Osteoarthrose oder rheumatoide Arthritis ist.

**26.** Verwendung einer Verbindung der Formel (I), wie in einem der Ansprüche 1 bis 16 definiert, bei der Herstellung eines Medikaments zur Behandlung von Erkrankungen, bei welchen die Hemmung einer Cysteinprotease ein Faktor ist.

**27.** Verfahren zur Herstellung einer Verbindung der Formel (I), wie in einem der Ansprüche 1 bis 16 definiert, wobei das Verfahren umfasst:

(A) für Verbindungen, in welchen A CH(OH) ist:

(i) Umsetzen einer Verbindung der Formel (III):

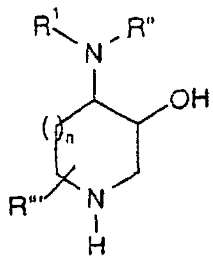

(III)

oder eines Salzes davon,
wobei $R^1$, R'', R''' und n wie in Formel (I) von Anspruch 1 definiert sind, und wobei irgendwelche reaktiven, funktionellen Gruppen geschützt sind, mit:

(a) $R^5C(O)Cl$, wobei $R^5$ wie in Formel (I) von Anspruch 1 definiert ist; oder
(b) $R^5C(O)OH$, wobei $R^5$ wie in Formel (I) von Anspruch 1 definiert ist, in Gegenwart von EDC und HOBT; oder
(c) $R^5C(O)H$, wobei $R^5$ wie in Formel (I) von Anspruch 1 definiert ist, und nachfolgernder Reduktion; oder
(d) $R^5OC(O)Cl$, wobei $R^5$ wie in Formel (I) von Anspruch 1 definiert ist, in Gegenwart einer Base; oder
(e) $R^5SO_2Cl$, wobei $R^5$ wie in Formel (I) von Anspruch 1 definiert ist, in Gegenwart einer Base; oder
(f)

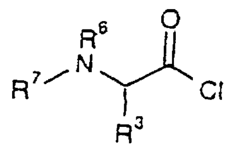

wobei $R^3$, $R^6$ und $R^7$ wie in Formel (I) von Anspruch 1 definiert sind; oder
(g) Adamantyl-C(O)Cl;

(ii) Umsetzen einer Verbindung der Formel (IV)

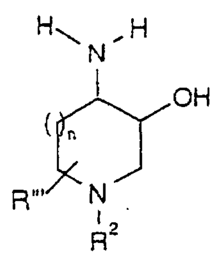

(IV)

wobei $R^2$, R''' und n wie in Formel (I) von Anspruch 1 definiert sind, und wobei irgendwelche reaktiven, funktionellen Gruppen geschützt sind, mit:

(a)

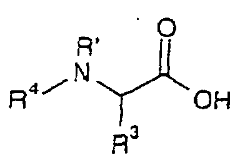

wobei $R^3$, $R^4$ und R' wie in Formel (I) von Anspruch 1 definiert sind, in Gegenwart von EDC und HOBT; oder

(b)

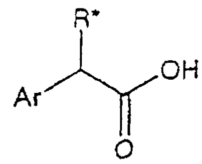

wobei R* wie in Formel (I) von Anspruch 1 definiert ist, in Gegenwart von EDC und HOBT; oder

(c)

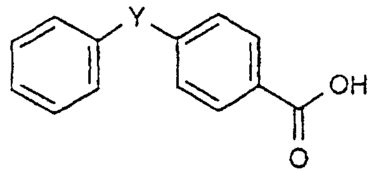

wobei Y wie in Formel (I) von Anspruch 1 definiert ist, in Gegenwart von EDC und HOBT; oder (d)

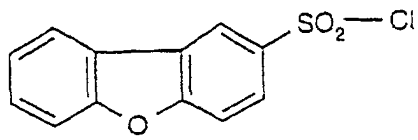

;

(iii) Umsetzen einer Verbindung der Formel (V):

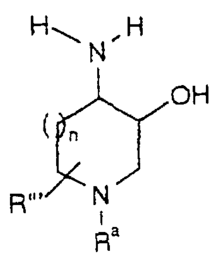

(V)

wobei R''' und n wie in Formel (I) von Anspruch 1 definiert sind, und wobei irgendwelche reaktiven, funktionellen Gruppen geschützt sind, mit:

(a)

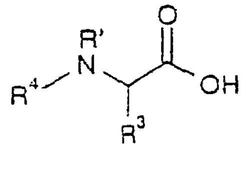

wobei $R^3$, $R^4$ und R' wie in Formel (I) von Anspruch 1 definiert sind, in Gegenwart von EDC und HOBT; oder

(b)

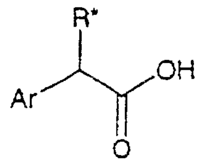

wobei R* wie in Formel (I) von Anspruch 1 definiert ist, in Gegenwart von EDC und HOBT; oder

(c)

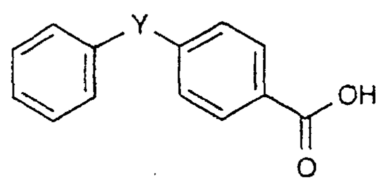

wobei Y wie in Formel (I) von Anspruch 1 definiert ist, in Gegenwart von EDC und HOBT; oder (d)

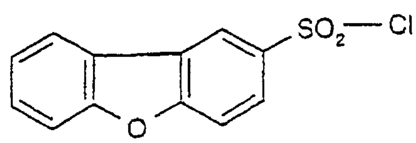

;

(B) für Verbindungen, in welchen A C(O) ist:

(i) Umsetzen einer Verbindung der Formel (VI):

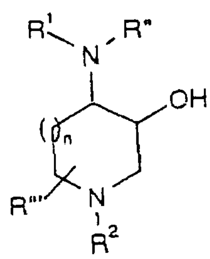

(VI).

wobei $R^1$, $R^2$, R'', R''' und n wie in Formel (I) von Anspruch 1 definiert sind, und wobei irgendwelche reaktiven, funktionellen Gruppen geschützt sind, mit einem Oxidationsmittel;

und nachfolgend Entfernen jeglicher Schutzgruppen und gegebenenfalls Bilden eines pharmazeutisch verträglichen Salzes.

**Revendications**

**1.** Composé de formule (I) :

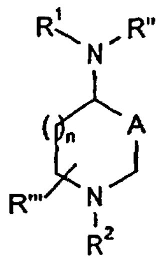

(I)

dans laquelle :

A représente un groupe C(O) ou CH(OH) ;
$R^1$ représente un groupe

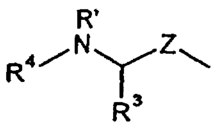

,

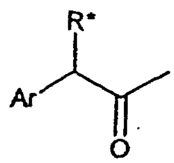

,

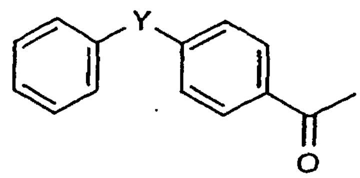

,

ou

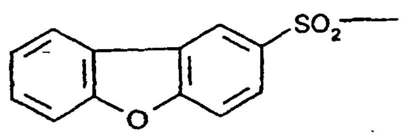

;

$R^2$ représente H, un groupe alkyle en $C_1$ à $C_6$ facultativement substitué indépendamment avec un ou deux substituants halogéno, SR, OR, $N(R)_2$, $C(O)N(R)_2$, carbamyle ou alkyle en $C_1$ à $C_4$, (cycloalkyle en $C_3$ à $C_6$) -(alkyle en $C_0$ à $C_6$), Ar-(alkyle en $C_0$ à $C_6$), Het-(alkyle en $C_0$ à $C_6$), $R^5C(O)$-, $R_5C(S)$-, $R^5SO_2$-, $R^5OC(O)$-, $R^5R'NC(O)$-, $R^5R'NC(S)$-, adamantyl-C(O)-, ou

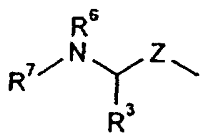

;

R" représente H, un groupe alkyle en $C_1$ à $C_6$ facultativement substitué indépendamment avec un ou deux substituants halogéno, SR, OR, $N(R)_2$, $C(O)N(R)_2$, carbamyle ou alkyle en $C_1$ à $C_4$, Ar-(alkyle en $C_0$ à $C_6$), ou Het-(alkyle en $C_0$ à $C_6$) ;
R" ' représente H, un groupe alkyle en $C_1$ à $C_6$ facultativement substitué indépendamment avec un ou deux substituants halogèno, SR, OR, $N(R)_2$, $C(O)N(R)_2$, carbamyle ou alkyle en $C_1$ à $C_4$, (cycloalkyle en $C_3$ à $C_6$) -(alkyle en $C_0$ à $C_6$), Ar-(alkyle en $C_0$ à $C_6$) ou Het-(alkyle en $C_0$ à $C_6$) ;
chaque groupe $R^3$ représente indépendamment H, un groupe alcényle en $C_2$ à $C_6$, alcynyle en $C_2$ à $C_6$, Het, Ar ou alkyle en $C_1$ à $C_6$ faculativement substitué avec un substituant OR', SR', $NR'_2$, $R'NC(O)OR^5$, $CO_2R'$ , $CO_2NR'_2$, $N(C=NH)NH_2$, Het ou Ar ;
$R^4$ représente H, un groupe alkyle en $C_1$ à $C_6$ facultativement substitué indépendamment avec un ou deux substituants halogéno, SR, OR, $N(R)_2$, $C(O)N(R)_2$, carbamyle ou alkyle en $C_1$ à $C_4$, (cycloalkyle en $C_3$ à $C_6$) -(alkyle en $C_0$ à $C_6$), Ar-(alkyle en $C_0$ à $C_6$), Het-(alkyle en $C_0$ à $C_6$), $R^5C(O)$-, $R^5C(S)$, $R^5SO_2$-, $R_5OC(O)$-,

$R^5R'NC(O)-$, $R^5R'NC(S)-$, $R'HNCH(R')C(O)-$ ou $R^5OC(O)NR'CH(R')C(O)-$ ;
chaque groupe $R^5$ représente indépendamment un groupe (cycloalkyle en $C_3$ à $C_6$)-(alkyle en $C_0$ à $C_6$), Ar-(alkyle en $C_0$ à $C_6$), Het- (alkyle en $C_0$ à $C_6$), Ar-(alkoxy en $C_0$ à $C_6$), Het-(alkoxy en $C_0$ à $C_6$), ou alkyle en $C_1$ à $C_6$ facultativement substitué avec un substituant OR', SR', $NR'_2$, $R'NC-(O)OR^5$, $CO_2R'$, $CO_2NR'_2$, N(C=NH) $NH_2$, Het ou Ar ;
$R^6$ représente H, un groupe alkyle en $C_1$ à $C_6$ facultativement substitué indépendamment avec un ou deux substituants halogéno, SR, OR, $N(R)_2$, $C(O)N(R)_2$, carbamyle ou alkyle en $C_1$ à $C_4$, Ar-(alkyle en $C_0$ à $C_6$) ou Het-(alkyle en $C_0$ à $C_6$), et $R^7$ représente H, un groupe alkyle en $C_1$ à $C_6$ facultativement substitué indépendamment avec un ou deux substituants halogéno, SR, OR, $N(R)_2$, $C(O)N(R)_2$, carbamyle ou alkyle en $C_1$ à $C_4$, (cycloalkyle en $C_3$ à $C_6$)-(alkyle en $C_0$ à $C_6$), Ar-(alkyle en $C_0$ à $C_6$), Het-(alkyle en $C_0$ à $C_6$), $R^5C(O)-$, $R^5C(S)-$, $R^5SO_2-$, $R^5OC(O)-$, $R^5R'NC(O)-$, $R^5R'NC(S)-$, $R'HNCH(R')C(O)-$ ou $R^5OC(O)NR'CH(R')C(O)-$ ; ou bien $R^6$ et $R^7$ sont connectés pour former un noyau pyrrolidine, un noyau pipéridine ou un noyau morpholine ;
chaque groupe R' représente indépendamment H, un groupe alkyle en $C_1$ à $C_6$, Ar-(alkyle en $C_0$ à $C_6$) ou Het-(alkyle en $C_0$ à $C_6$) ;
R* représente H, un groupe alkyle en $C_1$ à $C_6$, (cycloalkyle en $C_3$ à $C_6$)-(alkyle en $C_0$ à $C_6$), Ar- (alkyle en $C_0$ à $C_6$) ou Het-(alkyle en $C_0$ à $C_6$) ;
R représente H ou un groupe alkyle en $C_1$ à $C_6$ ;
Y représente une liaison simple ou O ;
chaque groupe Z représente indépendamment un groupe CO ou $CH_2$ ;
n est égal à 0, 1 ou 2 ;

le groupe cycloalkyle en $C_3$ à $C_6$ est facultativement substitué indépendamment avec un ou deux substituants halogéno, SR, OR, $N(R)_2$, $C(O)N(R)_2$, carbamyle ou alkyle en $C_1$ à $C_4$, dans lequel R représente H ou un groupe alkyle en $C_1$ à $C_6$ ;
Ar représente un groupe phényle ou naphtyle non substitué ; ou un groupe phényle ou naphtyle substitué avec un ou plusieurs substituants Ph-(alkyle en $C_0$ à $C_6$), Het-(alkyle en $C_0$ à $C_6$), alkoxy en $C_1$ à $C_6$, Ph- (alkoxy en $C_0$ à $C_6$), Het-(alkoxy en $C_0$ à $C_6$), OH, $(CH_2)_{1-6}NR^aR^a$, $O(CH_2)_{1-6}NR^aR^a$ ; ou un groupe phényle ou naphtyle substitué avec un à trois groupements choisis entre des groupements alkyle en $C_1$ à $C_4$, $OR^a$, $N(R^a)_2$, $SR^a$, $CF_3$, $NO_2$, CN, $CO_2R^a$, $CON(R^a)$, F, Cl, Br et I, ou substitué avec un groupe méthylènedioxy ; et
Het représente un noyau hétérocyclique stable penta- à heptagonal monocyclique ou stable hepta- à décagonal bicyclique, qui est saturé ou insaturé, et qui consiste en atomes de carbone et un à quatre hétéroatomes choisis dans le groupe consistant en N, O et S, dans lequel les hétéroatomes de N et S peuvent être facultativement oxydés, et l'hétéroatome de N peut être facultativement transformé en dérivé quaternaire, y compris n'importe quel groupe bicyclique dans lequel n'importe lequel des noyaux hétérocycliques définis ci-dessus est condensé avec un noyau benzénique ; le groupe Het pouvant être facultativement substitué avec un ou deux groupements choisis entre des groupements alkyle en $C_1$ à $C_4$, $OR^a$, $N(R^a)_2$, $SR^a$, $CF_3$, $NO_2$, CN, $CO_2R^a$, $CON(R^a)$, F, Cl, Br et I, et chaque groupe $R^a$ représentant indépendamment H, un groupe alkyle en $C_1$ à $C_6$, Ar-(alkyle en $C_0$ à $C_6$) ou Het-(alkyle en $C_0$ à $C_6$) ;
ou un de ses sels pharmaceutiquement acceptables.

**2.** Composé suivant la revendication 1, dans lequel $R^1$ représente un groupe

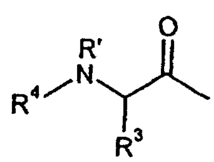

.

**3.** Composé suivant la revendication 1 ou la revendication 2, dans lequel $R^4$ représente un groupe $R^5C(O)-$, $R^5SO_2-$ ou $R^5OC(O)-$.

**4.** Composé suivant l'une quelconque des revendications 1 à 3, dans lequel $R^5$ dans ledit groupe $R^1$ est un groupe Ar-(alkyle en $C_0$ à $C_6$) ou Het-(alkyle en $C_0$ à $C_6$).

**5.** Composé suivant l'une quelconque des revendications 1 à 4, dans lequel $R^5$ dans ledit groupe $R^1$ est un groupe phényle ou benzyle qui est non substitué ou substitué avec un ou deux des groupes consistant en groupes Cl, Br,

F, $CF_3$, alkyle en $C_1$ à $C_4$, OH, alkoxy en $C_1$ à $C_4$, CN, $CONH_2$, $NH_2$ ou $NO_2$, ou qui est substitué avec un groupe méthylènedioxy ; ou

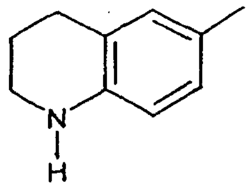
,
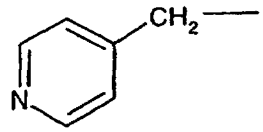
,
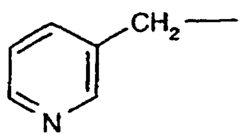
,
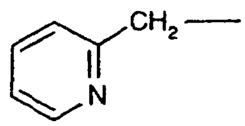
,
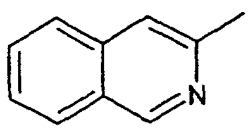
,
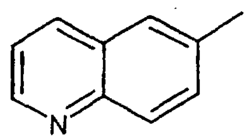
,
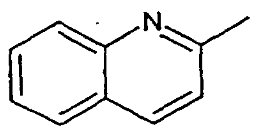
,
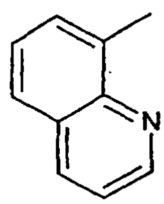
,
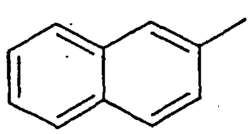
, ou
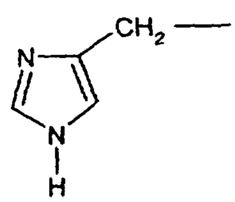
.

6. Composé suivant la revendication 1 ou la revendication 2, dans lequel, dans ledit groupe $R^1$, R' représente H ou un groupe $CH_3$ et $R^3$ représente un groupe isobutyle.

7. Composé suivant la revendication 1, dans lequel $R^1$ représente un groupe

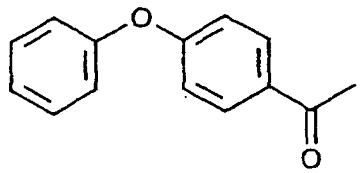

ou

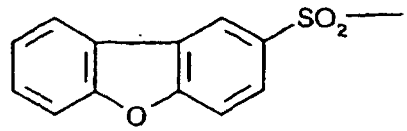

.

**8.** Composé suivant la revendication 1, dans lequel $R^2$ représente un groupe

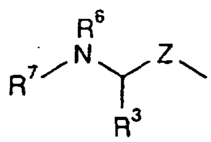

.

**9.** Composé suivant la revendication 1 ou la revendication 8, dans lequel $R^7$ représente un groupe $R^5OC(O)$-.

**10.** Composé suivant l'une quelconque des revendications 1, 8 et 9, dans lequel $R^5$ dans ledit groupe $R^7$ est un groupe Ar-(alkyle en $C_0$ à $C_6$) ou Het-(alkyle en $C_0$ à $C_6$).

**11.** Composé suivant l'une quelconque des revendications 1 et 8 à 10, dans lequel $R^5$ dans ledit groupe $R^7$ est un groupe phényle ou benzyle qui est non substitué ou substitué avec un ou deux des groupes consistant en des groupes Cl, Br, F, $CF_3$, alkyle en $C_1$ à $C_4$, OH, alkoxy en $C_1$ à $C_4$, CN, $CONH_2$, $NH_2$ et $NO_2$, ou qui est substitué avec un groupe méthylènedioxy ; ou un groupe 2-, 3- ou 4-pyridyl-$CH_2$-.

**12.** Composé suivant la revendication 1, dans lequel $R^2$ représente un groupe

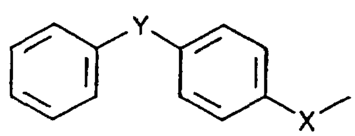

dans lequel X représente un groupe CO, $SO_2$ ou $CH_2$-CO et Y représente une liaison simple ou O.

**13.** Composé suivant la revendication 1 ou la revendication 8, dans lequel, dans ledit groupe $R^2$, $R^6$ représente H ou un groupe $CH_3$ et $R^3$ représente un groupe isobutyle.

**14.** Composé suivant la revendication 1, dans lequel $R^2$ représente un groupe Ar-(alkyle en $C_0$ à $C_6$), Het-(alkyle en $C_0$ à $C_6$), (cycloalkyle en $C_3$ à $C_6$)-(alkyle en $C_0$ à $C_6$), $R^5C(O)$-, $R^5C(S)$-, $R^5SO_2$-, $R^5OC(O)$-, $R^5R'NC(O)$-, $R^5R'NC(S)$-, R'HNCH(R')C(O)-, $R^5OC(O)NR'CH(R')C(O)$- ou adamantyl-C(O)-.

**15.** Composé suivant la revendication 1, dans lequel A représente un groupe C(O).

**16.** Composé suivant la revendication 1, qui consiste en :

(3RS, 4RS)-4-[[N$^{\alpha}$-(benzyloxycarbonyl)-L-leucinyl]-amino]-1-[(2S)-4-méthyl-2-[[(benzyloxycarbonyl)]amino]-pentanoyl]-3-pyrrolidinol ;
4-[[N$^{\alpha}$-(benzyloxycarbonyl)-L-leucinyl)amino]-1-[(2S)-4-méthyl-2-[[(benzyloxycarbonyl)]amino]pentanoyl]-3-pyrrolidinone ;
4-[[N$^{\alpha}$-(benzyloxycarbonyl)-L-leucinyl]amino]-1-[4-(phénoxybenzamide)]-3-pyrrolidinone ;
(3RS,4RS)-4-[[N$^{\alpha}$-(benzyloxycarbonyl)-L-leucinyl]-amino]-1-[4-(biphényléthanoyl)]-3-pyrrolidinol ;
4-[[N$^{\alpha}$-(benzyloxycarbonyl)-L-leucinyl]amino]-1-[4-(biphényléthanoyl)]-3-pyrrolidinone ;
(3RS,4RS)-4-[[N$^{\alpha}$-(benzyloxycarbonyl)-L-leucinyl]-amino]-1-[(2S)-4-méthyl-2-[[(benzyloxycarbonyl)]amino-méthyl]-pentanoyl]-3-pyrrolidinol ;
4-[[N$^{\alpha}$-(benzyloxycarbonyl)-L-leucinyl]amino]-1-[(2S)-4-méthyl-2-[[(benzyloxycarbonyl)]aminométhyl]-pentanoyl]-3-pyrrolidinone ;
(3RS,4RS)-4-[[N$^{\alpha}$-(benzyloxycarbonyl)-L-leucinyl]-amino]-1-[(2S)-4-méthyl-2-[[(tertiobutoxycarbonyl)]amino]-pentanoyl]-3-pyrrolidinol ;
4-[[N$^{\alpha}$-(benzyloxycarbonyl)-L-leucinyl]amino]-1-[(2S)-4-méthyl-2-[[(tertiobutoxycarbonyl)]aminométhyl]-pentanoyl]-3-pyrrolidinone ;
4-[[N$^{\alpha}$-(benzyloxycarbonyl)-L-leucinyl]amino]-1-[(2S)-4-méthyl-2-(aminométhyl)-pentanoyl]-3-pyrrolidinone ;
4-[[N$^{\alpha}$-(benzyloxycarbonyl)-L-leucinyl]amino]-1-tertiobutoxycarbonyl-3-pyrrolidinone ;
4-[[N$^{\alpha}$-(benzyloxycarbonyl)-L-leucinyl]amino]-3-pyrrolidinone ;
4-[[N$^{\alpha}$-(benzyloxycarbonyl)-L-leucinyl]amino]-1-[(2S)-4-méthyl-2-[[(N-tertiobutoxycarbonyl)éthanoyl]-aminométhyl]pentanoyl]-3-pyrrolidinone ;
4-[[N$^{\alpha}$-(benzyloxycarbonyl)-L-leucinyl]amino]-1-[(2S)-4-méthyl-2-[(éthanoyl)aminométhyl]-pentanoyl]-3-pyrrolidinone ;
(3RS,4RS)-4-[[N$^{\alpha}$-(benzyloxycarbonyl)-L-leucinyl]-amino]-1-[(2S)-4-méthyl-2-[[(tertiobutoxycarbonyl)]amino]-pentanoyl]-3-pyrrolidinol ;
4-[[N$^{\alpha}$-(benzyloxycarbonyl)-L-leucinyl]amino]-1-[(2S)-4-méthyl-2-[[(tertiobutoxycarbonyl)]amino]pentanoyl]-3-pyrrolidinone ;
(3RS, 4RS)-4-[[N$^{\alpha}$-(benzyloxycarbonyl)-L-leucinyl]-amino]-1-[(2R)-4-méthyl-2-[[(benzyloxycarbonyl)]amino]-pentanoyl]-3-pyrrolidinol ;
4-[[N$^{\alpha}$-(benzyloxycarbonyl)-L-leucinyl]amino]-1-[(2R)-4-méthyl-2-[[(benzyloxycarbonyl)]amino]pentanoyl]-3-pyrrolidinone ;
(3RS, 4RS)-4-[[N$^{\alpha}$-(benzyloxycarbonyl)-L-leucinyl]-amino]-1-[2-[(benzyloxycarbonyl)amino]-éthanoyl]-3-pyrrolidinol ;
4-[[[N$^{\alpha}$-(benzyloxycarbonyl)-L-leucinyl]amino]-1-[2-[(benzyloxycarbonyl)amino]-éthanoyl]-3-pyrrolidinone ;
(3RS,4RS)-4-[[N$^{\alpha}$-(benzyloxycarbonyl)-L-leucinyl]-amino]-1-[(2S)-3-tertiobutoxy[[(benzyloxycarbonyl)]amino]-propanoyl]-3-pyrrolidinol ;
(3RS,4RS)-4-[[N$^{\alpha}$-(benzyloxycarbonyl)-L-leucinyl]-amino]-1-[(2S)-2-[[(benzyloxycarbonyl)]amino]-propanoyl]-3-pyrrolidinol ;
4-[[N$^{\alpha}$-(benzyloxycarbonyl)-L-leucinyl]amino]-1-[(2S)-2-[[(benzyloxycarbonyl)]amino]propanoyl]-3-pyrrolidinone ;
(3RS,4RS)-4-[[N$^{\alpha}$-(benzyloxycarbonyl)-L-leucinyl]-amino]-1-[cyclohexanepropanoyl]-3-pyrrolidinol ;
4-[[N$^{\alpha}$-(benzyloxycarbonyl)-L-leucinyl]amino]-1-[cyclohexanepropanoyl]-3-pyrrolidinone ;
(3RS,4RS)-4-[[N$^{\alpha}$-(benzyloxycarbonyl)-L-leucinyl]-amino]-1-[(2S)-4-méthyl-2-[[(4-pyridinylméthoxycarbonyl)]-amino]-pentanoyl]-3-pyrrolidinol ;
4-[[N$^{\alpha}$-(benzyloxycarbonyl)-L-leucinyl]amino]-1-[(2S)-4-méthyl-2-[[(4-pyridinylméthoxycarbonyl)]-amino]-pentanoyl]-3-pyrrolidinone ;
(3RS,4RS)-4-[[N$^{\alpha}$-(benzyloxycarbonyl)-L-leucinyl]-amino]-1-[(2S)-4-méthyl-2-[[(2-pyridinylméthoxycarbonyl)]-amino]pentanoyl]-3-pyrrolidinol ;
4-[[N$^{\alpha}$-(benzyloxycarbonyl)-L-leucinyl]amino]-1-[(2S)-4-méthyl-2-[[(2-pyridinylméthoxycarbonyl)]-amino]-pentanoyl]-3-pyrrolidinone ;
(3RS,4RS)-4-[[N$^{\alpha}$-(benzyloxycarbonyl)-L-leucinyl]-amino]-1-[(2S)-4-méthyl-2-[[(3-pyridinylméthoxycarbonyl)]-amino]pentanoyl]-3-pyrrolidinol ;
4-[[N$^{\alpha}$-(benzyloxycarbonyl)-L-leucinyl]amino]-1-[(2S)-4-méthyl-2-[[(3-pyridinylméthoxycarbonyl)]-amino]-pentanoyl]-3-pyrrolidinone ;
(3RS,4RS)-4-[[N$^{\alpha}$-(benzyloxycarbonyl)-L-leucinyl]-amino]-1-(2-pyridylcarbonyl)-3-pyrrolidinol ;
4-[[N$^{\alpha}$-(benzyloxycarbonyl)-L-leucinyl]amino]-1-(2-pyridylcarbonyl)-3-pyrrolidinone;
(3RS,4RS)-4-[[N$^{\alpha}$-(benzyloxycarbonyl)-L-leucinyl]-amino]-1-[(2S)-4-méthyl-2-[[(benzyloxycarbonyl)]-amino] pentanoyl]-3-pipéridinol ;
4-[[N$^{\alpha}$-(benzyloxycarbonyl)-L-leucinyl]amino]-1-[(2S)-4-méthyl-2-[[(benzyloxycarbonyl)]-amino]pentanoyl]-3-pipéridinone ;
(3RS,4RS)-4-[[N$^{\alpha}$-(benzyloxycarbonyl)-L-leucinyl]-amino]-1-[4-biphényl)éthanoyl]-3-pipéridinol ;
4-[[N$^{\alpha}$-(benzyloxycarbonyl)-L-leucinyl]amino]-1-[4-(biphényl)éthanoyl]-3-pipéridinone ;
(3RS,4RS)-4-[[N$^{\alpha}$-(benzyloxycarbonyl)-L-leucinyl]-amino]-1-[(2S)-4-méthyl-2-[[(benzyloxycarbonyl)]-aminométhyl]pentanoyl]-3-pipéridinol ;
4-[[N$^{\alpha}$-(benzyloxycarbonyl)-L-leucinyl]amino]-1-[(2S)-4-méthyl-2-[[(benzyloxycarbonyl)]-aminométhyl]-pentanoyl]-3-pipéridinone ;
4-[[N$^{\alpha}$-(benzyloxycarbonyl)-L-leucinyl]amino]-1-tertiobutoxycarbonyl-3-pipéridinone ;

(3RS,4RS)-4-[[N$^{\alpha}$-(benzyloxycarbonyl)-L-leucinyl]-amino]-1-[2-[[(benzyloxycarbonyl)]isobutylamino)éthanoyl]-3-pipéridinol ;
4-[[N$^{\alpha}$-(benzyloxycarbonyl)-L-leucinyl]amino]-1-[2-[[(benzyloxycarbonyl)]isobutylamino]éthanoyl]-3-pipéridinone ;
(3RS,4RS)-4-[[N$^{\alpha}$-(benzyloxycarbonyl)-L-leucinyl]-amino]-1-[2-[(tertiobutoxycarbonyl)amino)éthanoyl]-3-pipéridinol ;
4-[[N$^{\alpha}$-(benzyloxycarbonyl)-L-leucinyl]amino]-1-[2-[[(tertiobutoxycarbonyl)amino]éthanoyl]-3-pipéridinone ;
4-[[N$^{\alpha}$-(benzyloxycarbonyl)-L-leucinyl]amino]-1-[2-(amino)éthanoyl]-3-pipéridinone ;
(3RS,4RS)-4-[[N$^{\alpha}$-(benzyloxycarbonyl)-L-leucinyl]-amino]-1-(4-méthylpentanoyl)-3-pipéridinol ;
4-[[N$^{\alpha}$-(benzyloxycarbonyl)-L-leucinyl]amino]-1-(4-méthylpentanoyl)-3-pipéridinone ;
(3RS,4RS)-4-[[N$^{\alpha}$-(benzyloxycarbonyl)-L-leucinyl]-amino]-1-(benzoyl)-3-pipéridinol ;
4-[[N$^{\alpha}$-(benzyloxycarbonyl)-L-leucinyl]amino]-1-(benzoyl)-3-pipéridinone ;
(3RS,4RS)-4-[[N$^{\alpha}$-(benzyloxycarbonyl)-L-leucinyl]-amino]-1-(acétyl)-3-pipéridinol ;
4-[[N$^{\alpha}$-(benzyloxycarbonyl)-L-leucinyl]amino]-1-(acétyl)-3-pipéridinone ;
(3RS,4RS)-4-[[N$^{\alpha}$-(benzyloxycarbonyl)-L-leucinyl]-amino]-1-(2-pyridoxyacétyl)-3-pipéridinol ;
4-[[N$^{\alpha}$-(benzyloxycarbonyl)-L-leucinyl]amino]-1-(2-pyridoxyacétyl)-3-pipéridinone ;
(3RS, 4RS)-4-[[N$^{\alpha}$-(benzyloxycarbonyl)-L-leucinyl]-amino]-1-[2-[(benzyloxycarbonyl)méthylamino]éthanoyl]-3-pipéridinol ;
4-[[N$^{\alpha}$-(benzyloxycarbonyl)-L-leucinyl]amino]-1-[2-[(benzyloxycarbonyl)méthylamino]éthanoyl]-3-pipéridinone ;
(3RS,4RS)-4-[[N$^{\alpha}$-(benzyloxycarbonyl)-L-leucinyl]-amino]-1-[3-(2-pyridyl)phénylacétyl)]-3-pipéridinol ;
4-[[N$^{\alpha}$-(benzyloxycarbonyl)-L-leucinyl]amino]-1-[3-(2-pyridyl)phénylacétyl)]-3-pipéridinone ;
(3RS,4RS)-4-[[N$^{\alpha}$-(benzyloxycarbonyl)-L-leucinyl]-amino]-1-[2-[(benzyloxycarbonyl)méthylamino]éthanoyl]-3-pyrrolidinol ;
4-[[N$^{\alpha}$-(benzyloxycarbonyl)-L-leucinyl]amino]-1-[2-[(benzyloxycarbonyl)méthylamino]éthanoyl]-3-pyrrolidinone ;
(3RS,4RS)-4-[[N$^{\alpha}$-(benzyloxycarbonyl)-L-leucinyl]-amino]-1-[2-(phénoxy)éthanoyl]-3-pyrrolidinol ;
4-[[N$^{\alpha}$-(benzyloxycarbonyl)-L-leucinyl]amino]-1-[2-[(phénoxy)éthanoyl]-3-pyrrolidinone ;
(3RS,4RS)-4-[[N$^{\alpha}$-(benzyloxycarbonyl)-L-leucinyl]-amino]-1-[2-(phénoxy)éthanoyl]-3-pyrrolidinol ;
4-[[N$^{\alpha}$-(benzyloxycarbonyl)-L-leucinyl]amino]-1-[2-(phénoxy)éthanoyl]-3-pyrrolidinone ;
(3RS,4RS)-4-[[N$^{\alpha}$-(pyridinylméthoxycarbonyl)-L-leucinyl]-amino]-1-[(2-phényl)éthanoyl]-3-pyrrolidinol ;
4-[[N$^{\alpha}$-(4-pyridinylméthoxycarbonyl)-L-leucinyl]amino]-1-[(2-phényl)éthanoyl]-3-pyrrolidinone ;
(3RS,4RS)-4-[[N$^{\alpha}$-(4-pyridinylméthoxycarbonyl)-L-leucinyl]amino]-1-éthanoyl-3-pyrrolidinol ;
4-[[N$^{\alpha}$-(4-pyridinylméthoxycarbonyl)-L-leucinyl]-amino]-1-éthanoyl-3-pyrrolidinone ;
(3RS,4RS)-4-[[N$^{\alpha}$-(4-pyridinylméthoxycarbonyl)-L-leucinyl]amino]-1-(4-cyanobenzoyl)-3-pyrrolidinol ;
4-[[N$^{\alpha}$-(4-pyridinylméthoxycarbonyl)-L-leucinyl]-amino]-1-(4-cyanobenzoyl)-3-pyrrolidinone ;
(3RS,4RS)-4-[[N$^{\alpha}$-(4-pyridinylméthoxycarbonyl)-L-leucinyl]amino]-1-tertiobutoxycarbonyl-3-pyrrolidinol ;
4-[[N$^{\alpha}$-(4-pyridinylméthoxycarbonyl)-L-leucinyl]-amino]-1-tertiobutylcarbonyl-3-pyrrolidinone ;
(3RS,4RS)-4-[[N$^{\alpha}$-(3-pyridinylméthoxycarbonyl)-L-leucinyl]amino]-1-tertiobutoxycarbonyl-3-pyrrolidinol ;
4-[[N$^{\alpha}$-(3-pyridinylméthoxycarbonyl)-L-leucinyl]-amino]-1-tertiobutoxycarbonyl-3-pyrrolidinone ;
4-[[N$^{\alpha}$-(3-pyridinylméthoxycarbonyl)-L-leucinyl]-amino]-3-pyrrolidinone ;
4-[[N$^{\alpha}$-(4-pyridinylméthoxycarbonyl)-L-leucinyl]-amino]-3-pyrrolidinone ;
(3RS,4RS)-4-[[N$^{\alpha}$-(2-pyridinylméthoxycarbonyl)-L-leucinyl]amino]-1-[(2S)-4-méthyl-2-[[(benzyloxycarbonyl))-aminométhyl]pentanoyl]-3-pyrrolidinone ;
4-[[N$^{\alpha}$-(2-pyridinylméthoxycarbonyl)-L-leucinyl]-amino]-1-[(2S)-4-méthyl-2-[[(benzyloxycarbonyl)]amino-méthyl]pentanoyl]-3-pyrrolidinone ;
(3RS,4RS)-4-[[N$^{\alpha}$-(4-pyridinylméthoxycarbonyl)-L-leucinyl]amino]-1-[(2S)-4-méthyl-2-[[(4-pyridinyl-méthoxycarbonyl)]-amino]pentanoyl]-3-pyrrolidinol ;
4-[[N$^{\alpha}$-(4-pyridinylméthoxycarbonyl)-L-leucinyl]-amino]-1-[(2S)-4-méthyl-2-[[(4-pyridinylméthoxy-carbonyl)]amino]pentanoyl]-3-pyrrolidinone ;
(3RS,4RS)-4-[[N$^{\alpha}$-(4-pyridinylméthoxycarbonyl)-L-leucinyl]amino]-1-[(2S)-4-méthyl-2-[[(benzyloxycarbonyl)]-aminométhyl)pentanoyl]-3-pyrrolidinol ;
4-[[N$^{\alpha}$-(4-pyridinylméthoxycarbonyl)-L-leucinyl]-amino]-1-[(2S)-4-méthyl-2-[[(benzyloxycarbonyl)]-aminométhyl]pentanoyl]-3-pyrrolidinone ;
(3RS,4RS)-4-[[N$^{\alpha}$-(3-isoquinoléinecarbonyl)-L-leucinyl)amino)-1-[(2S)-4-méthyl-2-[[(benzyloxycarbonyl)]-amino]pentyl]-3-pyrrolidinol ;
4-[[N$^{\alpha}$-(3-isoquinoléinecarbonyl)-L-leucinyl]-amino]-1-[(2S)-4-méthyl-2-[[(benzyloxycarbonyl)]amino]-pentanoyl)-3-pyrrolidinone ;

(3RS,4RS)-4-[[N$^{\alpha}$-(4-pyridinylméthoxycarbonyl)-L-leucinyl]amino]-1-[1-(adamantyl)carbonyl-3-pyrrolidinol ;
4-[[N$^{\alpha}$-(4-pyridinylméthoxycarbonyl)-L-leucinyl]-amino]-1-[1-(adamantyl)carbonyl]-3-pyrrolidinone ;
(3RS,4RS)-4-[[N$^{\alpha}$-(benzyloxycarbonyl)-L-leucinyl]-amino]-1-(4-méthylpentanoyl)-3-pyrrolidinol ;
4-[[N$^{\alpha}$-(benzyloxycarbonyl)-L-leucinyl]amino]-1-(4-méthylpentanoyl)-3-pyrrolidinone ;
(3RS,4RS)-4-[[N$^{\alpha}$-(benzyloxycarbonyl)-D-leucinyl]-amino]-1-[(2S)-4-méthyl-2-[[(benzyloxycarbonyl)]amino]-pentanoyl]-3-pipéridinol ;
4-[[N$^{\alpha}$-(benzyloxycarbonyl)-D-leucinyl]-amino]-1-[(2S)-4-méthyl-2-[[(benzyloxycarbonyl)]amino]-pentanoyl]-3-pipéridinone ;
(3RS,4RS)-4-[[N$^{\alpha}$-(tertiobutoxycarbonyl)-L-leucinyl]amino]-1-[(2S)-4-méthyl-2-[[(benzyloxycarbonyl)]-amino] pentanoyl]-3-pipéridinol ;
4-[[N$^{\alpha}$-(tertiobutoxycarbonyl)-L-leucinyl]amino]-1-[(2S)-4-méthyl-2-[[(benzyloxycarbonyl)]amino]pentanoyl]-3-pipéridinone ;
(3RS,4RS)-4-[[N$^{\alpha}$-(benzyloxycarbonyl)-N$^{\varepsilon}$-(tertiobutoxycarbonyl)-L-lysine]amino]-1-[(2S)-4-méthyl-2-[[(benzyloxycarbonyl)amino]pentanoyl]-3-pipéridinol ;
4-[[N$^{\alpha}$-(benzyloxycarbonyl)-N$^{\varepsilon}$-(tertiobutoxy-carbonyl)-L-lysine]amino]-1-[(2S)-4-méthyl-2-[[(benzyloxy-carbonyl)amino]pentanoyl]-3-pipéridinone ;
(3RS,4RS)-4-[[N$^{\alpha}$-(4-pyridinylméthoxycarbonyl)-L-leucinyl]amino]-1-tertiobutoxycarbonyl-3-pipéridinol ;
4-[[N$^{\alpha}$-(4-pyridinylméthoxycarbonyl)-L-leucinyl]-amino]-1-tertiobutoxycarbonyl-3-pipéridinone ;
(3RS,4RS)-4-[[N$^{\alpha}$-(4-pyridinylméthoxycarbonyl)-L-leucinyl]amino]-1-(4-méthylpentanoyl)-3-pipéridinol ;
4-[[N$^{\alpha}$-(4-pyridinylméthoxycarbonyl)-L-leucinyl]-amino]-1-(4-méthylpentanoyl)-3-pipéridinone ;
(3RS,4RS)-4-[[N$^{\alpha}$-(4-pyridinylméthoxycarbonyl)-L-leucinyl]amino]-1-[2-(benzyloxycarbonyl)isobutylamino]-éthanoyl]-3-pipéridinol ;
4-[[N$^{\alpha}$-(4-pyridinylméthoxycarbonyl)-L-leucinyl]-amino]-1-[2-(benzyloxycarbonyl)isobutylamino]-éthanoyl]-3-pipéridinone ;
(3RS,4RS)-4-[[N-2-(benzyloxycarbonyl)]isobutyl-amino]-éthanoyl]-1-[(2S)-4-méthyl-2-[[(benzyloxycarbonyl)]-amino]pentanoyl]-3-pipéridinol ;
4-[[N-2-(benzyloxycarbonyl)]isobutylamino]-éthanoyl]-1-[(2S)-4-méthyl-2-[[(benzyloxycarbonyl)]-amino]pentanoyl]-3-pipéridinone ;
(3RS,4RS)-4-[[N$^{\alpha}$-(benzyloxycarbonyl)-L-leucinyl]-amino]-1-(méthanesulfonyl)-3-pipéridinol ;
4-[[N$^{\alpha}$-(benzyloxycarbonyl)-L-leucinyl]-amino]-1-(méthanesulfonyl)-3-pipéridinone ;
(3RS,4RS)-4-[[N$^{\alpha}$-(benzyloxycarbonyl)-L-leucinyl]-amino]-1-(phénylsulfonyl)-3-pipéridinol ;
4-[[N$^{\alpha}$-(benzyloxycarbonyl)-L-leucinyl]amino]-1-(phénylsulfonyl)-3-pipéridinone ;
(3RS,4RS)-4-[[N$^{\alpha}$-(4-pyridinylméthoxycarbonyl)-L-leucinyl]-amino]-1-(8-quinoléinesulfonyl)-3-pyrrolidinol ;
4-[[N$^{\alpha}$-(4-pyridinylméthoxycarbonyl)-L-leucinyl]-amino]-1-(8-quinoléinesulfonyl)-3-pyrrolidinone ;
(3RS,4RS)-4-[[N$^{\alpha}$-(4-pyridinylméthoxycarbonyl)-L-leucinyl]amino]-1-(2-pyridylsulfonyl)-3-pyrrolidinol ;
4-[[N$^{\alpha}$-(4-pyridinylméthoxycarbonyl)-L-leucinyl]-amino]-1-(2-pyridylsulfonyl)-3-pyrrolidinone ;
(3RS,4RS)-4-[[N$^{\alpha}$-(4-pyridinylméthoxycarbonyl)-L-leucinyl]amino]-1-[(2-propoxy)carbonyl]-3-pyrrolidinol ;
4-[[N$^{\alpha}$-(4-pyridinylméthoxycarbonyl)-L-leucinyl]-amino]-1-[(2-propoxy)carbonyl]-3-pyrrolidinone ;
(3RS,4RS)-4-[[N$^{\alpha}$-(4-pyridinylméthoxycarbonyl)-L-leucinyl]amino]-1-[(3-méthyl-1-propoxy)carbonyl]-3-pyrrolidinol ;
4-[[N$^{\alpha}$-(4-pyridinylméthoxycarbonyl)-L-leucinyl]-amino]-1-[(3-méthyl-1-propoxy)carbonyl]-3-pyrrolidinone ;
(3RS,4RS)-4-[[N$^{\alpha}$-(benzyloxycarbonyl)-L-leucinyl]-amino]-1-[(4-phénoxy)phénylsulfonyl]-3-pyrrolidinol ;
4-[[N$^{\alpha}$-(benzyloxycarbonyl)-L-leucinyl]-amino]-1-[(4-phénoxy)phénylsulfonyl]-3-pyrrolidinone ;
(3RS, 4RS)-4-[[N$^{\alpha}$-(benzyloxycarbonyl)-L-leucinyl]-amino]-1-[(4-phénoxy)phénylsulfonyl]-3-pipéridinol ;
4-[[N$^{\alpha}$-(benzyloxycarbonyl)-L-leucinyl]-amino]-1-[(4-phénoxy)phénylsulfonyl]-3-pipéridinone ;
(3RS,4RS)-4-[[N$^{\alpha}$-(3,4-dichlorobenzoyl)-L-leucinyl]amino]-1-[(2S)-4-méthyl-2-[[(benzyloxycarbonyl)]-amino] pentyl]-3-pyrrolidinol ;
4-[[N$^{\alpha}$-(3,4-dichlorobenzoyl)-L-leucinyl]amino]-1-[(2S)-4-méthyl-2-[[(benzyloxycarbonyl)]amino]pentyl]-3-pyrrolidinone ;
(3RS,4RS)-4-[[N$^{\alpha}$-(6-quinoléinecarbonyl)-L-leucinyl]amino]-1-[(2S)-4-méthyl-2-[[(benzyloxycarbonyl)]-amino]pentyl]-3-pyrrolidinol ;
4-[[N$^{\alpha}$-(6-quinoléinecarbonyl)-L-leucinyl]amino]-1-[(2S)-4-méthyl-2-[[(benzyloxycarbonyl)]amino]pentyl]-3-pyrrolidinone ;
(3RS,4RS)-4-[(2-dibenzofurannesulfonyl)amino]-1-[(2S)-4-méthyl-2-[[(benzyloxycarbonyl)]amino]pentanoyl]-3-pyrrolidinol ;
4-[(2-dibenzofurannesulfonyl)amino]-1-[(2S)-4-méthyl-2-[[(benzyloxycarbonyl)]amino]pentanoyl]-3-pyrrolidinone ;
(3RS,4RS)-4-[(2-dibenzofurannesulfonyl)amino]-1-[(2S)-4-méthyl-2-[[(benzyloxycarbonyl)]méthylamino]penta-noyl]-3-pyrrolidinol ;
4-[(2-dibenzofurannesulfonyl)amino]-1-[(2S)-4-méthyl-2-[[(benzyloxycarbonyl)]méthylamino]pentanoyl]-3-pyrrolidinone ;
(3RS,4RS)-4-[[$N^{\alpha}$-(benzyloxycarbonyl)-L-leucinyl]-amino]-1-(4-méthylpentyl)-3-pipéridinol ;
4-[[$N^{\alpha}$-(benzyloxycarbonyl)-L-leucinyl]amino]-1-(4-méthylpentyl)-3-pipéridinone ;
(3RS, 4RS)-4-[[$N^{\alpha}$-(2-pyridylcarbonyl)-L-leucinyl]-amino]-1-(4-méthylpentyl)-3-pipéridinol ;
4-[[$N^{\alpha}$-(2-pyridylcarbonyl)-L-leucinyl]-amino]-1-(4-méthylpentyl)-3-pipéridinone ;
(3RS,4RS)-4-[[$N^{\alpha}$-(3-chlorobenzoyl)-L-leucinyl]-amino]-1-(4-méthylpentyl)-3-pipéridinol ;
4-[[$N^{\alpha}$-(3-chlorobenzoyl)-L-leucinyl]-amino]-1-(4-méthylpentyl)-3-pipéridinone ;
(3RS,4RS)-4-[[$N^{\alpha}$-(2-quinoléinecarbonyl)-L-leucinyl]-amino]-1-(4-méthylpentyl)-3-pipéridinol ;
4-[[$N^{\alpha}$-(2-quinoléinecarbonyl)-L-leucinyl]amino]-1-(4-méthylpentyl)-3-pipéridinone ;
(3RS,4RS)-4-[[$N^{\alpha}$-(3,4-dichlorobenzoyl)-L-leucinyl]-amino]-1-(4-méthylpentyl)-3-pipéridinol ;
4-[[$N^{\alpha}$-(3,4-dichlorobenzoyl)-L-leucinyl]-amino]-1-(4-méthylpentyl)-3-pipéridinone ;
(3RS,4RS)-4-[[$N^{\alpha}$-(8-quinoléinecarbonyl)-L-leucinyl]-amino]-1-(4-méthylpentyl)-3-pipéridinol ;
4-[[$N^{\alpha}$-(8-quinoléinecarbonyl)-L-leucinyl]-amino]-1-(4-méthylpentyl)-3-pipéridinone ;
(3RS,4RS)-4-[[$N^{\alpha}$-(3-isoquinoléinecarbonyl)-L-leucinyl]-amino]-1-(4-méthylpentyl)-3-pipéridinol ;
4-[[$N^{\alpha}$-(3-isoquinoléinecarbonyl)-L-leucinyl]-amino]-1-(4-méthylpentyl)-3-pipéridinone ;
(3RS,4RS)-4-[[$N^{\alpha}$-(2-pyridinyméthoxycarbonyl)-L-leucinyl]-amino]-1-(4-méthylpentyl)-3-pipéridinol ;
4-[[$^{\alpha}$-(2-pyridinyméthoxycarbonyl)-L-leucinyl]-amino]-1-(4-méthylpentyl)-3-pipéridinone ;
(3RS,4RS)-4-[[$N^{\alpha}$-(acétyl)-L-leucinyl]-amino]-1-(4-méthylpentyl)-3-pipéridinol ;
4-[[$N^{\alpha}$-(acétyl)-L-leucinyl]-amino]-1-(4-méthyl-pentyl)-3-pipéridinone ;
(3RS,4RS)-4-[[$N^{\alpha}$-(p-trifluorométhylbenzène-sulfonyl)-L-leucinyl]-amino]-1-(4-méthylpentyl)-3-pipéridinol ;
4-[[$N^{\alpha}$-(p-trifluorométhylbenzènesulfonyl)-L-leucinyl]-amino]-1-(4-méthylpentyl)-3-pipéridinone ;
(3RS,4RS)-4-[[$N^{\alpha}$-(6-quinoléinecarbonyl)-L-leucinyl]-amino]-1-(4-méthylpentyl)-3-pipéridinol ;
4-[[$N^{\alpha}$-(6-quinoléinecarbonyl)-L-leucinyl]-amino]-1-(4-méthylpentyl)-3-pipéridinone ;
(3RS,4RS)-4-[[2-(RS)-(3-biphényl)-4-méthyl]-amino]pentanoyl-1-(4-méthylpentyl)-3-pipéridinol ;
4-[[2-(RS)-(3-biphényl)-4-méthyl]-amino]-pentanoyl-1-(4-méthylpentyl)-3-pipéridinone ;
(3RS,4RS)-4-[[$N^{\alpha}$-(benzyloxycarbonyl)-L-leucinyl]-amino]-1-[2-[(benzyloxycarbonyl)méthylamino]éthyl]-3-pipéridinol ;
4-[[$N^{\alpha}$-(benzyloxycarbonyl)-L-leucinyl]-amino]-1-[2-[(benzyloxycarbonyl)méthylamino]éthyl]-3-pipéridinone ;
(3RS,4RS)-4-[[$N^{\alpha}$-($\alpha$-toluènesulfonyl)-L-leucinyl]-amino]-1-[3-(2-pyridyl)phénylacétyl)]-3-pipéridinol ;
4-[[$N^{\alpha}$-($\alpha$-toluènesulfonyl)-L-leucinyl]-amino]-1-[3-(2-pyridyl)phénylacétyl)]-3-pipéridinone ;
(3RS,4RS)-4-[[$N^{\alpha}$-(2-naphtylcarbonyl)-L-leucinyl]-amino]-1-[3-(2-pyridyl)phénylacétyl)]-3-pipéridinol ;
4-[[$N^{\alpha}$-(2-naphtylcarbonyl)-L-leucinyl]-amino]-1-[3-(2-pyridyl)phénylacétyl)]-3-pipéridinone ;
(3RS,4RS)-4-[[$N^{\alpha}$-(benzènesulfonyl)-L-leucinyl]-amino]-1-[3-(2-pyridyl)phénylacétyl)]-3-pipéridinol ;
4-[[$N^{\alpha}$-(benzènesulfonyl)-L-leucinyl]-amino]-1-[3-(2-pyridyl)phénylacétyl)]-3-pipéridinone ;
(3RS,4RS)-4-[[$N^{\alpha}$-(3-isoquinoléinecarbonyl)-L-leucinyl]-amino]-1-[3-(2-pyridyl)phénylacétyl)]-3-pipéridinol ;
4-[[$N^{\alpha}$-(3-isoquinoléinecarbonyl)-L-leucinyl]-amino]-1-[3-(2-pyridyl)phénylacétyl)]-3-pipéridinone ;
(3RS,4RS)-4-[3-[(2-pyridyl)phénylacétyl)]amino]-1-[(2S)-4-méthyl-[[(benzyloxycarbonyl)]amino]pentanoyl]-3-pipéridinol ;
4-[3-[(2-pyridyl)phénylacétyl)]amino]-1-[(2S)-4-méthyl-2-[[(benzyloxycarbonyl)]amino]pentanoyl]-3-pipéridinone ;
(3RS,4RS)-4-[3-[(2-pyridyl)phénylacétyl)]amino]-1-[(2S)-4-méthyl-2-[[(2-pyridinylméthoxycarbonyl)]-amino] pentanoyl]-3-pipéridinol ;
4-[3-[(2-pyridyl)phénylacétyl)]amino]-1-[(2S)-4-méthyl-2-[[(2-pyridinylméthoxycarbonyl)]-amino]pentanoyl]-3-pipéridinone ;
(3RS, 4RS)-4-[[$N^{\alpha}$-(2-phénylacétyl)-L-leucinyl]-amino]-1-[(2S)-4-méthyl-2-[[(benzyloxycarbonyl)]amino]-pentyl]-3-pyrrolidinol ;
4-[[$N^{\alpha}$-(2-phénylacétyl)-L-leucinyl]-amino]-1-[(2S)-4-méthyl-2-[[(benzyloxycarbonyl)]amino]-pentyl]-3-pyrrolidinone ;
4-[[$N^{\alpha}$-(tertiobutoxycarbonyl)-L-leucinyl]-amino]-1-[(2S)-4-méthyl-2-[[(benzyloxycarbonyl)]amino]-pentyl]-3-pyrrolidinone ;
4-[(L-leucinyl)amino]-1-[(2S)-4-méthyl-2-[[(benzyloxycarbonyl)]amino]pentyl]-3-pyrrolidinone ;
(3RS,4RS)-4-[[$N^{\alpha}$-(2-quinoléinecarbonyl)-L-leucinyl)-amino]-1-[(2S)-4-méthyl-2-[[(benzyloxycarbonyl)]-amino]-pentyl]-3-pyrrolidinol ;
4-[[$N^{\alpha}$-(2-quinoléinecarbonyl)-L-leucinyl]-amino]-1-[(2S)-4-méthyl-2-[[(benzyloxycarbonyl)]-amino]-pentyl]-3-pyrrolidinone ;
(3RS, 4RS)-4-[[$N^{\alpha}$-(pipéronylcarbonyl)-L-leucinyl]-amino]-1-[(2S)-4-méthyl-2-[[(benzyloxycarbonyl)]-amino]-

pentyl]-3-pyrrolidinol ;
4-[[N$^{\alpha}$-(pipéronylcarbonyl)-L-leucinyl]-amino]-1-[(2S)-4-méthyl-2-[[(benzyloxycarbonyl)]-amino]-pentyl]-3-pyrrolidinone ;
(3RS,4RS)-4-[[N$^{\alpha}$-(4-fluorobenzoyl)-L-leucinyl]-amino]-1-[(2S)-4-méthyl-2-[[(benzyloxycarbonyl)]-amino]-pentyl]-3-pyrrolidinol ;
4-[[N$^{\alpha}$-(4-fluorobenzoyl)-L-leucinyl]-amino]-1-[(2S)-4-méthyl-2-[[(benzyloxycarbonyl)]-amino]-pentyl]-3-pyrrolidinone ;
(3RS,4RS)-4-[[N$^{\alpha}$-(2-pyridinylcarbonyl)-L-leucinyl]-amino]-1-[(2S)-4-méthyl-2-[[(benzyloxycarbonyl)]-amino]-pentyl]-3-pyrrolidinol ;
4-[[N$^{\alpha}$-(2-pyridinylcarbonyl)-L-leucinyl]-amino]-1-[(2S)-4-méthyl-2-[[(benzyloxycarbonyl)]-amino]-pentyl]-3-pyrrolidinone ;
(3RS,4RS)-4-[[N$^{\alpha}$-(2-nitro-$\alpha$-toluènesulfonyl)-L-leucinyl]-amino]-1-[(2S)-4-méthyl-2-[[(benzyloxycarbonyl)]-amino]-pentyl]-3-pyrrolidinol ;
4-[[N$^{\alpha}$-(2-nitro-$\alpha$-toluènesulfonyl)-L-leucinyl]-amino]-1-[(2S)-4-méthyl-2-[[(benzyloxycarbonyl)]-amino]-pentyl]-3-pyrrolidinone ;
(3RS,4RS)-4-[[N$^{\alpha}$-(8-quinoléinesulfonyl)-L-leucinyl]-amino]-1-[(2S)-4-méthyl-2-[[(benzyloxycarbonyl)]-amino]-pentyl]-3-pyrrolidinol ;
4-[[N$^{\alpha}$-(8-quinoléinesulfonyl)-L-leucinyl]-amino]-1-[(2S)-4-méthyl-2-[[(benzyloxycarbonyl)]-amino]-pentyl]-3-pyrrolidinone ;
(3RS, 4RS)-4-[[N$^{\alpha}$-(2-naphtylcarbonyl)-L-leucinyl]-aminométhyl]-1-[(2S)-4-méthyl-2-[[(benzyloxycarbonyl)]-amino]-pentyl]-3-pyrrolidinol ;
4-[[N$^{\alpha}$-(2-naphtylcarbonyl)-L-leucinyl]-aminométhyl]-1-[(2S)-4-méthyl-2-[[(benzyloxycarbonyl)]-amino]-pentyl]-3-pyrrolidinone ;
(3RS,4RS)-4-[[N$^{\alpha}$-(2-quinolinylcarbonyl)-L-leucinyl]-aminométhyl]-1-[(2S)-4-méthyl-2-[[(benzyloxycarbonyl)]-amino]-pentyl]-3-pyrrolidinol ;
4-[[N$^{\alpha}$-(2-quinolinylcarbonyl)-L-leucinyl]-aminométhyl]-1-[(2S)-4-méthyl-2-[[(benzyloxy-carbonyl)]-amino]-pentyl]-3-pyrrolidinone ;
(3RS,4RS)-4-[[N$^{\alpha}$-(phénylacétyl)-L-leucinyl]-amino]-1-[(2S)-4-méthyl-2-[[(benzyloxycarbonyl)]-amino]-pentyl]-3-pipéridinol ;
4-[[N$^{\alpha}$-(phénylacétyl)-L-leucinyl]-amino]-1-[(2S)-4-méthyl-2-[[(benzyloxycarbonyl)]-amino]-pentyl]-3-pipéridinone ;
(3RS,4RS)-4-[[N$^{\alpha}$-(4-pyridinylméthoxycarbonyl)-L-leucinyl]-amino]-1-[(2S)-4-méthyl-2-[[(benzyloxycarbonyl)]-amino]-pentyl]-3-pipéridinol ;
4-[[N$^{\alpha}$-(4-pyridinylméthoxycarbonyl)-L-leucinyl]-amino]-1-[(2S)-4-méthyl-2-[[(benzyloxycarbonyl)]-amino]-pentyl]-3-pipéridinone ;
(3RS,4RS)-4-[[N$^{\alpha}$-(4-pyridinylméthoxycarbonyl)-L-leucinyl]-amino]-1-[(2R)-4-méthyl-2-[[(benzyloxycarbonyl)]-amino]-pentyl]-3-pipéridinol ;
4-[[N$^{\alpha}$-(4-pyridinylméthoxycarbonyl)-L-leucinyl]-amino]-1-[(2R)-4-méthyl-2-[[(benzyloxycarbonyl)]-amino]-pentyl]-3-pipéridinone ;
(3RS, 4RS)-4-[[N$^{\alpha}$-(phénylacétyl)-L-leucinyl]-amino]-1-[(2R)-4-méthyl-2-[[(benzyloxycarbonyl)]-amino]-pentyl]-3-pipéridinol ;
4-[[N$^{\alpha}$-(phénylacétyl)-L-leucinyl]-amino]-1-[(2R)-4-méthyl-2-[[(benzyloxycarbonyl)]-amino]-pentyl]-3-pipéridinone ;
(3RS, 4RS)-4-[[N$^{\alpha}$-(4-imidazolacétyl)-L-leucinyl]-amino]-1-[(2R)-4-méthyl-2-[[(benzyloxycarbonyl)]-amino]-pentyl]-3-pipéridinol ;
4-[[N$^{\alpha}$-(4-imidazolacétyl)-L-leucinyl]-amino]-1-[(2R)-4-méthyl-2-[[(benzyloxycarbonyl)]-amino]-pentyl]-3-pipéridinone ;
, (3RS,4RS)-4-[[N$^{\alpha}$-(4-imidazolacétyl)-L-leucinyl]-amino]-1-[(2S)-4-méthyl-2-[[(benzyloxycarbonyl)]-amino]-pentyl]-3-pipéridinol ;
4-[[N$^{\alpha}$-(4-imidazolacétyl)-L-leucinyl]-amino]-1-[(2S)-4-méthyl-2-[[(benzyloxycarbonyl)]-amino]-pentyl]-3-pipéridinone ;
(3RS,4RS)-4-[(N$^{\alpha}$-(4-pyridinylcarbonyl)-L-leucinyl]-amino]-1-[(2S)-4-méthyl-2-[[(benzyloxycarbonyl)]-amino]-pentyl]-3-pipéridinol ;
4-[[N$^{\alpha}$-(4-pyridinylcarbonyl)-L-leucinyl]-amino]-1-[(2S)-4-méthyl-2-[[(benzyloxycarbonyl)]-amino]-pentyl]-3-pipéridinone ;
4-[[N$^{\alpha}$-(tertiobutoxycarbonyl)-L-leucinyl]-amino]-1-(benzyloxycarbonyl)-3-pipéridinone ;
(3RS,4RS)-4-[(N$^{\alpha}$-(8-quinoléinesulfonyl)-L-leucinyl]-amino]-1-(benzyloxycarbonyl)-3-pipéridinol ;
4-[[N$^{\alpha}$-(8-quinoléinesulfonyl)-L-leucinyl]-amino]-1-(benzyloxycarbonyl)-3-pipéridinone ;

(3RS, 4RS)-4-[[N$^{\alpha}$-(pyridinylacétyl)-L-leucinyl]-amino]-1-(benzyloxycarbonyl)-3-pipéridinol ;
4-[[N$^{\alpha}$-(pyridinylacétyl)-L-leucinyl]-amino]-1-(benzyloxycarbonyl)-3-pipéridinone ;
(3RS,4RS)-4-[[N$^{\alpha}$-(4-imidazolacétyl)-L-leucinyl]-amino]-1-(benzyloxycarbonyl)-3-pipéridinol ;
4-[[N$^{\alpha}$-(4-imidazolacétyl)-L-leucinyl]-amino]-1-(benzyloxycarbonyl)-3-pipéridinone ;
(3RS,4RS)-4-[[N$^{\alpha}$-(4-pyridinylcarbonyl)-L-leucinyl]-amino]-1-(benzyloxycarbonyl)-3-pipéridinol ;
4-[[N$^{\alpha}$-(4-pyridinylcarbonyl)-L-leucinyl]-amino]-1-(benzyloxycarbonyl)-3-pipéridinone ;
(3RS,4RS)-1-benzyl-4-[[N$^{\alpha}$-(3-isoquinolinylcarbonyl)-L-leucinyl]-amino]-3-pyrrolidinol ;
1-benzyl-4-[[N$^{\alpha}$-(3-isoquinolinylcarbonyl)-L-leucinyl]-amino]-3-pyrrolidinone ;
(3RS,4RS)-1-benzyl-4-[[N$^{\alpha}$-(3,4-dichlorobenzoyl)-L-leucinyl]-amino]-3-pyrrolidinol ;
1-benzyl-4-[[N$^{\alpha}$-(3,4-dichlorobenzoyl)-L-leucinyl]-amino]-3-pyrrolidinone ;
(3RS,4RS)-1-benzyl-4-[[N$^{\alpha}$-(2-naphtylcarbonyl)-L-leucinyl]-aminométhyl]-3-pyrrolidinol ;
1-benzyl-4-[[N$^{\alpha}$-(2-naphtylcarbonyl)-L-leucinyl]-amino]-3-pyrrolidinone ;
(3RS, 4RS)-1-benzyl-4-[[N$^{\alpha}$-(2-quinolinylcarbonyl)-L-leucinyl]-aminométhyl]-3-pyrrolidinol ;
1-benzyl-4-[[N$^{\alpha}$-(2-quinolinylcarbonyl)-L-leucinyl]-aminométhyl]-3-pyrrolidinone ;
(3RS,4RS)-1-benzyl-4-[[N$^{\alpha}$-(2-quinolinylcarbonyl)-L-leucinyl]-amino]-3-pyrrolidinol ;
1-benzyl-4-[[N$^{\alpha}$-(2-quinolinylcarbonyl)-L-leucinyl]-amino]-3-pyrrolidinone ;
(3RS,4RS)-1-benzyl-4-[[N$^{\alpha}$-(pipéronylcarbonyl)-L-leucinyl]-amino]-3-pyrrolidinol ;
1-benzyl-4-[[N$^{\alpha}$-(pipéronylcarbonyl)-L-leucinyl]-amino]-3-pyrrolidinone ;
(3RS,4RS)-1-benzyl-4-[[N$^{\alpha}$-(4-fluorobenzoyl)-L-leucinyl]-amino]-3-pyrrolidinol ;
1-benzyl-4-[[N$^{\alpha}$-(4-fluorobenzoyl)-L-leucinyl]-amino]-3-pyrrolidinone ;
(3RS,4RS)-1-benzyl-4-[[N$^{\alpha}$-(6-hydroxy-2-naphtyl-carbonyl)-L-leucinyl]-amino]-3-pyrrolidinol ;
1-benzyl-4-[[N$^{\alpha}$-(6-hydroxy-2-naphtylcarbonyl)-L-leucinyl]-amino]-3-pyrrolidinone ;
(3RS,4RS)-1-benzyl-4-[[N$^{\alpha}$-(2-naphtylcarbonyl)-L-leucinyl]-amino]-3-pyrrolidinol ;
1-benzyl-4-[[N$^{\alpha}$-(2-naphtylcarbonyl)-L-leucinyl]-amino]-3-pyrrolidinone ;
(3RS,4RS)-1-benzyl-4-[[N$^{\alpha}$-(6-quinolinylcarbonyl)-L-leucinyl]-amino]-3-pyrrolidinol ;
1-benzyl-4-[[N$^{\alpha}$-(6-quinolinylcarbonyl)-L-leucinyl]-amino]-3-pyrrolidinone ;
(3RS,4RS)-1-benzyl-4-[[N$^{\alpha}$-(4-imidazolacétyl)-L-leucinyl]-amino]-3-pyrrolidinol ;
1-benzyl-4-[[N$^{\alpha}$-(4-imidazolacétyl)-L-leucinyl]-amino]-3-pyrrolidinone ;
(3RS,4RS)-1-benzyl-4-[[N$^{\alpha}$-(4-pyridinylcarbonyl)-L-leucinyl]-amino]-3-pyrrolidinol ;
1-benzyl-4-[[N$^{\alpha}$-(4-pyridinylcarbonyl)-L-leucinyl]-amino]-3-pyrrolidinone ;
4-[[N$^{\alpha}$-(tertiobutoxycarbonyl)-L-leucinyl]-amino]-1-benzyloxycarbonyl-3-pyrrolidinone ;
(3RS,4RS)-4-[[N$^{\alpha}$-(4-pyridinylméthoxy)carbonyl]-L-leucinyl]-amino]-1-[(2R)-4-méthyl-2-[[(benzyloxycarbonyl)]-amino]pentyl]-3-pyrrolidinol ;
4-[[N$^{\alpha}$-(4-pyridinylméthoxy)carbonyl]-L-leucinyl]-amino]-1-[(2R)-4-méthyl-2-[[(benzyloxycarbonyl)]-amino]-pentyl]-3-pyrrolidinone ;
(3RS,4RS)-4-[[N$^{\alpha}$-(4-pyridinylméthoxy)carbonyl]-L-leucinyl]-amino]-1-[(2S)-4-méthyl-2-[[(benzyloxycarbonyl)]-amino]pentyl]-3-pyrrolidinol ;
4-[[N$^{\alpha}$-(4-pyridinylméthoxy)carbonyl]-L-leucinyl]-amino]-1-[(2R)-4-méthyl-2-[[(benzyloxycarbonyl)]-amino]-pentyl]-3-pyrrolidinone ;
(3RS,4RS)-4-[[N$^{\alpha}$-(4-pyridinylméthoxy)carbonyl]-L-leucinyl]-amino]-1-[(2S)-4-méthyl-2-[[(tertiobutoxycarbonyl)]-amino]pentyl]-3-pyrrolidinol ;
4-[[N$^{\alpha}$-(4-pyridinylméthoxy)carbonyl]-L-leucinyl]-amino]-1-[(2S)-4-méthyl-2-(amino)pentyl]-3-pyrrolidinone ;
(3RS,4RS)-4-[[N$^{\alpha}$-(2-méthylpropoxy)carbonyl]-L-leucinyl]amino]-1-[(2S)-4-méthyl-2-[[(benzyloxycarbonyl)]-amino]pentyl]-3-pyrrolidinol ;
4-[[N$^{\alpha}$-(2-méthylpropoxy)carbonyl]-L-leucinyl]-amino]-1-[(2S)-4-méthyl-2-[[(benzyloxycarbonyl)]-amino]-pentyl]-3-pyrrolidinone ;
(3RS,4RS)-4-[[N$^{\alpha}$-(méthylamino)thiocarbonyl]-L-leucinyl]amino]-1-[(2S)-4-méthyl-2-[[(benzyloxycarbonyl)]-amino]pentyl]-3-pyrrolidinol ;
4-[[N$^{\alpha}$-(méthylamino)thiocarbonyl]-L-leucinyl]-amino]-1-[(2S)-4-méthyl-2-[[(benzyloxycarbonyl)]-amino]-pentyl]-3-pyrrolidinone ;
(3RS,4RS)-4-[[N$^{\alpha}$-(phénylméthylamino)carbonyl]-L-leucinyl]amino]-1-[(2S)-4-méthyl-2-[[(benzyloxycarbonyl)]-amino]pentyl]-3-pyrrolidinol ;
4-[[N$^{\alpha}$-(phénylméthylamino)carbonyl]-L-leucinyl]-amino]-1-[(2S)-4-méthyl-2-[[(benzyloxycarbonyl)]-amino]-pentyl]-3-pyrrolidinone ;
(3RS,4RS)-4-[[N$^{\alpha}$-(3,4-dichlorophénylamino)-carbonyl]-L-leucinyl]amino]-1-[(2S)-4-méthyl-2-[[(benzyl-oxycarbonyl)]-amino]pentyl]-3-pyrrolidinol ;
4-[[N$^{\alpha}$-(3,4-dichlorophénylamino)-carbonyl]-L-leucinyl]amino]-1-[(2S)-4-méthyl-2-[[(benzyloxycarbonyl)]-amino]pentyl]-3-pyrrolidinone ;

(3RS,4RS)-1-benzyl-4-[[Nα-(3,4-dichlorophényl-amino)-L-leucinyl]amino]-3-pyrrolidinol ;
1-benzyl-4-[[Nα-(3,4-dichlorophénylamino)-L-leucinyl]amino]-3-pyrrolidinone ;
(3RS,4RS)-4-[[Nα-(1,2,3,4-tétrahydro-6-quinoléinecarbonyl)-L-leucinyl]amino]-1-[(2S)-4-méthyl-2-[(p-toluènesulfonyl)amino]pentyl]-3-pyrrolidinol ;
4-[[Nα-(1,2,3,4-tétrahydro-6-quinoléinecarbonyl)-L-leucinyl]amino]-1-[(2S)-4-méthyl-2-[(p-toluènesulfonyl)-amino]pentyl]-3-pyrrolidinone ;
(3RS,4RS)-4-[[Nα-(1,2,3,4-tétrahydro-6-quinoléinecarbonyl)-L-leucinyl]amino]-1-[(2S)-4-méthyl-2-[(acétyl) amino]pentyl]-3-pyrrolidinol ;
4-[[Nα-(1,2,3,4-tétrahydro-6-quinoléinecarbonyl)-L-leucinyl]amino]-1-[(2S)-4-méthyl-2-[(acétyl)amino]-pentyl]-3-pyrrolidinone ;
(3RS,4RS)-4-[[Nα-(4-fluorobenzoyl)-L-leucinyl]-amino]-1-[(2S)-4-méthyl-2-[(acétyl)amino]pentyl]-3-pyrrolidinol ;
4-[[[Nα-(4-fluorobenzoyl)-L-leucinyl]-amino]-1-[(2S)-4-méthyl-2-[(acétyl)amino]pentyl]-3-pyrrolidinone ;
(3RS,4RS)-4-[[Nα-(4-fluorobenzoyl)-L-leucinyl]-amino]-1-[(2S)-4-méthyl-2-[(p-toluènesulfonyl)amino]-pentyl]-3-pyrrolidinol ;
4-[[Nα-(4-fluorobenzoyl)-L-leucinyl]-amino]-1-[(2S)-4-méthyl-2-[(p-toluènesulfonyl)amino]-pentyl]-3-pyrrolidinone ;
(3RS,4RS)-4-[[Nα-(4-fluorobenzoyl)-L-leucinyl]-amino]-1-[(2S)-4-méthyl-2-[(méthanesulfonyl)amino]-pentyl]-3-pyrrolidinone ;
4-[[Nα-(4-fluorobenzoyl)-L-leucinyl]-amino]-1-[(2S)-4-méthyl-2-[(méthanesulfonyl)amino]-pentyl]-3-pyrrolidinone ;
(3RS,4RS)-4-[[Nα-(4-fluorobenzoyl)-L-leucinyl]-amino]-1-[(2S)-4-méthyl-2-[(α-toluènesulfonyl)amino]-pentyl]-3-pyrrolidinone ;
4-[[Nα-(4-fluorobenzoyl)-L-leucinyl]-amino]-1-[(2S)-4-méthyl-2-[(α-toluènesulfonyl)amino]-pentyl]-3-pyrrolidinone ;
(3RS,4RS)-1-(2-phénéthyl)-4-[[Nα-(4-fluorobenzoyl)-L-leucinyl]amino]-3-pyrrolidinol ;
1-(2-phénéthyl)-4-[[Nα-(4-fluorobenzoyl)-L-leucinyl]amino]-3-pyrrolidinone ;
(3RS,4RS)-1-(2-phénéthyl)-4-[[Nα-(2-quinolinyl-carbonyl)-L-leucinyl]amino]-3-pyrrolidinol ;
1-(2-phénéthyl)-4-[[Nα-(2-quinolinylcarbonyl)-L-leucinyl]amino]-3-pyrrolidinone ;
(3RS,4RS)-1-(2-phénéthyl)-4-[[Nα-(2-naphtyl-carbonyl)-L-leucinyl]amino]-3-pyrrolidinol ;
1-(2-phénéthyl)-4-[[Nα-(2-naphtylcarbonyl)-L-leucinyl]amino]-3-pyrrolidinone ;
(3RS,4RS)-1-(2-phénéthyl)-4-[[Nα-(α-toluènesulfonyl)-L-leucinyl]amino]-3-pyrrolidinol ;
1-(2-phénéthyl)-4-[[Nα-(α-toluènesulfonyl)-L-leucinyl]amino]-3-pyrrolidinone ;
(3RS,4RS)-1-(2-phénéthyl)-4-[[Nα-(2-nitro-α-toluènesulfonyl)-L-leucinyl]amino]-3-pyrrolidinol ;
1-(2-phénéthyl)-4-[[Nα-(2-nitro-α-toluènesulfonyl)-L-leucinyl]amino]-3-pyrrolidinone ;
(3RS,4RS)-4-[[Nα-(2-phénylacétyl)-L-leucinyl]-amino]-1-[(2S)-4-méthyl-2-[[(4-pyridinylcarbonyl)]-amino]-pentyl]-3-pyrrolidinol ;
4-[[Nα-(2-phénylacétyl)-L-leucinyl]-amino]-1-[(2S)-4-méthyl-2-[[(4-pyridinylcarbonyl)]-amino]-pentyl]-3-pyrrolidinone ;
(3RS,4RS)-4-[[Nα-(2-phénylacétyl)-L-leucinyl]-amino]-1-[(2S)-4-méthyl-2-[[(p-toluènesulfonyl)]-amino]-pentyl]-3-pyrrolidinol ;
4-[[Nα-(2-phénylacétyl)-L-leucinyl]-amino]-1-[(2S)-4-méthyl-2-[[(p-toluènesulfonyl)]-amino]-pentyl]-3-pyrrolidinone ;
(3RS,4RS)-4-[[Nα-(2-phénylacétyl)-L-leucinyl]-amino]-1-[(2S)-4-méthyl-2-[[(4-imidazolacétyl)]-amino]-pentyl]-3-pyrrolidinol ;
4-[[Nα-(2-phénylacétyl)-L-leucinyl]-amino]-1-[(2S)-4-méthyl-2-[[(4-imidazolacétyl)]-amino]-pentyl]-3-pyrrolidinone ;
(3RS,4RS)-4-[(4-phénoxybenzoyl)amino]-1-[(2S)-4-méthyl-2-[[(benzyloxycarbonyl)]amino]pentanoyl]-3-pyrrolidinolycarbonyl)-L-leucinyl]amino]-3-pyrrolidinol ;
1-(3-nitrobenzyl)-4-[[Nα-(4-pyridinylméthoxycarbonyl)-L-leucinyl]amino]-3-pyrrolidinone ;
(3RS,4RS)-1-(2-nitrobenzyl)-4-[[Nα-(4-pyridinylméthoxycarbonyl)-L-leucinyl]amino]-3-pyrrolidinol ;
1-(2-nitrobenzyl)-4-[[Nα-(4-pyridinylméthoxycarbonyl)-L-leucinyl]amino]-3-pyrrolidinone ;
(3RS,4RS)-1-(4-cyanobenzyl)-4-[[Nα-(4-pyridinylméthoxycarbonyl)-L-leucinyl]amino]-3-pyrrolidinol ;
1-(4-cyanobenzyl)-4-[[Nα-(4-pyridinylméthoxycarbonyl)-L-leucinyl]amino]-3-pyrrolidinone ;
(3RS,4RS)-1-(4-bromobenzyl)-4-[[Nα-(4-pyridinylméthoxycarbonyl)-L-leucinyl]amino]-3-pyrrolidinol ;
1-(4-bromobenzyl)-4-[[Nα-(4-pyridinylméthoxycarbonyl)-L-leucinyl]amino]-3-pyrrolidinone ;
(3RS,4RS)-1-phénéthyl-4-[[Nα-(4-pyridinylméthoxycarbonyl)-L-leucinyl]amino]-3-pyrrolidinol ;
1-phénéthyl-4-[[Nα-(4-pyridinylméthoxycarbonyl)-L-leucinyl]amino]-3-pyrrolidinone ;

1-(3-aminobenzyl)-4-[[N$^{\alpha}$-(4-pyridinylméthoxycarbonyl)-L-leucinyl]amino]-3-pyrrolidinone ;
(3RS,4RS)-1-(3-benzyloxybenzyl)-4-[[N$^{\alpha}$-(4-pyridinylméthoxycarbonyl)-L-leucinyl]amino]-3-pyrrolidinol ;
1-(3-benzyloxybenzyl)-4-[[N$^{\alpha}$-(4-pyridinylméthoxycarbonyl)-L-leucinyl]amino]-3-pyrrolidinone ;
(3RS,4RS)-1-(3-hydroxybenzyl)-4-[[N$^{\alpha}$-(4-pyridinylméthoxycarbonyl)-L-leucinyl]amino]-3-pyrrolidinol ;
1-(3-hydroxybenzyl)-4-[[N$^{\alpha}$-(4-pyridinylméthoxycarbonyl)-L-leucinyl]amino]-3-pyrrolidinone ;
(3RS,4RS)-1-éthyl-4-[[N$^{\alpha}$-(4-pyridinylméthoxycarbonyl)-L-leucinyl]amino]-3-pyrrolidinol ;
1-éthyl-4-[[N$^{\alpha}$-(4-pyridinylméthoxy-carbonyl)-L-leucinyl]amino]-3-pyrrolidinone ;
(3RS,4RS)-1-cyclopropylméthyl-4-[[N$^{\alpha}$-(4-pyridinylméthoxycarbonyl)-L-leucinyl]amino]-3-pyrrolidinol ;
1-cyclopropylméthyl-4-[[N$^{\alpha}$-(4-pyridinylméthoxycarbonyl)-L-leucinyl]amino]-3-pyrrolidinone ;
(3RS,4RS)-1-(2-N,N-diméthylaminoéthyl)-4-[[N$^{\alpha}$-(4-pyridinylméthoxycarbonyl)-L-leucinyl]amino]-3-pyrrolidinol ;
1-(2-N,N-diméthylaminoéthyl)-4-[[N$^{\alpha}$-(4-pyridinylméthoxycarbonyl)-L-leucinyl]amino]-3-pyrrolidinone ;
(3RS,4RS)-1-(2-morpholinoéthyl)-4-[[N$^{\alpha}$-(4-pyridinylméthoxycarbonyl)-L-leucinyl]amino]-3-pyrrolidinol ;
1-(2-morpholinoéthyl)-4-[[N$^{\alpha}$-(4-pyridinylméthoxycarbonyl)-L-leucinyl]amino]-3-pyrrolidinone ;
(3RS,4RS)-1-(2-bromobenzyl)-4-[[N$^{\alpha}$-(2-pyridinylméthoxycarbonyl)-L-leucinyl]amino]-3-pyrrolidinonol ;
1-(2-bromobenzyl)-4-[[N$^{\alpha}$-(2-pyridinylméthoxycarbonyl)-L-leucinyl]amino]-3-pyrrolidinonone ;
(3RS,4RS)-4-[[N$^{\alpha}$-(4-pyridinylméthoxy)carbonyl)-L-leucinyl]amino]-1-[(2S)-4-méthyl-2-[[(benzyloxycarbonyl)]-amino]pentyl]-3-pyrrolidinol ;
4-[[N$^{\alpha}$-(4-pyridinylméthoxy)carbonyl)-L-leucinyl]-amino]-1-[(2S)-4-méthyl-2-[[(benzyloxycarbonyl)]-amino]-pentyl]-3-pyrrolidinone ;
(3RS,4RS)-4-[[N$^{\alpha}$-(4-pyridinylméthoxy)carbonyl]-L-leucinyl]amino]-1-[(2S)-4-méthyl-2-[[(benzyloxycarbonyl)]-aminométhyl]pentyl]-3-pyrrolidinol ;
4-[[N$^{\alpha}$-(4-pyridinylméthoxy)carbonyl)-L-leucinyl]-amino]-1-[(2S)-4-méthyl-2-[[(benzyloxycarbonyl)]-aminométhyl]pentyl]-3-pyrrolidinone ;
(3RS,4RS)-4-[[N$^{\alpha}$-(2-pyridinylméthoxy)carbonyl)-L-leucinyl]amino]-1-[(2S)-4-méthyl-2-[[(benzyloxycarbonyl)]-amino]pentyl]-3-pyrrolidinol ;
4-[[N$^{\alpha}$-(2-pyridinylméthoxy)carbonyl)-L-leucinyl]-amino]-1-[(2S)-4-méthyl-2-[[(benzyloxycarbonyl)]-amino]-pentyl]-3-pyrrolidinone ;
(3RS,4RS)-4-[[N$^{\alpha}$-(3-pyridinylméthoxy)carbonyl)-L-leucinyl]amino]-1-[(2S)-4-méthyl-2-[[(benzyloxycarbonyl)]-amino]pentyl]-3-pyrrolidinol ;
4-[[N$^{\alpha}$-(3-pyridinylméthoxy)carbonyl)-L-leucinyl]-amino]-1-[(2S)-4-méthyl-2-[[(benzyloxycarbonyl)]-amino]-pentyl]-3-pyrrolidinone ;
(3RS,4RS)-4-[[N$^{\alpha}$-(benzyloxycarbonyl)-L-leucinyl]-amino]-1-[(2S)-4-méthyl-2-[[(benzyloxycarbonyl)]-amino]-pentyl]-3-pyrrolidinol ;
4-[[N$^{\alpha}$-(benzyloxycarbonyl)-L-leucinyl]-amino]-1-[(2S)-4-méthyl-2-[[(benzyloxycarbonyl)]-amino]-pentyl]-3-pyrrolidinone ;
(3RS,4RS)-4-[[N$^{\alpha}$-(4-pyridinylméthoxycarbonyl)-L-leucinyl]-aminométhyl]-1-[(2S)-4-méthyl-2-[[(benzyloxycarbonyl)]-amino]-pentyl]-3-pyrrolidinol ;
4-[[N$^{\alpha}$-(4-pyridinylméthoxycarbonyl)-L-leucinyl]-aminométhyl)]-1-[(2S)-4-méthyl-2-[[(benzyloxycarbonyl)]-amino]-pentyl]-3-pyrrolidinone ;
(3RS,4RS)-1-benzyl-4-[[N$^{\alpha}$-(2-nitro-$\alpha$-toluènesulfonyl)-L-leucinyl]amino]-3-pyrrolidinol ;
1-benzyl-4-[N$^{\alpha}$-(2-nitro-$\alpha$-toluène-sulfonyl)-L-leucinyl]amino]-3-pyrrolidinone ;
(3RS,4RS)-1-benzyl-4-[[N$^{\alpha}$-(phénylsulfonyl)-L-leucinyl]amino]-3-pyrrolidinol ;
1-benzyl-4-[[N$^{\alpha}$-(phénylsulfonyl)-L-leucinyl]amino]-3-pyrrolidinone ;
(3RS,4RS)-1-benzyl-4-[[N$^{\alpha}$-($\alpha$-toluènesulfonyl)-L-leucinyl]amino]-3-pyrrolidinol ;
1-benzyl-4-[[N$^{\alpha}$-($\alpha$-toluènesulfonyl)-L-leucinyl]-amino]-3-pyrrolidinone ;
(3RS,4RS)-1-benzyl-4-[[N$^{\alpha}$-(2-naphtylsulfonyl)-L-leucinyl]amino]-3-pyrrolidinol ;
1-benzyl-4-[[N$^{\alpha}$-(2-naphtylsulfonyl)-L-leucinyl]amino]-3-pyrrolidinone ;
(3RS,4RS)-1-benzyl-4-[[N$^{\alpha}$-(2-naphtylcarbonyl)-L-leucinyl]amino]-3-pipéridinol ;
1-benzyl-4-[[N$^{\alpha}$-(2-naphtylcarbonyl)-L-leucinyl]-amino]-3-pipéridinone ;
(3RS,4RS)-1-benzyl-4-[[N$^{\alpha}$-(2-quinolinylcarbonyl)-L-leucinyl]amino]-3-pipéridinol ;
1-benzyl-4-[[N$^{\alpha}$-(2-quinolinylcarbonyl)-L-leucinyl]amino]-3-pipéridinone ;
(3RS,4RS)-1-benzyl-4-[[N$^{\alpha}$-(2-naphtylsulfonyl)-L-leucinyl]-amino]-3-pipéridinol ;
1-benzyl-4-[[N$^{\alpha}$-(2-naphtylsulfonyl]-L-leucinyl]-amino]-3-pipéridinone ;
(3RS, 4RS)-4-[[N$^{\alpha}$-(benzyloxycarbonyl)-L-leucinyl)-aminométhyl]-1-[(2S)-4-méthyl-2-[[(benzyloxycarbonyl)]-aminométhyl]pentanoyl]-3-pyrrolidinol ;
4-[[N$^{\alpha}$-(benzyloxycarbonyl)-L-leucinyl)-aminométhyl]-1-[[2S)-4-méthyl-2-[[(benzyloxycarbonyl)]-aminométhyl]pentanoyl]-3-pyrrolidinone ;

(3RS,4RS)-4-[(2S)-4-méthyl-2-[(benzyloxycarbonyl)]-amino]pentyl]-1-[(2S)-4-méthyl-2-[[(benzyloxycarbonyl)]amino]pentanoyl]3-pyrrolidinol ;
4-[(2S)-4-méthyl-2-[(benzyloxy-carbonyl)]-amino]pentyl]-1-[(2S)-4-méthyl-2-[[(benzyloxycarbonyl)]amino] pentanoyl]3-pyrrolidinone ;
(3RS,4RS)-4-[[2S)-4-[[N$^{\alpha}$-(4-fluorobenzoyl)-L-leucinyl]amino]-1-[2-[($\alpha$-toluènesulfonyl)amino]éthyl]-3-pyrrolidinol ;
4-[[N$^{\alpha}$-(4-fluorobenzoyl)-L-leucinyl]-amino]-1-[2-[($\alpha$-toluènesulfonyl)amino]éthyl]-3-pyrrolidinone ;
(3RS,4RS)-4-[[N$^{\alpha}$-(4-fluorobenzoyl)-L-leucinyl]-amino]-1-benzoyl-3-pyrrolidinol ;
4-[[N$^{\alpha}$-(4-fluorobenzoyl)-L-leucinyl]-amino]-1-benzoyl-3-pyrrolidinone ;
(3RS,4RS)-4-[[N$^{\alpha}$-(pipéronylcarbonyl)-L-leucinyl]-amino]-1-benzoyl-3-pyrrolidinol ;
4-[[N$^{\alpha}$-(pipéronylcarbonyl)-L-leucinyl]-amino]-1-benzoyl-3-pyrrolidinone ;
(3RS,4RS)-4-[[N$^{\alpha}$-(4-fluorobenzoyl)-L-leucinyl]-amino]-1-[2-[(4-fluorobenzoyl)amino]éthyl]-3-pyrrolidinol ;
4-[[N$^{\alpha}$-(4-fluorobenzoyl)-L-leucinyl]-amino]-1-[2-[(4-fluorobenzoyl)amino]éthyl]-3-pyrrolidinone ;
(3RS,4RS)-4-[[N$^{\alpha}$-(tertiobutoxycarbonyl)-L-leucinyl]-amino]-1-benzoyl-3-pyrrolidinol ;
4-[[N$^{\alpha}$-(tertiobutoxycarbonyl)-L-leucinyl]-amino]-1-benzoyl-3-pyrrolidinone ;
(3RS,4RS)-4-[[N$^{\alpha}$-(4-fluorobenzoyl)-L-leucinyl]-amino]-1-[(2S)-4-méthyl-2-[(4-fluorobenzoyl)amino]pentyl]-3-pyrrolidinol ;
4-[[N$^{\alpha}$-(4-fluorobenzoyl)-L-leucinyl]-amino]-1-[(2S)-4-méthyl-2-[(4-fluorobenzoyl)amino]pentyl]-3-pyrrolidinone ;
4-[[N$^{\alpha}$-(4-carboxybenzoyl)-L-leucinyl]-amino]-1-[(2S)-4-méthyl-2-[(4-fluorobenzoyl)amino]pentyl]-3-pyrrolidinone ;
1-benzyl-4-[[N$^{\alpha}$-(4-carboxybenzoyl)-L-leucinyl]-amino]-3-pyrrolidinone ;
(3RS,4RS)-1-benzyl-4-[[N$^{\alpha}$-(4-carboxyméthyl)-benzoyl)]-L-leucinyl]-amino]-3-pyrrolidinol ;
1-benzyl-4-[[N$^{\alpha}$-(4-carboxyméthyl)-benzoyl)]-L-leucinyl]-amino]-3-pyrrolidinone ;
(3RS,4RS)-4-[[N$^{\alpha}$-[(4-carboxyméthyl)-benzoyl)-L-leucinyl]-amino]-1-[(2S)-4-méthyl-2-[[(4-fluorobenzoyl)-amino]pentyl]-3-pyrrolidinol ;
4-[[N$^{\alpha}$-[(4-carboxyméthyl)-benzoyl)-L-leucinyl]-amino]-1-[(2S)-4-méthyl-2-[[(4-fluorobenzoyl)-amino]pentyl]-3-pyrrolidinone ;
(3RS,4RS)-1-phénéthyl-4-[[N$^{\alpha}$-(2-amino-$\alpha$-toluènesulfonyl)-L-leucinyl]-amino]-3-pyrrolidinol ;
1-phénéthyl-4-[[N$^{\alpha}$-(2-amino-$\alpha$-toluènesulfonyl)-L-leucinyl]-amino]-3-pyrrolidinone ;
(3RS,4RS)-4-[[N$^{\alpha}$-(2-naphtylcarbonyl)-L-leucinyl]-amino]-1-benzoyl-3-pyrrolidinol ;
4-[[N$^{\alpha}$-(2-naphtylcarbonyl)-L-leucinyl]-amino]-1-benzoyl-3-pyrrolidinone ;
(3RS,4RS)-4-[[N$^{\alpha}$-(2-quinolinecarbonyl)-L-leucinyl]-amino]-1-benzoyl-3-pyrrolidinol ;
4-[[N$^{\alpha}$-(2-quinolinecarbonyl)-L-leucinyl]-amino]-1-benzoyl-3-pyrrolidinone ;
(3RS,4RS)-4-[[N$^{\alpha}$-(3-isoquinolinecarbonyl)-L-leucinyl]-amino]-1-benzoyl-3-pipéridinol ;
4-[[N$^{\alpha}$-(3-isoquinolinecarbonyl)-L-leucinyl]-amino]-1-benzoyl-3-pipéridinone ;
(3RS,4RS)-4-[(2S)-4-méthyl-2-(benzyl)oxy)-pentanoyl]-[(2S)-4-méthyl-2-[[(benzyloxycarbonyl)]-amino]pentanoyl]-3-pipéridinol ;
4-[(2S)-4-méthyl-2-(benzyl)oxy)-pentanoyl]-1-[(2S)-4-méthyl-2-[[(benzyloxycarbonyl)]-amino]pentanoyl]-3-pipéridinone ;
(3RS,4RS)-4-[[3-(2-pyridyl)phénylacétyl]amino]-1-[3-(2-pyridyl)phénylacétyl]-3-pipéridinol ;
4-[[3-(2-pyridyl)phénylacétyl]amino]-1-[3-(2-pyridyl)phénylacétyl]-3-pipéridinone ;
(3RS,4RS)-4-[[N$^{\alpha}$-(p-trifluorométhanephénylsulfonyl)-L-leucinyl]-amino]-1-[3-(2-pyridyl)phénylacétyl]-3-pipéridinol ;
4-[[N$^{\alpha}$-(p-trifluorométhanephénylsulfonyl)-L-leucinyl]-amino]-1-[3-(2-pyridyl)phénylacétyl]-3-pipéridinone ;
(3RS, 4RS)-4-[[N$^{\alpha}$-(2-naphtylsulfonyl)-L-leucinyl]-amino]-1-[3-(2-pyridyl)phénylacétyl]-3-pipéridinol ;
4-[[N$^{\alpha}$-(2-naphtylsulfonyl)-L-leucinyl]-amino]-1-[3-(2-pyridyl)phénylacétyl]-3-pipéridinone ;
(3RS,4RS)-4-[[N$^{\alpha}$-(3,4-dichlorophénylsulfonyl)-L-leucinyl]-amino]-1-[3-(2-pyridyl)phénylacétyl]-3-pipéridinol ;
4-[[N$^{\alpha}$-(3,4-dichlorophénylsulfonyl)-L-leucinyl]-amino]-1-[3-(2-pyridyl)phénylacétyl)]-3-pipéridinone ;
(3RS,4RS)-4-[(N$^{\alpha}$-(méthanesulfonyl)-L-leucinyl]-amino]-1-[3-(2-pyridyl)phénylacétyl]-3-pipéridinol ;
4-[[N$^{\alpha}$-(méthanesulfonyl)-L-leucinyl]-amino]-1-[3-(2-pyridyl)phénylacétyl)]-3-pipéridinone ;
(3RS,4RS)-4-[[N$^{\alpha}$-(4-fluorophénylsulfonyl)-L-leucinyl]-amino]-1-[3-(2-pyridyl)phénylacétyl]-3-pipéridinol ; ou
4-[[N$^{\alpha}$-(4-fluorophénylsulfonyl)-L-leucinyl]-amino]-1-[3-(2-pyridyl)phénylacétyl)]-3-pipéridinone ;

ou un de ses sels pharmaceutiquement acceptables.

**17.** Composé suivant l'une quelconque des revendications 1 à 16, destiné à être utilisé en thérapeutique.

**18.** Composition pharmaceutique comprenant un composé suivant l'une quelconque des revendications 1 à 16 et un support pharmaceutiquement acceptable.

**19.** Composé suivant l'une quelconque des revendications 1 à 16, destiné à être utilisé dans le traitement de maladies dans lesquelles l'inhibition d'une cystéine-protéase est un facteur.

**20.** Utilisation suivant la revendication 19, dans laquelle la cystéine-protéase est la cathepsine K.

**21.** Composé suivant l'une quelconque des revendications 1 à 16, destiné à être utilisé dans l'inhibition de la perte de tissu osseux.

**22.** Composé suivant l'une quelconque des revendications 1 à 16, destiné à être utilisé dans le traitement de l'ostéoporose.

**23.** Composé suivant l'une quelconque des revendications 1 à 16, destiné à être utilisé dans le traitement d'une maladie gingivale ou périodontique.

**24.** Composé suivant l'une quelconque des revendications 1 à 16, destiné à être utilisé dans le traitement d'une maladie **caractérisée par** une dégradation excessive du cartilage ou de la matrice.

**25.** Utilisation suivant la revendication 24, dans laquelle ladite maladie est l'ostéo-arthrite ou l'arthrite rhumatoïde.

**26.** Utilisation d'un composé de formule (I) répondant à la définition suivant l'une quelconque des revendications 1 à 16 dans la production d'un médicament destiné au traitement de maladies dans lesquelles l'inhibition d'une cystéine-protéase est un facteur.

**27.** Procédé pour la préparation d'un composé de formule (I) répondant à la définition suivant l'une quelconque des revendications 1 à 16, procédé qui comprend :

(A) pour des composés dans lesquels A représente un groupe CH(OH)

(i) la réaction d'un composé de formule (III) :

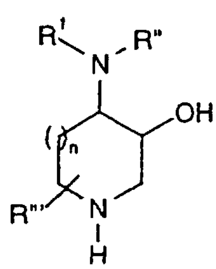

(III)

ou d'un de ses sels,

formule dans laquelle $R^1$, R", R" ' et n répondent aux définitions mentionnées pour la formule (I) dans la revendication 1, n'importe quels groupes fonctionnels réactifs étant protégés, avec :

(a) un composé de formule $R^5C(O)Cl$, dans laquelle $R^5$ répond à la définition mentionnée pour la formule (I) dans la revendication 1 ; ou
(b) un composé de formule $R^5C(O)OH$, dans laquelle $R^5$ répond à la définition mentionnée pour la formule (I) dans la revendication 1, en présence de EDC et de HOBT ; ou
(c) un composé de formule $R^5C(O)H$, dans laquelle $R^5$ répond à la définition mentionnée pour la formule (I) dans la revendication 1, avec ensuite une réduction ; ou
(d) un composé de formule $R^5OC(O)Cl$, dans laquelle $R^5$ répond à la définition mentionnée pour la formule (I) dans la revendication 1, en présence d'une base ; ou
(e) un composé de formule $R^5SO_2Cl$, dans laquelle $R^5$ répond à la définition mentionnée pour la formule (I) dans la revendication 1, en présence d'une base ; ou

(f) un composé de formule

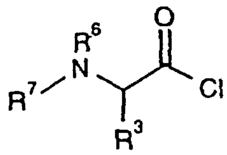

dans laquelle $R^3$, $R^6$ et $R^7$ répondent aux définitions mentionnées pour la formule (I) dans la revendication 1 ;
ou
(g) un composé de formule adamantyl-C(O)Cl ;

(ii) la réaction d'un composé de formule (IV) :

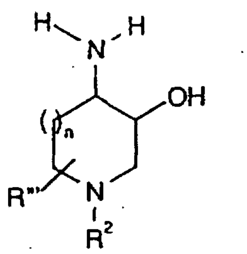

(IV)

dans laquelle $R^2$, R''' et n répondent aux définitions mentionnées pour la formule (I) dans la revendication 1, n'importe quel groupe fonctionnel réactif étant protégé, avec :

(a) un composé de formule

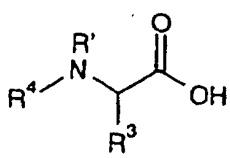

dans laquelle $R^3$, $R^4$ et R' répondent aux définitions mentionnées pour la formule (I) dans la revendication 1, en présence de EDC et HOBT ; ou
(b) un composé de formule

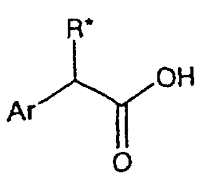

dans laquelle R* répond à la définition mentionnée pour la formule (I) dans la revendication 1, en présence de EDC et de HOBT ; ou
(c) un composé de formule

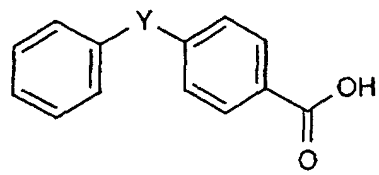

dans laquelle Y répond à la définition mentionnée pour la formule (I) dans la revendication 1, en présence de EDC et de HOBT ; ou

(d) un composé de formule

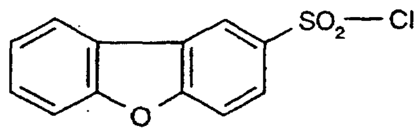

;

(iii) la réaction d'un composé de formule (V) :

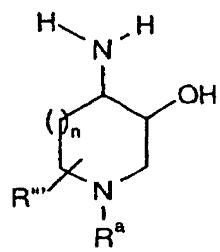

(V)

dans laquelle R''' et n répondent aux définitions mentionnées pour la formule (I) dans la revendication 1, n'importe quels groupes fonctionnels réactifs étant protégés, et $R^a$ représente un groupe alkyle en $C_1$ à $C_6$, (cycloalkyle en $C_3$ à $C_6$)-(alkyle en $C_0$ à $C_6$), Ar-(alkyle en $C_0$ à $C_6$) ou Het-(alkyle en $C_0$ à $C_6$), avec :

(a) un composé de formule

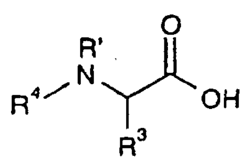

,

dans laquelle $R^3$, $R^4$ et R' répondent aux définitions mentionnées pour la formule (I) dans la revendication 1, en présence de EDC et de HOBT ; ou

(b) un composé de formule

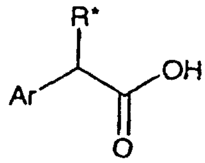

.

dans laquelle R* répond à la définition mentionnée pour la formule (I) dans la revendication 1, en présence de EDC et de HOBT ; ou
(c) un composé de formule

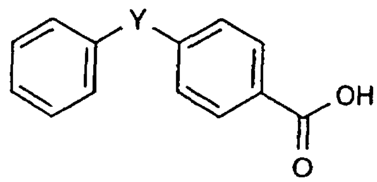

,

dans laquelle Y répond à la définition mentionnée pour la formule (I) dans la revendication 1, en présence de EDC et de HOBT ; ou
(d) un composé de formule

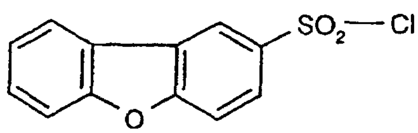

;

(B) pour des composés dans lesquels A représente un groupe C(O) :

(i) la réaction d'un composé de formule (VI) :

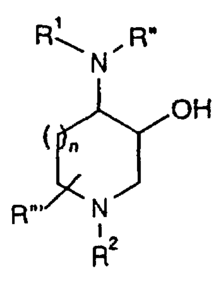

(VI)

dans laquelle $R^1$, $R^2$, R", R"' et n répondent aux définitions mentionnées pour la formule (I) dans la revendication 1, n'importe quels groupes fonctionnels réactifs étant protégés, avec un agent oxydant ;

et ensuite l'élimination de n'importe quels groupes protecteurs et, facultativement, la formation d'un sel pharmaceutiquement acceptable.